# EUROPEAN PATENT APPLICATION

(11) **EP 4 840 868 A1**
(43) Date of publication of application: **18.11.2026**
(21) Application number: 25738506.2
(22) Date of filing: 09.01.2025
(51) Int. Cl.: C07H 9/06, A61K 47/68, A61K 47/54, A61P 35/00

(54) **NOVEL DISACCHARIDE LINKER, PREPARATION METHOD THEREFOR AND USE THEREOF**

(30) Priority: 10.01.2024 CN 202410038405; 03.01.2025 CN 202510013650
(71) Applicant: Shanghai Qilu Pharmaceutical Research and Development Centre Ltd., Shanghai 201203 (CN)
(72) Inventor: QIN, Ken, Shanghai 201203 (CN); HUANG, Guoyin, Shanghai 201203 (CN); HUANG, Ying, Shanghai 201203 (CN); YANG, Yang, Shanghai 201203 (CN); TAO, Weikang, Shanghai 201203 (CN)
(74) Representative: Dai, Simin
(86) International application number: PCT/CN2025/071513
(87) International publication number: WO 2025/148977

(57) **Abstract**

Disclosed are a disaccharide linker represented by formula (I), a non-natural sugar-engineered antibody obtained using the disaccharide linker, a fixed-point quantitative antibody drug conjugate based on glycosyl modification, a preparation method therefor and a medical use thereof.

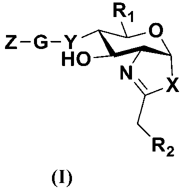

## Description

### CROSS-REFERENCE TO RELATED APPLICATIONS

The present application claims the benefits and priorities of patent application No. 202410038405.8 filed with the China National Intellectual Property Administration on January 10, 2024 and patent application No. 202510013650.8 filed with the China National Intellectual Property Administration on January 3, 2025, the entire contents of which are hereby incorporated by reference herein in their entirety.

### TECHNICAL FIELD

The present disclosure relates to the fields of medicinal chemistry and biotechnological drugs, and specifically relates to a novel class of disaccharide linkers, non-natural glycoengineered antibodies obtained using this class of disaccharide linkers and novel enzymes, site-specific and quantitative antibody-drug conjugates based on glycoengineering, as well as preparation methods therefor and uses thereof.

### BACKGROUND

Antibody-based conjugate drugs are a class of biotechnological drugs that utilize antibodies as targeting carriers to load effector payloads, thereby achieving enrichment of the effector payloads in targeted regions. Loadable effector payloads include cytotoxins, radioisotopes, oligonucleotides, immunomodulators, peptides, or protein fragments, etc., forming antibody-toxin conjugates (*e.g*., Trastuzumab deruxtecan), antibody-radioisotope conjugates (*e.g.,* BAY 2315497), antibody-nucleic acid conjugates *(e.g., AOC* 1001), antibody-immunomodulator conjugates (*e.g.,* XMT-2056), and antibody-peptide conjugates *(e.g.,* AMG 133), respectively.

Antibody-drug conjugates (ADCs) are a class of biotechnological drugs composed of antibodies, cytotoxins, and linkers. ADCs combine the targeting capability of antibodies with the killing capability of cytotoxins, and control the release of the cytotoxin via the linker to achieve targeted killing of tumor cells. Compared with traditional chemotherapeutic drugs, ADCs have lower systemic toxicity and a broader therapeutic window; compared with traditional antibody drugs, ADCs have a more direct tumor-killing capability that is less dependent on the immune system. Currently marketed ADCs primarily rely on naturally occurring lysine (*e.g.,* Trastuzumab emtansine) or cysteine (*e.g.,* Enfortumab vedotin) in antibodies. ADCs produced by such random conjugation methods using natural amino acids exhibit poor homogeneity and poor stability at conjugation sites, resulting in inferior drug safety and a narrow therapeutic window. Currently, various methods are available for preparing site-specific ADCs, including exogenous cysteine insertion technology, non-natural amino acid insertion technology, enzyme-catalyzed conjugation technology, glycosite-specific conjugation technology, and the like.

A conserved *N*-glycosylation site is present in the Fc domain of natural antibodies. Utilizing this site, various site-specific conjugation technologies have been developed. Kathrin Zuberbühler *et al.* utilized sodium periodate to oxidize fucose, introducing an aldehyde group to achieve payload attachment. Qun Zhou *et al.* utilized the glycosyltransferases beta-1,4-Gal-T1 and Sialyltransferase (Sia T) to process antibodies into antibodies with homogeneous sialic acid at the termini, followed by oxidation with sodium periodate to introduce an aldehyde group and achieve conjugation. Using wild-type endoglycosidase, the *N*-glycosylation of antibodies can be processed into a truncated and homogeneous form. On this basis, Laixi Wang *et al.* employed mutants of endoglycosidases *(e.g.,* Endo S D233Q, Endo S2 D184M) to transfer oxazoline substrates bearing bioorthogonal reactive groups to the *N-*glycan termini, followed by payload conjugation through bioorthogonal reactions. Based on glycan-truncated antibodies, Pradman K. Qasba utilized the beta-1,4-galactosyltransferase1-Y289L mutant to achieve the transfer of galactose and *N*-acetylgalactosamine bearing bioorthogonal reactive groups, thereby achieving payload conjugation. The teams of Huang Wei and Laixi Wang respectively reported the use of wild-type endoglycosidases Endo S and Endo S2 with a disaccharide linker as a substrate to achieve the preparation of glycoengineered antibodies and ADCs. Currently, the disaccharide structures that can serve as disaccharide linkers for glycosyl transfer, as well as the transfer specificities of different enzymes toward these disaccharide substrates, remain to be explored.

### DETAILED DESCRIPTION OF THE INVENTION

The purpose of the present disclosure is to provide a novel disaccharide linker capable of site-specifically introducing a small-molecule drug into an antibody.

In a first aspect, the present disclosure provides a disaccharide linker represented by formula (I) 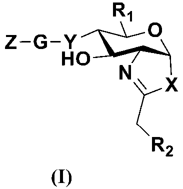 wherein
X is an oxygen atom or a sulfur atom;
Y is an oxygen atom or a sulfur atom;
R₁ is selected from the group consisting of: 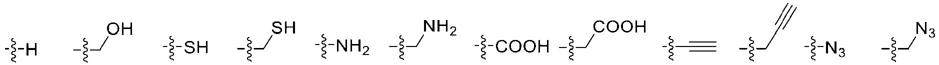 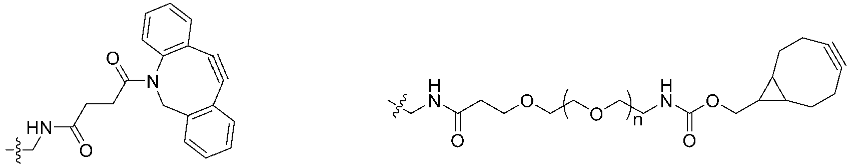 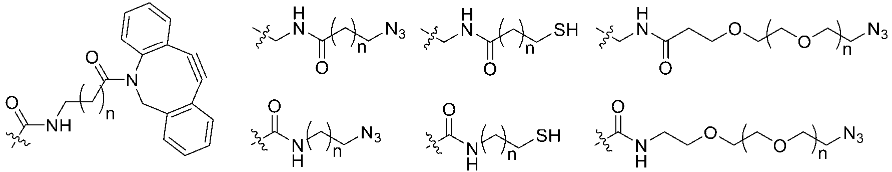 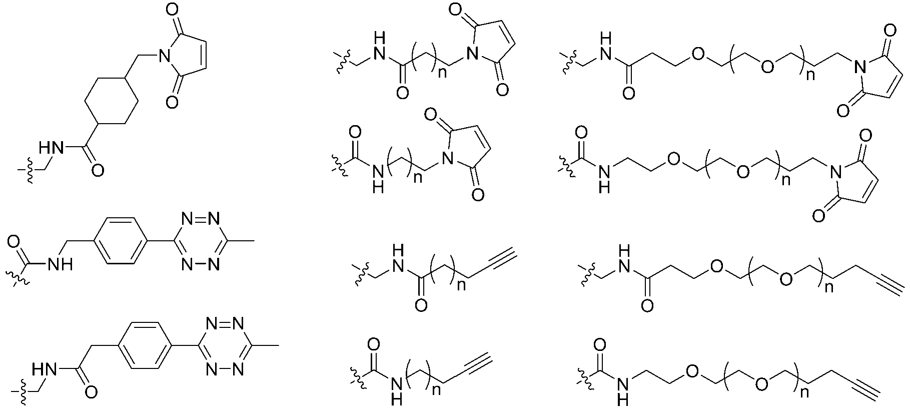
wherein n is an integer selected from the group consisting of 0-30, or an integer from 0-20, or an integer from 0-10;
R₂ is selected from the group consisting of: hydrogen, -N₃, C₁-C₆ alkyl-N₃, -C=CH, C₁-C₆ alkyl-C=CH, -SH, C₁-C₆ alkyl-SH, and C₁-C₆ alkyl;
ring G represents a structure derived from a monosaccharide molecule, which is linked to a monosaccharide via a thioether bond or a glycosidic bond; the monosaccharide molecule is selected from the group consisting of galactose, N-acetylgalactosamine, glucose, *N-*acetylglucosamine, mannose, N-acetylmannosamine, fucose, and sialic acid; when Y is a sulfur atom, ring G is linked to 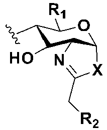 via a thioether bond, wherein the thioether bond is a 1,4-thioether bond, a 2,4-thioether bond, or a 3,4-thioether bond; when Y is an oxygen atom, ring G is linked to 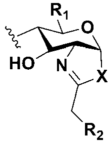 via a glycosidic bond, wherein the glycosidic bond is a 1,4-glycosidic bond, a 2,4-glycosidic bond, or a 3,4-glycosidic bond; and
Z is hydrogen, a reactive group containing an azide residue, a reactive group containing a cycloalkyne residue, a reactive group containing a tetrazine residue, a reactive group containing an alkyne residue, a reactive group containing a cycloalkene residue, or a reactive group containing a maleimide residue.

In some embodiments of the present disclosure, R₂ is selected from the group consisting of: hydrogen, -N₃, -CH₂-N₃, -CH₂-CH₂-N₃, -C=CH, -CH₂-C≡CH, -CH₂-CH₂-C≡CH, -SH, -CH₂-SH, -CH₂-CH₂-SH, and C₁-C₆ alkyl.

In some embodiments of the present disclosure, the expression "ring G represents a structure derived from a monosaccharide molecule, which is linked to a monosaccharide via a thioether bond or a glycosidic bond" means that ring G represents a structure derived from a monosaccharide molecule, which may be linked to another monosaccharide via a thioether bond or a glycosidic bond.

Z is a reactive group containing an azide residue, a reactive group containing a cycloalkyne residue, a reactive group containing a tetrazine residue, a reactive group containing an alkyne residue, or a reactive group containing a cycloalkene residue.

In some embodiments of the present disclosure, in the disaccharide linker represented by formula (I), Y is an oxygen atom, and the ring G is linked to 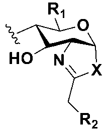 via a glycosidic bond, wherein the glycosidic bond is a 1,4-glycosidic bond, a 2,4-glycosidic bond, or a 3,4-glycosidic bond, preferably a 1,4-glycosidic bond.

In some embodiments of the present disclosure, in the disaccharide linker represented by formula (I), Y is a sulfur atom, and the ring G is linked to 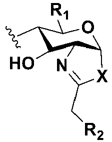 via a thioether bond, wherein the thioether bond is a 1,4-thioether bond, a 2,4-thioether bond, or a 3,4-thioether bond, preferably a 1,4-thioether bond.

In some embodiments of the present disclosure, "ring G represents a structure derived from a monosaccharide molecule" means that the ring G may be a galactosyl group, an *N-*acetylgalactosaminyl group, a glucosyl group, an N-acetylglucosaminyl group, a mannosyl group, an N-acetylmannosaminyl group, a fucosyl group, or a sialyl group. In some embodiments of the present disclosure, "ring G may be linked to another monosaccharide via a thioether bond or a glycosidic bond" means that the ring G may be linked to another monosaccharide other than 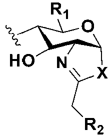 via a thioether bond or a glycosidic bond.

In some embodiments of the present disclosure, in the disaccharide linker represented by formula (I), the monosaccharide molecule from which the ring G is derived is galactose, glucose, mannose, N-acetylglucosamine, N-acetylgalactosamine, or N-acetylmannosamine.

In some embodiments of the present disclosure, in the disaccharide linker represented by formula (I), R₁ is selected from the group consisting of: 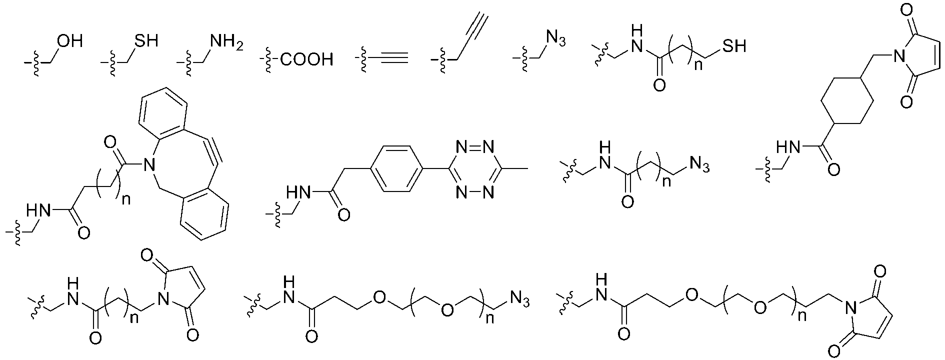 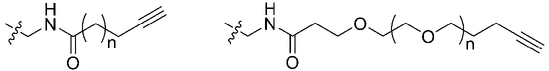 wherein n is an integer selected from the group consisting of 0-30, or an integer from 0-20, or an integer from 0-10.

In some embodiments of the present disclosure, in the disaccharide linker represented by formula (I), R₂ is selected from the group consisting of hydrogen, -N₃, -CH₂-N₃, -CH₂-CH₂-N₃, -C≡CH, -SH, -CH₃, -CH₂-CH₃, and -CH₂-CH₂-CH₃.

In some embodiments of the present disclosure, in the disaccharide linker represented by formula (I), when Z is a reactive group containing an azide residue, it is selected from the group consisting of: 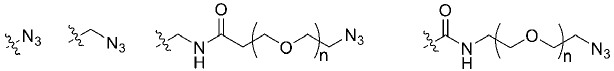 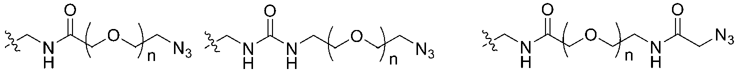 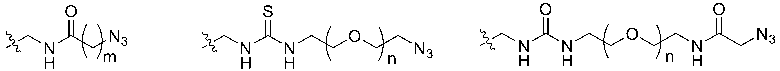 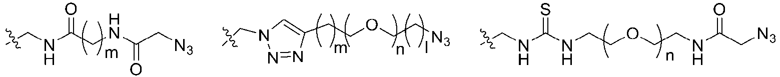 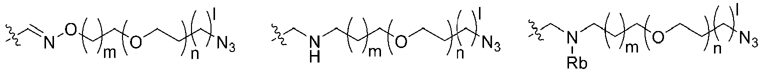 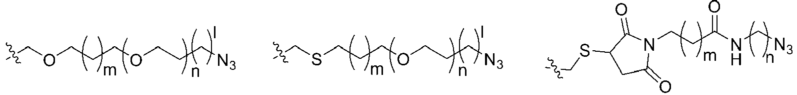 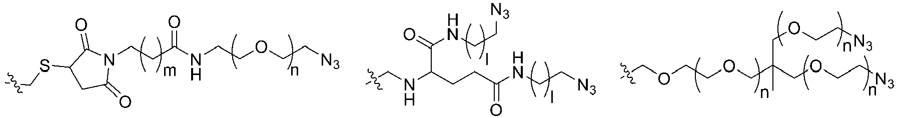 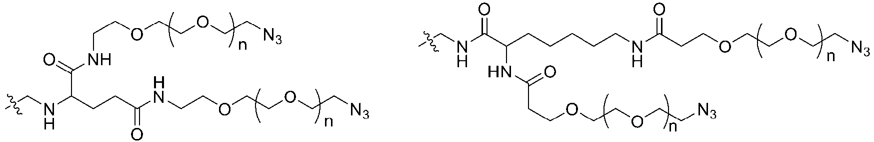 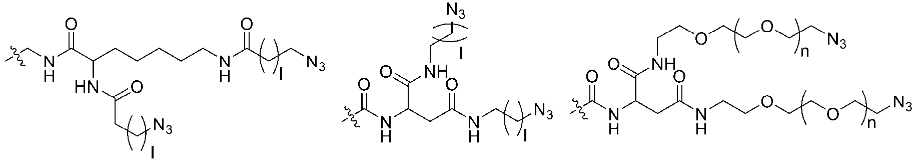 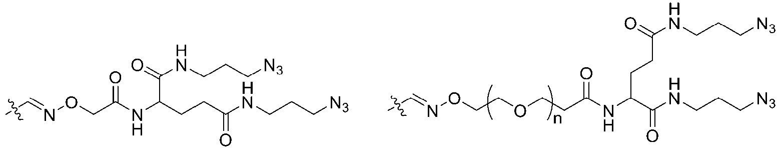 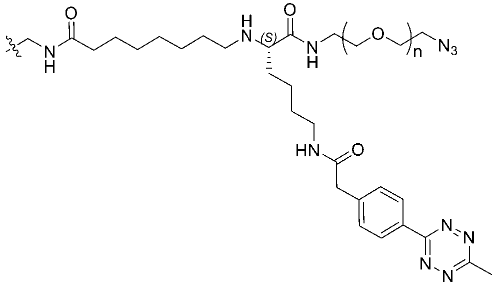
wherein n, m, and I are each independently an integer selected from the group consisting of 0-30, or an integer from 0-20, or an integer from 0-10;
Rb is hydrogen, C₁-C₆ alkyl, C₆-C₁₀ aryl, or C₅-C₁₀ heteroaryl.

In some embodiments of the present disclosure, in the disaccharide linker represented by formula (I), when Z is a reactive group containing an azide residue, it is selected from the group consisting of: 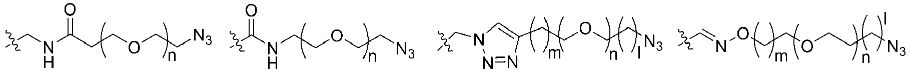 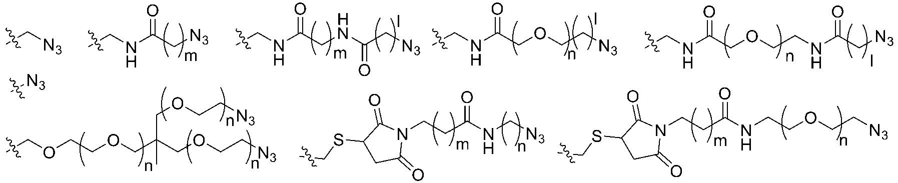 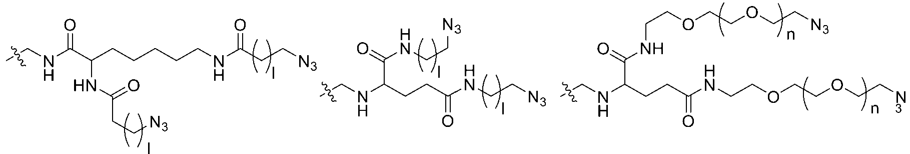 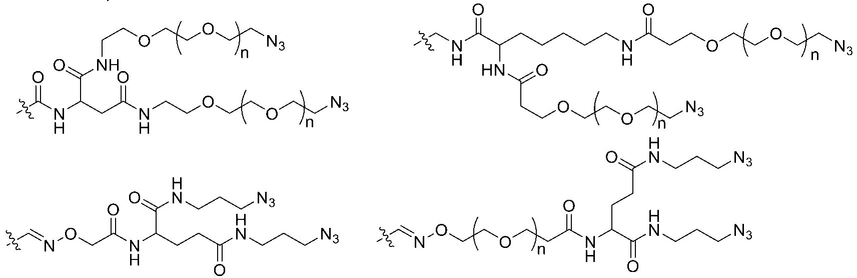 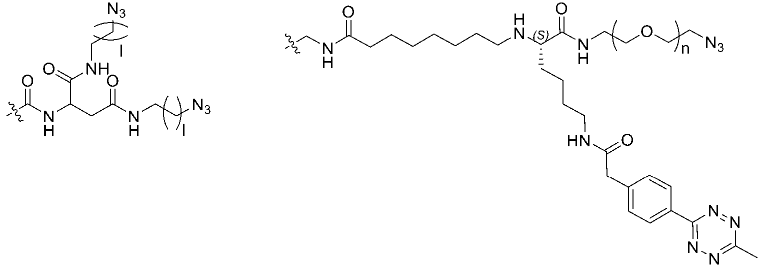 wherein n, m, and I are each independently an integer selected from the group consisting of 0-30, or an integer from 0-20, or an integer from 0-10.

In some embodiments of the present disclosure, in the disaccharide linker represented by formula (I), when Z is a reactive group containing a cycloalkyne residue, it is selected from the group consisting of: 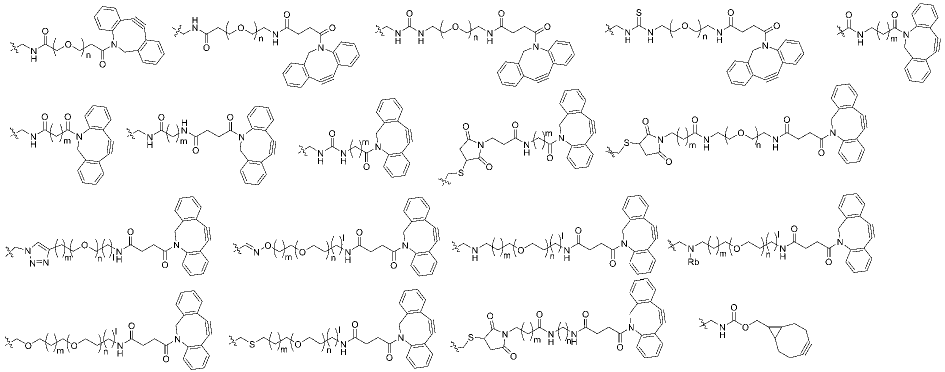 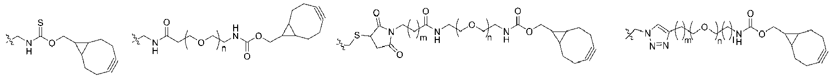 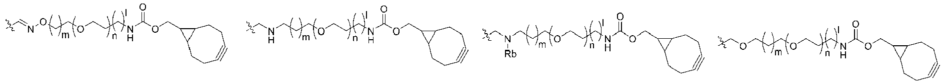 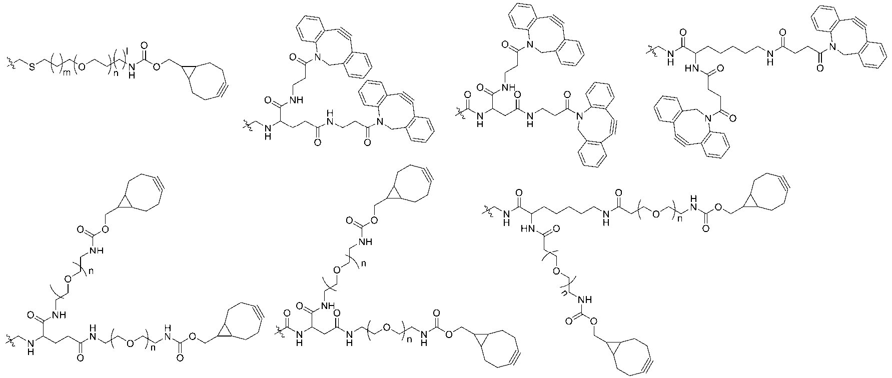
wherein n, m, and I are each independently an integer selected from the group consisting of 0-30, or an integer from 0-20, or an integer from 0-10;
Rb is hydrogen, C₁-C₆ alkyl, C₆-C₁₀ aryl, or C₅-C₁₀ heteroaryl.

In some embodiments of the present disclosure, in the disaccharide linker represented by formula (I), when Z is a reactive group containing a cycloalkyne residue, it is selected from the group consisting of: 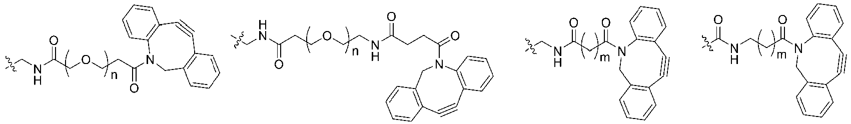 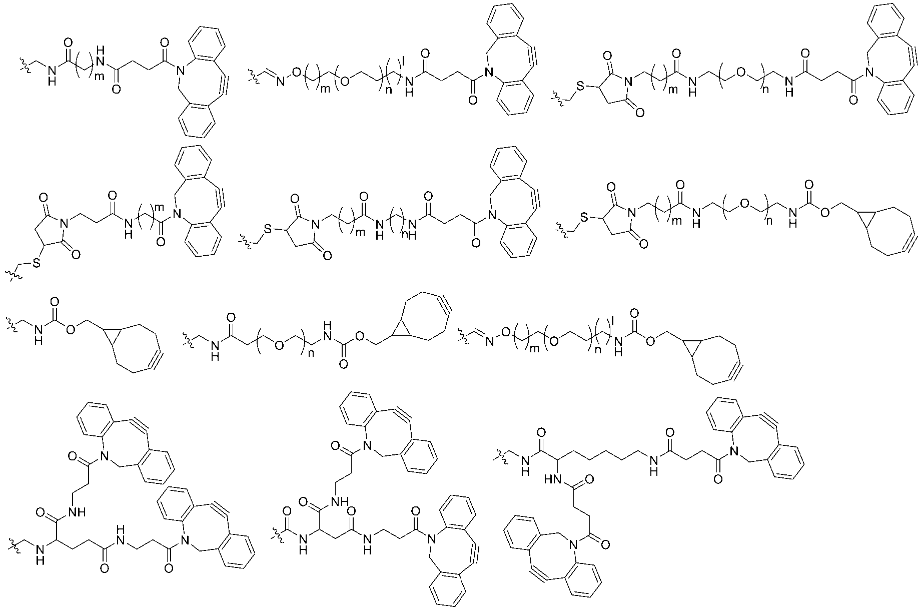 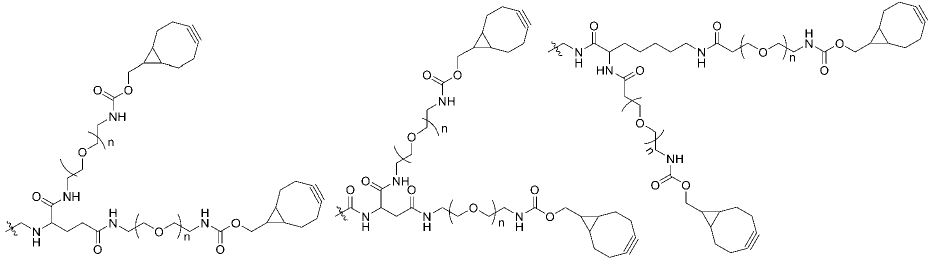 wherein n, m, and I are each independently an integer selected from the group consisting of 0-30, or an integer from 0-20, or an integer from 0-10.

In some embodiments of the present disclosure, in the disaccharide linker represented by formula (I), when Z is a reactive group containing an alkyne residue, it is selected from the group consisting of: 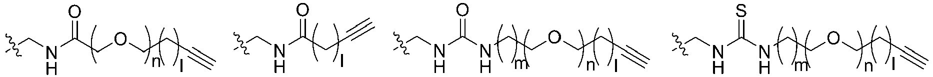 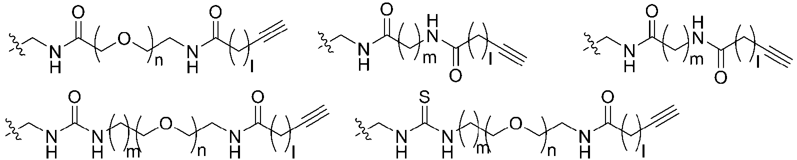 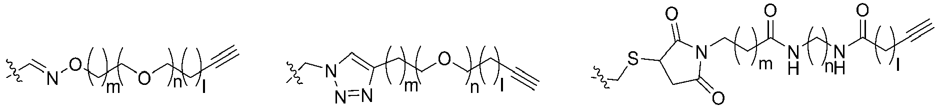 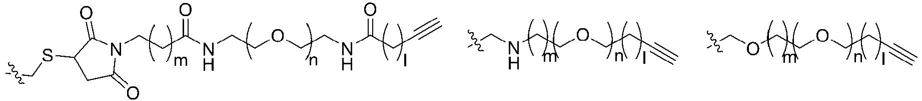 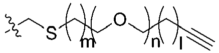 wherein n, m, and I are each independently an integer selected from the group consisting of 0-30, or an integer from 0-20, or an integer from 0-10.

In some embodiments of the present disclosure, in the disaccharide linker represented by formula (I), when Z is a reactive group containing an alkyne residue, it is selected from the group consisting of: 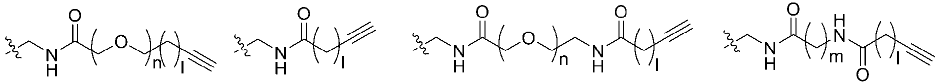 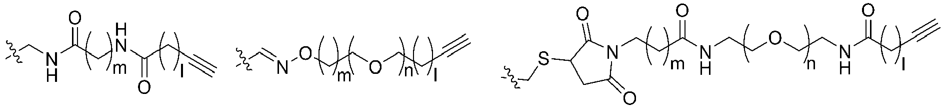 wherein n, m, and I are each independently an integer selected from the group consisting of 0-30, or an integer from 0-20, or an integer from 0-10.

In some embodiments of the present disclosure, in the disaccharide linker represented by formula (I), when Z is a reactive group containing a tetrazine residue, it is selected from the group consisting of: 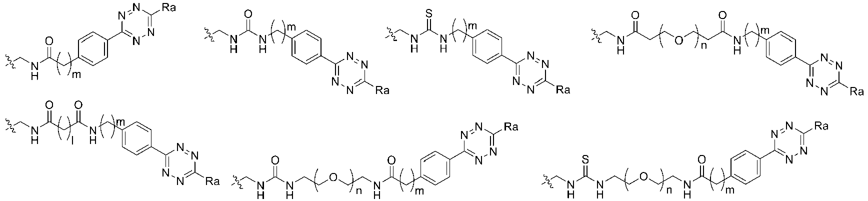 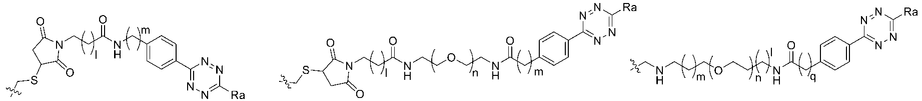 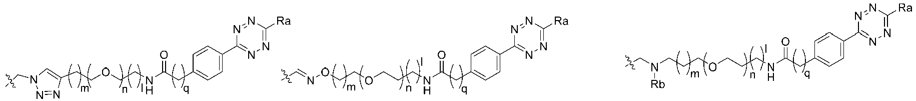 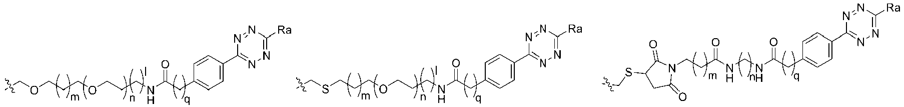 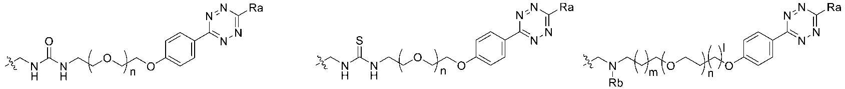 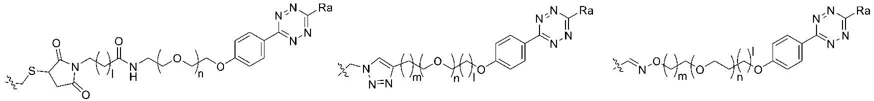 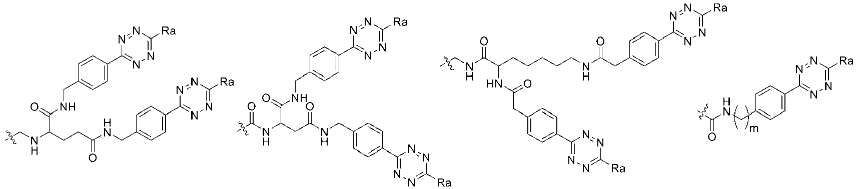 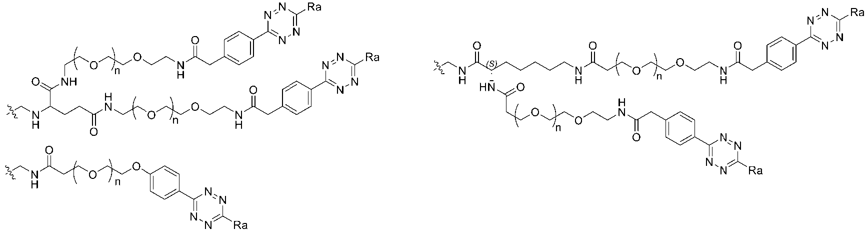
wherein n, m, I, and q are each independently an integer selected from the group consisting of 0-30, or an integer from 0-20, or an integer from 0-10;
Ra is hydrogen or C₁-C₆ alkyl; Rb is hydrogen, C₁-C₆ alkyl, C₆-C₁₀ aryl, or C₅-C₁₀ heteroaryl.

In some embodiments of the present disclosure, in the disaccharide linker represented by formula (I), when Z is a reactive group containing a tetrazine residue, it is selected from the group consisting of: 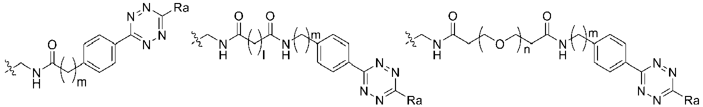 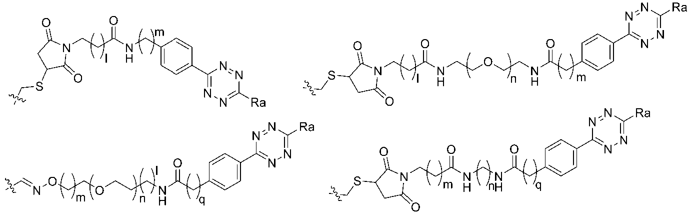 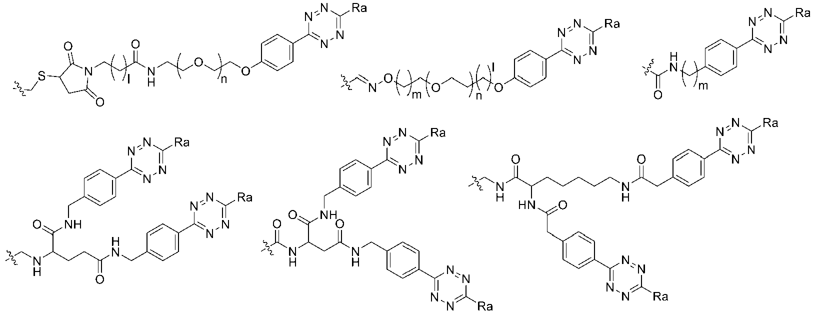 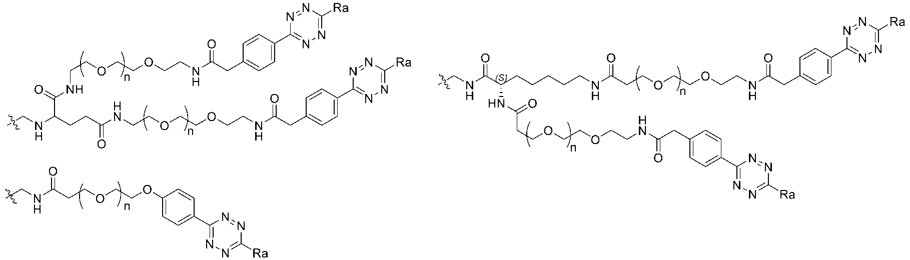 wherein n, m, I, and q are each independently an integer selected from the group consisting of 0-30, or an integer from 0-20, or an integer from 0-10; Ra is hydrogen or C₁-C₆ alkyl.

In some embodiments of the present disclosure, in the disaccharide linker represented by formula (I), when Z is a reactive group containing a cycloalkene residue, it is selected from the group consisting of: 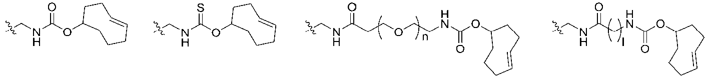 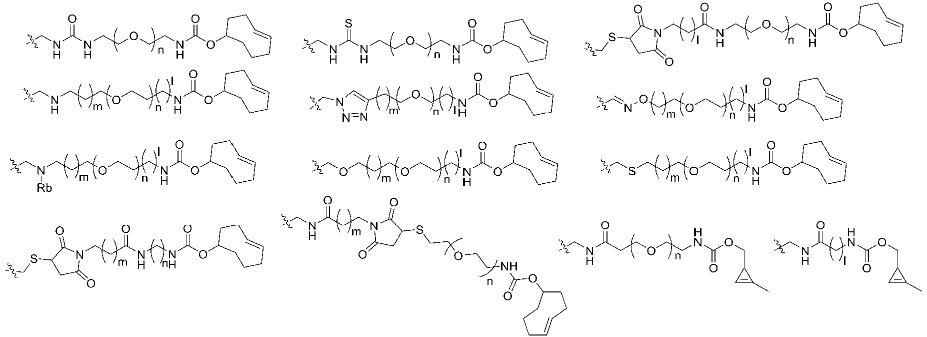
wherein n, m, and I are each independently an integer selected from the group consisting of 0-30, or an integer from 0-20, or an integer from 0-10;
Rb is hydrogen, C₁-C₆ alkyl, C₆-C₁₀ aryl, or C₅-C₁₀ heteroaryl.

In some embodiments of the present disclosure, in the disaccharide linker represented by formula (I), when Z is a reactive group containing a cycloalkene residue, it is selected from the group consisting of: 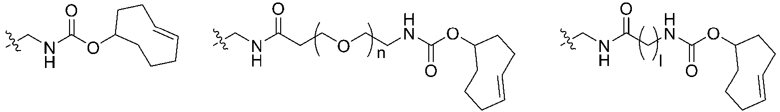 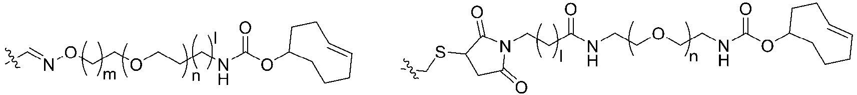 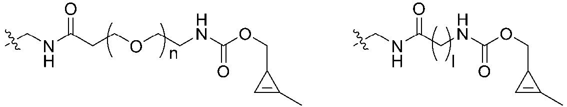 wherein n, m, and I are each independently an integer selected from the group consisting of 0-30, or an integer from 0-20, or an integer from 0-10.

In some embodiments of the present disclosure, in the disaccharide linker represented by formula (I), when Z is a reactive group containing a maleimide residue, it is selected from the group consisting of:

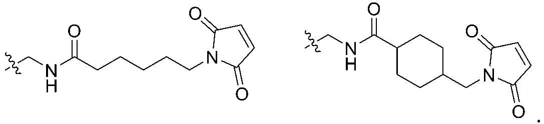

In some embodiments of the present disclosure, the disaccharide linker represented by formula (I) is selected from the group consisting of the following specific compounds: 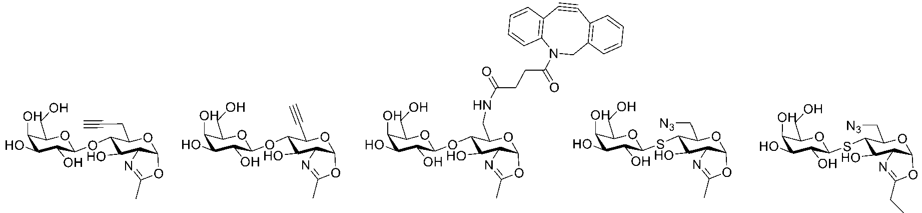 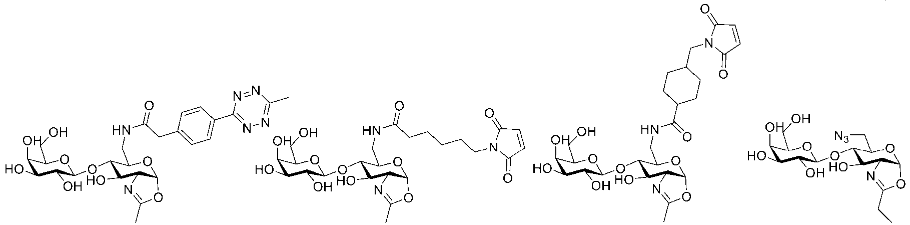 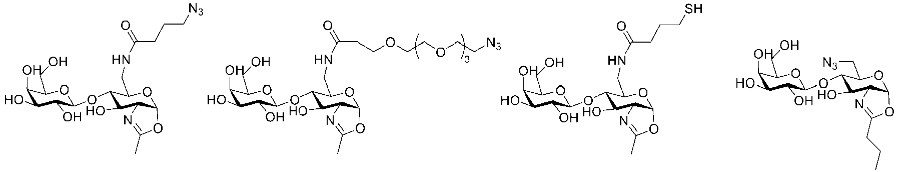 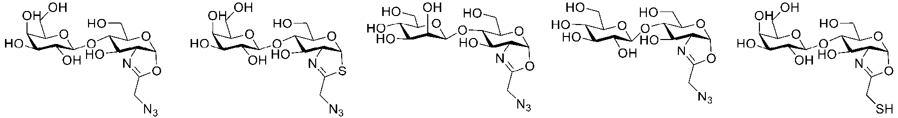 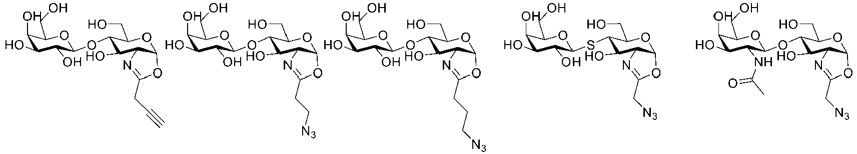 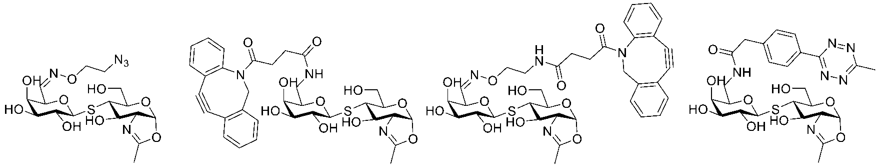 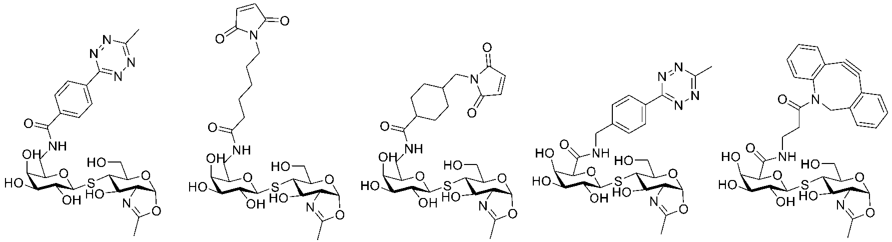 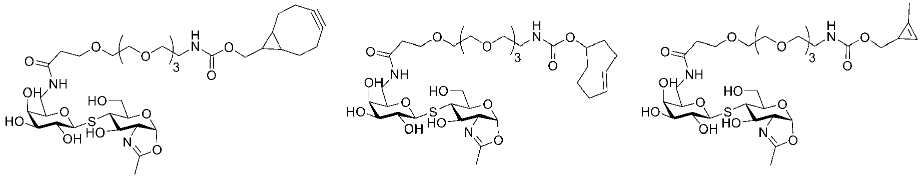 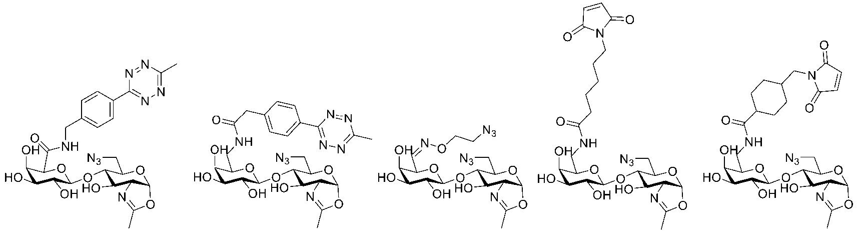 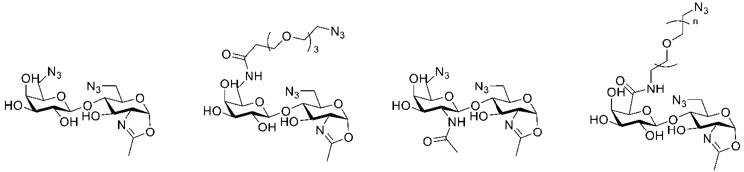 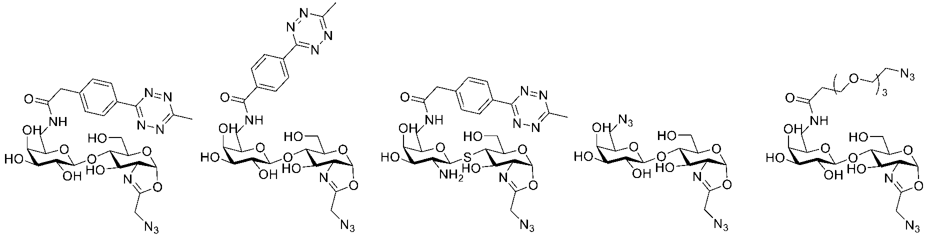 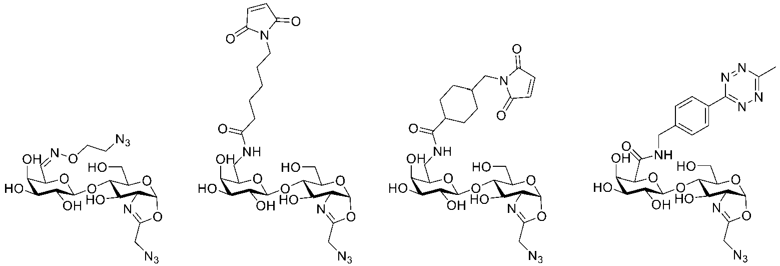 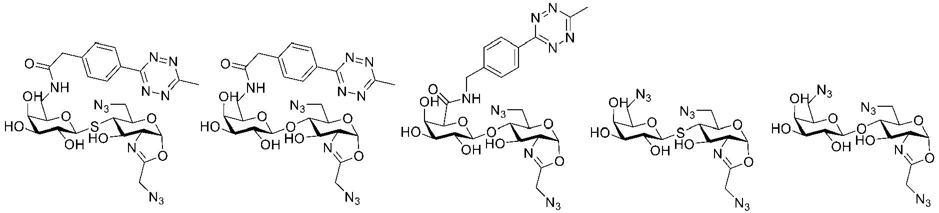 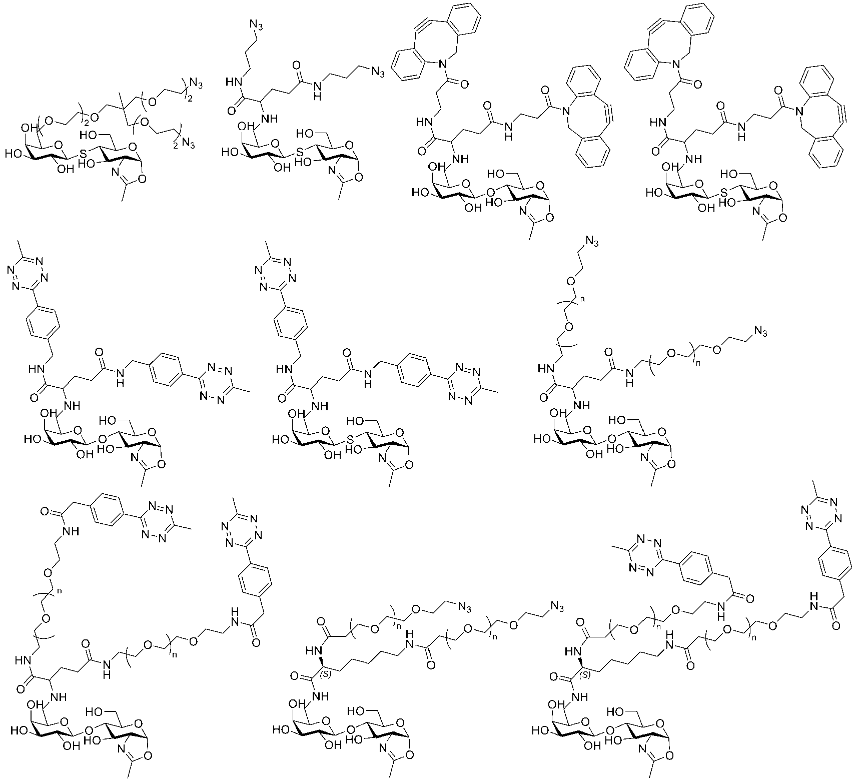 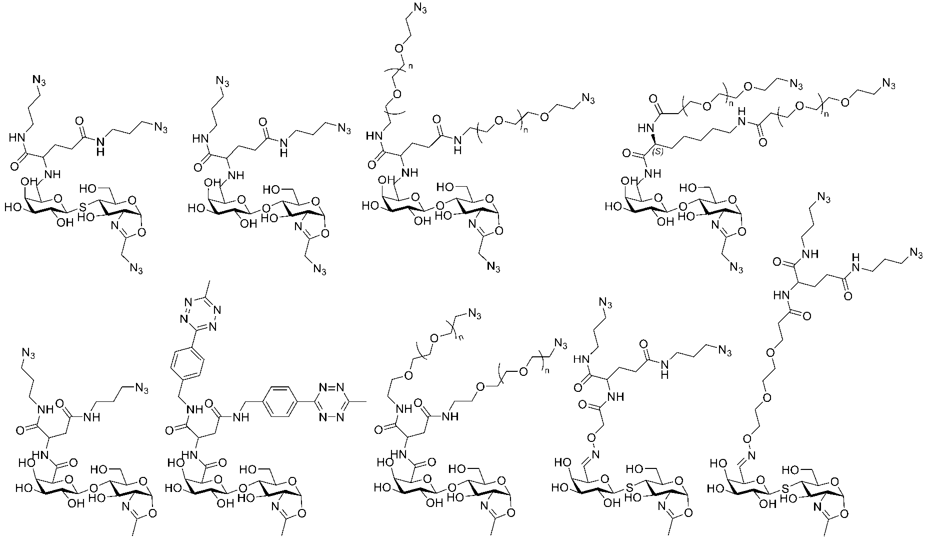 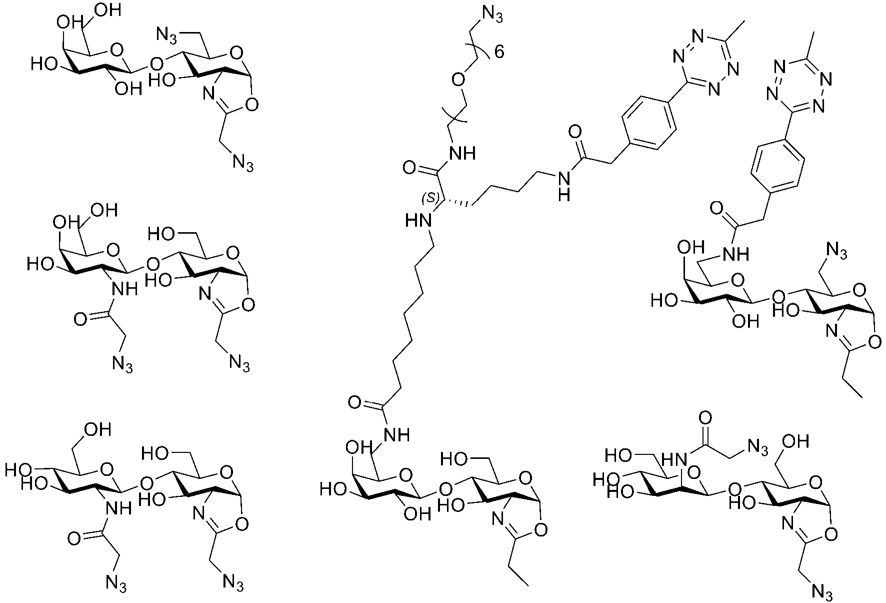 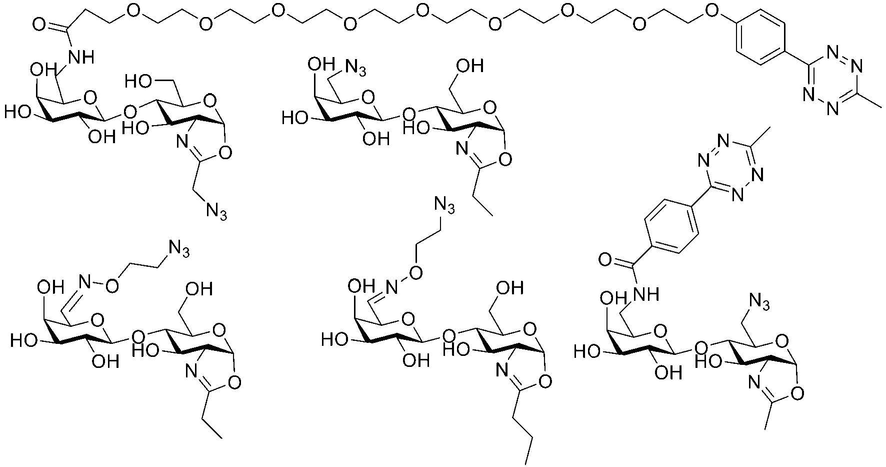 wherein n is an integer selected from the group consisting of 0-30, or an integer from 0-20, or an integer from 0-10.

In a second aspect, the present disclosure provides a disaccharide conjugate having a structure represented by formula (II): 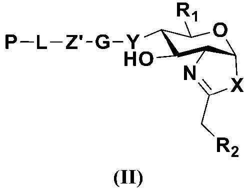 wherein
X, Y, R₁, R₂, and ring G are as defined above;
P-L-Z'- represents a substituent on the ring G, and the substitution position of P-L-Z'-is any position other than position 1 of the ring G derived from the monosaccharide molecule;
L is a divalent or multivalent linker connecting Z' and P, comprising a cleavable linker, a non-cleavable linker, or a combination thereof;
P is selected from the group consisting of a cytotoxin, a small-molecule drug, a near-infrared or fluorescent probe, a peptide, RNA and related drugs, a radioisotope label, a contrast agent, and a magnetic resonance imaging agent; the small-molecule drug is preferably selected from the group consisting of maytansine, DM-1, DM-4, MMAE, MMAF, Auristatin 0101, SN-38, Dxd, exatecan, duocarmycin, amanitin, PBDs, VP-16, camptothecin, paclitaxel, docetaxel, anthracyclines, and derivatives of the above compounds, or the small-molecule drug is a radiotherapeutic agent;
Z' is a linking moiety connecting L and ring G, which is independently absent or one or any combination of two or more selected from the group consisting of -(CH₂)ₚ-, -(CH₂-CH₂-O)ₚ-, -(phenyl)ₚ-, and the following groups: 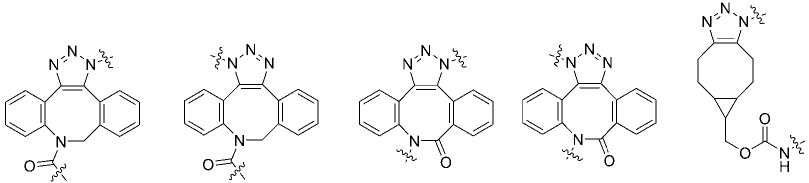 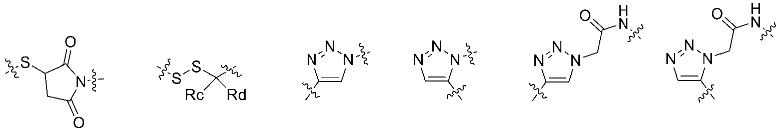 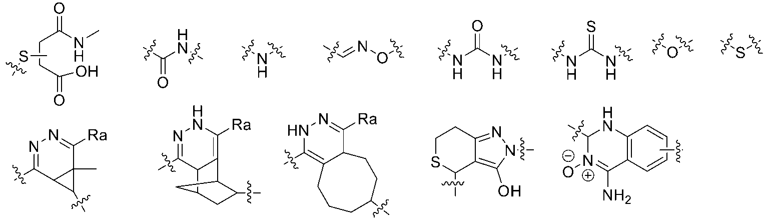
wherein Ra, Rc, and Rd are each independently selected from the group consisting of hydrogen, C₁-C₆ alkyl, C₆-C₁₀ aryl, and C₅-C₁₀ heteroaryl; and
each p is an integer from 1 to 5, for example, 1, 2, 3, 4, and 5.

In some embodiments of the present disclosure, in the disaccharide conjugate represented by formula (II), Z' is a linking moiety connecting L and ring G, which is independently absent or is -(CH₂)ₚ- or -(CH₂-CH₂-O)ₚ-, wherein p is an integer from 1 to 5, or comprises the specific groups enumerated above.

In some embodiments of the present disclosure, in the disaccharide conjugate represented by formula (II), L is selected from the group consisting of or comprises one or a combination of more than one of the following: Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Gly-Gly-Gly, Gly-Gly-Phe-Gly, and Gly-Gly-Gly-Gly-Gly.

In some embodiments of the present disclosure, in the disaccharide conjugate represented by formula (II), L is selected from the group consisting of or comprises one or a combination of more than one of the following: Val-Cit, Val-Ala, Ala-Ala, and Gly-Gly-Phe-Gly.

In some embodiments of the present disclosure, in the disaccharide conjugate represented by formula (II), L is selected from the group consisting of or comprises one or a combination of more than one of the following cleavable linkers:

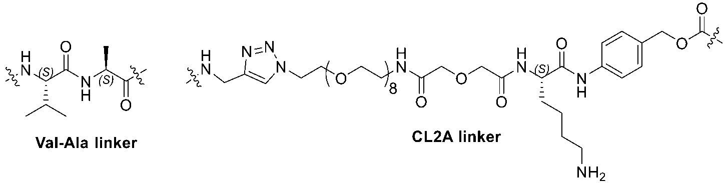

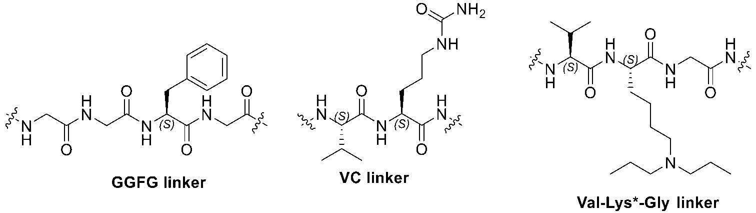

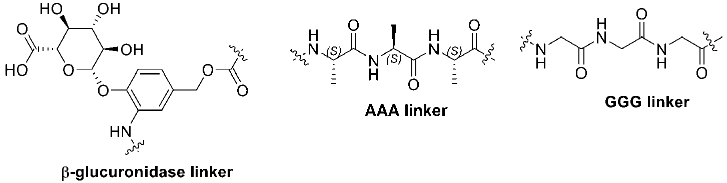

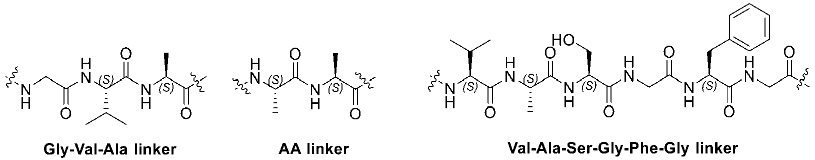

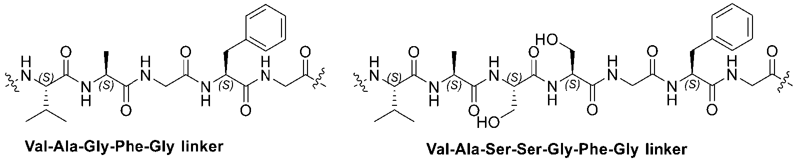

In some embodiments of the present disclosure, in the disaccharide conjugate represented by formula (II), L is selected from the group consisting of or comprises one or a combination of more than one of the following non-cleavable linkers: -(CH₂)ₘ-, -(CH₂-CH₂-O)ₘ-, and -(PO₄)ₙ-, wherein m and n are each independently an integer selected from the group consisting of 0-30, or an integer from 0-20, or an integer from 0-10.

In some embodiments of the present disclosure, in the disaccharide conjugate represented by formula (II), when P is a toxin, it is selected from the group consisting of:

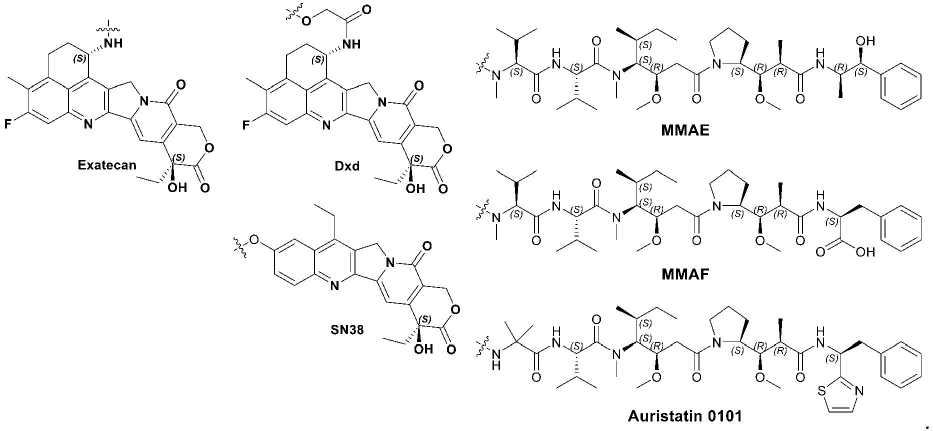

In some embodiments of the present disclosure, the disaccharide conjugate represented by formula (II) is selected from the group consisting of the following specific compounds: 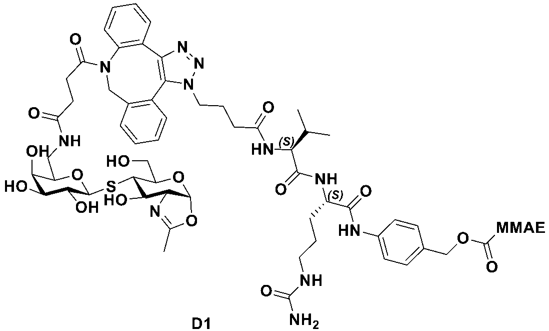 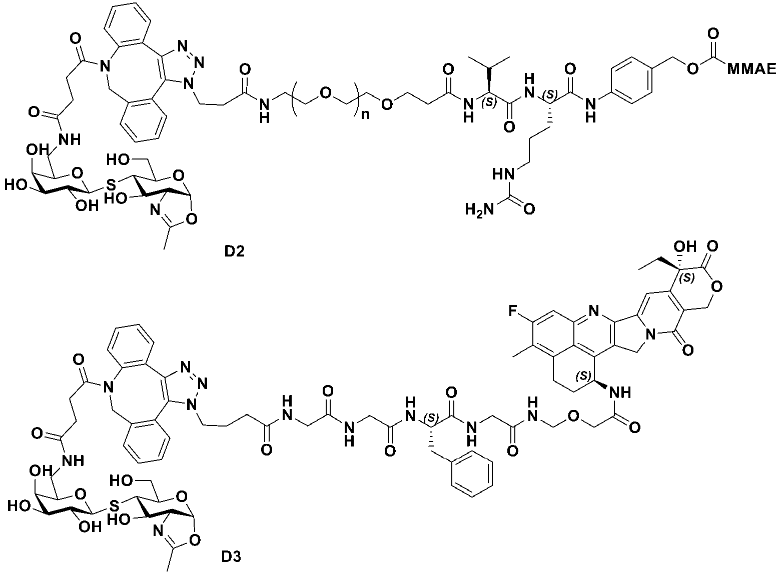 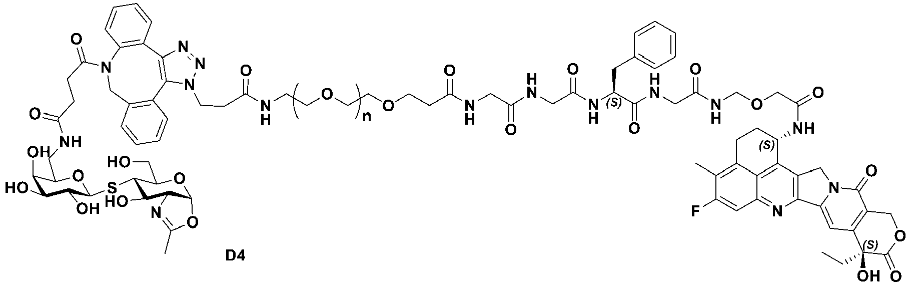 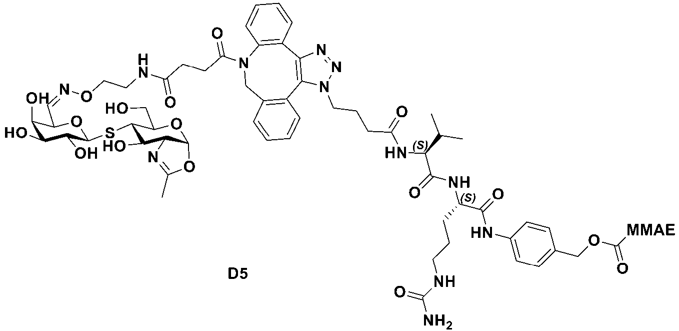 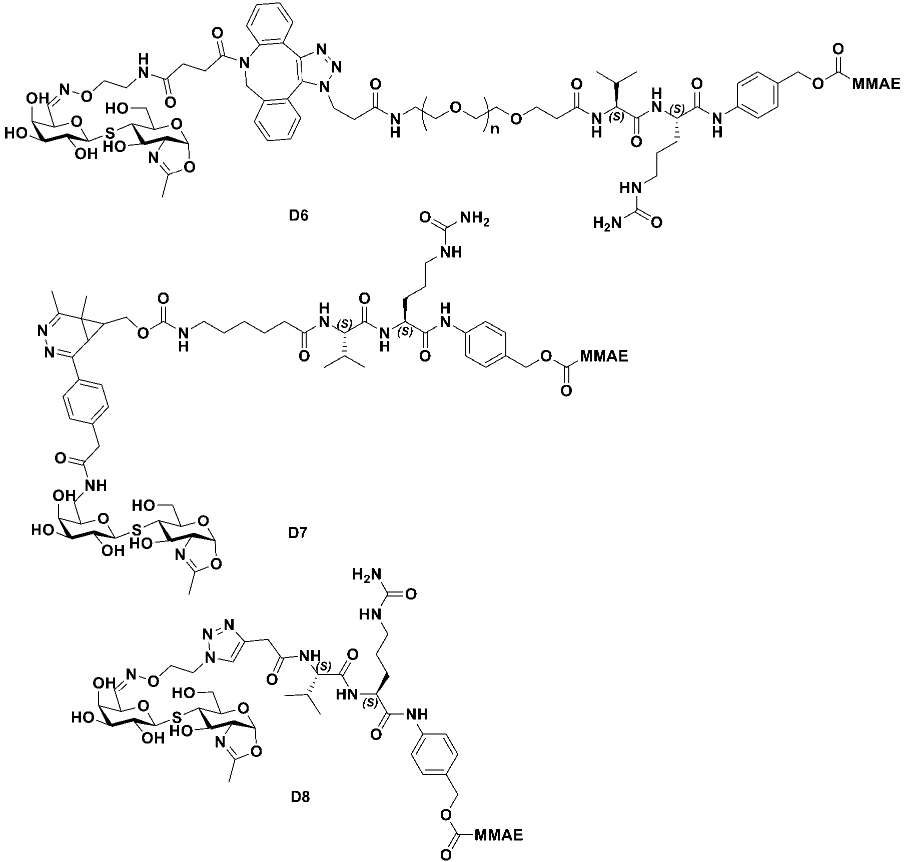 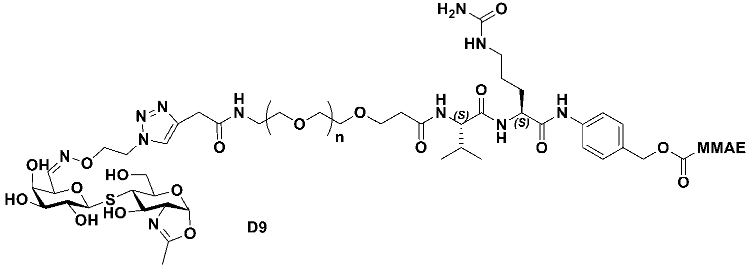 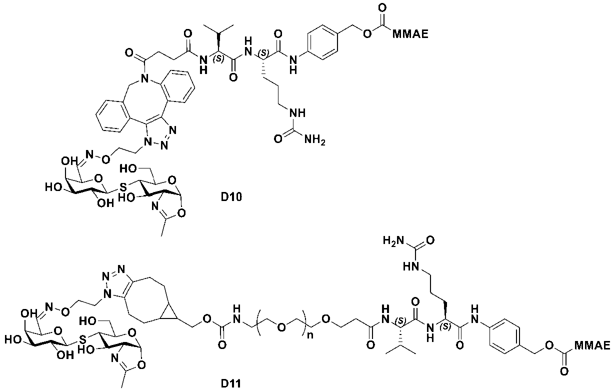 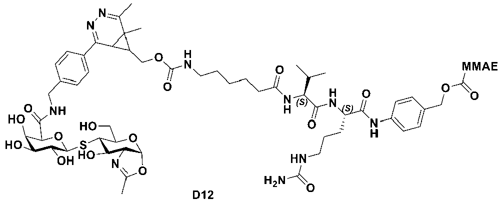 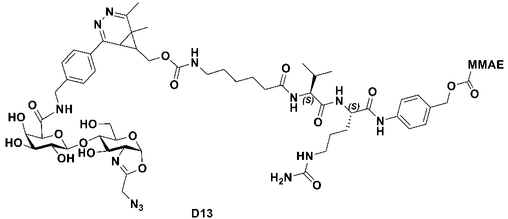 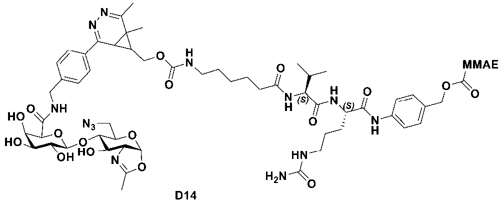 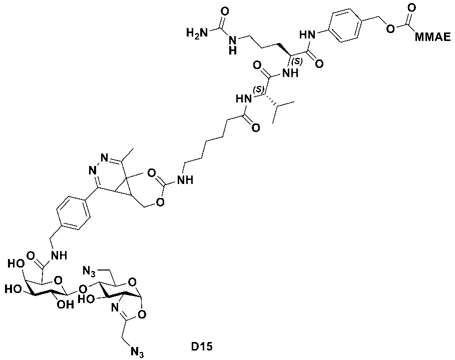 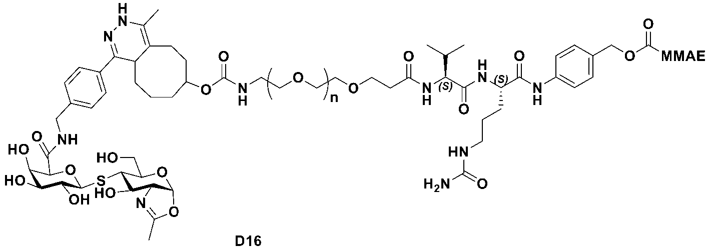 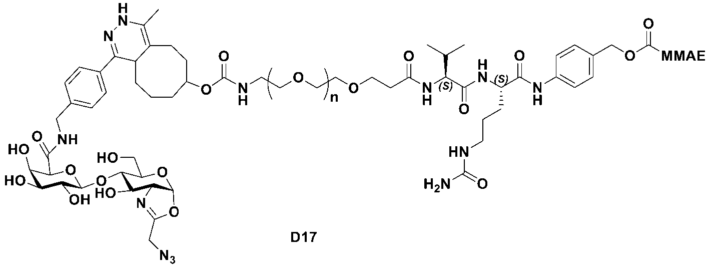 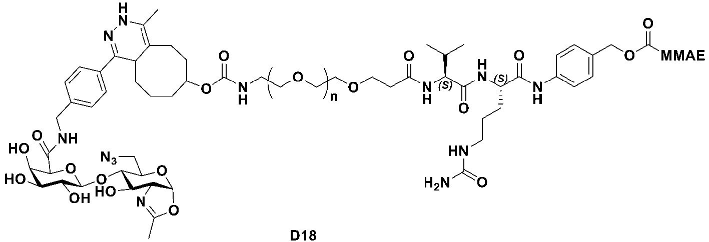 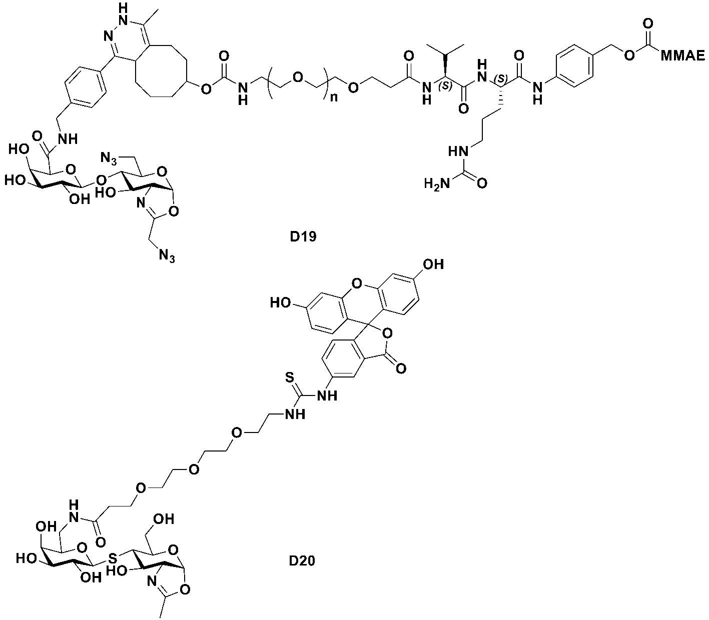 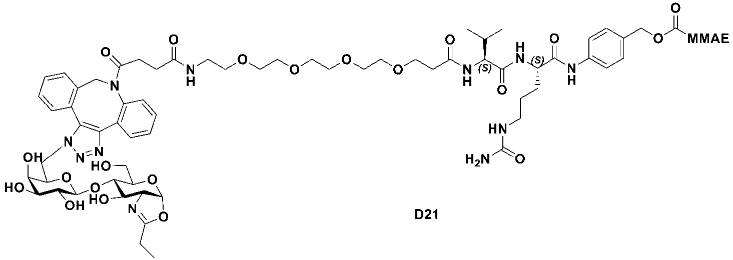 wherein n is an integer selected from the group consisting of 0-30, or an integer from 0-20, or an integer from 0-10.

In a third aspect, the present disclosure provides an antibody-based conjugate drug represented by formula (III) below: 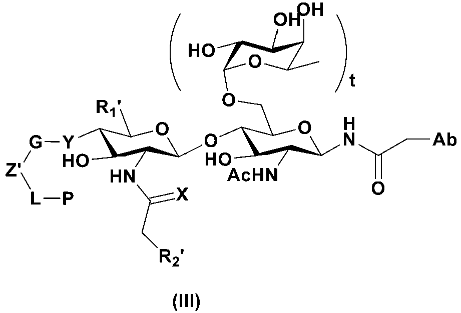
wherein X, Y, and ring G are as defined above;
Z', L, and P are as defined above, or P-L-Z'- is hydrogen;
t is 0 or 1;
R₁' is R₁ or a group formed after R₁ reacts with a drug linker E-L-P, and R₂' is R₂ or a group formed after R₂ reacts with a drug linker E-L-P; wherein R₁ and R₂ are as defined above; L and P in the drug linker E-L-P are as defined above, and E comprises the following groups: 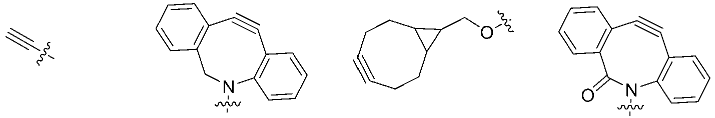 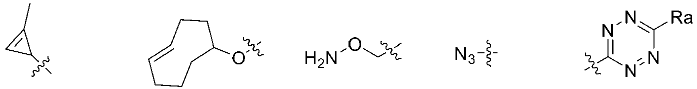 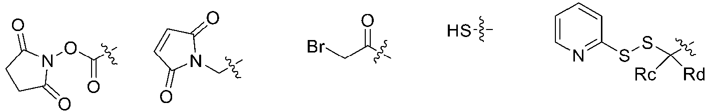 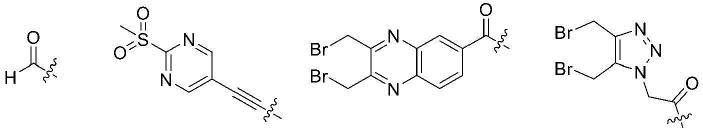
wherein Ra, Rc, and Rd are each independently selected from the group consisting of hydrogen, C₁-C₆ alkyl, C₆-C₁₀ aryl, and C₅-C₁₀ heteroaryl;
Ab is an antibody or antigen-binding fragment, selected from the group consisting of a monoclonal antibody, a bispecific antibody, and a multispecific antibody.

In some embodiments of the present disclosure, in the antibody-based conjugate drug represented by formula (III), the antibody is selected from the group consisting of: Pertuzumab, Trastuzumab, Rituximab, Cetuximab, Margetuximab, Abciximab, Daclizumab, Adalimumab, Palivizumab, Basiliximab, Bevacizumab, Panitumumab, Nimotuzumab, Denosumab, Zolbetuximab, Ramucirumab, Necitumumab, Ipilimumab, Daratumumab, Alemtuzumab, Elotuzumab, Blinatumomab, Nivolumab, Pembrolizumab, Atezolizumab, Avelumab, Durvalumab, Toripalimab, Catumaxomab, Emicizumab, and Amivantamab.

In some embodiments of the present disclosure, in the antibody-based conjugate drug represented by formula (III), the drug linker E-L-P is selected from the group consisting of the following specific compounds: 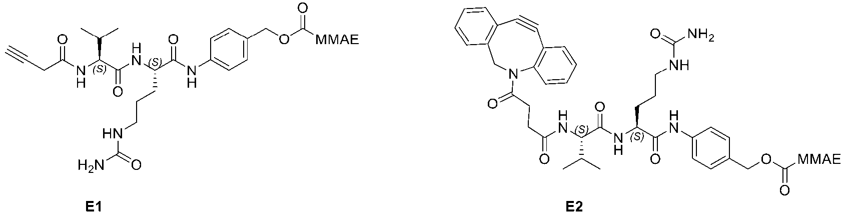 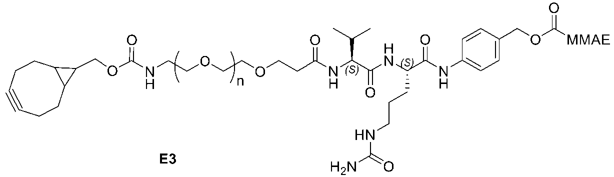 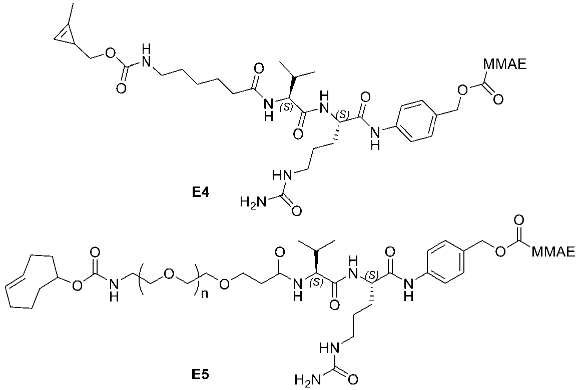 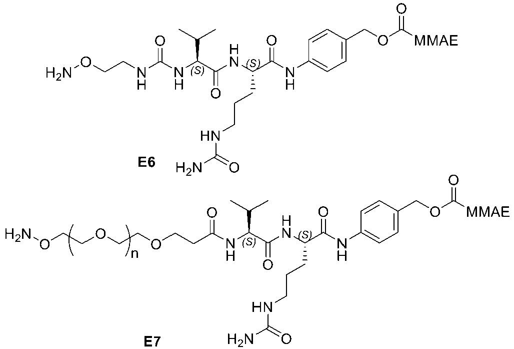 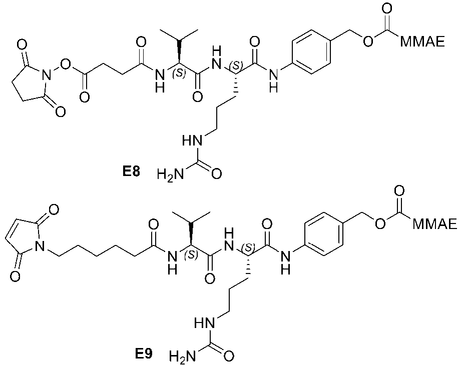 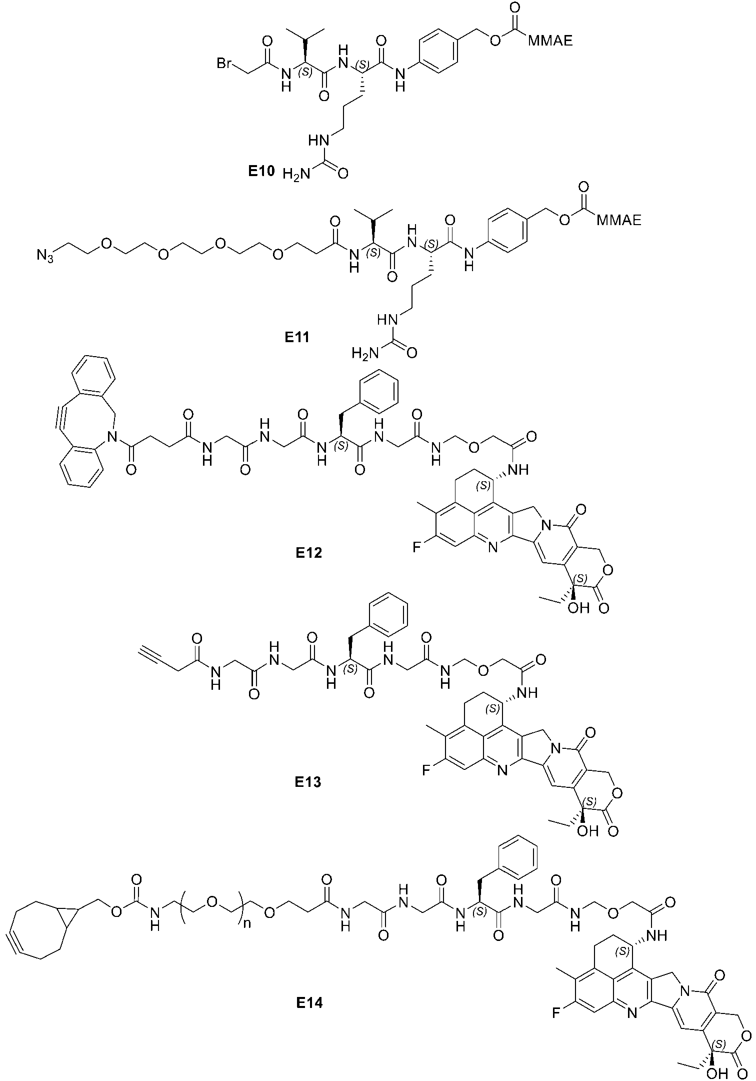 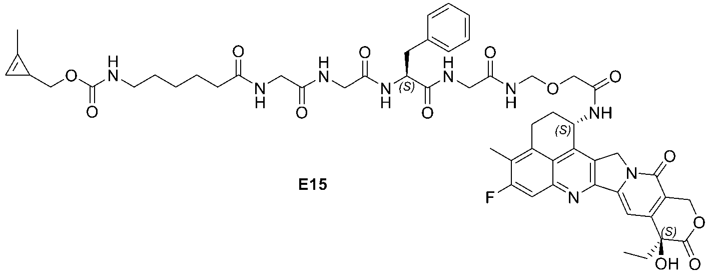 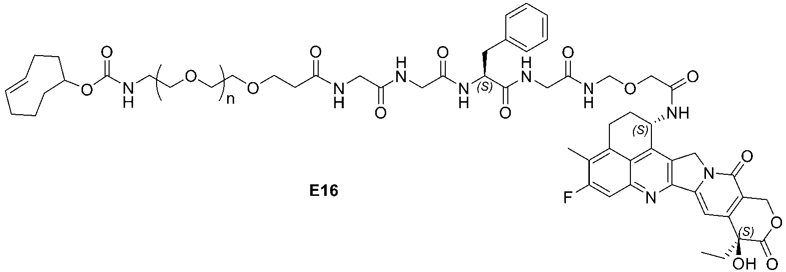 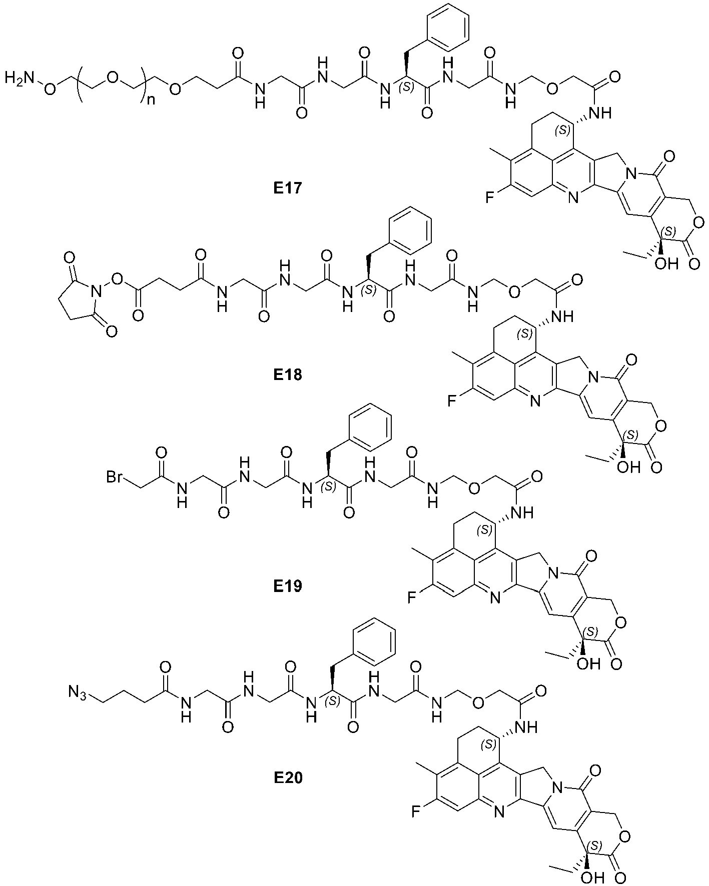 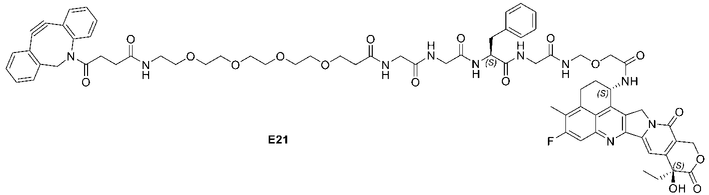 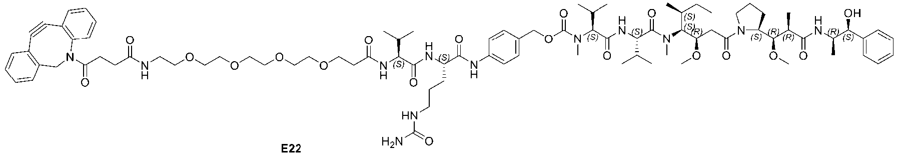 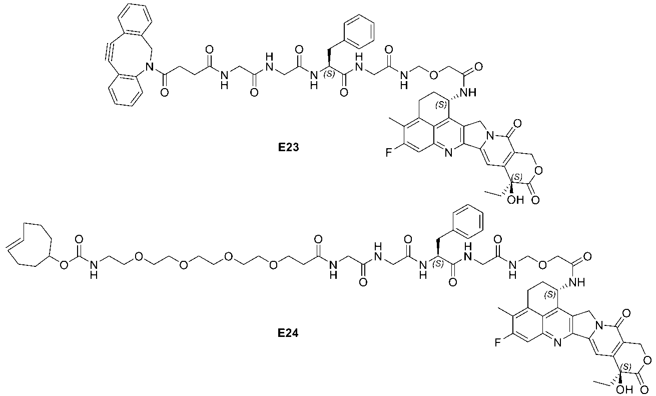 wherein n is an integer selected from the group consisting of 0-30, or an integer from 0-20, or an integer from 0-10.

In some embodiments of the present disclosure, when the antibody-based conjugate drug represented by formula (III) has a single payload, the antibody-based conjugate drug represented by formula (III) is selected from the group consisting of the following specific structures: 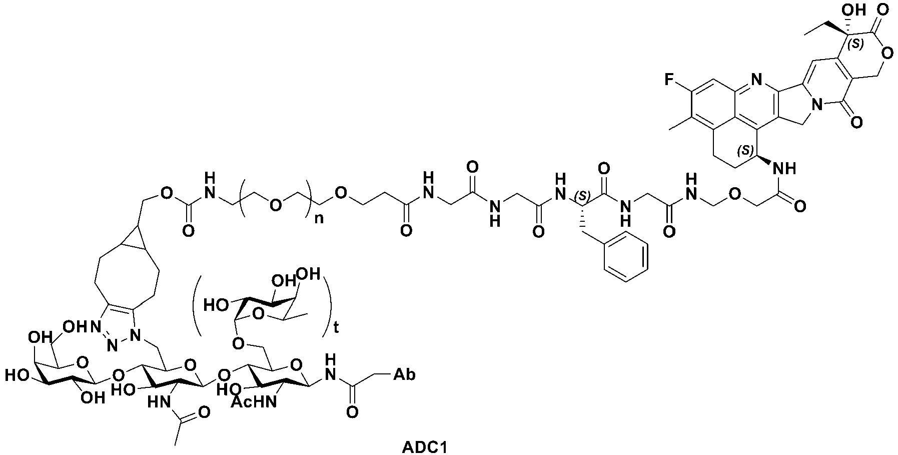 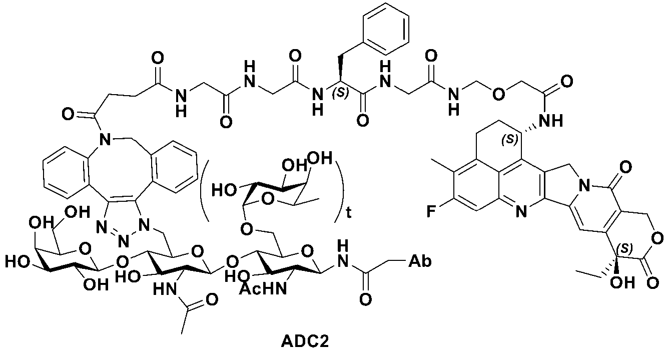 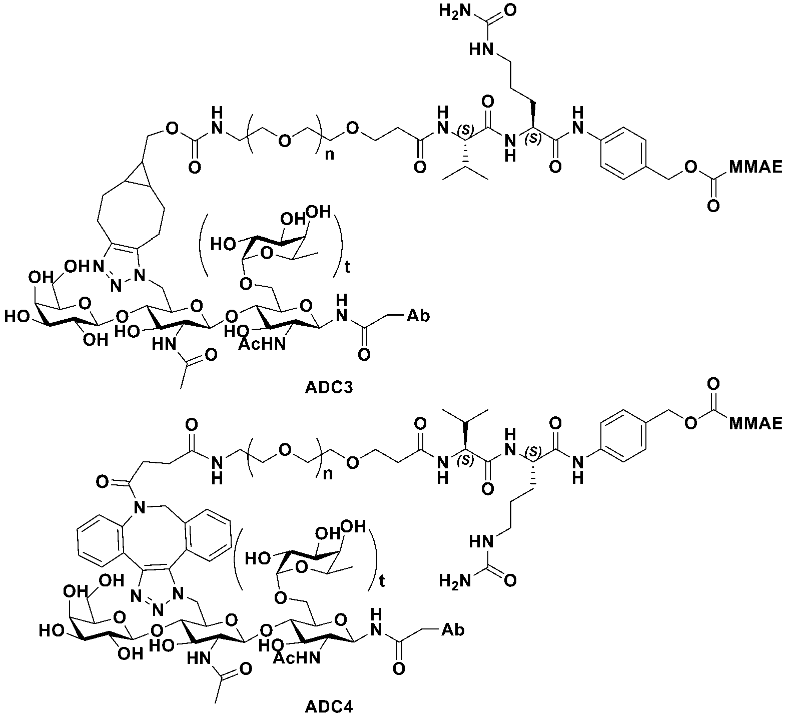 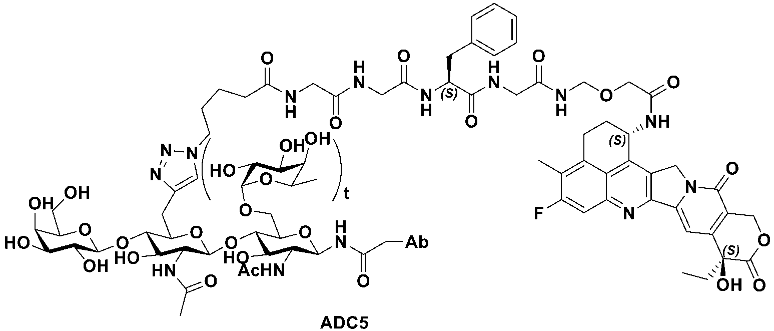 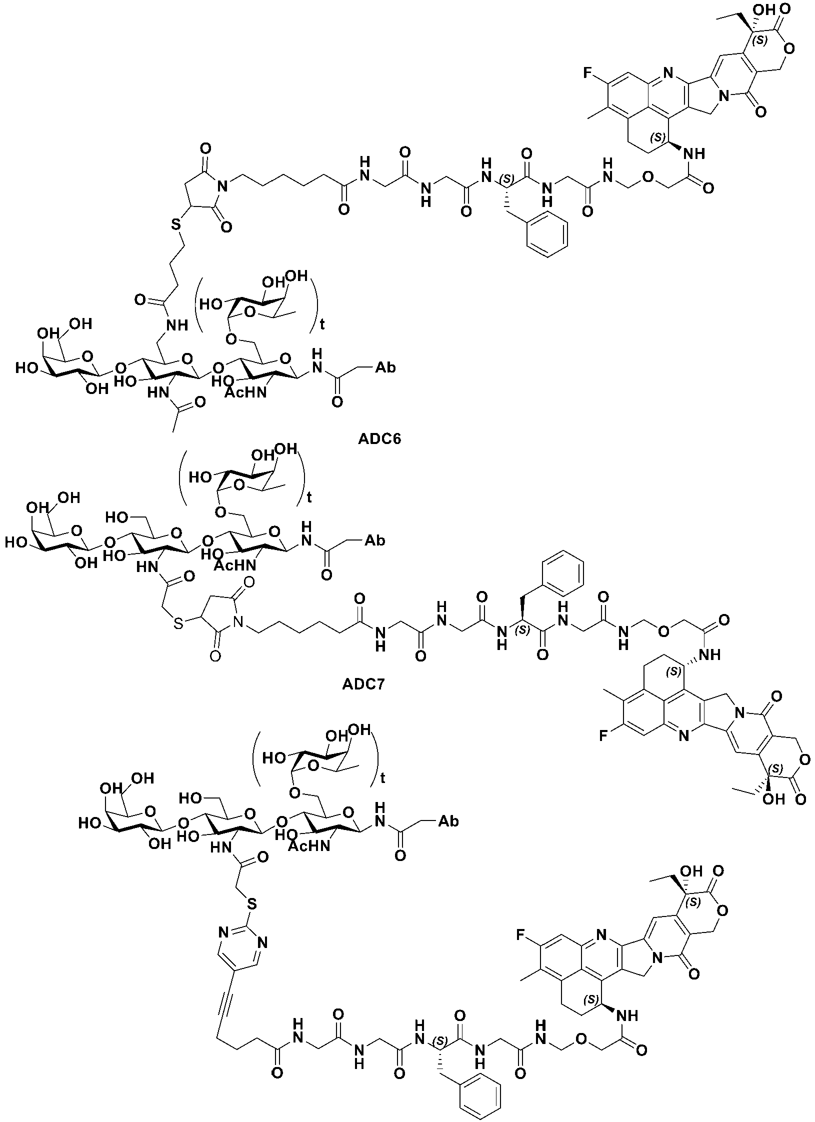 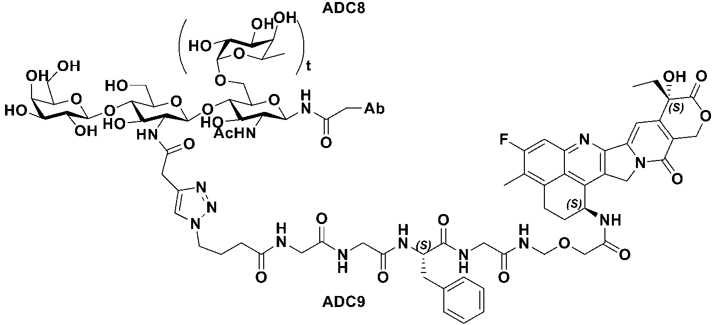 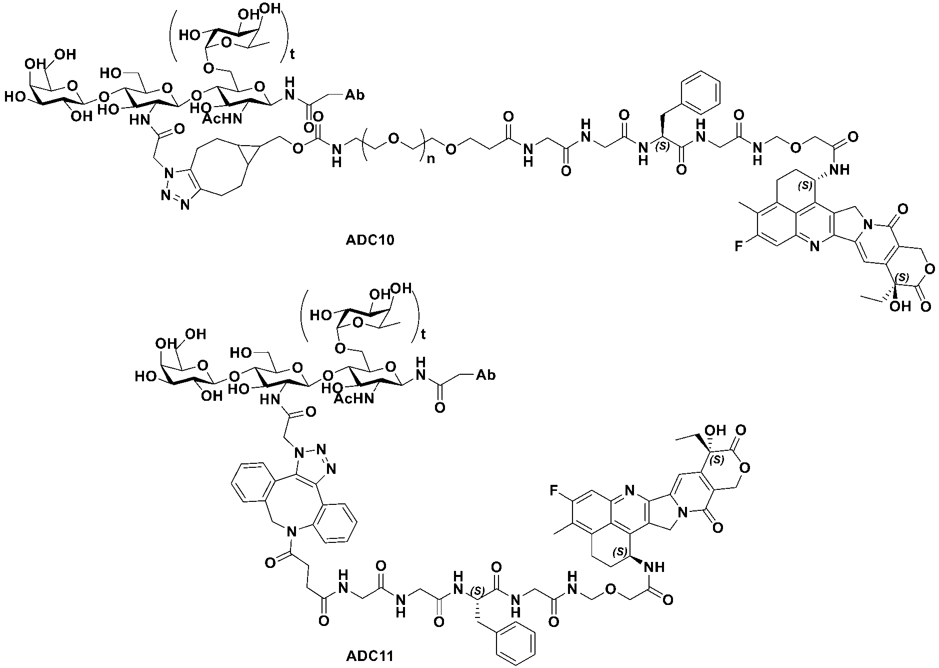 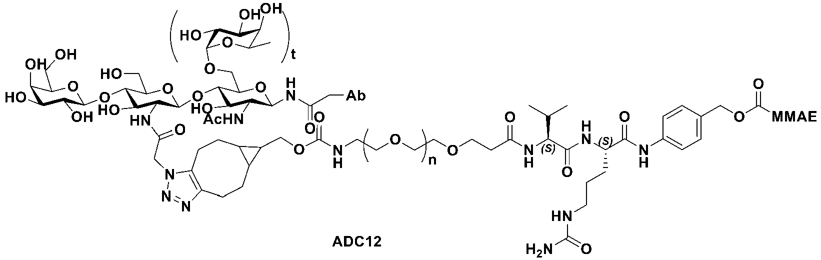 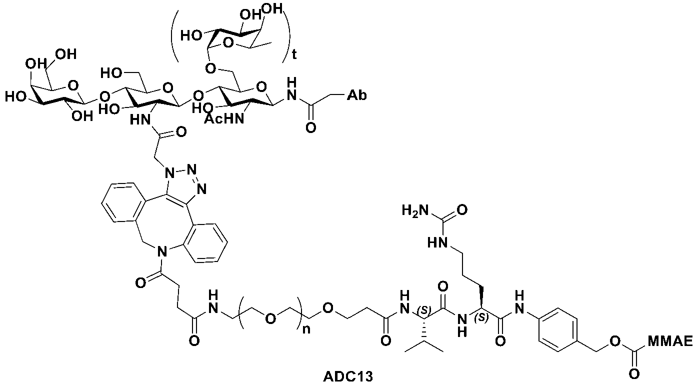 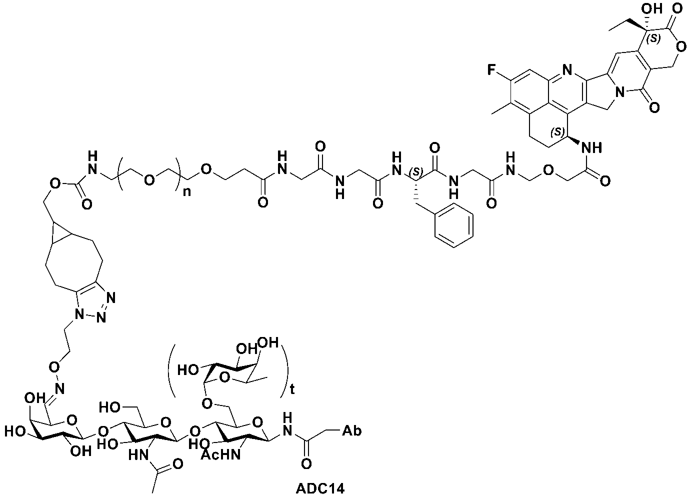 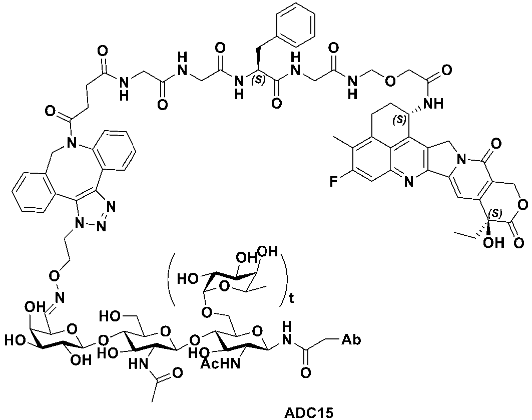 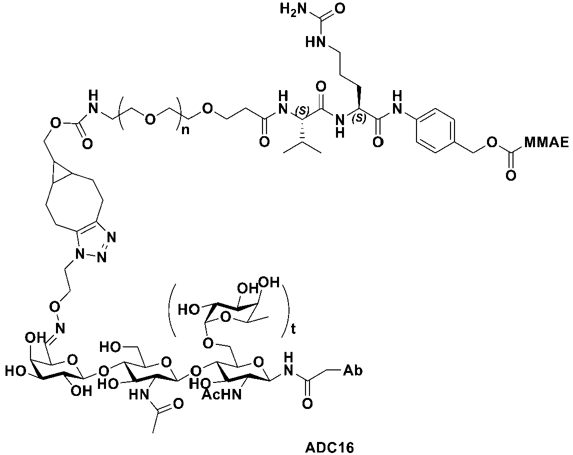 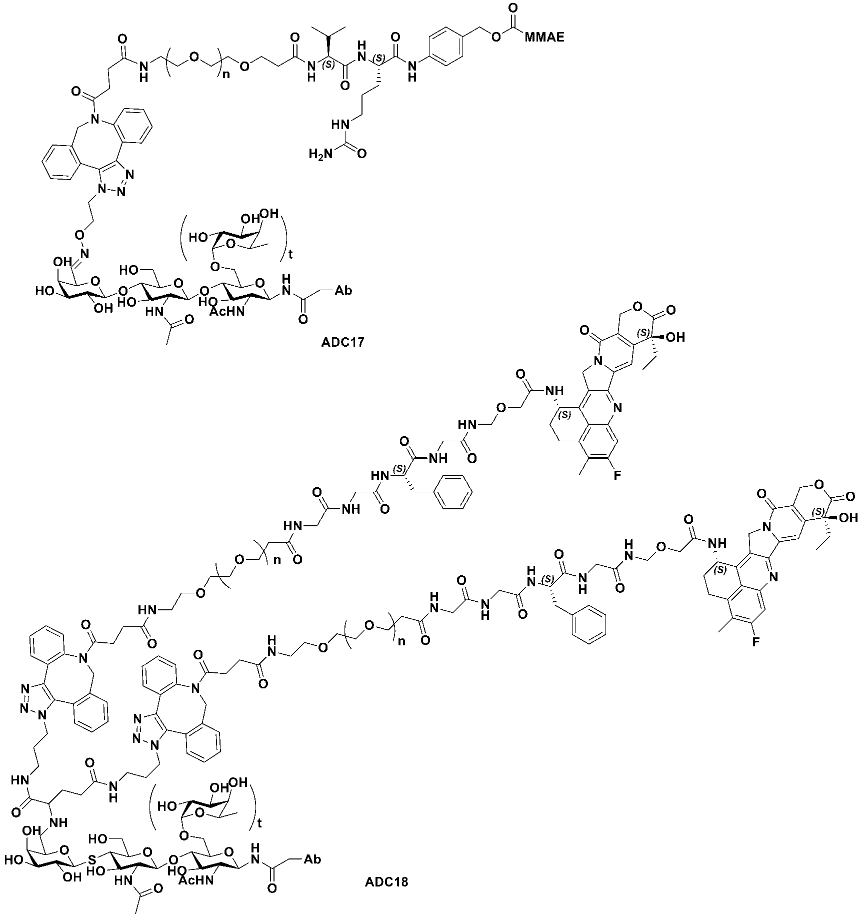 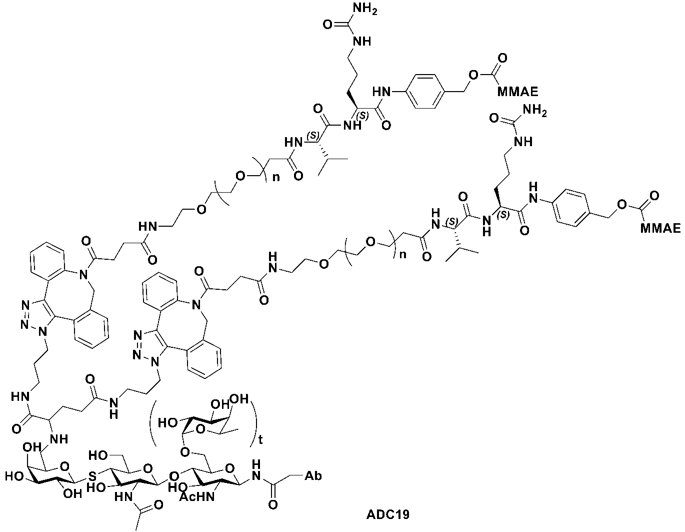 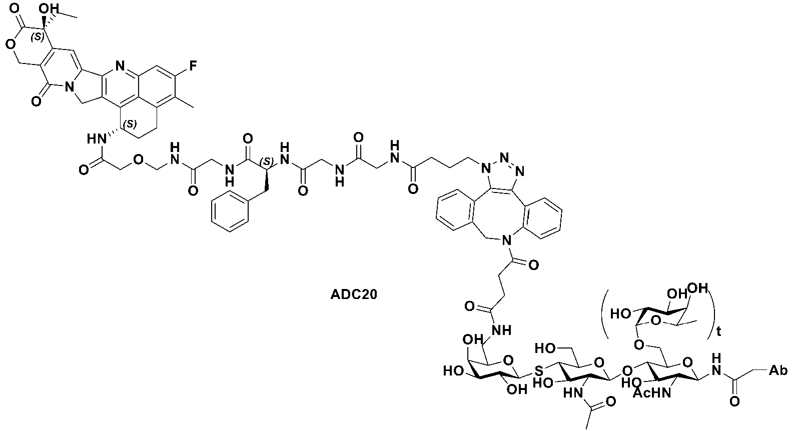 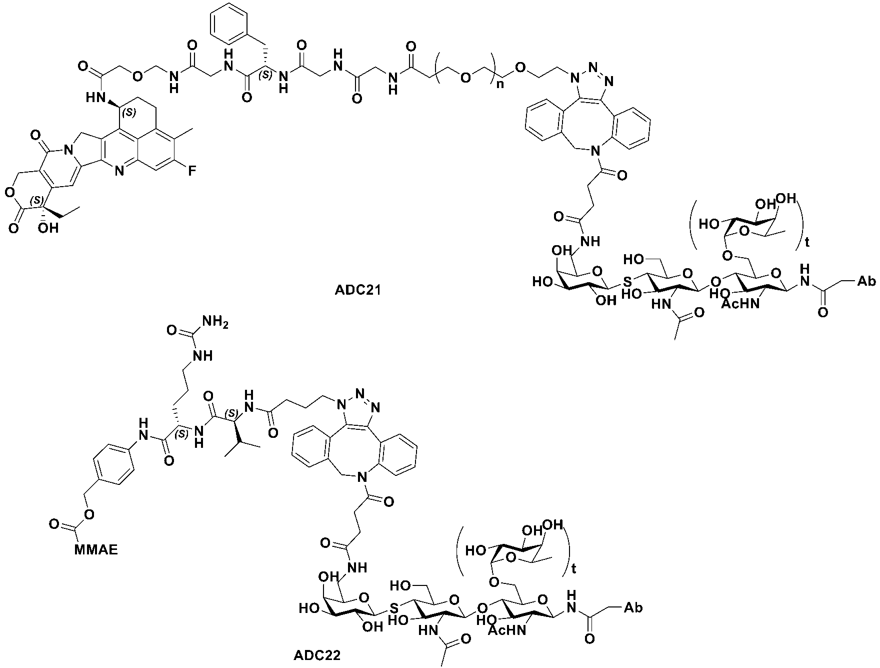 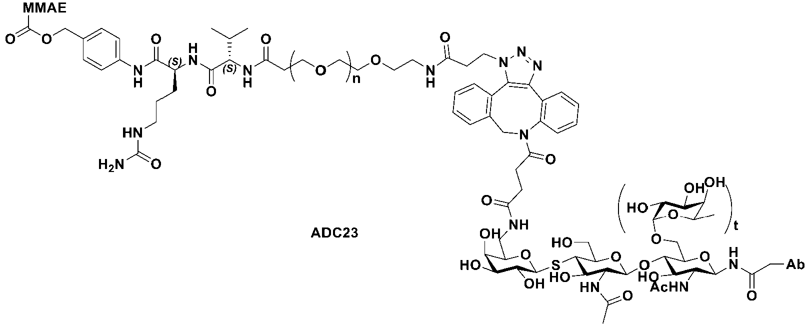 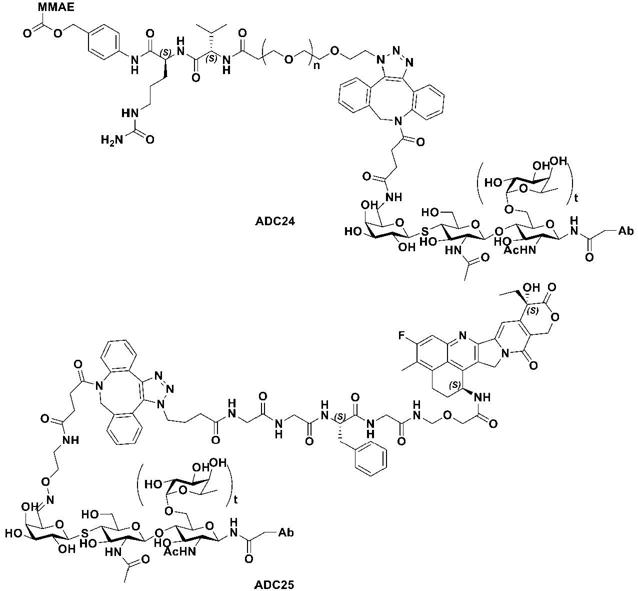 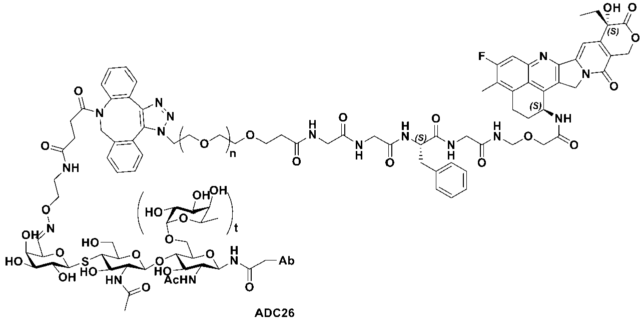 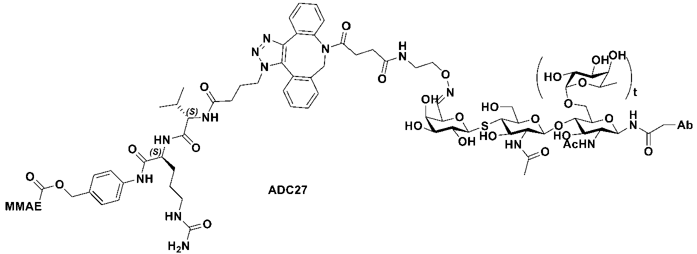 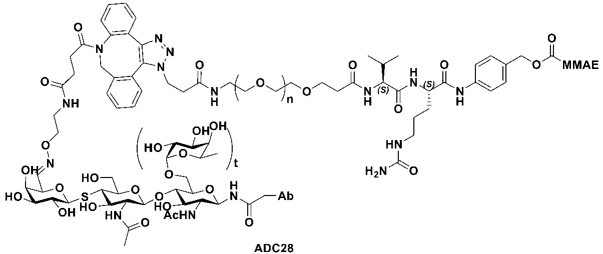 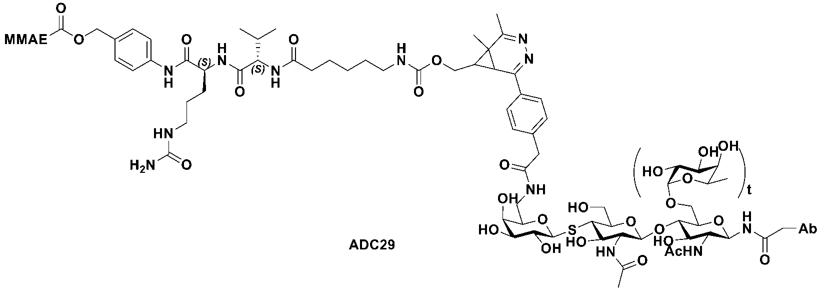 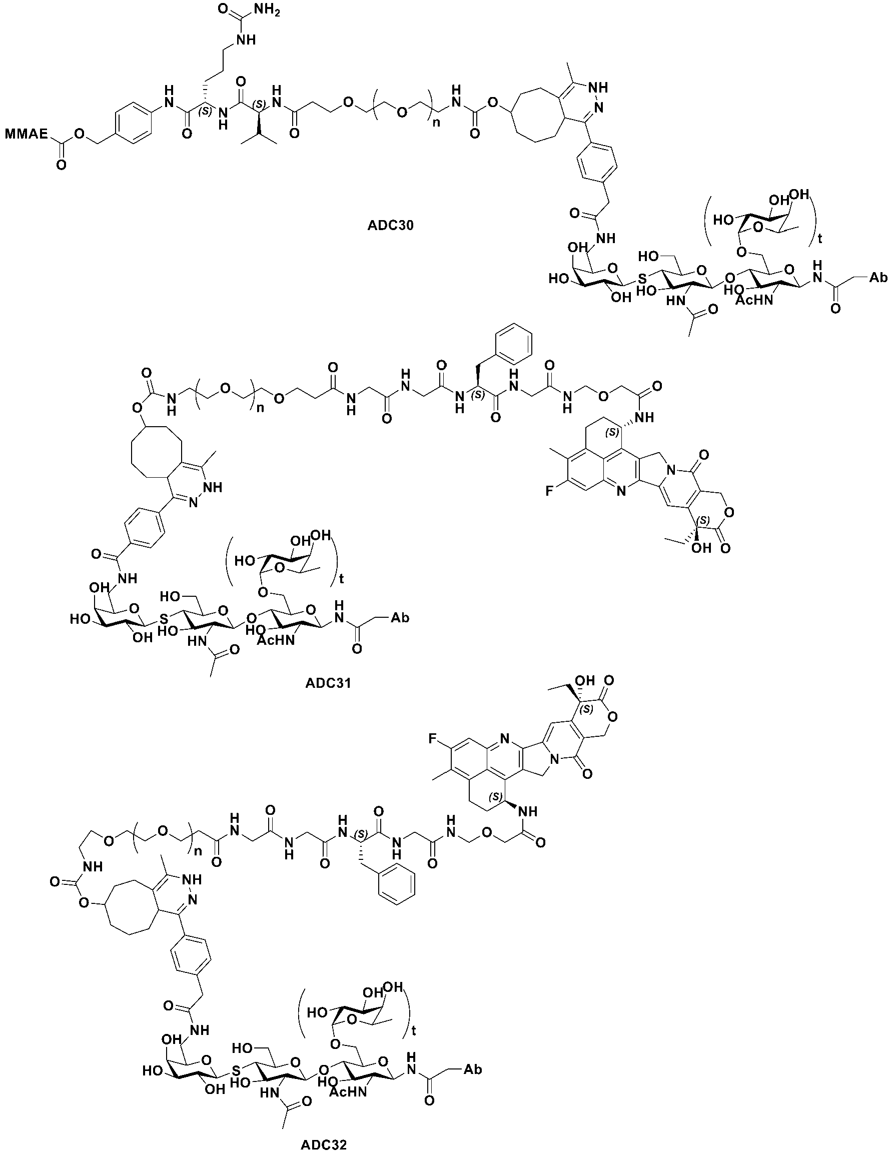 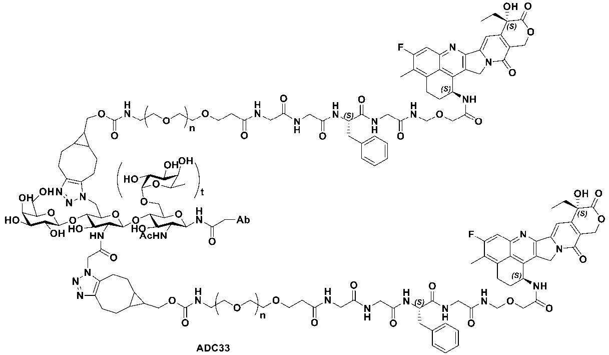 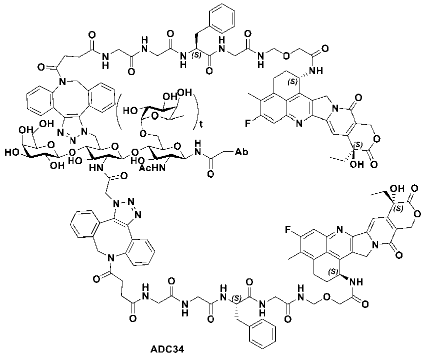 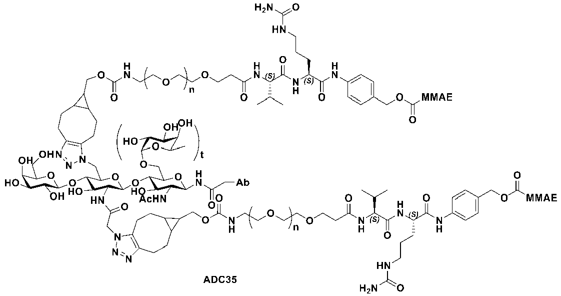 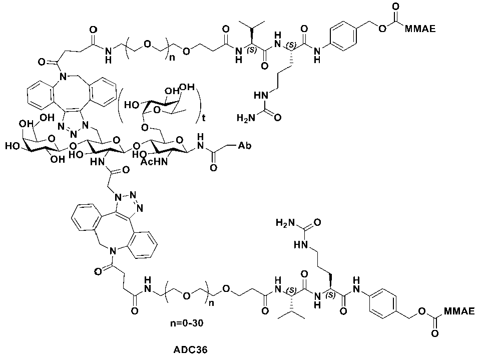 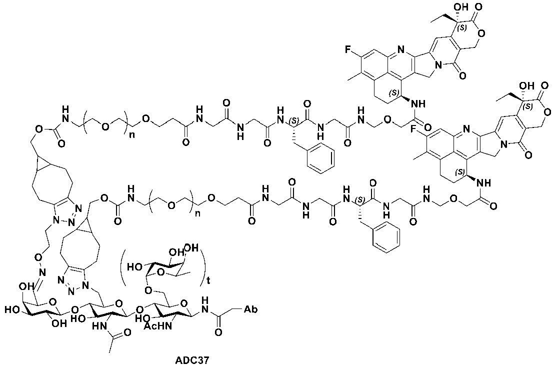 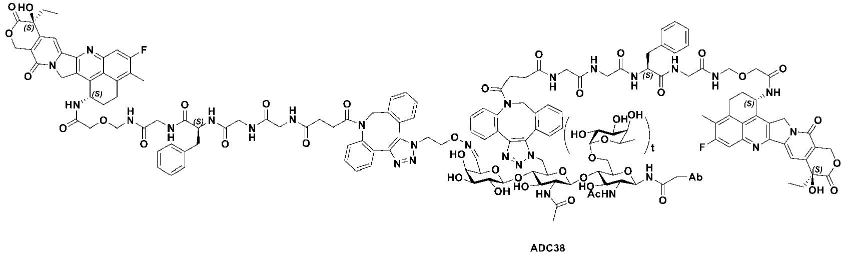 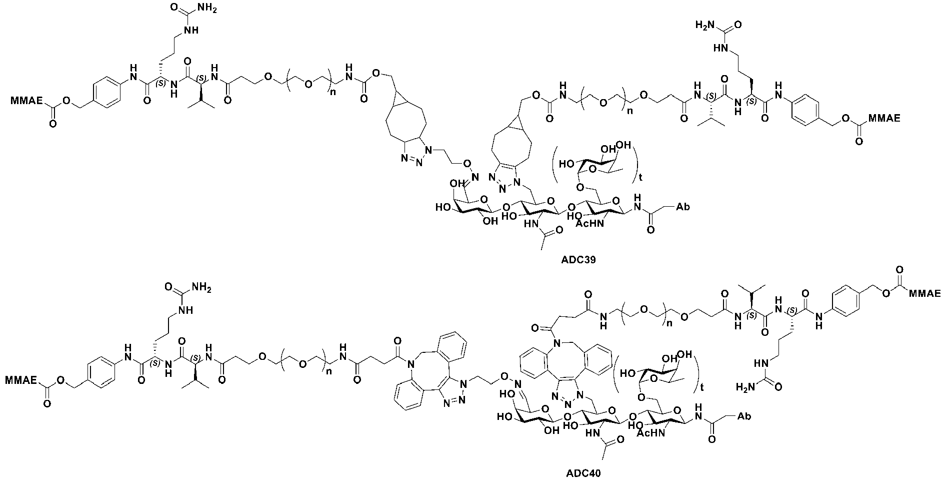 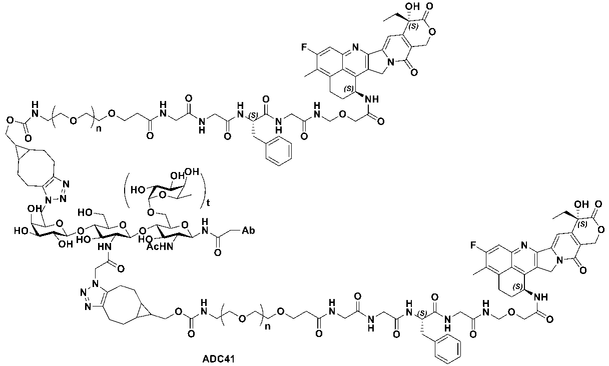 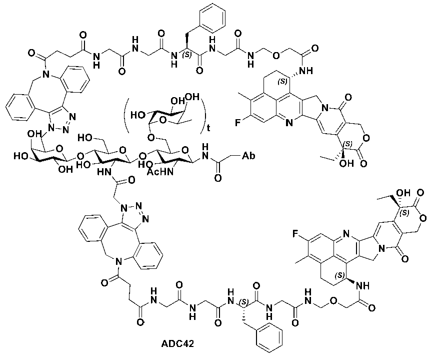 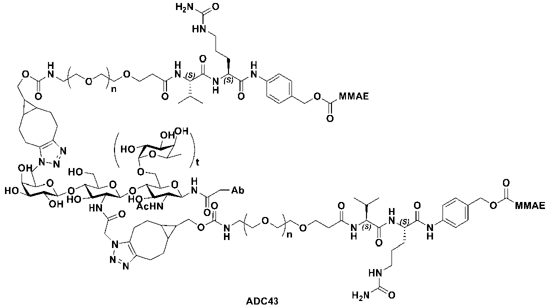 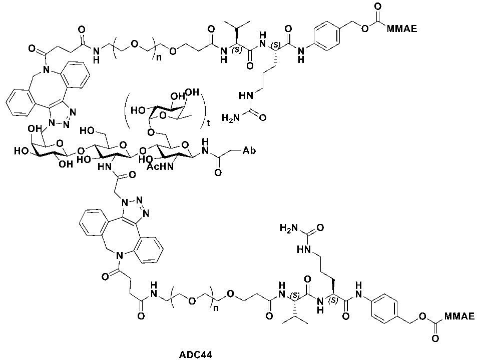 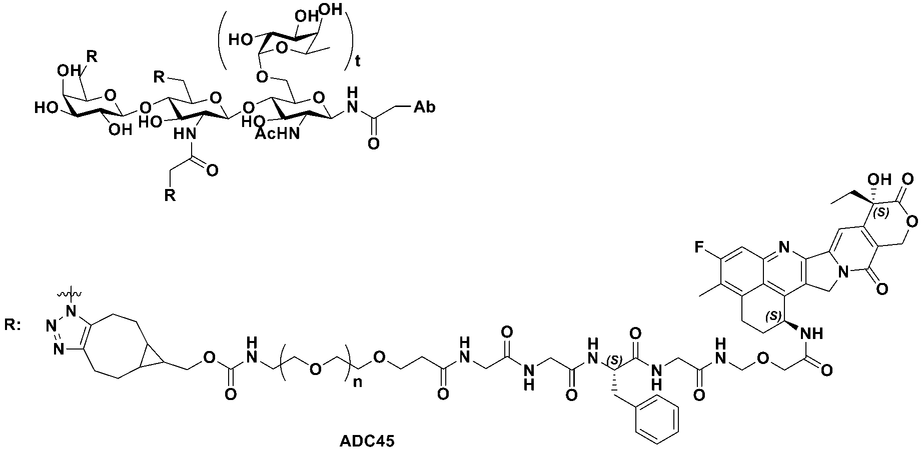 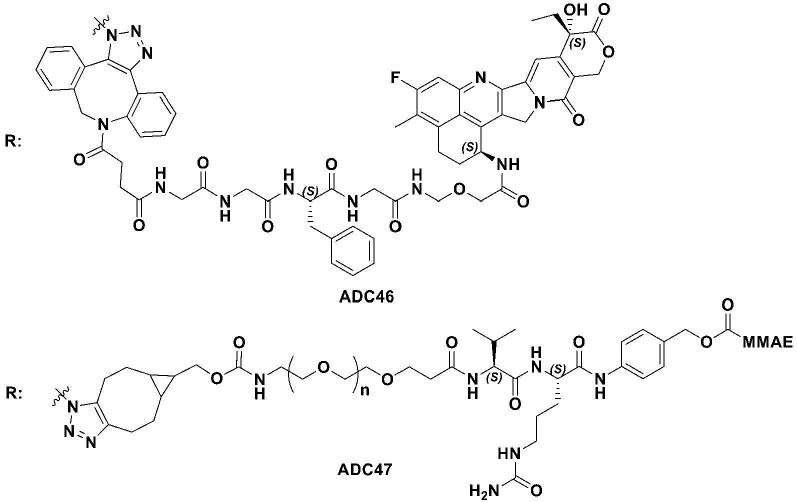 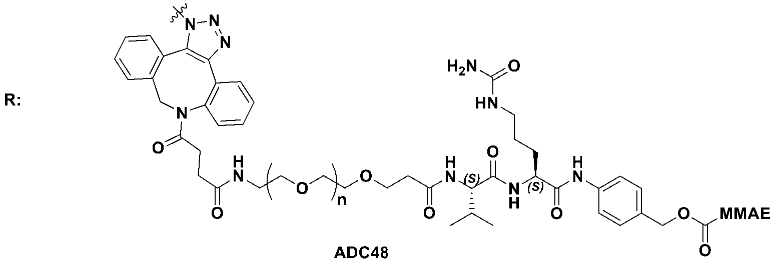 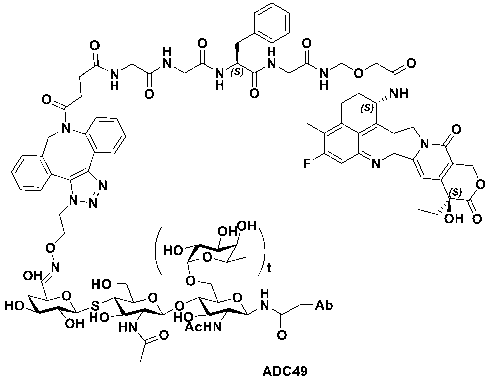 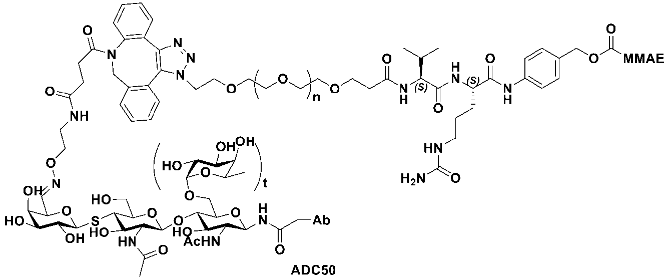 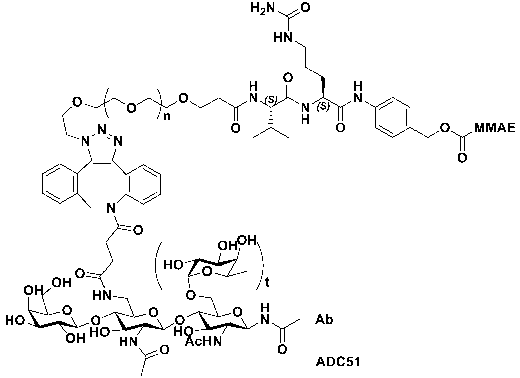 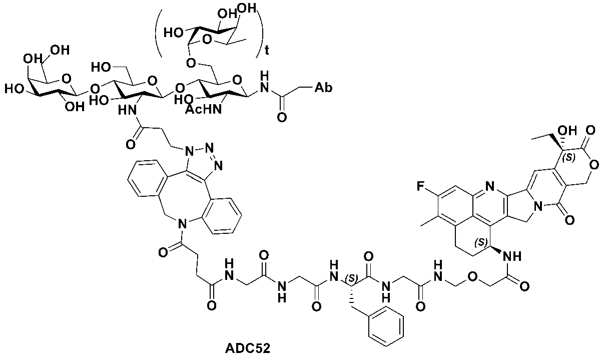 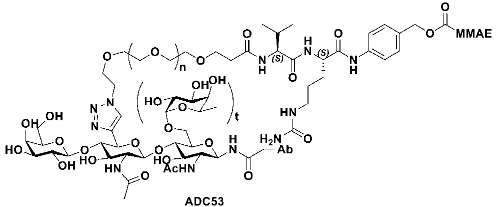 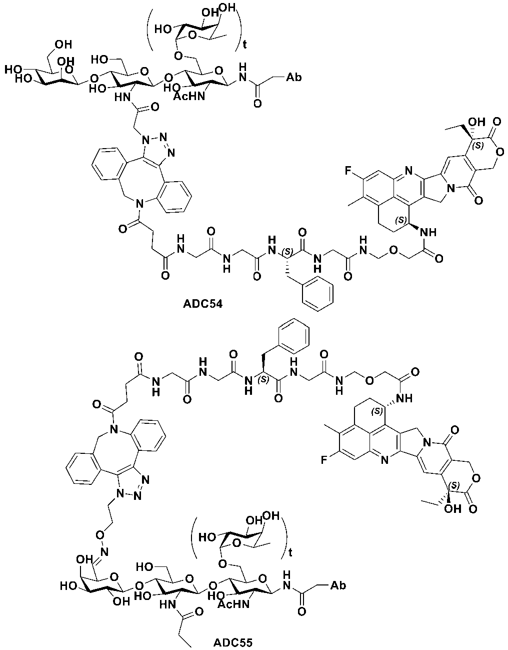 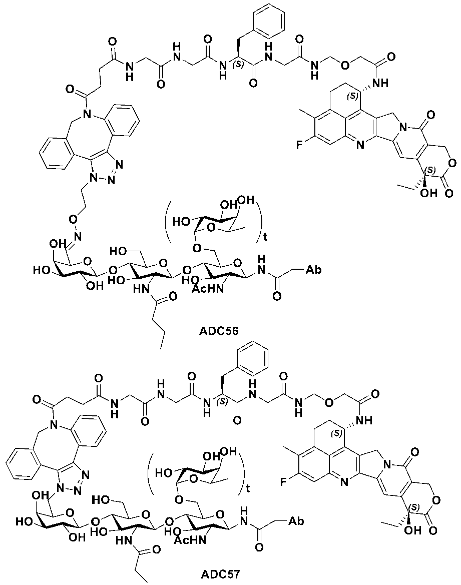 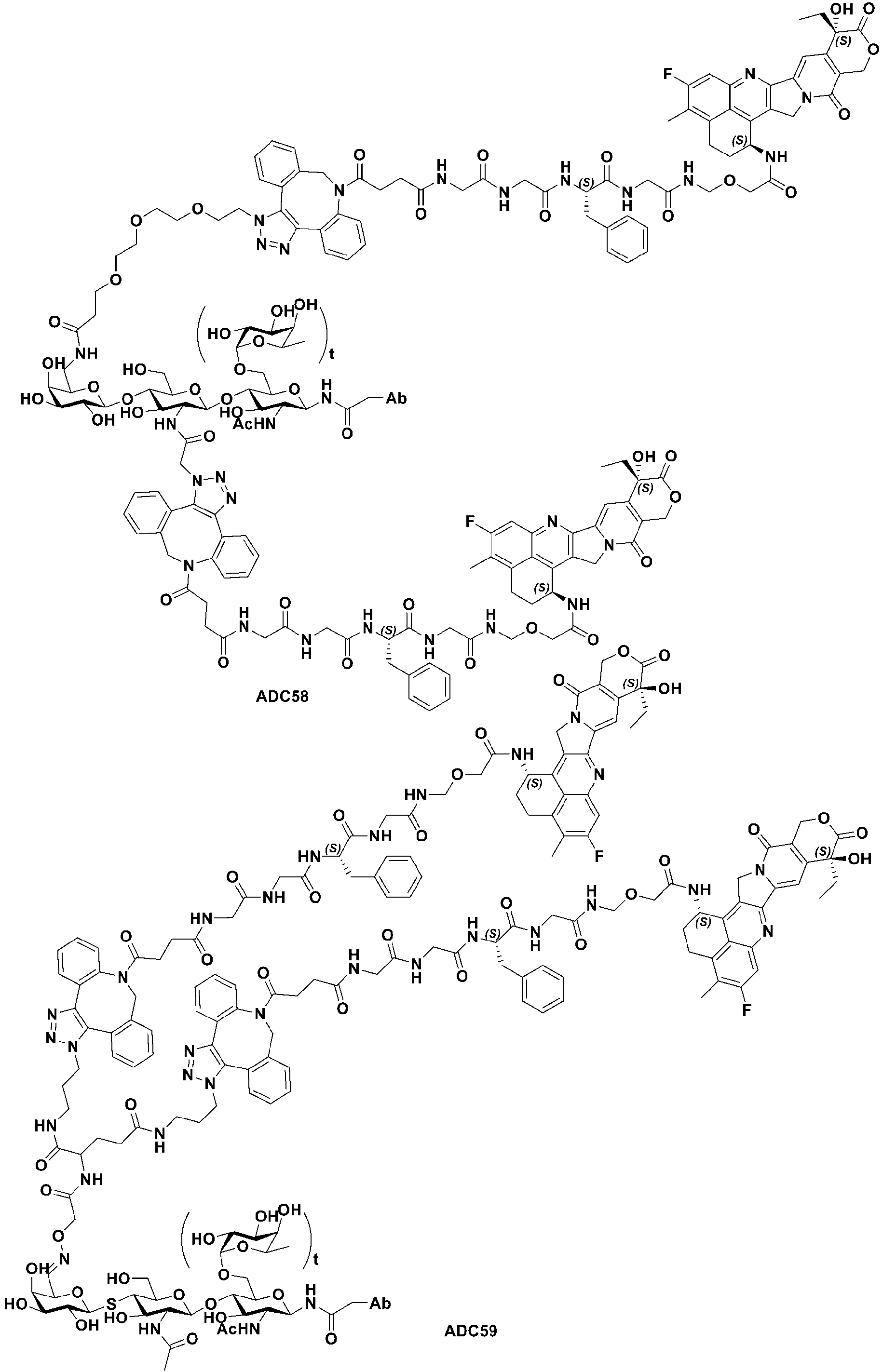 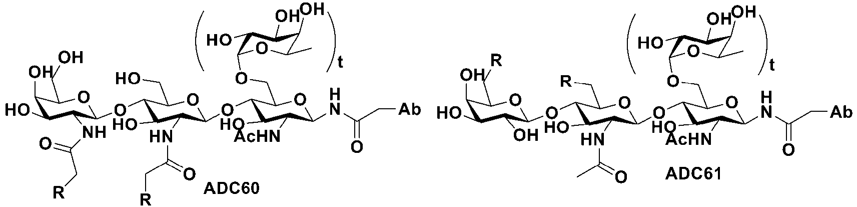 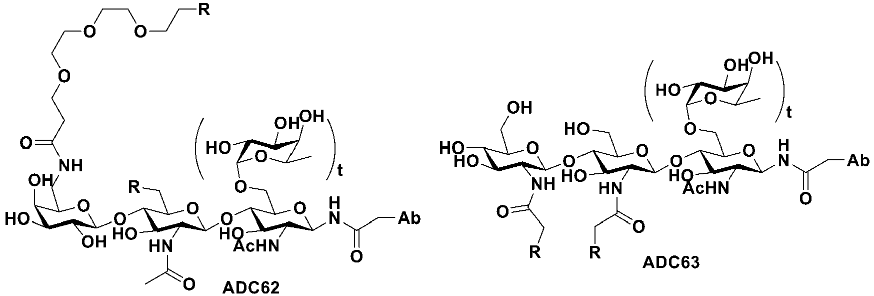 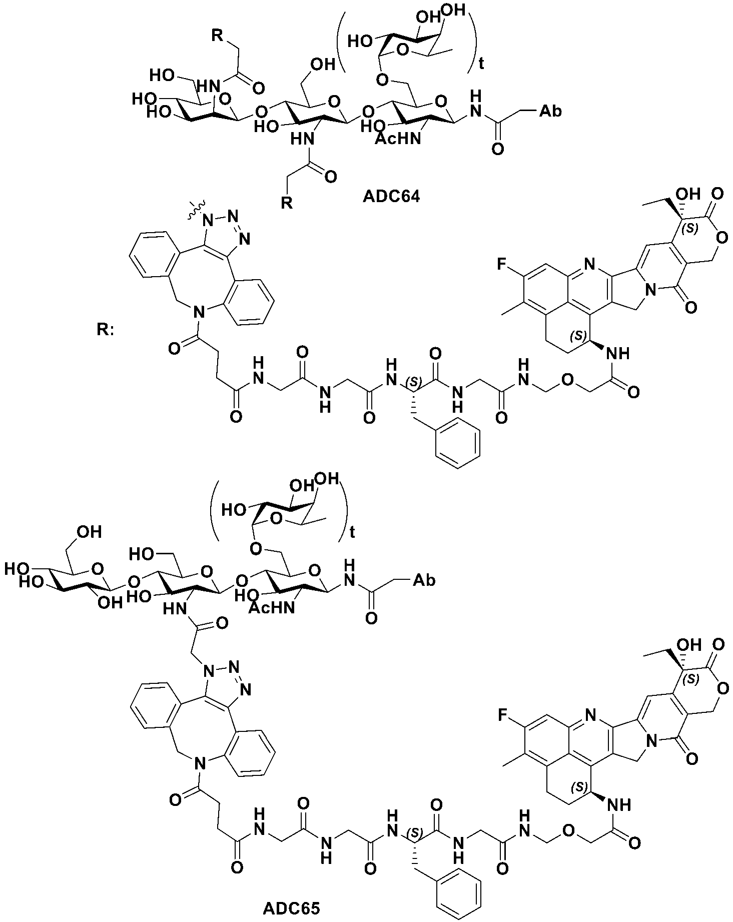 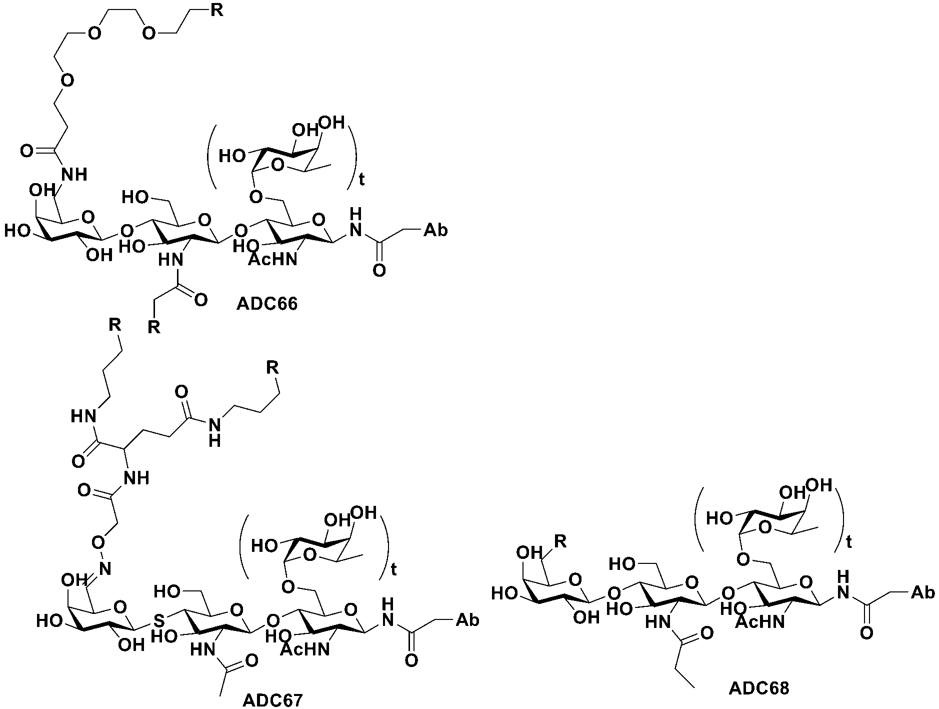 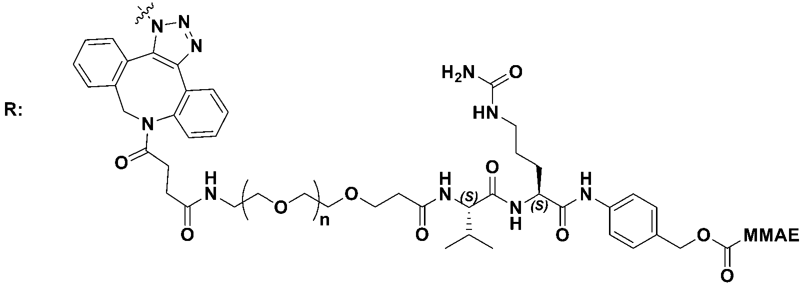 wherein n is an integer selected from the group consisting of 0-30, or an integer from 0-20, or an integer from 0-10; t is 0 or 1.

In some embodiments of the present disclosure, when the antibody-based conjugate drug represented by formula (III) has a dual payload, the antibody-based conjugate drug represented by formula (III) is selected from the group consisting of the following specific compounds: 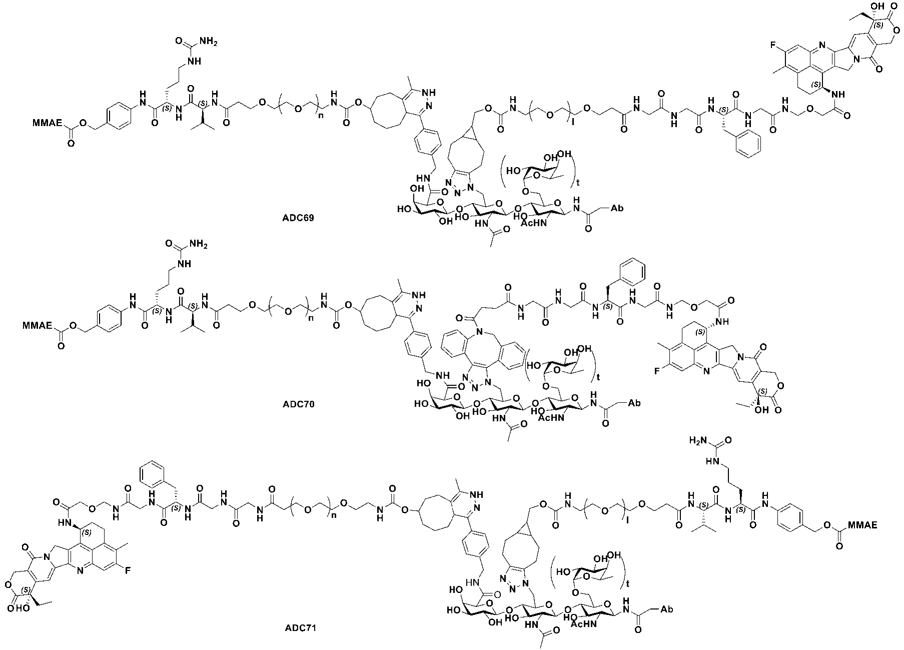 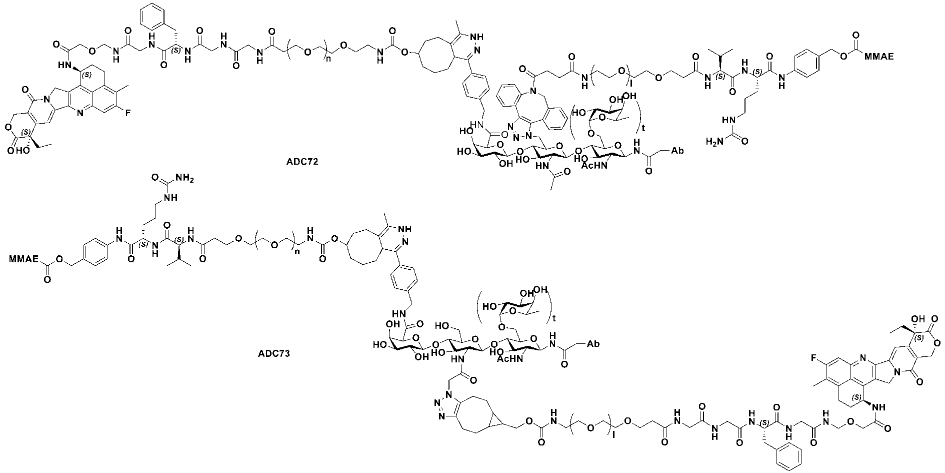 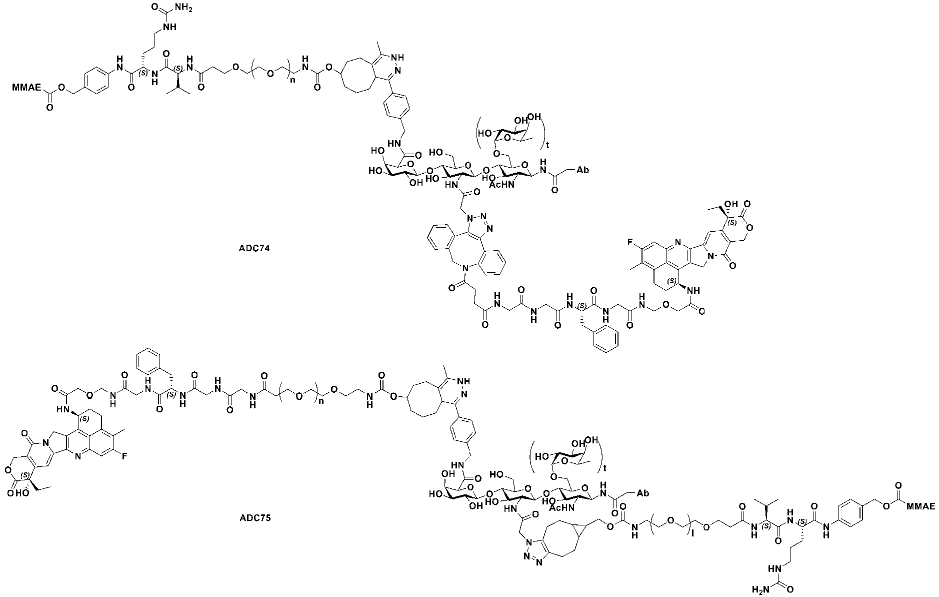 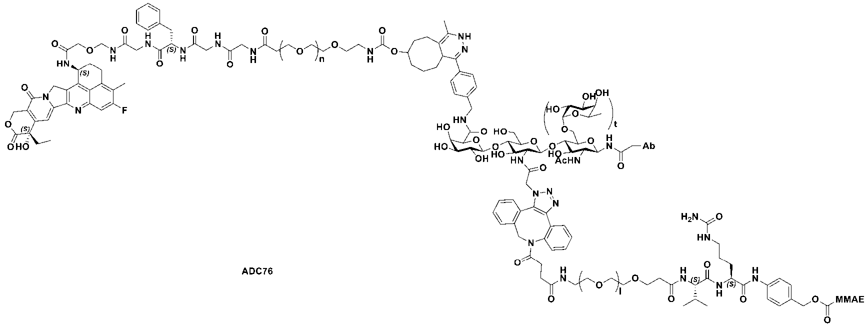 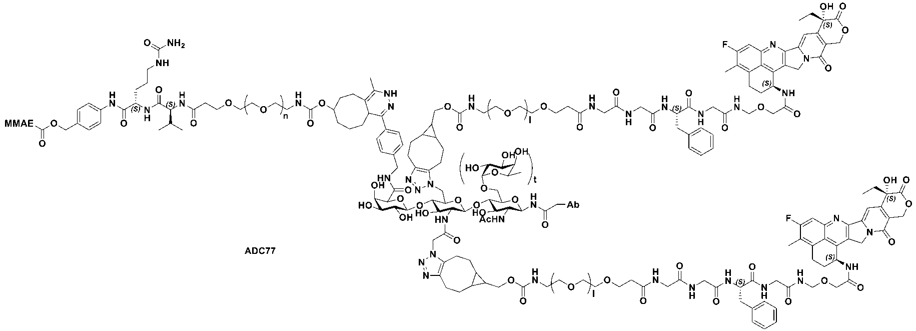 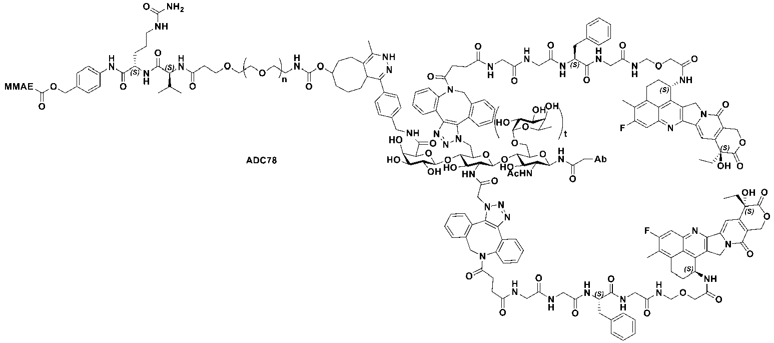 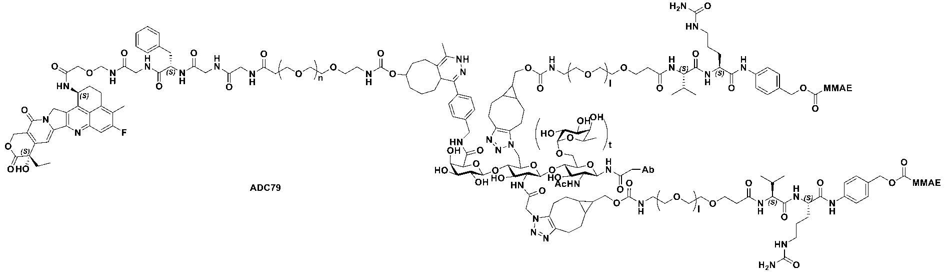 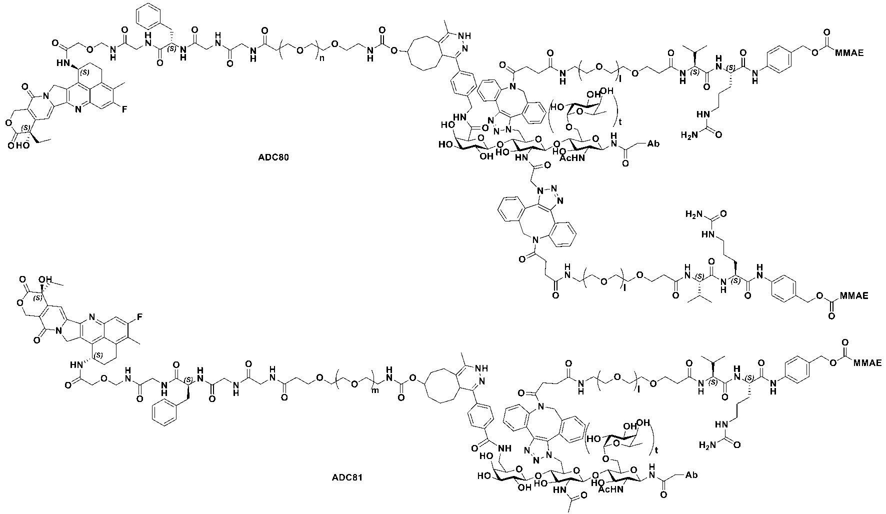 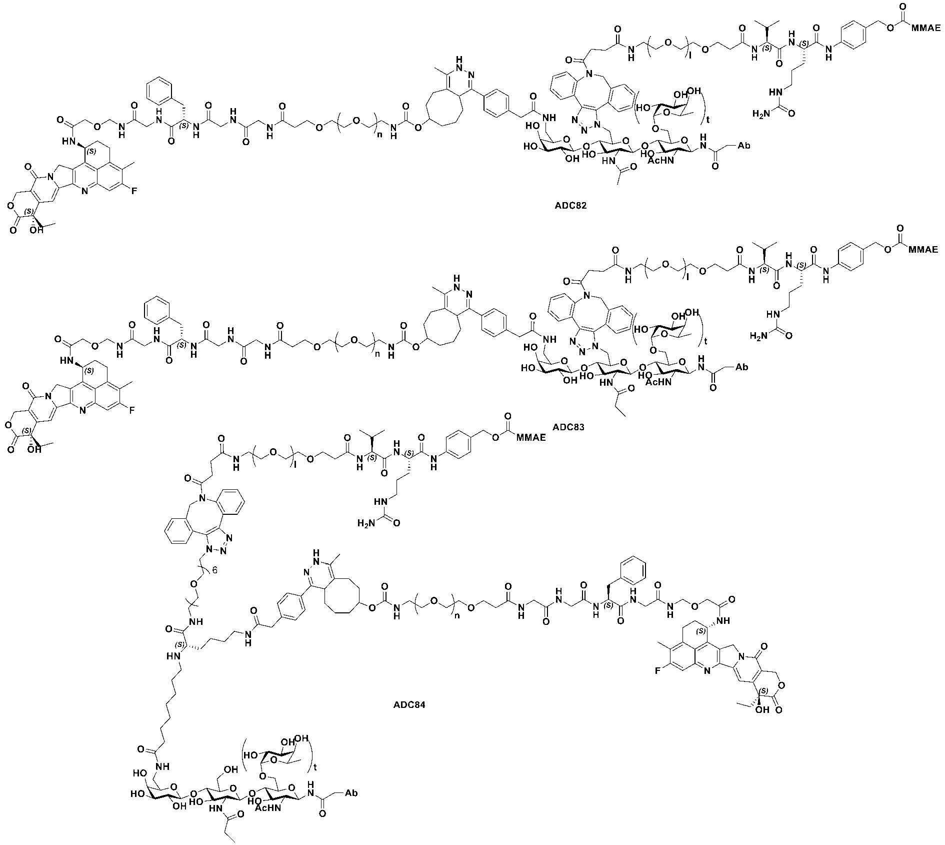 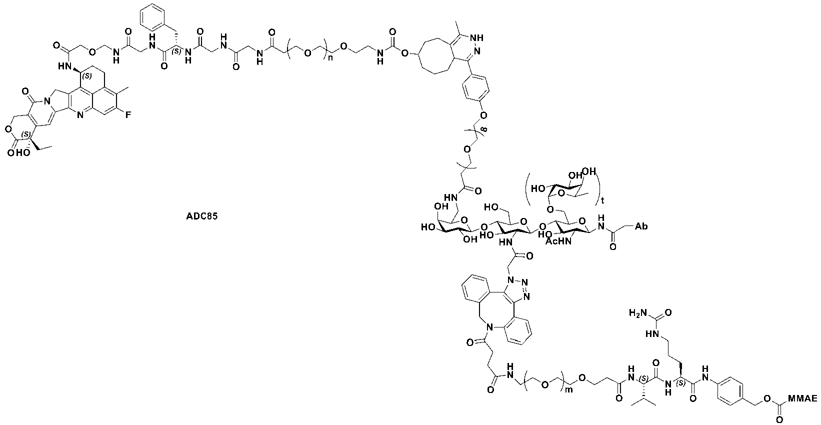 wherein m, n, and I are each independently an integer selected from the group consisting of 0-30, or an integer from 0-20, or an integer from 0-10; t is 0 or 1.

In some embodiments of the present disclosure, each n, each m, each l, and each q is independently an integer selected from the group consisting of 0-30, for example, selected from the group consisting of 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, and 30.

In some embodiments of the present disclosure, each C₁-C₆ alkyl is, for example, C₁ alkyl, C₂ alkyl, C₃ alkyl, C₄ alkyl, C₅ alkyl, and C₆ alkyl; each C₆-C₁₀ aryl is, for example, phenyl and naphthyl; each C₅-C₁₀ heteroaryl is, for example, a 6-, 7-, 8-, 9-, 10-, 11-, 12-, 13-, and 14-membered heteroaryl, containing 1 or more *(e.g.,* 2, 3, 4, and 5) heteroatoms selected from the group consisting of N, O, and S.

In a fourth aspect, the present disclosure provides a method for preparing an antibody-based conjugate drug, the method comprising the following three methods:

### Method 1:

a) under the action of an endoglycosidase having beta-N-acetylglucosaminidase hydrolytic activity, or an endoglycosidase and a fucosidase, hydrolyzing a wild-type antibody to form a deglycosylated antibody containing one N-acetylglucosamine or core-fucosylated *N-*acetylglucosamine;
b) conjugating the deglycosylated antibody obtained in step a) with the disaccharide linker described above under the catalysis of an endoglycosidase having transglycosylation activity or a mutant thereof, to obtain a glycoengineered antibody;
c) conjugating the glycoengineered antibody containing an orthogonally reactive group obtained in step b) with the drug linker E-L-P, which is modified with a corresponding group capable of undergoing a specific conjugation reaction with the orthogonally reactive group, to prepare the antibody-based conjugate drug;

### Method 2:

a) under the catalysis of an endoglycosidase having transglycosylation activity or a mutant thereof, conjugating a wild-type antibody with the disaccharide linker described above to obtain a glycoengineered antibody;
b) conjugating the glycoengineered antibody containing an orthogonally reactive group obtained in step a) with the drug linker E-L-P, which is modified with a corresponding group capable of undergoing a specific conjugation reaction with the orthogonally reactive group, to prepare the antibody-based conjugate drug;

### Method 3:

under the catalysis of an endoglycosidase having transglycosylation activity or a mutant thereof, co-incubating a wild-type antibody and the disaccharide-small-molecule drug conjugate described above, or co-incubating a deglycosylated antibody and the disaccharide-small-molecule drug conjugate, to prepare the antibody-based conjugate drug.

In some embodiments of the present disclosure, in the Method 1 and Method 2, the orthogonally reactive group and the corresponding group capable of undergoing a specific conjugation reaction with the orthogonally reactive group are selected from the group consisting of any one of the following combinations: azido and alkynyl, tetrazinyl and trans-cyclooctene (TCO), thiol and maleimide, thiol and thiol or an activated form of thiol, aldehyde and amino, and aldehyde and aminooxy or hydrazino.

In some embodiments of the present disclosure, the endoglycosidase having beta-N-acetylglucosaminidase hydrolytic activity is selected from the group consisting of Endo Se2, Endo Si, Endo S, Endo S2, Endo F3, Endo A, Endo D, Endo CC, and mutants thereof.

In some embodiments of the present disclosure, the endoglycosidase having transglycosylation activity is selected from the group consisting of Endo Se2, Endo Si, Endo S, Endo S2, Endo F3, and mutants thereof.

In some embodiments of the present disclosure, when preparing a deglycosylated antibody lacking core fucosylation, a glycoengineered antibody, or an antibody-based conjugate drug, the endoglycosidase having beta-N-acetylglucosaminidase hydrolytic activity is used in combination with a fucosidase.

In a fifth aspect, the present disclosure provides a use of the disaccharide linker provided in the first aspect of the present disclosure or the disaccharide conjugate provided in the second aspect of the present disclosure in the manufacture of the antibody-based conjugate drug of the present disclosure.

In some embodiments of the present disclosure, the antibody-based conjugate drug is the antibody-based conjugate drug provided in the third aspect of the present disclosure.

In a sixth aspect, the present disclosure provides a use of the antibody-based conjugate drug provided in the third aspect of the present disclosure in the manufacture of a medicament for treating and/or preventing tumors, inflammation, viruses, infectious diseases, or other immune diseases.

In a seventh aspect, the present disclosure provides a pharmaceutical composition comprising a therapeutically effective amount of the antibody-based conjugate drug provided in the third aspect of the present disclosure.

In some embodiments of the present disclosure, the pharmaceutical composition is used for treating and/or preventing diseases, including tumors, inflammation, viruses, infectious diseases, or other immune diseases.

In an eighth aspect, the present disclosure provides a method of treating and/or preventing a disease, the method comprising administering to a patient a therapeutically and/or prophylactically effective amount of the antibody-based conjugate drug provided in the third aspect of the present disclosure.

In some embodiments of the present disclosure, the disease includes tumors, inflammation, viruses, infectious diseases, or other immune diseases.

The present disclosure prepares homogenized sugar chain-modified antibodies through glycoengineering methods. The present disclosure relates to the Fc region of an antibody molecule, wherein the Fc region is modified with a specific sugar chain, and the sugar chain is capable of enhancing the function of the Fc region. In some embodiments, the Fc region modified with the specific sugar chain comprises a monoclonal antibody, preferably a humanized monoclonal antibody. Disclosed herein are methods for producing such antibodies or antibody fragments comprising specific sugar chain modifications through glycoengineering methods.

Unless specifically defined, all academic terms used in the present disclosure have the same meanings as commonly understood by those skilled in the art to which the present disclosure pertains. All publications and patent documents mentioned in the present disclosure may be regarded as guidance for those of ordinary skill in the art.

In the present disclosure, the term "glycan" refers to a polysaccharide or an oxidized or non-oxidized carbohydrate-containing molecule, including but not limited to a monosaccharide, disaccharide, trisaccharide, oligosaccharide, or polysaccharide. "Glycan" as used herein also refers to the carbohydrate moiety of a saccharide-containing conjugate, such as a glycoprotein, glycolipid, glycopeptide, glycoproteome, peptidoglycan, lipopolysaccharide, or proteoglycan. The term "sugar chain" may be used interchangeably with "glycan" herein.

As used in the present disclosure, a "sugar chain linker," as an activated donor molecule for glycosyl conjugation, may be a synthetic saccharide containing oxazoline or thiazoline, for example, an oligosaccharide with an activated reducing end, preferably an oligosaccharide molecule structured with oxazoline; or may be a natural N-glycan oxazoline. The sugar chain linker may also be chemically modified, for example, by introducing functional groups through azidation, alkynylation, aldehydation, thiolation, and the like. The term "disaccharide linker" refers to a sugar chain linker containing at least two monosaccharide units, preferably two monosaccharide units linked via a glycosidic bond or a thioether bond.

In the present disclosure, the term "antibody" includes monoclonal antibodies, polyclonal antibodies, multispecific antibodies (e.g., bispecific antibodies), and antibody fragments, as long as they exhibit the desired biological activity. The term "immunoglobulin" may be used interchangeably with "antibody" herein. The term "monoclonal antibody" as used herein refers to an antibody obtained from a substantially homogeneous population of antibodies.

Core-fucosylated and non-fucosylated glycoproteins are important classes of molecules, which play key roles in many biological events such as tumor metastasis, cell adhesion, pathogen infection, and immune responses. Natural and recombinant fucosylated and non-fucosylated glycoproteins are typically produced as mixtures of glycoforms that differ only in the structure of the side-chain oligosaccharides.

The term "antibody-based conjugate drug" in the present disclosure broadly refers to all conjugates formed by covalently linking a polypeptide/protein targeting specific cells to a payload. The polypeptide/protein targeting specific cells may be an antibody or an antigen-binding fragment thereof, such as a monoclonal antibody, a bispecific antibody, or a multispecific antibody; the payload may be a cytotoxin, a small-molecule drug, a near-infrared or fluorescent probe, a peptide, RNA and related drugs, a radioisotope label, a contrast agent, or a magnetic resonance imaging agent; the resulting covalent conjugate may be used for treatment or detection. The term "antibody-drug conjugate" in the present disclosure refers to all conjugates formed by covalently linking a polypeptide/protein targeting specific cells to a cytotoxin.

The term "endoglycosidase" in the present disclosure refers to a glycoside hydrolase having beta-N-acetylglucosaminidase hydrolytic activity, and such enzymes typically belong to glycoside hydrolase family 18 or 85. Some endoglycosidases known in the art include, for example, Endo S, Endo S2, Endo F3, Endo A, Endo D, Endo CC, and mutants thereof, such as those described in WO2022/226420. Such enzymes may also simultaneously have transglycosylation activity. Enzymes having such activity known in the art include, for example, Endo S, Endo S2, Endo F3, and mutants thereof, such as those described in WO2022/226420.

The term "wild-type antibody" used in the present disclosure typically refers to a naturally occurring antibody or an antibody produced by recombinant expression, having an N-glycosylation site (N-glycan). For example, all antibodies whose Fc region has an N297 site fall within the scope of "wild-type antibody," wherein the Fc region is derived from IgG1, IgG2, IgG3, or IgG4. The term "deglycosylated antibody" in the present disclosure refers to an antibody containing one N-acetylglucosamine or core-fucosylated N-acetylglucosamine, formed from a wild-type antibody under the action of a glycoside hydrolase.

The term "drug linker E-L-P" used in the present disclosure refers to a covalent conjugate comprising an orthogonal reactive group, a linker, and a payload. In a typical E-L-P structure, E comprises any one of the following structures:

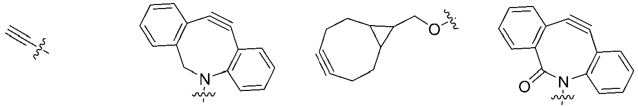

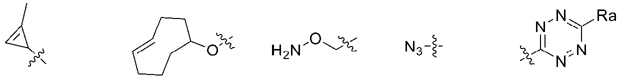

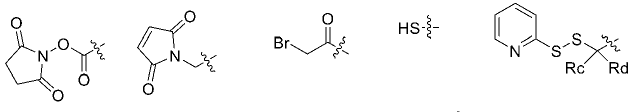

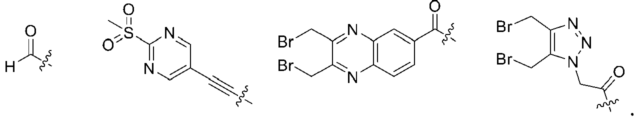

The preparation routes of the glycoengineered antibody and the sugar-chain site-specific ADC of the present disclosure are schematically illustrated below by reaction schemes.

General method for glycoengineering modification of antibodies:

### General procedure I: Method for preparing a glycoengineered antibody

A wild-type antibody, the prepared disaccharide linker, and an endoglycosidase (Endo Si or Endo Se2) are used such that the concentrations of the respective components are sequentially 10 mg/mL, 2 mM (30 equivalents relative to the antibody concentration), and 0.4 mg/mL. The pH of the reaction system is adjusted to 7.4, followed by incubation at 25°C for 12 h. After purification with protein A, the desired non-natural glycoengineered antibody is obtained.

### General procedure II: Method for preparing a site-specific ADC based on an azide-modified glycoengineered antibody

The prepared azide-modified glycoengineered antibody and a cycloalkyne linker-payload are used such that the concentrations of the respective components are sequentially 5 mg/mL and 0.33 mM. The pH of the reaction system is adjusted to 7.4, followed by incubation overnight at 25°C. After conversion to the product is confirmed by LC-MS, the desired glycosite-specific conjugated ADC is obtained by protein A purification or ultrafiltration.

### General procedure III: Method for preparing a site-specific ADC based on a cycloalkyne-modified glycoengineered antibody

The prepared cycloalkyne-modified glycoengineered antibody and an azide linker-payload are used such that the concentrations of the respective components are sequentially 5 mg/mL and 0.33 mM. The pH of the reaction system is adjusted to 7.4, followed by incubation overnight at 25°C. After conversion to the product is confirmed by LC-MS, the desired glycosite-specific conjugated ADC is obtained by protein A purification or ultrafiltration.

### General procedure IV: Method for preparing a site-specific ADC based on a tetrazine-modified glycoengineered antibody

The prepared tetrazine-modified glycoengineered antibody and a trans-cyclooctene (TCO) linker-payload are used such that the concentrations of the respective components are sequentially 5 mg/mL and 0.33 mM. The pH of the reaction system is adjusted to 7.4, followed by incubation overnight at 25°C. After conversion to the product is confirmed by LC-MS, the desired glycosite-specific conjugated ADC is obtained by protein A purification or ultrafiltration.

### General procedure V: Method for preparing a glycosite-specific conjugated ADC by a one-step method

The prepared payload-bearing disaccharide linker, wild-type antibody, and an endoglycosidase (Endo Si or Endo Se2) are used such that the concentrations of the respective components are sequentially 0.4 mM, 5 mg/mL, and 0.4 mg/mL. The pH of the reaction system is adjusted to 7.4, followed by incubation overnight at 25°C. After conversion to the product is confirmed by LC-MS, the desired glycosite-specific conjugated ADC is obtained by protein A purification or ultrafiltration.

### General procedure VI: Method for preparing a dual-payload site-specific ADC based on an azide- and tetrazine-modified glycoengineered antibody

The prepared azide- and tetrazine-modified glycoengineered antibody, a cycloalkyne linker-payload, and a trans-cyclooctene (TCO) linker-payload are used such that the concentrations of the respective components are sequentially 5 mg/mL, 0.33 mM, and 0.33 mM. The pH of the reaction system is adjusted to 7.4, followed by incubation overnight at 25°C. After conversion to the product is confirmed by LC-MS, the desired glycosite-specific conjugated ADC is obtained by protein A purification or ultrafiltration.

### General procedure VII: Method for preparing a site-specific ADC based on a linear alkynyl-modified glycoengineered antibody

Copper sulfate, tris(3-hydroxypropyltriazolylmethyl)amine (THPTA), and sodium ascorbate are pre-mixed such that the concentrations of the respective components are respectively 20 mM, 100 mM, and 150 mM, and set aside as a catalyst for later use. The prepared linear alkynyl-modified glycoengineered antibody, an azide linker-payload, and the catalyst are mixed such that the concentrations of the respective components are sequentially 5 mg/mL, 0.5 mM, 0.67 mM (copper sulfate), 3.33 mM (THPTA), and 5 mM (sodium ascorbate). The pH of the reaction system is adjusted to 7.4, followed by incubation overnight at 25°C. After conversion to the product is confirmed by LC-MS, the desired glycosite-specific conjugated ADC is obtained by protein A purification or ultrafiltration.

### Technical effects

The present disclosure achieves at least one of the following beneficial technical effects:
Based on glycosite-specific structural modification, multiple novel modification sites available for disaccharide-linkers have been enriched and developed, providing various novel disaccharide-linker structures.

Based on the development of endoglycosidases, the present disclosure discovers and tests two wild-type endoglycosidases with broader substrate specificity and better transfer efficiency.

The present disclosure achieves site-specific introduction of orthogonal reactive groups onto antibodies through enzyme-catalyzed reactions, thereby enabling site-specific conjugation with small-molecule drugs, with simple operation and suitability for industrial production. Moreover, the site-specifically conjugated antibody-drug conjugates exhibits favorable druggability and can be used for treating tumors, inflammation, viruses, infectious diseases, or other immune diseases.

### BRIEF DESCRIPTION OF THE DRAWINGS

FIG. 1. Detection of transglycosylation activity of different endoglycosidases on compound G0;
FIG. 2. Detection of transglycosylation activity of different endoglycosidases on compound G1;
FIG. 3. Detection of transglycosylation activity of different endoglycosidases on compound G2;
FIG. 4. Efficacy of a low-dose glycosite-specific conjugated ADC in an NCI-N87 xenograft tumor model;
FIG. 5. Efficacy of a high-dose glycosite-specific conjugated ADC in an NCI-N87 xenograft tumor model.

### EXAMPLES

The specific processes for preparing disaccharide linkers, glycoengineered antibodies, and glycosite-specific conjugated ADCs by the preparation methods of the present disclosure are described below by way of specific examples.

In the present disclosure, the synthesis of the sugar chain linker was commissioned to Wuhan Glycogene Pharmaceutical Co., Ltd. DBCO (dibenzocyclooctyne) compounds and tetrazine compounds were purchased from Shanghai Bide Pharmatech Co., Ltd.; D-glucosamine hydrochloride and azido acid compounds were purchased from Energy Chemical Technology Co., Ltd.; acetonitrile was purchased from Wuhan Shenshi Co., Ltd.; galactose oxidase, catalase, and horseradish peroxidase were purchased from Shanghai Yingxin Laboratory Co., Ltd. Other compounds and reagents, unless otherwise specified, were purchased from Sinopharm Chemical Reagent Co., Ltd. The instruments and chromatographic columns used include: LC-MS Agilent 1290+6135B, analytical high-performance liquid chromatograph (Thermo Ultimate 3000), Tongwei ELSD-UM500, preparative high-performance liquid chromatograph (Wuhan Ruihe Chromatography Technology, LC 2100); Agilent SB C18 (5 µm, 4.6×150 mm), Waters XBridge BEH Amide (250 mm×4.6 mm, 5.0 µm), Welch HILIC Amide (10 µm, 21.2×250 mm).

The antibody used in the examples of the present disclosure is a wild-type antibody comprising an Fc segment, wherein the sequence of the Fc segment is, for example, as set forth in SEQ ID NO:5, typically such as Pertuzumab. In the present disclosure, Trastuzumab was purchased from Sanyou Biopharmaceuticals (Shanghai) Co., Ltd. In the present disclosure, the fillers, chromatographic columns, and instruments used for antibody purification, sugar chain -modified antibody purification, ADC purification, and property determination include: 5 ml cOmplete^{™} His-Tag Purification Column (Roche), HiLoad^{™} 26/600 Superdex^{™} 200 prep grade chromatographic column (Cytive), AmMag^{™} Protein A Magnetic Beads (GenScript), SDA030 protein purification system (Sepure), Acquity I-Class/RDa (Waters) liquid chromatography-mass spectrometry system, Arc Premier high-performance liquid chromatograph, ACCQUITY UPLC BEH PROTEIN C4 (Waters, 1.7 µm, 2.1 mm×50 mm) chromatographic column, TSKgel G3000SWXL (7.8 mm×30 cm, 5 µm) SEC chromatographic column, and TSKgel Butyl-NPR (4.6 mm×10 cm, 2.5 µm) HIC chromatographic column.

In the present disclosure, the drug linkers DBCO-GGFG-Dxd and TCO-PEG₄-GGFG-Dxd were purchased from Shanghai Tekanbio Pharm-Tech Co., Ltd.; the drug linkers DBCO-PEG₄-GGFG-Dxd, DBCO-PEG₄-VC-PAB-MMAE, and N₃-PEG₄-VC-PAB-MMAE were purchased from MedChemExpress LLC.

### 1. Preparation of disaccharide linkers

### Example 1: Synthesis of compound G0

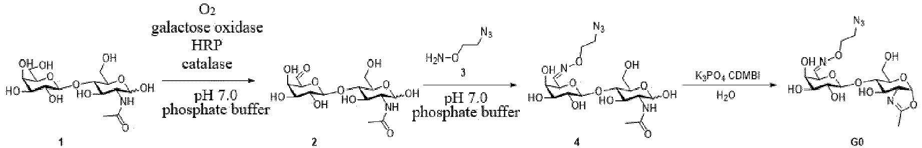

**Step 1:** Method for preparing 50 mM pH 7.0 phosphate buffer: 0.68 g of potassium dihydrogen phosphate was dissolved in 100 mL of deionized water, and then the pH of the phosphate buffer was adjusted to 7.0±0.2 with 1 N sodium hydroxide solution. At room temperature, 40 mL of the 50 mM pH 7.0 phosphate buffer and compound 1 (800 mg, 2.09 mmol) were added to a 100 mL single-neck reaction flask, respectively. The mixture was stirred, and oxygen (O₂) was continuously introduced using a syringe needle for about 0.5-1 hour. Subsequently, 1 kU of galactose oxidase, 10 kU of horseradish peroxidase (HRP), and 200 kU of catalase were added sequentially. The reaction was carried out at room temperature for 6 hours. Upon completion of the reaction, the introduction of oxygen was stopped. The prepared compound **2** was used directly in the next step without purification.

**Step 2:** 250 µL of compound **3** (O-(2-azidoethyl)hydroxylamine, CAS: 1397706-70-5, 250 µL, 2.45 mmol) was added to the reaction mixture obtained from the previous step, and the reaction was carried out at 15-25°C overnight. The reaction progress was monitored by thin-layer chromatography (TLC) (isopropanol:concentrated aqueous ammonia:deionized water = 10:3:2). Upon completion of the reaction, most of the water (>95%) was removed by rotary evaporation. Then, 40 mL of ethanol was added, and insoluble materials were removed by filtration. The filtrate was subjected to rotary evaporation until dryness, and the residue was purified by silica gel column chromatography (dichloromethane:methanol = 10:2 to 10:3, containing 1% concentrated aqueous ammonia). The collected fractions were concentrated and then lyophilized to obtain compound **4** (350 mg, yield over two steps: 36%).

ESI-MS calc. for C₁₆H₂₇N₅O₁₁ [M+H]⁺ m/z= 466.2, found m/z= 466.2. ¹HNMR (400 MHz, deuterium oxide) *δ* 7.47 (d, *J =* 4.1 Hz, 0.7H), 6.81 (d, *J =* 4.6Hz, 0.3H), 5.16 (q, *J= 1.5* Hz, 0.7H), 4.80 (ddd, *J* = 4.7, 3.5, 1.2 Hz, 0.3H), 4.67-4.65 (m, 0.3H), 4.49 (d, *J* = 7.9 Hz, 0.65H), 4.46 (dd, *J* = 7.9, 1.6 Hz, 0.35H), 4.42 (ddd, *J =* 4.1, 2.8, 1.2 Hz, 0.7H), 4.29-4.19 (m, 2H), 4.04 (dt, *J =* 2.8*,* 1.3 Hz, 1H), 3.97-3.89 (m, 1H), 3.88-3.78 (m, 3H), 3.73-3.60 (m, 3H), 3.54 (m, 3H), 2.00 (d, *J* = 1.8 Hz, 3H).

**Step 3:** To a 50 mL centrifuge tube were added 5 mL of deionized water, compound **4** (100 mg, 0.21 mmol), and potassium phosphate (684 mg, 3.22 mmol), respectively. The mixture was cooled to 0°C in an ice-water bath, stirred, and then 2-chloro-1,3-dimethyl-1H-benzoimidazol-3-ium chloride (CDMBI, 232 mg, 1.07 mmol) was slowly added. The reaction was carried out at 0°C for half an hour, then warmed to room temperature and stirred overnight. The reaction endpoint was monitored by TLC (dichloromethane:methanol = 10:2, containing 1% concentrated aqueous ammonia). Upon completion of the reaction, the reaction mixture was purified by C18 column chromatography (deionized water/methanol = 20:1-10:1). The collected fractions were concentrated and then lyophilized to obtain compound **G0** (63 mg, yield: 66%).

ESI-MS calc. for C₁₆H₂₆N₅O₁₀ [M+H]⁺ m/z= 448.2, found m/z= 448.2. ¹HNMR (400MHz, deuterium oxide) *δ* 7.49 (d, *J =* 4.8 Hz, 0.7H), 6.81 (d, *J =* 4.6 Hz, 0.3H), 6.09 (dd, *J* = 7.3*,* 2.3 Hz, 1H), 4.43 (d, *J* = 7.8 Hz, 0.7H), 4.38 (d, *J* = 7.9 Hz, 0.3H), 4.34 (m, 0.3H), 4.33-4.3 (m, 1H), 4.25-4.15 (m, 3H), 4.11 (m, 1H), 3.98 (dd, *J =* 3.4, 1.1 Hz, 0.7H), 3.74 (m, 1H), 3.67-3.57 (m, 3H), 3.53-3.44 (m, 3H), 3.40-3.35 (m, 1H), 1.99 (t, *J=* 1.9 Hz, 3H).

### Example 2: Synthesis of compound G1

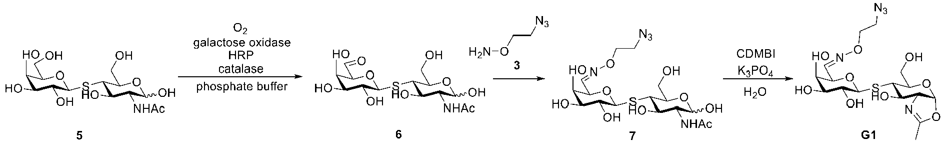

**Step 1:** To a 100 mL single-neck reaction flask were added 5 mL of 50 mM pH 7.0 phosphate buffer and compound **5** (230 mg, 0.58 mmol), respectively. The mixture was stirred in a 30°C water bath while oxygen (O₂) was continuously introduced using a syringe needle for about 0.5 hours. To another 5 mL centrifuge tube were sequentially added 1 mL of 50 mM pH 7.0 phosphate buffer, 280 U of galactose oxidase, 10 kU of horseradish peroxidase (HRP), 200 kU of catalase, and 0.16 mL of 1 mM aqueous copper sulfate solution, and the mixture was activated in a 30°C water bath for 0.5 hours. Subsequently, the activated mixed enzyme was added to the 100 mL single-neck reaction flask, and the reaction was carried out in a 30°C water bath for 6 hours. Upon completion of the reaction, the introduction of oxygen was stopped, and the prepared compound **6** was used directly in the next step without purification.

**Step 2:** Compound 3 (130 µL, 1.27 mmol) was added to the reaction mixture from the previous step, and the reaction was carried out at 25°C overnight. The reaction progress was monitored by TLC (isopropanol:concentrated aqueous ammonia:deionized water = 10:3:2). Upon completion of the reaction, most of the water (>90%) was removed by rotary evaporation. Then, 20 mL of methanol was added, and the insoluble materials were removed by filtration. The precipitate was washed twice with 10 mL of methanol each time. The filtrate was subjected to rotary evaporation until dryness, and the residue was purified by silica gel column chromatography (dichloromethane:methanol = 10:1 to 5:1). The collected fractions were concentrated to obtain compound **7** (150 mg, yield over two steps: 54%).

**Step 3:** To a 100 mL single-neck reaction flask were added 10 mL of deionized water and compound 7 (150 mg, 0.31 mmol), respectively. The mixture was cooled to 0°C and stirred, and potassium phosphate (1020 mg, 4.81 mmol) was added. The reaction mixture was further cooled to -3 to 0°C, and CDMBI (330 mg, 1.52 mmol) was added. The reaction was carried out at 0°C overnight. The reaction endpoint was monitored by TLC (isopropanol:concentrated aqueous ammonia:deionized water = 50:5:4). Upon completion of the reaction, the insoluble material was removed by filtration, and the insoluble material was washed with 3 mL of water. The filtrate was purified by C18 column chromatography (5%-15% methanol in water) using a preparative high-performance liquid chromatograph (Wuhan Ruihe Chromatography Technology, LC 2100). The collected fractions were concentrated and then lyophilized to obtain compound **G1** (86 mg, yield: 60%).

ESI-MS calc. for C₁₆H₂₆N₅O₉S [M+H]⁺ m/z= 464.1, found m/z= 464.1. ¹HNMR (400MHz, deuterium oxide) *δ* 7.48 (d, *J* = 5.4 Hz, 0.7H), 6.91 (d, *J = 5.6* Hz, 0.3H), 6.01 (d, *J* = 7.56, 2.3 Hz, 1H), 4.51 (d, *J* = 8.6 Hz, 1H), 4.46 (d, *J* = 8.4 Hz, 0.3H), 4.41-4.44 (m, 1H), 4.25 (d, *J =* 5.2 Hz, 0.7H), 4.17 (d, *J* = 5.6 Hz, 0.3H), 4.17-4.15 (m, 2H), 4.10-4.04(m, 1H), 3.98 (d, *J = 5.4* Hz, 0.7H), 3.76-3.74 (m, 1H), 3.64-3.53 (m, 2H),3.49-3.34 (m, 4H), 2.91 (t, *J* = 8.6 Hz, 1H). 1.95 (d, J= 1.6 Hz, 3H).

### Example 3: Synthesis of compound G2

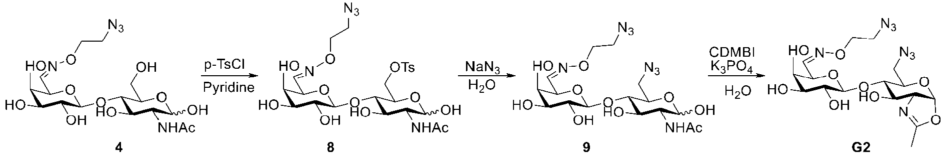

**Step 1:** To a 100 mL three-neck reaction flask were added 10 mL of pyridine and compound 4 (200 mg, 0.43 mmol), respectively. The system was purged with nitrogen and cooled to -15°C. p-Toluenesulfonyl chloride (p-TsCl, 410 mg, 2.15 mmol) was dissolved in 5 mL of pyridine, and the resulting solution was added dropwise to the 100 mL three-neck reaction flask via a syringe, and the reaction was carried out at about -15°C overnight. The reaction endpoint was monitored by TLC (dichloromethane:methanol = 10:1, containing 0.1% concentrated aqueous ammonia). Upon completion of the reaction, 15 mL of dichloromethane was added for dilution, and then the mixture was purified by silica gel column chromatography (dichloromethane:methanol = 20:1-10:1). The collected fractions were concentrated to obtain a crude product of compound **8** (140 mg, yield: 53%).

**Step 2:** To a 100 mL three-neck reaction flask were added 8 mL of deionized water, the crude product of compound **8** (140 mg, 0.23 mmol), and sodium azide (235 mg, 3.61 mmol), respectively. The system was purged with nitrogen, and the reaction was stirred at about 60°C for 40 hours. Upon completion of the reaction, the reaction mixture was filtered. The filtrate was purified by C18 column chromatography (deionized water:methanol = 10:1-5:1). The collected fractions were concentrated to obtain compound **9** (90 mg, yield: 81%).

**Step 3:** To a 100 mL single-neck reaction flask were added 10 mL of deionized water and compound **9** (90 mg, 0.18 mmol), and the reaction mixture was cooled to about 0°C. To the single-neck reaction flask was added potassium phosphate (584 mg, 2.75 mmol), and then the reaction mixture was further cooled to -3 to 0°C, followed by the addition of CDMBI (200 mg, 0.92 mmol). The reaction was stirred at about 0°C overnight. The reaction endpoint was monitored by TLC (isopropanol:concentrated aqueous ammonia: deionized water = 50:5:4). Upon completion of the reaction, the reaction mixture was filtered to remove insoluble materials, and the insoluble materials were washed with 3 mL of water. The filtrate was washed three times with 15 mL of toluene to remove impurities. The aqueous phase was purified by C18 column chromatography (deionized water:methanol = 5:1-3:1, containing 0.1% concentrated NH₃·H₂O). The collected fractions were concentrated and then lyophilized to obtain compound G2 (46 mg, yield: 53%).

ESI-MS calc. for C₁₆H₂₅N₈O₉ [M+H]⁺ m/z= 473.1, found m/z= 473.1. ¹HNMR (400MHz, deuterium oxide) *δ* 7.54 (d, *J* = 5.2 Hz, 0.7H), 6.87 (d, *J* = 5.8 Hz, 0.3H), 6.01 (d, *J* = 7.6 Hz, 1H), 4.38 (d, *J= 8.4* Hz, 0.7H), 4.34 (d, *J* = 8.2 Hz, 0.3H), 4.31 (d, *J=* 5.8 Hz, 0.3H), 4.29-4.26 (m, 1H), 4.21-4.14 (m, 3H), 4.08 (d, *J= 4.4* Hz, 1H), 3.94 (d, *J= 4.2* Hz, 0.7H), 3.68 (t, *J=* 8.2 Hz, 1H), 3.61-3.47 (m, 3H), 3.47-3.42 (m, 3H), 3.39-3.35 (m, 1H), 1.96 (d, *J= 1.4* Hz, 3H).

### Example 4: Synthesis of compound G3

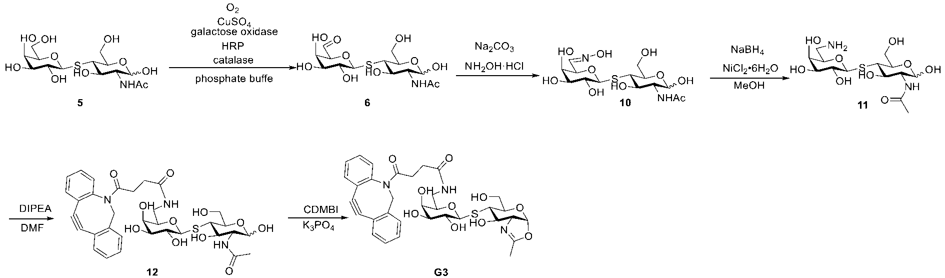

**Step 1:** To a 1000 mL single-neck reaction flask were added compound 5 (2.83 g, 7.1 mmol) and 115 mL of 50 mM pH 7.0 phosphate buffer, respectively, and the mixture was stirred in a 30°C water bath. Oxygen (O₂) was continuously introduced using a syringe needle for about 0.5 hours. To another 50 mL centrifuge tube were sequentially added 10 mL of 50 mM pH 7.0 phosphate buffer, 3 kU of galactose oxidase, 30 kU of horseradish peroxidase (HRP), 600 kU of catalase, and 1.8 mL of 1 mM aqueous copper sulfate solution, and the mixture was activated in a 30°C water bath for 0.5 hours. Subsequently, the activated mixed enzyme was added to the 1000 mL single-neck reaction flask, and the reaction was carried out in a 30°C water bath for 6 hours. Upon completion of the reaction, the introduction of oxygen was stopped, and the prepared compound 6 was used directly in the next step without purification.

**Step 2:** To the reaction system from the previous step were added sodium carbonate (Na₂CO₃, 376 mg, 3.5 mmol) and hydroxylamine hydrochloride (NH₂OH·HCl, 542 mg, 6.4 mmol), respectively. The reaction was carried out at 25°C overnight, and the reaction progress was monitored by TLC (isopropanol:concentrated aqueous ammonia:deionized water = 10:2:1). Upon completion of the reaction, 100 mL of methanol was added to the system, and then the insoluble materials were removed by filtration. The filtrate was subjected to rotary evaporation until dryness and purified on a C18 column (5% methanol in water). The collected fractions were concentrated to obtain compound 10 (2.68 g, yield: 92%).

**Step 3:** To a 500 mL single-neck reaction flask were added compound 10 (2.68 g, 6.5 mmol), 50 mL of 50 mM pH 7.0 phosphate buffer, and 100 mL of methanol, respectively. The mixture was stirred and cooled to 0°C. Nickel(II) chloride hexahydrate (NiCl₂ 6H₂O, 3.2 g, 13.5 mmol) and sodium borohydride (NaBH₄, 1.2 g, 31.7 mmol) were added respectively, and the reaction was carried out at 0°C overnight. Upon completion of the reaction, the reaction mixture was filtered. The filtrate was purified by amide column chromatography (Welch HILIC Amide, 10 µm, 21.2×250 mm, 90%-50% acetonitrile in water). The collected fractions were concentrated to obtain compound **11** (1.85 g, yield: 71%).

**Step 4:** To a 100 mL single-neck reaction flask were added 5 mL of *N,N-*dimethylformamide (DMF), compound **11** (113 mg, 0.28 mmol), *N*,*N*-diisopropylethylamine (DIPEA, 181 µL, 0.742 g/mL at 25°C, 1.04 mmol), and compound **20** (DBCO-COOSu, 167 mg, 0.42 mmol), respectively. The reaction was carried out at room temperature overnight. The reaction endpoint was monitored by TLC (isopropanol:concentrated aqueous ammonia:deionized water = 25:5:3). Upon completion of the reaction, the solvent was subjected to rotary evaporation until dryness, and the residue was purified by silica gel column chromatography (methanol:dichloromethane = 1:9-1:4). The collected fractions were concentrated to obtain compound **12** (163 mg, yield: 91%).

**Step 5:** To a 100 mL single-neck reaction flask were added 16 mL of deionized water and compound **12** (163 mg, 0.24 mmol), respectively. The reaction system was cooled to about 0°C, and potassium phosphate (756 mg, 3.56 mmol) was added. The reaction temperature was maintained at 0°C, and CDMBI (285 mg, 1.31 mmol) was added. The reaction was stirred at about 0°C overnight. The reaction endpoint was monitored by TLC (isopropanol:concentrated aqueous ammonia:deionized water = 25:5:3). Upon completion of the reaction, the reaction mixture was filtered, and the precipitate was washed with 3 mL of water. The filtrate was collected and washed four times with 30 mL of toluene, respectively, to remove impurities. The aqueous phase was purified by C18 column chromatography (20%-50% acetonitrile in water). The collected fractions were concentrated and then lyophilized to obtain compound **G3** (white solid product, 70 mg, yield: 44%).

ESI-MS calc. for C₃₃H₃₈N₃O₁₀S [M+H]⁺ m/z= 668.2, found m/z= 668.2. ¹HNMR (400MHz, deuterium oxide) *δ* 8.21 (d, *J* = 7.6 Hz, 2H), 7.42 (d, *J =* 7.6 Hz, 1H), 7.30-7.16 (m, 5H), 7.09(d, *J* = 7.2 Hz, 1H), 5.91 (d, *J* = 7.8 Hz, 1H), 4.87 (d, *J* = 7.2 Hz, 1H), 4.32-4.10 (m, 2H), 3.91 (d, *J =* 8.2 Hz, 1H), 3.71-3.62(m, 2H), 3.52 (d, *J =* 6.8 Hz, 2H), 3.46-3.28(m, 3H), 3.26-3.02(m, 3H), 3.01-2.91 (m, 1H), 2.74 (d, *J =* 6.4 Hz, 1H), 2.41-2.29 (m, 1H), 2.06 (s, 2H), 1.86 (d, *J =* 1.6 Hz, 3H).

### Example 5: Synthesis of compound G7

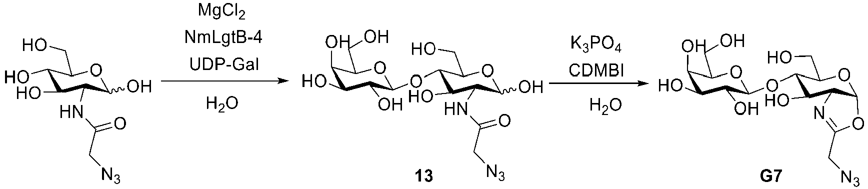

**Step 1:** 2-[(Azidoacetyl)amino]-2-deoxy-D-glucose (26.2 g, 100 mmol) and 64.1 g of UDP-Gal (uridine 5'-diphosphate galactose disodium salt, 64.1 g, 105 mmol) were weighed and added to a 1000 mL single-neck reaction flask, respectively. 600 mL of deionized water was added, and the mixture was stirred until dissolved. MgCl₂ (4 g, 42 mmol) was added, followed by the addition of 0.3 g of β-1,4-galactosyltransferase (NmLgtB-4) to catalyze the reaction. The pH of the system was 8.0, the reaction temperature was 37°C, and the stirring speed was 200 rpm. The reaction was carried out overnight. Upon completion of the reaction, an equal volume of ethanol was added to remove the enzyme. After centrifugation, the supernatant was concentrated. The concentrate was purified by electrodialysis, concentrated by membrane filtration, and then the residue was purified by silica gel column chromatography (dichloromethane:methanol = 10:1). The collected fractions were concentrated to obtain compound **13** (8.2 g, yield: 19%).

**Step 2:** To a 100 mL single-neck reaction flask were added 10 mL of deionized water and compound **13** (100 mg, 0.24 mmol), respectively. The reaction system was cooled to about 0°C, and potassium phosphate (610 mg, 2.87 mmol) was added. The temperature was further lowered to -3 to 0°C, and CDMBI (220 mg, 1.01 mmol) was added. The reaction was stirred at about 0°C overnight. The reaction endpoint was monitored by TLC. Upon completion of the reaction, the reaction mixture was filtered to remove insoluble materials, and the insoluble materials were washed with 3 mL of water. The filtrate was washed three times with 15 mL of toluene. The aqueous phase was purified by C18 column chromatography (deionized water:methanol=5:1-3:1, containing 0.1% concentrated aqueous ammonia). The collected fractions were concentrated and then lyophilized to obtain compound G7 (51 mg, yield: 52%).

HRMS calc. for C₁₄H₂₃N₄O₁₀ [M+H]⁺ m/z= 407.14153, found m/z= 407.14003. ¹HNMR (400MHz, deuterium oxide) *δ* 5.99 (d, *J* = 7.2 Hz, 1H), 4.22-4.17 (m, 2H), 4.07-4.05 (m, 1H), 3.96 (dd, *J =* 11.2, 3.4 Hz, 1H), 3.7 (d, *J =* 3.4 Hz, 1H), 3.61-3.38 (m, 8H), 3.27-3.21 (m, 2H).

### Example 6: Synthesis of compound G9

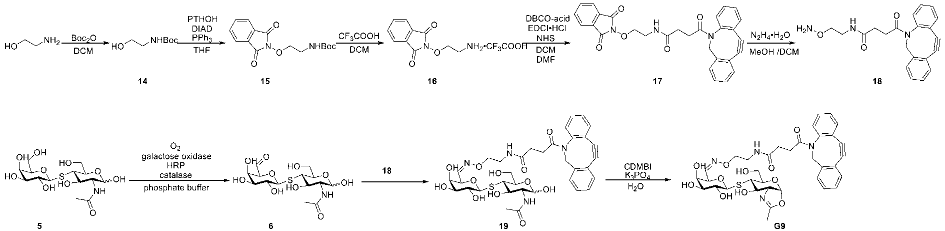

**Step 1:** To a 1000 mL three-neck reaction flask were added 500 mL of dichloromethane, 2-aminoethanol (10 g, 164 mmol), and triethylamine (34 mL, 245 mmol), respectively. The system was purged with nitrogen, and di-tert-butyl dicarbonate (Boc₂O, 35.7 g, 164 mmol) was added at 0°C. The reaction was stirred at room temperature for 4-5 hours. Upon completion of the reaction, the dichloromethane was subjected to rotary evaporation until dryness. The residue was dissolved in 300 mL of ethyl acetate, washed with 200 mL of saturated ammonium chloride solution and 100 mL of water, dried over anhydrous sodium sulfate, and concentrated to obtain compound 14 (20 g, yield: 76%).

**Step 2:** To a 2 L three-neck reaction flask were added 1 L of tetrahydrofuran (THF), compound **14** (20 g, 124 mmol), N-hydroxyphthalimide (PHTOH, 21.2 g, 130 mmol), and triphenylphosphine (PPh₃, 35.8 g, 136 mmol), respectively. The system was purged with nitrogen, stirred, and the temperature of the reaction system was lowered to about 0°C. Diisopropyl azodicarboxylate (DIAD, 26.3 mL, 130 mmol) was added dropwise, and the reaction was carried out at 0°C overnight. TLC indicated that the reaction was complete (petroleum ether:ethyl acetate = 2:1). Upon completion of the reaction, 500 mL of ethyl acetate was added to the reaction mixture. The organic phase was washed with 200 mL of saturated aqueous sodium chloride solution and 200 mL of deionized water, respectively, dried over anhydrous sodium sulfate, concentrated, and purified by silica gel column chromatography (petroleum ether:ethyl acetate = 3:1-1:1) to obtain 41 g of a crude product of compound **15,** which was used directly in the next step.

**Step 3:** To a 100 mL single-neck reaction flask were added 6 mL of dichloromethane, the crude product of compound **15** (1.25 g, 4.08 mmol), and trifluoroacetic acid (CF₃COOH, 3.0 mL, 39.18 mmol), respectively, and the reaction was carried out at room temperature for 2 hours. The reaction endpoint was monitored by TLC (petroleum ether:ethyl acetate = 10:1). Upon completion of the reaction, the reaction mixture was concentrated and dried under reduced pressure with an oil pump to obtain 1.3 g of a crude product of compound **16,** which was used directly in the next step without purification.

**Step 4:** To a 100 mL three-neck reaction flask were added 6 mL of dichloromethane, DBCO-acid (CAS:1353016-70-2, 0.5 g, 1.64 mmol), N-hydroxysuccinimide (HNS, CAS: 6066-82-6, 0.28 g, 2.43 mmol), and EDCI HCl (CAS: 7084-11-9, 0.47 g, 2.45 mmol), respectively. The mixture was stirred at room temperature for 3-4 hours. Then, the crude product of compound **16** (1.3 g, 4.06 mmol) and N,N-diisopropylethylamine (DIPEA, 3 mL, 17.22 mmol) were added, and the mixture was stirred at room temperature for 2-3 hours. The reaction endpoint was monitored by TLC (petroleum ether:ethyl acetate = 10:1). Upon completion of the reaction, insoluble materials were removed by filtration, and the insoluble materials were rinsed twice with 10 mL of dichloromethane. The filtrate was washed twice with 20 mL of saturated aqueous sodium bicarbonate solution. The organic phase was dried over anhydrous sodium sulfate, concentrated, and subjected to silica gel column chromatography (petroleum ether:ethyl acetate = 1:1-1:2) to obtain compound 17 (0.23 g, yield: 29%).

**Step 5:** To a 100 mL single-neck reaction flask were added 4 mL of dichloromethane, 2 mL of methanol, and compound **17** (0.42 g, 0.85 mmol), respectively, followed by the addition of hydrazine hydrate (N₂H₄ H₂O, 36 µL, 0.94 mmol). The reaction was carried out at room temperature for 2 hours, and the reaction endpoint was monitored by TLC (dichloromethane:methanol = 5:1). Upon completion of the reaction, 40 mL of deionized water was added. The aqueous phase was extracted three times with 20 mL of dichloromethane, and the organic phases were combined. The combined organic phase was washed twice with 20 mL of saturated aqueous sodium bicarbonate solution, dried over anhydrous sodium sulfate, concentrated, and subjected to silica gel column chromatography (methanol:dichloromethane = 1:19-1:9) to obtain compound **18** (0.2 g, yield: 65%).

**Step 6:** To a 100 mL single-neck reaction flask were added 5 mL of 50 mM pH 7.0 phosphate buffer and compound **5** (120 mg, 0.30 mmol), respectively. The mixture was stirred in a 30°C water bath while oxygen (O₂) was continuously introduced using a syringe needle for about 0.5 hours. To another 5 mL centrifuge tube were sequentially added 1 mL of 50 mM pH 7.0 phosphate buffer, 280 U of galactose oxidase, 10 kU of horseradish peroxidase (HRP), 200 kU of catalase, and 0.16 mL of 1 mM aqueous copper sulfate solution, and the mixture was activated in a 30°C water bath for 0.5 hours. Subsequently, the activated mixed enzyme was added to the 100 mL single-neck reaction flask, and the reaction was carried out in a 30°C water bath for 6 hours. Upon completion of the reaction, the introduction of oxygen was stopped, and the prepared compound **6** was used directly in the next step without purification.

**Step 7:** To a 100 mL single-neck reaction flask was added compound **18** (120 mg, 0.33 mmol), and the reaction was carried out at 25°C overnight. The reaction progress was monitored by TLC (isopropanol:concentrated aqueous ammonia:deionized water = 10:2:1). Upon completion of the reaction, most of the water (>90%) was removed by rotary evaporation. Then, 20 mL of methanol was added for thorough dissolution, and the insoluble materials were removed by filtration. The insoluble materials were washed twice with 10 mL of methanol. The filtrate was concentrated, and then the residue was purified by silica gel column chromatography (dichloromethane:methanol = 15:1-6:1). The collected fractions were concentrated again and purified by C18 column chromatography (30%-70% acetonitrile in water). The fractions were collected to obtain compound **19** (120 mg, yield: 54%).

**Step 8:** To a 100 mL single-neck reaction flask were added 15 mL of deionized water and compound **19** (120 mg, 0.16 mmol), respectively. The reaction system was cooled to about 0°C, and potassium phosphate (514 mg, 2.42 mmol) was added. The temperature was further lowered to 0°C, and CDMBI (175 mg, 0.81 mmol) was added. The reaction was stirred at about 0°C overnight. The reaction endpoint was monitored by TLC (isopropanol:concentrated aqueous ammonia:deionized water = 50:5:3). Upon completion of the reaction, the insoluble material was removed by filtration, and the insoluble material was washed with 3 mL of water. The filtrate was purified by C18 column chromatography (30%-70% acetonitrile in water), and the collected fractions were concentrated and then lyophilized to obtain compound **G9** (white solid product, 45 mg, yield: 39%).

ESI-MS calc. for C₃₅H₄₃N₄O₁₂S [M+H₂O+H]⁺ m/z= 743.3, found m/z= 743.3. ¹HNMR (400MHz, deuterium oxide) *δ* 7.68 (d, *J* = 5.2 Hz, 0.7H), 7.56-7.35 (m, 8H), 6.84 (d, *J =* 5.2Hz, 0.3H), 6.18-6.13 (m, 1H), 5.10 (d, *J =* 16.8 Hz, 1H), 4.61 (d, *J =* 7.4 Hz, 0.7H), 4.54-4.52 (m, 1H), 4.33 (t, *J=* 5.2Hz, 1H), 4.25 (d, *J=* 8.2 Hz, 0.3H), 4.18-4.16 (m, 1H), 4.07 (s, 1H), 4.03-3.80 (m, 4H), 3.76-3.58 (m, 3H), 3.54-3.46 (m, 1H), 3.34-3.19 (m, 2H), 3.04 (d, J = 9.8 Hz, 1H), 2.57-2.49 (m, 1H), 2.29-2.15 (m, 3H), 2.08-2.01 (m, 3H).

### Example 7: Synthesis of compound G10

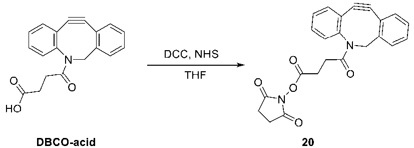

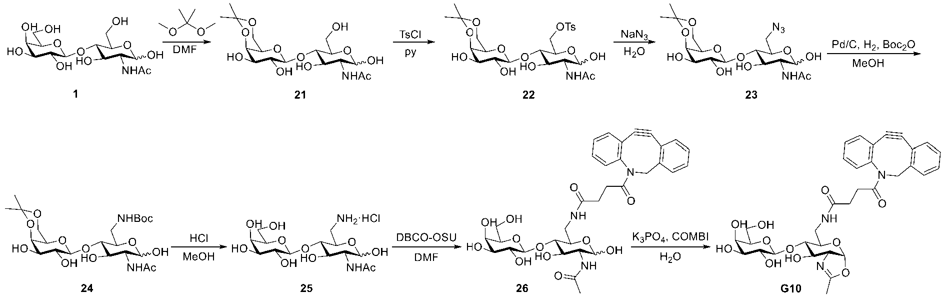

**Step 1:** To a 100 mL single-neck flask were added 10 mL of tetrahydrofuran, 11,12-didehydro-γ-oxodibenzo[b,f]azocine-5(6H)-butanoic acid (DBCO-acid, 0.5 g, 1.64 mmol), N,N'-dicyclohexylcarbodiimide (DCC, 0.35 g, 1.70 mmol) and N-hydroxysuccinimide (NHS, 0.2 g, 1.74 mmol), respectively. The system was purged with nitrogen and the reaction was carried out at room temperature overnight. Upon completion of the reaction, the solid was removed by filtration. The filtrate was concentrated, and then the residue was purified by silica gel column chromatography (petroleum ether:ethyl acetate = 2:1-1:1). The collected fractions were concentrated to obtain compound **20** (0.4 g, yield: 61%).

**Step 2:** To a 500 mL single-neck reaction flask were added 260 mL of *N,N-*dimethylformamide, compound 1 (5 g, 13 mmol), and 2,2-dimethoxypropane (18.8 g, 181 mmol), respectively. The mixture was stirred, and p-toluenesulfonic acid (TsOH, 0.2 g, 1.2 mmol) was added at room temperature. The mixture was stirred at 25°C for 30 minutes. Upon completion of the reaction, sodium bicarbonate solid was added in an ice bath to quench the reaction. The mixture was warmed to room temperature and stirred for an additional 1 hour. The solid was removed by filtration. The filtrate was concentrated, then slurried with ethyl acetate, subjected to ultrasonic oscillation for 30 minutes, filtered, rinsed, and the filter cake was collected and dried with an oil pump to obtain compound **21** (3.5 g, yield: 64%).

**Step 3:** To a 250 mL three-neck reaction flask were added 100 mL of pyridine and compound **21** (3.5 g, 8.27 mmol), respectively. The system was purged with nitrogen, and the reaction system was cooled to -20°C. Stirring was initiated, and p-toluenesulfonyl chloride (5.5 g, 28.85 mmol) was added in portions. The reaction was carried out at -20°C overnight. TLC detection indicated that the main spot of the reaction was the product (dichloromethane:methanol = 20:1). Upon completion of the reaction, 100 mL of saturated sodium bicarbonate solution was added to quench the reaction. The mixture was concentrated, and then the residue was purified by silica gel column chromatography (methanol:dichloromethane = 1:9-2:8). The collected fractions were concentrated to obtain compound **22** (2.6 g, yield: 54%).

**Step 4:** To a 100 mL three-neck reaction flask were added 30 mL of deionized water, compound **22** (1.5 g, 2.60 mmol), and sodium azide (2.5 g, 38.46 mmol), respectively. The reaction was carried out at 60°C for 36 hours, and the reaction endpoint was monitored by TLC (dichloromethane:methanol = 20:1). Upon completion of the reaction, the mixture was purified by C18 column chromatography (5%-15% methanol in water). The collected fractions were subjected to rotary evaporation until a volume of 20 mL was obtained, followed by lyophilization, to obtain compound **23** (0.8 g, yield: 68%).

**Step 5:** To a 100 mL three-neck reaction flask were added 10 mL of methanol, compound **23** (0.8 g, 1.78 mmol), and di-tert-butyl dicarbonate (Boc₂O, 0.6 g, 2.75 mmol), respectively. The system was purged with nitrogen three times, followed by the addition of Pd/C (80 mg, 0.18 mmol). The system was purged with hydrogen three times, and the reaction was stirred at room temperature under a hydrogen balloon for 2 hours. The reaction endpoint was monitored by TLC (dichloromethane:methanol = 10:1). Upon completion of the reaction, the mixture was filtered through diatomite, concentrated, and then the residue was purified by C18 column chromatography (5%-20% methanol in water). The collected fractions were subjected to rotary evaporation until dryness to obtain compound **24** (0.4 g, yield: 43%).

**Step 6:** To a 50 mL two-neck reaction flask were added 2 mL of methanol, a 10 mM solution of hydrogen chloride in ethanol (0.2 mL), and compound **24** (0.1 g, 0.19 mmol), respectively. The mixture was stirred at room temperature for 2 hours. The reaction endpoint was monitored by TLC (isopropanol:water:concentrated aqueous ammonia = 20:4:5). Upon completion of the reaction, the solvent was subjected to rotary evaporation until dryness, and the residue was purified by C18 column chromatography (5% methanol in water). The collected fractions were subjected to rotary evaporation until a volume of 10 mL was obtained, followed by lyophilization to obtain compound **25** (30 mg, yield: 38%).

**Step 7:** To a 100 mL three-neck reaction flask were added 2 mL of *N,N-*dimethylformamide, compound **25** (40 mg, 0.10 mmol), compound **20** (64 mg, 0.16 mmol), and triethylamine (TEA, 80 mg, 0.50 mmol). The reaction was stirred at room temperature for 2 hours. Upon completion of the reaction, DMF was subjected to rotary evaporation until dryness, and the residue was purified by C18 column chromatography (20%-50% methanol in water). The eluent was concentrated to about 5 mL to obtain compound **26** (30 mg, yield: 45%).

**Step 8:** To a 100 mL single-neck reaction flask were added 5 mL of water and compound **26** (30 mg, 0.04 mmol), respectively. The reaction system was cooled to about 0°C, and potassium phosphate (142 mg, 0.67 mmol) was added. The temperature was further lowered to -3 to 0°C, and CDMBI (48 mg, 0.22 mmol) was added. The reaction was stirred at about 0°C overnight. The reaction endpoint was monitored by TLC (isopropanol:concentrated aqueous ammonia:deionized water = 50:5:4). Upon completion of the reaction, the reaction mixture was filtered to remove insoluble materials, and the filter cake was washed with 3 mL of deionized water. The filtrate was washed three times with 15 mL of toluene to remove impurities. The product was purified by C18 column chromatography (20%-50% methanol in water). The eluent was subjected to rotary evaporation to remove methanol, and then lyophilized to obtain compound **G10** (26 mg, yield: 99%).

ESI-MS calc. for C₃₃H₃₈N₃O₁₁ [M+H]⁺ m/z= 652.3, found m/z= 652.3. ¹HNMR (400MHz, deuterium oxide) *δ* 7.68-7.34 (m, 8H), 6.00 (d, *J =* 8.6 Hz, 1H), 5.12 (d, *J* = 14.8 Hz, 1H), 4.44-4.36 (m, 2H), 3.92 (d, *J* = 8.4 Hz, 1H), 3.86 (d, *J =* 14.8 Hz, 1H), 3.81-3.74 (m, 2H), 3.68-3.49 (m, 5H), 3.46-3.32 (m, 3H), 2.64-2.53 (m, 1H), 2.34-2.13 (m, 3H), 2.00-1.95 (m, 3H).

### Example 8: Synthesis of compound G13

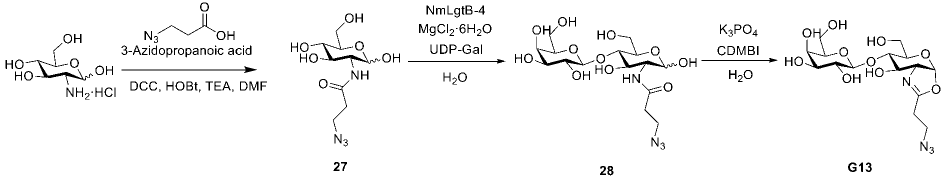

**Step 1:** To a 100 mL three-neck reaction flask were added 10 mL of *N,N-*dimethylformamide, D-glucosamine hydrochloride (1.1 g, 5.10 mmol), *N,N'-*dicyclohexylcarbodiimide (DCC, 1.16 g, 5.62 mmol), 1-hydroxybenzotriazole (HOBt, 0.76 g, 5.62 mmol), and triethylamine (TEA, 0.57 g, 5.62 mmol), respectively. The mixture was stirred uniformly, and 3-azidopropanoic acid (0.65 g, 5.62 mmol) was added in an ice-water bath. After the addition, the mixture was returned to room temperature and stirred overnight. The reaction mixture was directly concentrated, purified by silica gel column chromatography (dichloromethane:methanol = 10:1), and concentrate under reduced pressure to obtain compound **27** (380 mg, yield: 27%).

**Step 2:** To a 100 mL three-neck reaction flask were added 20 mL of deionized water, compound **27** (380 mg, 1.38 mmol), and UDP-Gal (790 mg, 1.29 mmol), respectively. The mixture was stirred, and an appropriate amount of metal salt ion MgCl₂·6H₂O (0.1 g) was added. The reaction was carried out in the presence of β-1,4-galactosyltransferase (NmLgtB-4, 0.06 g). The pH of the reaction mixture was 8.0, the reaction temperature was 37°C, and the stirring speed was 200 rpm for overnight reaction. Upon completion of the reaction, an equal volume of ethanol was added to remove the enzyme, and the mixture was concentrated. The concentrated mixture was then purified by electrodialysis and concentrated by membrane filtration. The product was purified by silica gel column chromatography (dichloromethane:methanol = 10:1) and concentrated under reduced pressure to obtain compound **28** (120 mg, yield: 21%).

**Step 3:** To a 100 mL single-neck reaction flask were added 10 mL of deionized water and compound **28** (100 mg, 0.23 mmol), respectively. The reaction system was cooled to about 0°C, and potassium phosphate (580 mg, 2.73 mmol) was added. The temperature was further lowered to -3 to 0°C, and CDMBI (210 mg, 0.97 mmol) was added. The reaction was maintained at about 0°C and stirred overnight. The reaction endpoint was monitored by TLC. Upon completion of the reaction, insoluble materials were removed by filtration, and the filter cake was washed with 3 mL of water. The filtrate was washed with 15 mL of toluene to remove impurities. The aqueous phase was purified by C18 column chromatography (deionized water:methanol = 5:1-3:1, containing 0.1% concentrated aqueous ammonia). The collected fractions were concentrated and then lyophilized to obtain compound **G13** (58 mg, yield: 60%).

ESI-MS calc. for C₁₅H₂₄KN₄O₁₀ [M+K]⁺ m/z= 459.1, found m/z= 459.1. ¹HNMR (400MHz, deuterium oxide) *δ* 6.01 (d, *J= 7.8* Hz, 1H), 4.33-4.28 (m, 2H), 4.12 (d, *J= 7.2* Hz, 1H), 3.78 (d, *J* = 5.6 Hz, 1H), 3.71-3.46 (m, 9H), 3.42-3.435 (m, 2H), 2.58 (t, *J* = 7.4 Hz, 2H).

### Example 9: Synthesis of compound G14

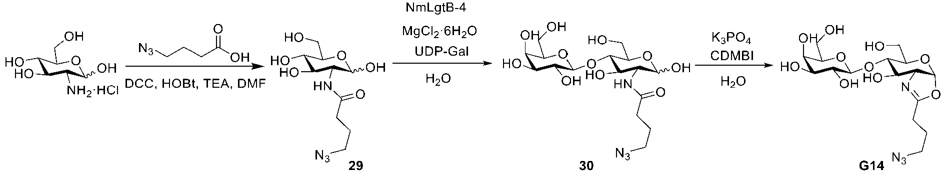

**Step 1:** To a 100 mL three-neck reaction flask were added 10 mL of *N,N-*dimethylformamide, D-glucosamine hydrochloride (1.1 g, 5.10 mmol), *N,N'-*dicyclohexylcarbodiimide (DCC, 1.16 g, 5.62 mmol), 1-hydroxybenzotriazole (HOBt, 0.76 g, 5.62 mmol), and triethylamine (TEA, 0.57 g, 5.62 mmol), respectively. The mixture was stirred uniformly, and 4-azidobutyric acid (0.73 g, 5.62 mmol) was added in an ice-water bath. After the addition, the mixture was returned to room temperature and stirred overnight. The reaction mixture was directly concentrated, purified by silica gel column chromatography (dichloromethane:methanol = 10:1), and concentrate under reduced pressure to obtain compound **29** (320 mg, yield: 22%).

**Step 2:** To a 100 mL three-neck reaction flask were added 20 mL of deionized water, compound **29** (320 mg, 1.10 mmol), and UDP-Gal (730 mg, 1.20 mmol), respectively. The mixture was stirred, and an appropriate amount of metal salt ion MgCl₂·6H₂O (0.1 g) was added. The reaction was carried out in the presence of β-1,4-galactosyltransferase (NmLgtB-4, 0.06 g). The pH of the reaction mixture was 8.0, the reaction temperature was 37°C, and the stirring speed was 200 rpm for overnight reaction. Upon completion of the reaction, an equal volume of ethanol was added to remove the enzyme, and the mixture was concentrated. The concentrated mixture was then purified by electrodialysis and concentrated by membrane filtration. The product was purified by silica gel column chromatography (dichloromethane:methanol = 10:1) and concentrated under reduced pressure to obtain compound **30** (130 mg, yield: 26%).

**Step 3:** To a 100 mL single-neck reaction flask were added 10 mL of deionized water and compound **30** (100 mg, 0.22 mmol), respectively. The reaction system was cooled to about 0°C, and potassium phosphate (580 mg, 2.73 mmol) was added. The temperature was further lowered to -3 to 0°C, and CDMBI (210 mg, 0.97 mmol) was added. The reaction was maintained at about 0°C and stirred overnight. The reaction endpoint was monitored by TLC. Upon completion of the reaction, insoluble materials were removed by filtration, and the filter cake was washed with 3 mL of water. The filtrate was washed with 15 mL of toluene to remove impurities. The aqueous phase was purified by C18 column chromatography (deionized water:methanol = 5:1-3:1, containing 0.1% concentrated aqueous ammonia). The collected fractions were concentrated and then lyophilized to obtain compound **G14** (61 mg, yield: 64%).

ESI-MS calc. for C₁₆H₂₆N₄O₁₀ [M+K]⁺ m/z= 473.1, found m/z= 473.1. ¹HNMR (400MHz, deuterium oxide) *δ* 5.099 (d, *J* = 7.6 Hz, 1H), 4.33-4.28 (m, 2H), 4.11 (d, *J* = 7.2 Hz, 1H), 3.77 (d, *J* = 5.8 Hz, 1H), 3.71-3.46 (m, 7H), 3.40-3.34 (m, 1H), 3.31 (t, *J =* 7.4 Hz, 3H), 2.37 (t, *J=* 6.8 Hz, 2H), 1.77-1.86 (m, 2H).

### Example 10: Synthesis of compound G27

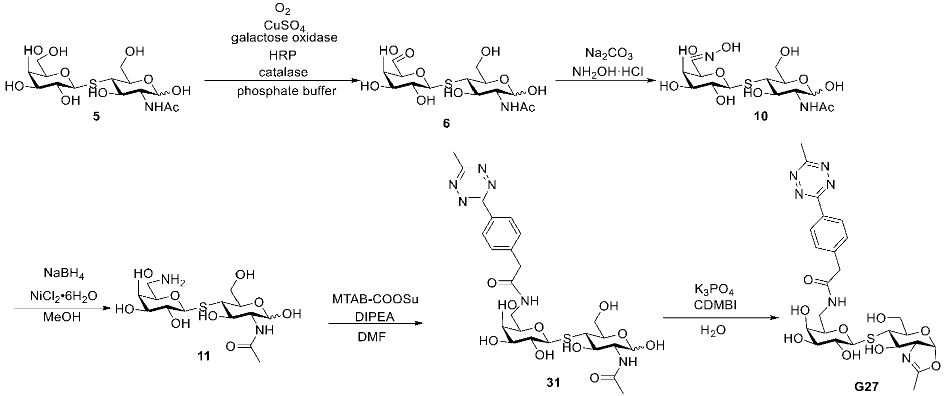

**Step 1:** To a 1000 mL single-neck reaction flask were added 115 mL of 50 mM pH 7.0 phosphate buffer and compound 5 (2.83 g, 7.09 mmol), respectively. The mixture was stirred in a 30°C water bath while oxygen (O₂) was continuously introduced using a syringe needle for about 0.5 hours. To another 50 mL centrifuge tube were sequentially added 10 mL of 50 mM pH 7.0 phosphate buffer, 3 kU of galactose oxidase, 30 kU of horseradish peroxidase (HRP), 600 kU of catalase, and 1.8 mL of 1 mM aqueous copper sulfate solution, and the mixture was activated in a 30°C water bath for 0.5 hours. Subsequently, the activated mixed enzyme was added to the 1000 mL single-neck reaction flask, and the reaction was carried out in a 30°C water bath for 6 hours. Upon completion of the reaction, the introduction of oxygen was stopped, and the prepared compound **6** was used directly in the next step without purification.

**Step 2:** To the reaction mixture from the previous step were added sodium carbonate (Na₂CO₃, 306 mg, 2.89 mmol) and hydroxylamine hydrochloride (NH₂OH·HCl, 442 mg, 6.36 mmol), respectively. The reaction was carried out at 25°C overnight, and the reaction progress was monitored by TLC (isopropanol:concentrated aqueous ammonia:deionized water = 10:2:1). Upon completion of the reaction, 100 mL of methanol was added to the reaction system, and the insoluble materials were removed by filtration. The filtrate was subjected to rotary evaporation until dryness, and then the residue was purified by C18 column chromatography (5% methanol in water) to obtain compound **10** (2.68 g, yield: 92%).

**Step 3:** To a 500 mL single-neck reaction flask were added compound **10** (2.68 g, 6.5 mmol), 50 mL of 50 mM pH 7.0 phosphate buffer, and 100 mL of methanol, respectively. The mixture was stirred and cooled to 0°C. Nickel(II) chloride hexahydrate (NiCl₂ 6H₂O, 3.2 g, 13.5 mmol) and sodium borohydride (NaBH₄, 1.2 g, 31.7 mmol) were added respectively, and the reaction was carried out at 0°C overnight. Upon completion of the reaction, the reaction mixture was filtered. The filtrate was purified by amide column chromatography (Welch HILIC Amide, 10 µm, 21.2×250 mm, 90%-50% acetonitrile in water). The collected fractions were concentrated to obtain compound **11** (1.85 g, yield: 71%).

**Step 4:** To a 100 mL single-neck reaction flask were added 5 mL of *N,N-*dimethylformamide (DMF), compound **11** (250 mg, 0.63 mmol), N,N-diisopropylethylamine (DIPEA, 330 µL, 0.742 g/mL at 25°C, 1.89 mmol), and 2,5-dioxopyrrolidin-1-yl 2-(4-(6-methyl-1,2,4,5-tetrazin-3-yl)phenyl)acetate (MTAB-COOSu, 310 mg, 0.95 mmol), respectively. The reaction was carried out at room temperature overnight. The reaction endpoint was monitored by TLC (isopropanol:concentrated aqueous ammonia:deionized water = 25:5:3). Upon completion of the reaction, the solvent was subjected to rotary evaporation until dryness, and the residue was purified by silica gel column chromatography (methanol:dichloromethane = 3:17-3:7). The collected fractions were concentrated to obtain compound **31** (300 mg, yield: 78%).

**Step 5:** To a 100 mL single-neck reaction flask were added 30 mL of deionized water and compound **31** (300 mg, 0.49 mmol), respectively. The reaction system was cooled to about 0°C, and potassium phosphate (1.56 g, 7.35 mmol) was added. The temperature was further lowered to 0°C, and CDMBI (533 mg, 2.46 mmol) was added. The reaction was stirred at about 0°C overnight. The reaction endpoint was monitored by TLC (isopropanol:concentrated aqueous ammonia:deionized water = 25:5:3). Upon completion of the reaction, insoluble materials were removed by filtration, and the insoluble materials were washed with 3 mL of deionized water. The filtrate was washed three times with 30 mL of toluene 30 mL to remove impurities from the aqueous phase. The aqueous phase was purified by C18 column chromatography (20%-50% acetonitrile in water). The collected fractions were concentrated and then lyophilized to obtain compound G27 (46 mg, yield: 16%).

ESI-MS calc. for C₂₅H₃₃N₆O₉S [M+H]⁺ m/z= 593.2, found m/z= 593.2. ¹HNMR (400MHz, deuterium oxide) *δ* 7.44 (d, *J* = 7.8 Hz, 2H), 7.32(d, *J =* 7.8 Hz, 2H), 5.92(d, *J =* 7.2 Hz, 1H), 4.32(d, *J =* 7.2 Hz, 2H), 3.99(d, *J =* 6.8 Hz, 1H), 3.77(d, *J =* 6.4 Hz, 1H), 3.67(d, *J =* 7.8 Hz, 1H), 3.69 (s, 2H), 3.54-3.49(m, 2H), 3.47-3.43(m, 1H), 3.38-3.32(m, 2H), 3.28-3.20(m, 2H), 2.89 (s, 3H), 2.82(d, *J* = 6.8 Hz, 1H), 1.86 (d, *J =* 1.6 Hz, 3H).

### Example 11: Synthesis of compound G4

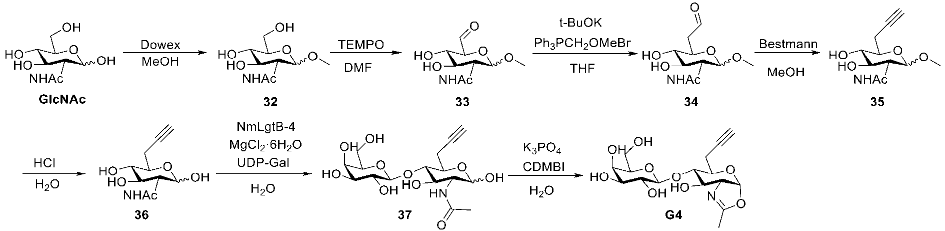

**Step 1:** To a 2 L single-neck reaction flask were added 1 L of methanol and 50 g of *N-*acetyl-D-glucosamine (GlcNAc, 0.23 mol), respectively, and the mixture was stirred at room temperature until dissolved. Then, 100 g of cation exchange resin Dowex (H⁺) was added, and the system was heated to 70°C and stirred for 24 hours. The temperature was cooled to room temperature, and the filter residue was removed by filtration. The filtrate was concentrated under reduced pressure to obtain 38.4 g of compound **32** as a white solid, which was used directly in the next step without further purification.

**Step 2:** To a 2 L three-neck reaction flask were added 1 L of DMF and compound **32** (20 g, 85.02 mmol), respectively, and the mixture was stirred at room temperature until dissolved. Subsequently, sodium bicarbonate (214.5 g, 2.55 mol) and 2,2,6,6-tetramethylpiperidine-1-oxyl (0.4 g, 2.56 mmol) were added sequentially. The system was purged with nitrogen, and the temperature was lowered to about 0°C. Then, trichloroisocyanuric acid (19.8 g, 85.02 mmol) was added. The reaction mixture was stirred at 0°C for 4 hours, and then the solid was removed by filtration. The filtrate was concentrated under reduced pressure, and then the residue was purified by silica gel column chromatography (dichloromethane:methanol = 20:1-10:1) to obtain 9.5 g of compound **33** as an off-white solid in 48% yield.

**Step 3:** Under a nitrogen atmosphere and at 0°C, to a 500 mL three-neck reaction flask were added 75 mL of THF, (methoxymethyl)triphenylphosphonium bromide (Ph₃PCH₂OMeBr, 15.0 g, 38.86 mmol), and a solution of potassium tert-butoxide (t-BuOK, 4.33 g, 38.59 mmol) in THF (70 mL), respectively. The mixture was then stirred at 0°C for 2 hours. To this mixture was added a solution of compound **33** (5.0 g, 21.44 mmol) in THF (37 mL), and then the temperature was raised to room temperature, with stirring continued for an additional 3 hours. The reaction mixture was concentrated under reduced pressure to about 40% of its original volume, and then 30% sulfuric acid (30 mL) was added thereto. The mixture was stirred at 20°C for 3 hours. The pH of the reaction mixture was adjusted to about 7 with a 2 M aqueous sodium hydroxide solution at a low temperature, and then the mixture was concentrated under reduced pressure, redissolved in methanol (150 mL), and filtered to remove the solid. The filtrate was concentrated under reduced pressure, and then the residue was purified by silica gel column chromatography (dichloromethane:methanol = 30:1-10:1) to obtain 1.37 g of compound **34** as a yellow solid in 25% yield.

**Step 4:** Under a nitrogen atmosphere and at 20°C, to a 50 mL three-neck reaction flask were added 14 mL of methanol, compound **34** (1.37 g, 5.54 mmol), potassium carbonate (1.53 g, 11.1 mmol), and Bestmann reagent (1.49 g, 7.76 mmol), respectively. The reaction mixture was stirred at 20°C for 12 hours, and the solid was removed by filtration. The filtrate was concentrated under reduced pressure, and then the residue was purified by C18 column chromatography (acetonitrile:water = 1:20-1:2) to obtain 439 mg of compound **35** as a yellow solid in 32% yield.

**Step 5:** To a 100 mL two-neck reaction flask were added 12 mL of 6 M hydrochloric acid solution and compound **35** (1.37 g, 5.63 mmol), respectively, and the reaction was stirred at 60°C for 3 hours. After the reaction mixture was cooled to room temperature, the pH was adjusted to about 7 with 1 M sodium hydroxide solution, and the reaction mixture was concentrated under reduced pressure, and then the residue was purified by C18 column chromatography (methanol:water = 1:20) to obtain 222 mg of compound **36** as an off-white solid in 53% yield.

**Step 6:** At room temperature, to a 50 mL two-neck reaction flask were sequentially added compound **36** (222 mg, 0.97 mmol), UDP-Gal (590 mg, 1.06 mmol), a solution of MgCl₂·6H₂O (100 mg) in deionized water (20 mL), and β1,4-galactosyltransferase NmLgtB-4 (60 mg). The system was warmed to 37°C and the reaction was stirred overnight. An equal volume of ethanol was added, and the solid was removed by filtration. The filtrate was concentrated under reduced pressure, and then the residue was purified by silica gel column chromatography (dichloromethane:methanol = 10:1) to obtain 214 mg of solid compound **37** in 56% yield.

**Step 7:** To a 100 mL three-neck reaction flask were sequentially added 10 mL of water, compound **37** (214 mg, 0.55 mmol), potassium phosphate (1.74 g, 8.20 mmol), and CDMBI (0.6 g, 2.76 mmol) at 0°C. The reaction mixture was stirred at 0°C overnight, and the solid was removed by filtration. The filtrate was extracted with toluene (10 mL × 3), and the aqueous phases were combined. The aqueous phase was purified by preparative C18 chromatography (acetonitrile:water = 1:10-1:3, preparative high-performance liquid chromatograph (Wuhan Ruihe Chromatography Technology, LC 2100)) to obtain 78 mg of compound **G4** as a white solid in 38% yield.

ESI-MS calc. for C₁₆H₂₄NO₉ [M+H]⁺ m/z= 374.1, found m/z= 374.1. ¹H NMR (400 MHz, D₂O) *δ* 5.93 (d, *J* = 7.4 Hz, 1H), 4.33 (d, *J =* 7.8 Hz, 1H), 4.23 (s, 1H), 4.07 - 4.03 (m, 1H), 3.78 (s, 1H), 3.74 - 3.53 (m, 5H), 3.51 (s, 1H), 3.42 - 3.35 (m, 2H), 2.56 (s, 1H), 2.52 - 2.46 (m, 1H), 1.92 (s, 3H).

### Example 12: Synthesis of compound G4b

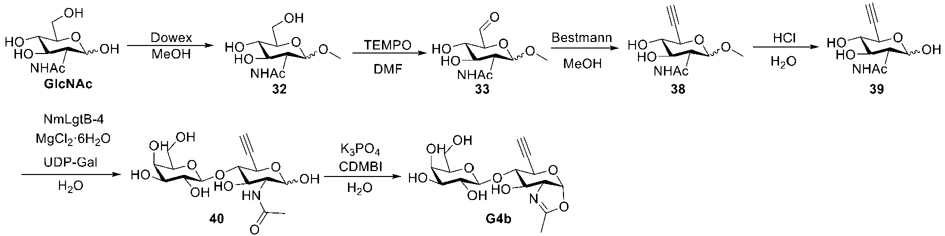

**Step 1:** To a 2 L single-neck reaction flask were added 1 L of methanol and 50 g of *N-*acetyl-D-glucosamine (GlcNAc, 0.23 mol), respectively, and the mixture was stirred at room temperature until dissolved. Then, 100 g of cationic resin Dowex (H⁺) was added, the system temperature was heated to 70°C, and the mixture was stirred for 24 hours. The temperature was cooled to room temperature, and the filter residue was removed by filtration. The filtrate was concentrated under reduced pressure to obtain 38.4 g of compound **32** as a white solid, which was used directly in the next step without further purification.

**Step 2:** To a 2 L three-neck reaction flask were added 1 L of DMF and compound **32** (20 g, 85.02 mmol), respectively, and the mixture was stirred at room temperature until dissolved. Subsequently, sodium bicarbonate (214.5 g, 2.55 mol) and 2,2,6,6-tetramethylpiperidine-1-oxyl (0.4 g, 2.56 mmol) were added sequentially. The system was purged with nitrogen, and the temperature was lowered to about 0°C. Then, trichloroisocyanuric acid (19.8 g, 85.02 mmol) was added. The reaction mixture was stirred at 0°C for 4 hours, and then the solid was removed by filtration. The filtrate was concentrated under reduced pressure, and then the residue was purified by silica gel column chromatography (dichloromethane:methanol = 20:1-10:1) to obtain 9.5 g of compound **33** as an off-white solid in 48% yield.

**Step 3:** Under a nitrogen atmosphere and at 20°C, to a 250 mL three-neck reaction flask were added 95 mL of methanol, compound **33** (9.5 g, 40.73 mmol), potassium carbonate (11.3 g, 81.76 mmol), and Bestmann reagent (10.2 g, 53.09 mmol), respectively. The reaction mixture was stirred at 20°C for 14 hours, and the solid was removed by filtration. The filtrate was concentrated under reduced pressure, and then the residue was purified by silica gel column chromatography (dichloromethane:methanol = 20:1-15:1) to obtain 5.95 g of compound **38** as a yellow solid in 63% yield.

**Step 4:** To a 100 mL single-neck reaction flask were added 29 mL of 6 M hydrochloric acid solution and compound **38** (1.0 g, 4.36 mmol), respectively, and the reaction was stirred at 60°C for 5 hours. After the reaction mixture was cooled to room temperature, the pH was adjusted to about 7 with 1 M sodium hydroxide solution, and the reaction mixture was concentrated under reduced pressure, and then the residue was purified by C18 column chromatography (methanol:water = 1:20) to obtain 460 mg of compound **39** as an off-white solid in 49% yield.

**Step 5:** At room temperature, to a 100 mL single-neck reaction flask were sequentially added compound **39** (460 mg, 2.14 mmol), UDP-Gal (1.31 g, 2.30 mmol), a solution of MgCl₂·6H₂O (100 mg) in deionized water (20 mL), and β1,4-galactosyltransferase NmLgtB-4 (60 mg). The system was warmed to 37°C and the reaction was stirred overnight. An equal volume of ethanol was added, and the solid was removed by filtration. The filtrate was concentrated under reduced pressure, and then the residue was purified by silica gel column chromatography (dichloromethane:methanol = 10:1) to obtain 320 mg of solid compound **40** in 39% yield.

**Step 6:** To a 100 mL three-neck reaction flask were sequentially added 15 mL of water, compound **40** (0.32 g, 0.85 mmol), potassium phosphate (2.7 g, 12.72 mmol), and CDMBI (0.92 g, 4.24 mmol) at 0°C. The reaction mixture was stirred at 0°C overnight. TLC (isopropanol:concentrated aqueous ammonia:water = 25:5:3) indicated that the reaction was complete. The solid was removed by filtration, and the filtrate was extracted with toluene (10 mL × 3). The aqueous phases were combined. The aqueous phase was purified by preparative C18 chromatography (acetonitrile:water = 1:10-1:3, preparative high-performance liquid chromatograph (Wuhan Ruihe Chromatography Technology, LC 2100)) to obtain 116 mg of compound **G4b** as a white solid in 38% yield.

ESI-MS calc. for C₁₅H₂₂NO₉ [M+H]⁺ m/z= 360.1, found m/z= 360.1. ¹H NMR (400 MHz, D₂O) *δ* 5.95 (d, *J* = 7.2 Hz, 1H), 4.47 (d, *J* = 7.8 Hz, 1H), 4.17 - 4.08 (m, 2H), 4.05 (d, *J =* 8.7 Hz, 1H), 3.98 - 3.88 (m, 1H), 3.78 (d, *J* = 3.1 Hz, 1H), 3.73 - 3.43 (m, 5H), 3.44 - 3.27 (m, 1H), 1.94 (s, 3H).

### Example 13: Synthesis of compound G20

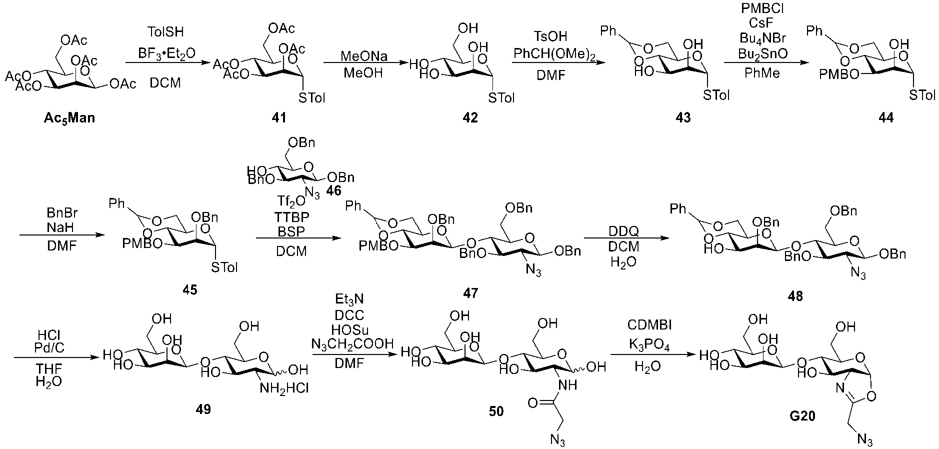

**Step 1**: To a 500 mL single-neck reaction flask were added 150 mL of dichloromethane, pentaacetylmannose (**Ac₅Man,** 50 g, 0.128 mol), and 4-toluenethiol (TolSH, 25 g, 0.201 mol), respectively, and the mixture was stirred at room temperature until dissolved. Then, boron trifluoride diethyl etherate (BF₃•Et₂O, 40 mL, 0.32 mol) was added, and the reaction was stirred at room temperature for 3 hours. TLC (petroleum ether:ethyl acetate = 1:1) indicated that the reaction was complete. The reaction mixture was diluted with dichloromethane (450 mL) and washed with saturated sodium bicarbonate (1000 mL). The organic phase was concentrated under reduced pressure to obtain a crude product of compound **41,** which was used in the next step with a theoretical yield of 58 g.

**Step 2:** To a 2.0 L single-neck reaction flask were added 600 mL of methanol and compound **41** (58 g, 0.175 mol), respectively, and the mixture was stirred at room temperature until dissolved. Then, a 5 M solution of MeONa in methanol (40 mL) was added, and the reaction was stirred at room temperature for 2 hours. TLC (dichloromethane:methanol = 20:1) indicated that the reaction was complete. The pH was adjusted to 7 with 1 M dilute hydrochloric acid. After concentration under reduced pressure, the residue was purified by silica gel column chromatography (dichloromethane:methanol = 20:1) to obtain 30.8 g of compound **42** as an off-white solid in 82% yield.

**Step 3:** To a 1.0 L single-neck reaction flask were added 300 mL of DMF, compound **42** (30 g, 0.105 mol), and benzaldehyde dimethyl acetal (40 mL), respectively, and the mixture was stirred at room temperature until dissolved. To the mixture was added TsOH (0.54 g, 0.003 mol), and the reaction was stirred at room temperature overnight. Saturated sodium bicarbonate (40 mL) was added to quench the reaction. After concentration under reduced pressure, the concentrate was slurried with 300 mL of a mixture of petroleum ether and ethyl acetate (1:1), resulting in the precipitation of a solid. The filtered solid was further slurried with 250 mL of a mixture of water and ethanol (4:1), and filtered to obtain compound **43** as a white solid (17.8 g) in 45% yield.

**Step 4:** Under a nitrogen atmosphere, to a 1.0 L single-neck reaction flask were added 350 mL of toluene and compound **43** (17.8 g, 0.048 mol), respectively, and the mixture was stirred at room temperature until dissolved. To the mixture was then added dibutyltin oxide (Bu₂SnO, 12.1 g, 0.049 mol), and the reaction was carried out at 125°C for 3 hours. After cooling to room temperature, tetrabutylammonium bromide (Bu₄NBr, 16.2 g, 0.050 mol), cesium fluoride (CsF, 7.4 g, 0.049 mol), and p-methoxybenzyl chloride (PMBCl, 7.8 g, 0.050 mol) were added to the reaction system, and the reaction was carried out at 125°C for 3 hours under a nitrogen atmosphere. After cooling, ethyl acetate (800 mL) was added, and the mixture was washed with saturated sodium bicarbonate (600 mL). The organic phase was concentrated under reduced pressure, and the concentrate was purified by silica gel column chromatography (petroleum ether:ethyl acetate = 4:1) to obtain compound **44** as a colorless oil, 18 g, in 76% yield.

**Step 5:** Under a nitrogen atmosphere, to a 1.0 L single-neck reaction flask were added 350 mL of DMF and compound **44** (18 g, 0.036 mol), respectively, and the mixture was stirred at room temperature until dissolved. Then, sodium hydride (NaH, 2.2 g, 60 wt%, 0.055 mol) was added, and the mixture was stirred at room temperature for 10 minutes. Then, benzyl bromide (BnBr, 6.5 mL, 0.055 mol) was added, and the reaction was carried out at room temperature for 6 hours. Saturated ammonium chloride (20 mL) was added to quench the reaction. After the solution was concentrated under reduced pressure, ethyl acetate (800 mL) was added to the concentrate, and the mixture was washed with water (300 mL × 2). The organic phase was concentrated under reduced pressure, and then the residue was purified by silica gel column chromatography (petroleum ether:ethyl acetate = 30:1-20:1) to obtain compound **45** as a colorless gum (19.2 g) in 90% yield.

**Step 6:** Under a nitrogen atmosphere, to a 500 mL three-neck reaction flask were added 150 mL of dichloromethane and compound **45** (5.6 g, 0.010 mol), respectively, and the mixture was stirred at room temperature until dissolved. Dried 4Å molecular sieves (28 g) were then added, and the mixture was stirred at room temperature for 2 hours. The system was further cooled to -60°C, and 1-(phenylsulfonyl)pyrrole (BSP, 2.0 g, 0.010 mol) and tri-tert-butylphosphine (TTBP, 5.0 g, 0.025 mol) were added, followed by stirring for 45 minutes. Trifluoromethanesulfonic anhydride (Tf₂O, 2.0 mL, 0.012 mol) was then slowly added dropwise, and the reaction was maintained at -60°C for 30 minutes. A solution of compound **46** (3.2 g, 0.007 mol) in dichloromethane (10 mL) was then added, and the reaction was carried out at -60°C for 3 hours. TLC (petroleum ether:ethyl acetate = 5:1) indicated that the reaction was complete. The reaction mixture was filtered, and the filter residue was rinsed with dichloromethane (300 mL). The filtrate was washed once with water (200 mL). The organic phase was concentrated under reduced pressure, and then the residue was purified by silica gel column chromatography (petroleum ether:ethyl acetate = 8:1) to obtain compound **47** as a colorless gum (14.1 g) in 65% yield.

**Step 7:** To a 250 mL single-neck reaction flask were added 56 mL of dichloromethane, 3.2 mL of water, and compound **47** (1.6 g, 1.71 mmol), respectively, and the mixture was stirred at room temperature until completely dissolved. The system was cooled to 0°C, and 2,3-dichloro-5,6-dicyano-1,4-benzoquinone (DDQ, 0.9 g, 3.96 mmol) was added. The reaction was carried out at 0°C for 0.5 hours, then the temperature was raised to room temperature, and the mixture was stirred for an additional 1 hour. The reaction mixture was diluted with dichloromethane (50 mL) and washed with saturated sodium bicarbonate (30 mL × 2). The organic phase was concentrated under reduced pressure, and then the residue was purified by silica gel column chromatography (petroleum ether:ethyl acetate = 10:1-7:1) to obtain compound **48** as a yellow gum (1.39 g) in 99% yield.

**Step 8:** To a 250 mL single-neck reaction flask were added 75 mL of tetrahydrofuran, 20 mL of water, and compound **48** (1.39 g, 1.73 mmol), respectively, and the mixture was stirred at room temperature until completely dissolved. The system was purged with nitrogen, and then Pd/C (2 g, 10 wt%, 1.88 mmol) and an aqueous solution of HCl (5 mL, 1 M) were added to the system, respectively. The system was charged with hydrogen (15 psi). The reaction was stirred at room temperature overnight. TLC (isopropanol:concentrated aqueous ammonia:water = 4:2:1) indicated that the reaction was complete. The reaction mixture was filtered, and the filtrate was concentrated under reduced pressure to obtain compound **49** as a yellow solid, which was used directly in the next step without purification (estimated product amount: 0.6 g, yield: 93%).

**Step 9:** Under a nitrogen atmosphere, to a 100 mL single-neck flask were added compound **49** (600 mg, 1.59 mmol), azidoacetic acid (N₃CH₂COOH, 241 mg, 2.38 mmol), *N-*hydroxysuccinimide (HOSu, 329 mg, 2.86 mmol), a solution of triethylamine (Et₃N, 0.66 mL, 4.77 mmol) in DMF (10 mL), and N,N'-dicyclohexylcarbodiimide (DCC, 721 mg, 3.49 mmol), respectively. The mixture was stirred at room temperature overnight. TLC (isopropanol:concentrated aqueous ammonia:water = 25:5:3) indicated that the reaction was complete. The mixture was filtered, and the filtrate was concentrated under reduced pressure. The residue was purified by amide column chromatography to obtain compound **50** as a white solid (0.5 g) in 74% yield.

**Step 10:** At 0°C, to a 100 mL three-neck reaction flask were added 10 mL of water, compound 50 (160 mg, 0.38 mmol), potassium phosphate (K₃PO₄, 1200 mg, 5.65 mmol), and 2-chloro-1,3-dimethyl-1H-benzimidazol-3-ium chloride (CDMBI, 409 mg, 1.88 mmol), respectively. The reaction mixture was stirred at 0°C overnight. TLC (isopropanol:concentrated aqueous ammonia:water = 25:5:3) indicated that the reaction was complete. The precipitate was removed by filtration, and the filtrate was purified by amide column chromatography to obtain 120 mg of compound **G20** as a white solid in 78% yield.

ESI-MS calc. for C₁₄H₂₃N₄O₁₉ [M+H]⁺ m/z= 407.1, found m/z= 407.1. ¹H NMR (400 MHz, D₂O) *δ* 6.02 (d, *J* = 7.1 Hz, 1H), 4.54 (s, 1H), 4.24 (s, 1H), 4.14 - 4.07 (m, 1H), 4.02 (s, 1H), 3.82 - 3.67 (m, 3H), 3.65 - 3.53 (m, 3H), 3.52 - 3.34 (m, 3H), 3.32 - 3.23 (m, 1H), 3.24 - 3.12 (m, 1H).

### Example 14: Synthesis of compound G21

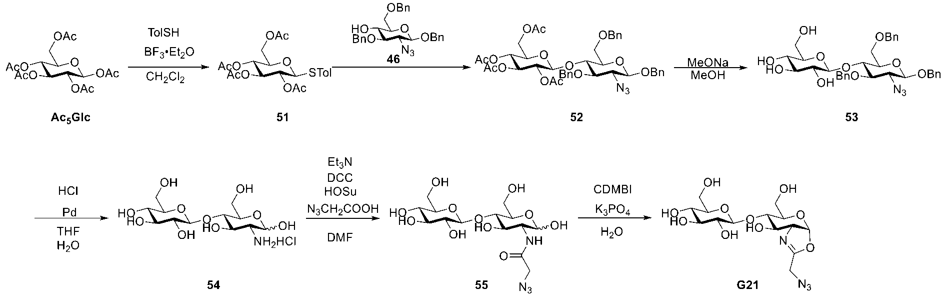

**Step 1**:To a 500 mL single-neck reaction flask were added 150 mL of dichloromethane, pentaacetylglucose (**Ac₅Glc,** 50 g, 0.128 mol), and 4-toluenethiol (TolSH, 27 g, 0.217 mol), respectively, and the mixture was stirred at room temperature until dissolved. Then, boron trifluoride diethyl etherate (BF₃•Et₂O, 40 mL, 0.32 mol) was added, and the reaction was stirred at room temperature for 3 hours. TLC (petroleum ether:ethyl acetate = 3:1) indicated that the reaction was complete. The reaction mixture was diluted with dichloromethane (800 mL) and washed with saturated sodium bicarbonate (1000 mL). The organic phase was concentrated under reduced pressure, and then the residue was purified by silica gel column chromatography (petroleum ether:ethyl acetate = 7:1-3:1) to obtain 48 g of compound **51** as a white solid in 82% yield.

**Step 2:** Under a nitrogen atmosphere, to a 500 mL three-neck reaction flask were added 200 mL of dichloromethane, compound **51** (12.5 g, 0.028 mol), and compound **46** (10.1 g, 0.021 mol), respectively, and the mixture was stirred at room temperature until dissolved. Dried 4Å molecular sieves (35 g) were then added, and the mixture was stirred at room temperature for 2 hours. The temperature was lowered to -10°C, and *N*-iodosuccinimide (NIS, 8.8 g, 0.039 mol) and trimethylsilyl trifluoromethanesulfonate (TMSOTf, 2.9 mL, 0.016 mol) were added to the reaction mixture. After the addition was completed, the mixture was warmed to room temperature and stirred at room temperature overnight. TLC (petroleum ether:ethyl acetate = 3:1) indicated that the reaction was complete. The reaction mixture was filtered, and the filter residue was rinsed with dichloromethane (300 mL). Water (300 mL) was added to the filtrate, and sodium thiosulfate was slowly added with vigorous stirring until the organic phase was decolorized. The organic phase was concentrated under reduced pressure, and the residue was purified by silica gel column chromatography (petroleum ether:ethyl acetate = 7:1-4:1) to obtain 17 g of compound **52** as a white solid in 99% yield.

**Step 3:** To a 1.0 L single-neck reaction flask were added 200 mL of methanol and compound **52** (17 g, 21.09 mmol), respectively, and the mixture was stirred at room temperature until dissolved. Then, a 5 M solution of MeONa in methanol (1 mL) was added, and the reaction was stirred at room temperature for 2 hours. TLC (petroleum ether:ethyl acetate = 10:1) indicated that the reaction was complete. Cation exchange resin (H⁺) was added for neutralization, and the pH was adjusted to 6-7. The mixture was filtered, and the filtrate was concentrated under reduced pressure to obtain 12.6 g of compound **53** as a light yellow solid in 93% yield, which was used directly in the next step without further purification.

**Step 4:** To a 250 mL single-neck reaction flask were added 60 mL of tetrahydrofuran, 7.5 mL of water, and compound **53** (2 g, 3.14 mmol), respectively, and the mixture was stirred at room temperature until completely dissolved. The system was purged with nitrogen, and then Pd/C (2 g, 10 wt%, 1.88 mmol) and an aqueous solution of HCl (12.5 mL, 1 M) were added to the system. The mixture was charged with H₂ (15 psi) and stirred at room temperature overnight. TLC (isopropanol:concentrated aqueous ammonia:water = 4:2:1) indicated that the reaction was complete. The reaction mixture was filtered, and the filtrate was concentrated under reduced pressure to obtain compound **54** as a yellow solid, which was used directly in the next step without purification (the theoretical yield of 1.18 g was used in the next step).

**Step 5:** Under a nitrogen atmosphere, to a 100 mL single-neck flask were added compound **54** (1.18 g, 3.12 mmol), azidoacetic acid (N₃CH₂COOH, 473 mg, 4.68 mmol), *N-*hydroxysuccinimide (HOSu, 647 mg, 5.62 mmol), a solution of triethylamine (Et₃N, 1.3 mL, 9.40 mmol) in DMF (15 mL), and *N,N*'-dicyclohexylcarbodiimide (DCC, 1.42 g, 6.88 mmol), respectively, and the mixture was stirred at room temperature overnight. TLC (isopropanol:concentrated aqueous ammonia:water = 25:5:3) indicated that the reaction was complete. The mixture was filtered, and the filtrate was concentrated under reduced pressure. The residue was purified by amide column chromatography to obtain 370 mg of compound **55** as a white solid in 28% yield.

**Step 6:** At 0°C, to a 100 mL three-neck reaction flask were added 10 mL of water, compound **55** (120 mg, 0.28 mmol), potassium phosphate (K₃PO₄, 900 mg, 4.24 mmol), and 2-chloro-1,3-dimethyl-1H-benzimidazol-3-ium chloride (CDMBI, 307 mg, 1.41 mmol), respectively. The reaction mixture was stirred at 0°C overnight. TLC (isopropanol:concentrated aqueous ammonia:water = 25:5:3) indicated that the reaction was complete. The precipitate was removed by filtration, and the filtrate was purified by amide column chromatography to obtain 60 mg of compound **G21** as a white solid in 52% yield.

ESI-MS calc. for C₁₄H₂₃N₄O₁₀ [M+H]⁺ m/z= 407.1, found m/z= 407.2. ¹H NMR (400 MHz, D₂O) *δ* 6.04 (dd, *J* = 7.3, 2.9 Hz, 1H), 4.38 - 4.22 (m, 2H), 4.17 - 4.07 (m, 1H), 4.02 (d, *J* = 22.6 Hz, 2H), 3.80 - 3.61 (m, 3H), 3.61 - 3.44 (m, 2H), 3.35 - 3.25 (m, 3H), 3.25 - 3.16 (m, 1H), 3.15 - 3.03 (m, 1H).

### Example 15: Synthesis of compound G22

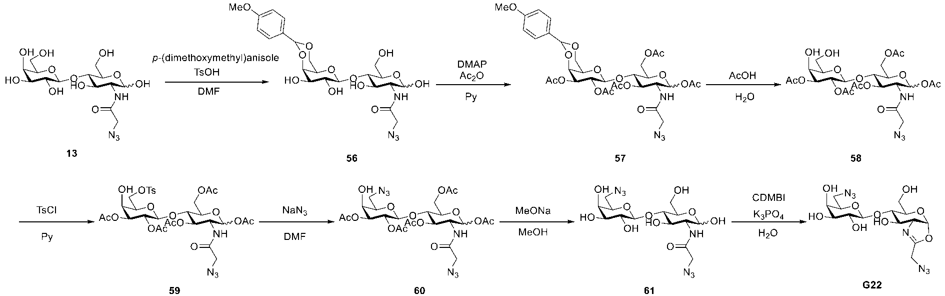

**Step 1:** To a 500 mL single-neck reaction flask were added 125 mL of DMF, compound **13** (5 g, 0.012 mol), and 4-methoxybenzaldehyde dimethyl acetal (6.4 g, 0.035 mol), respectively, and the mixture was stirred at room temperature until dissolved. Then, *p-*toluenesulfonic acid (TsOH, 0.2 g, 0.001 mol) was added, and the mixture was stirred at room temperature for 2 hours. Subsequently, saturated sodium bicarbonate (100 mL) was added to quench the reaction, and the solid was removed by filtration. The filtrate was concentrated under reduced pressure to obtain a crude product of compound **56,** which was directly used in the next step with a calculated amount of 6.39 g.

**Step 2:** To a 500 mL single-neck reaction flask were added 100 mL of pyridine, compound **56** (6.39 g, 0.012 mol), and 4-dimethylaminopyridine (DMAP, 0.14 g, 0.001 mol), respectively, and the mixture was stirred at room temperature until dissolved. Then, acetic anhydride (Ac₂O, 20 mL, 0.216 mol) was added to the system, and the mixture was stirred at room temperature overnight. TLC (petroleum ether:ethyl acetate = 1:1) indicated that the reaction was complete. The reaction mixture was concentrated under reduced pressure, and then the residue was purified by silica gel column chromatography (petroleum ether:ethyl acetate = 1:1-0:1) to obtain 7.8 g of compound **57** as an off-white solid in 88% yield.

**Step 3:** To a 250 mL single-neck reaction flask were added 80 mL of acetic acid (AcOH), 20 mL of water, and compound **57** (7.8 g, 0.010 mol), respectively, and the mixture was stirred at room temperature for 3 hours. TLC (petroleum ether:ethyl acetate = 1:4) indicated that the reaction was complete. After concentration under reduced pressure, water (150 mL) was added to the concentrate, and the mixture was then extracted with ethyl acetate (150 mL × 2). The organic phases were combined and concentrated under reduced pressure to obtain 5.9 g of compound **58** as an off-white solid, which was used directly in the next step without further purification.

**Step 4:** To a 250 mL single-neck reaction flask were added 60 mL of pyridine and compound **58** (5.9 g, 9.30 mmol), respectively. The mixture was stirred at room temperature until dissolved, and then the temperature was lowered to 20°C to -15°C. At -20°C to -15°C, *p*-toluenesulfonyl chloride (TsCl, 6.8 g, 35.67 mmol) was further added, and the mixture was stirred overnight. The reaction was quenched by adding 20 mL of methanol, and then the reaction mixture was concentrated under reduced pressure. After concentration, ethyl acetate (400 mL) and 1 M HCl (1000 mL) were added, and the organic layer was separated. The aqueous phase was extracted with ethyl acetate (400 mL), and then the organic phases were combined and washed with saturated aqueous sodium bicarbonate solution (200 mL). The organic phase was concentrated under reduced pressure to obtain 10 g of compound **59** as a white solid, which was used directly in the next step without purification (estimated product amount: 5 g, yield: 68%).

**Step 5:** Under a nitrogen atmosphere and at room temperature, to a 250 mL three-neck reaction flask were added 120 mL of DMF and compound **59** (5 g, 7.55 mmol), respectively. Sodium azide (NaN₃, 6.18 g, 95.06 mmol) was then added, and then the temperature was raised to 60°C, and the mixture was stirred for 60 hours. The temperature was cooled to room temperature, the mixture was filtered, and the filtrate was concentrated under reduced pressure. To the residue was added water (200 mL), and the mixture was extracted with ethyl acetate (100 mL × 2). The organic phases were combined and concentrated under reduced pressure to obtain 2.4 g of compound **60** as a white solid, which was used directly in the next step without further purification.

**Step 6:** To a 250 mL single-neck reaction flask were added 100 mL of methanol and compound **60** (2.4 g, 3.64 mmol), respectively, and the mixture was stirred at room temperature until completely dissolved. Then, a solution of sodium methoxide (MeONa, 0.7 mL) in methanol was added, and the mixture was stirred at room temperature overnight. The pH was adjusted to 6-7 with a cation exchange resin (H⁺), and the mixture was filtered. The filtrate was concentrated under reduced pressure, and then the residue was purified by C18 column chromatography (methanol:water = 1:20) to obtain 500 mg of compound **61** as an off-white solid in 30% yield.

**Step 7:** At 0°C, to a 50 mL three-neck reaction flask were added 10 mL of water, compound **61** (125 mg, 0.28 mmol), potassium phosphate (K₃PO₄, 905 mg, 4.26 mmol), and 2-chloro-1,3-dimethyl-1H-benzimidazol-3-ium chloride (CDMBI, 295 mg, 1.36 mmol), respectively, and then the mixture was stirred at 0°C overnight. TLC (isopropanol:aqueous ammonia:water = 25:5:3) indicated that the reaction was complete. The reaction mixture was warmed to room temperature, and then impurities were extracted with toluene (20 mL × 4). The aqueous layer was directly purified by C18 column chromatography (acetonitrile:water = 1:10-1:3, preparative high-performance liquid chromatograph (Wuhan Ruihe Chromatography Technology, LC 2100)) to obtain 60 mg of compound **G22** as a white solid in 50% yield.

ESI-MS calc. for C₁₄H₂₂N₇O₉ [M+H]⁺ m/z= 432.1, found m/z= 432.1. ¹H NMR (400 MHz, D₂O) *δ* 6.04 (d, *J* = 7.2 Hz, 1H), 4.38 - 4.24 (m, 2H), 4.11 (d, *J* = 6.8 Hz, 1H), 4.02 (d, *J* = 14.8 Hz, 1H), 3.99 - 3.79 (m, 1H), 3.70 (s, 1H), 3.67 - 3.53 (m, 3H), 3.54 - 3.38 (m, 3H), 3.36 - 3.27 (m, 2H), 3.24 (d, *J* = 13.1 Hz, 1H).

### Example 16: Synthesis of compound G23

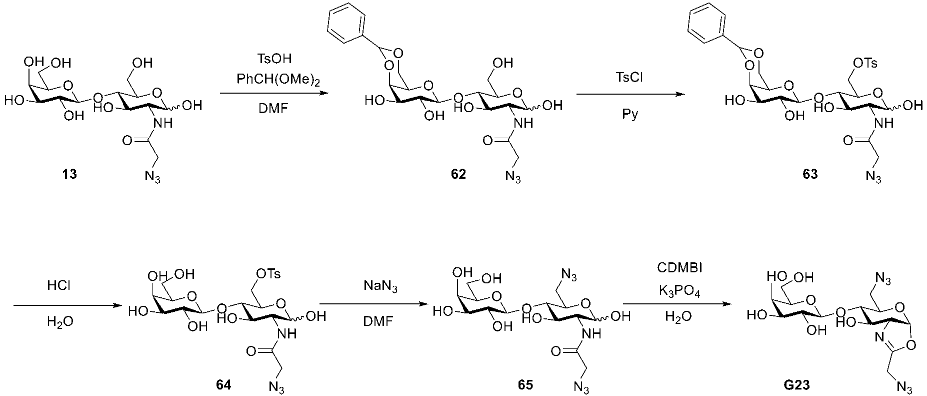

**Step 1:** To a 500 mL single-neck reaction flask were added 200 mL of DMF, compound **13** (5 g, 0.012 mol), and benzaldehyde dimethyl acetal (2.15 g, 0.014 mol), respectively, and the mixture was stirred at room temperature until dissolved. Then, p-toluenesulfonic acid (TsOH, 0.2 g, 0.001 mol) was added, and the reaction was stirred at room temperature overnight. Subsequently, saturated sodium bicarbonate (100 mL) was added to quench the reaction, and the solid was removed by filtration. The filtrate was concentrated under reduced pressure, and the residue was purified by C18 column chromatography (methanol:water=2:3-1:1) to obtain 4.0 g of compound **62** as a white solid in 66% yield.

**Step 2:** At -20°C to -15°C, to a 250 mL single-neck reaction flask were added 100 mL of pyridine and compound **62** (4.0 g, 0.008 mol), and the mixture was stirred until dissolved. Then, *p*-toluenesulfonyl chloride (TsCl, 2.98 g, 0.016 mol) was added, and the mixture was stirred overnight. The reaction was quenched by adding 20 mL of methanol, and then concentrated under reduced pressure. The residue was used directly in the next step without purification (calculated based on a theoretical yield of 5.2 g for charging).

**Step 3:** To a 250 mL single-neck reaction flask were added 100 mL of 1 M aqueous HCl solution and compound **63** (5.2 g, 0.007 mol), respectively, and the mixture was stirred at room temperature for 2 hours. The reaction mixture was then concentrated under reduced pressure and purified by C18 column chromatography (methanol:water = 1:4 to 1:1) to obtain 1 g of compound **64** as a yellow solid in 22% yield.

**Step 4:** Under a nitrogen atmosphere, to a 100 mL three-neck reaction flask were added 20 mL of DMF and compound **64** (1 g, 0.0016 mol), respectively, and the mixture was stirred at room temperature until dissolved. Sodium azide (NaN₃, 1.05 g, 0.016 mol) was then added, and then the temperature was raised to 60°C, and the mixture was stirred for 48 hours. The reaction mixture was filtered, and the filtrate was concentrated under reduced pressure, and then the residue was purified by C18 column chromatography (acetonitrile:water = 1:20-1:2) to obtain 0.22 g of compound **65** in 30% yield.

**Step 5:** To a 50 mL three-neck reaction flask were added 10 mL of water and compound **65** (150 mg, 0.334 mmol), respectively. The mixture was stirred at room temperature until dissolved. The temperature was lowered to 0°C, and then potassium phosphate (K₃PO₄, 1.06 g, 5.0 mmol) and 2-chloro-1,3-dimethyl-1H-benzimidazol-3-ium chloride (CDMBI, 362 mg, 1.67 mmol) were added. The mixture was then stirred at 0°C overnight. TLC (isopropanol:aqueous ammonia:water = 25:5:3) indicated that the reaction was complete. The reaction mixture was warmed to room temperature, and then impurities were extracted with toluene (20 mL × 4). The aqueous layer was directly purified by C18 column chromatography (acetonitrile:water = 1:20-1:2, preparative high-performance liquid chromatograph (Wuhan Ruihe Chromatography Technology, LC 2100)) to obtain 100 mg of compound **G23** as a white solid in 69% yield.

ESI-MS calc. for C₁₄H₂₂N₇O₉ [M+H]⁺ m/z = 432.1, found m/z = 432.1. ¹H NMR (400 MHz, D₂O) *δ* 6.30 - 6.21 (m, 1H), 4.50 - 4.41 (m, 2H), 4.36 - 4.29 (m, 1H), 4.30 - 4.14 (m, 1H), 3.95 - 3.90 (m, 1H), 3.87 - 3.59 (m, 8H), 3.59 - 3.48 (m, 2H).

### Example 17: Synthesis of compound G24

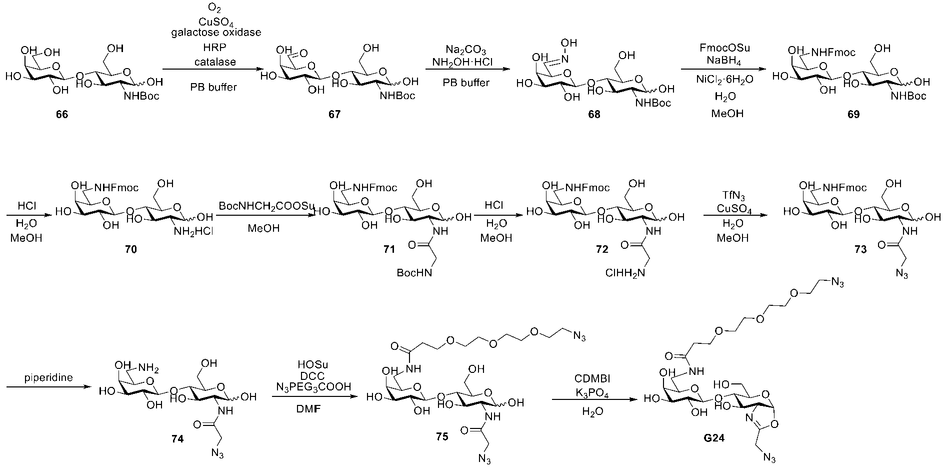

**Step 1:** To a phosphate buffer (40 mL) were sequentially added galactose oxidase (18 mL), copper sulfate (CuSO₄, 2 mM, 10 mL), horseradish peroxidase (HRP, 340 mg), and catalase (10 mL), and then the mixture was activated in a 30°C water bath for 30 minutes. At 30°C, to a 2.0 L three-neck reaction flask were added phosphate buffer (200 mL) and compound **66** (13 g, 29.45 mmol), respectively. Oxygen (O₂) was introduced for 15-30 minutes, and then the mixed enzyme was added to the reaction system. The reaction was carried out in a 30°C water bath for 6 hours. TLC (isopropanol:concentrated aqueous ammonia:water = 25:5:3) indicated that the reaction was complete. The introduction of oxygen was stopped, and the reaction mixture was used directly in the next step without further treatment.

**Step 2:** To the reaction mixture were added Na₂CO₃ (1.56 g, 14.72 mmol) and hydroxylamine hydrochloride (NH₂OH HCl, 2.25 g, 32.38 mmol), and the reaction was carried out at room temperature for 2 hours. TLC (isopropanol:concentrated aqueous ammonia:water = 25:5:3) indicated that the reaction was complete. Methanol (300 mL) was added, and the precipitate was removed by filtration through diatomite. The precipitate was rinsed with methanol (300 mL), and the obtained filtrate was used directly in the next step (compound 68 therein: 13.0 g, 97% yield).

**Step 3:** At 0°C, to a 2.0 L single-neck reaction flask were added 600 mL of methanol, 280 mL of phosphate buffer, compound **68** (13 g, 0.029 mol), and nickel(II) chloride hexahydrate (NiCl₂ 6H₂O, 17.0 g, 0.072 mol), respectively, and the mixture was stirred. Then, sodium borohydride (NaBH₄, 5.4 g, 0.143 mol) and 9-fluorenylmethyl *N*-succinimidyl carbonate (FmocOSu, 19.3 g, 0.057 mol) were further added, and the mixture was stirred at 0°C overnight. TLC (isopropanol:concentrated aqueous ammonia:water = 50:5:3) indicated that the reaction was complete. The precipitate was removed by filtration. The filtrate was concentrated under reduced pressure, and the residue was purified by C18 column chromatography (methanol:water = 1:3-1:2) to obtain 16 g of compound **69** as a white solid in 84% yield.

**Step 4:** At room temperature, to a 250 mL single-neck reaction flask were added 45 mL of methanol, compound **69** (3.3 g, 0.005 mol), and an aqueous HCl solution (20 mL, 5 M), respectively, and the reaction was carried out for 4 hours. TLC (isopropanol:concentrated aqueous ammonia:water = 50:5:3) indicated that the reaction was complete. The mixture was concentrated under reduced pressure to obtain a crude product of compound **70,** which was used directly in the next step based on a theoretical yield of 2.98 g.

**Step 5:** At room temperature, to a 250 mL reaction flask were added 100 mL of methanol and compound **70** (2.98 g, 0.005 mol), respectively. Sodium methoxide was added to adjust the pH to 7. Under a nitrogen atmosphere, Boc-glycine-N-hydroxysuccinimide ester (BocNHCH₂COOSu, 6.1 g, 0.022 mol) was then added, and the mixture was stirred overnight. The reaction mixture was concentrated under reduced pressure, and then the residue was purified by C18 column chromatography (methanol:water = 1:3-2:3) to obtain 3.1 g of compound **71** as a white solid in 86% yield.

**Step 6:** At room temperature, to a 250 mL single-neck reaction flask were added 40 mL of methanol, compound **71** (3.3 g, 0.005 mol), and an aqueous HCl solution (17 mL, 5 M), respectively, and the reaction was carried out for 4 hours. TLC (isopropanol:concentrated aqueous ammonia:water = 50:5:3) indicated that the reaction was complete. The mixture was concentrated under reduced pressure to obtain a crude product of compound **72,** which was used directly in the next step based on a calculated amount of 2.8 g.

**Step 7:** Preparation of trifluoromethanesulfonyl azide (TfN₃): To a mixture of NaN₃ (3.5 g, 53.2 mmol, 3 eq) in H₂O (25 mL) and DCM (40 mL) (in a 250 mL three-neck reaction flask) was added Tf₂O (3 mL, 17.7 mmol, 1 eq) at -5 to 0°C, and then the mixture was stirred for 2.5 hours. The phases were separated, and the aqueous phase was extracted with DCM (10 mL × 2). The combined DCM extracts were washed with saturated sodium bicarbonate (40 mL) to obtain a DCM solution of TfN₃, wherein TfN₃ was used as charged based on 3.0 g (17.1 mmol, 97%).

To a solution of compound **72** (2.8 g, 0.004 mol), K₂CO₃ (0.65 g, 0.005 mol), and CuSO₄ (6.8 mg, 0.043 mmol) in water (60 mL) and methanol (120 mL) (in a 1.0 L single-neck flask) was added the above-obtained DCM solution of TfN₃ at room temperature, and the reaction was carried out at room temperature overnight. TLC (isopropanol:concentrated aqueous ammonia:water = 25:5:3) indicated that the reaction was complete. The mixture was filtered, and the filtrate was evaporated under reduced pressure to remove methanol and dichloromethane. The product precipitated from the water and was filtered to obtain 3 g of a crude product of compound **73,** which was used directly in the next step (estimated actual content: 80%, yield: 87%).

**Step 8:** At room temperature, to a 100 mL single-neck flask were added 20 mL of DMF and compound **73** (1.5 g, 80 wt%, 0.002 mol), respectively, and the mixture was stirred until dissolved. To the system was added piperidine (2 mL, 0.020 mol), and the reaction was carried out for 2 hours. TLC (isopropanol:concentrated aqueous ammonia:water = 25:5:3) indicated that the reaction was complete. The mixture was concentrated under reduced pressure to obtain a crude product of compound **74,** which was used directly in the next step based on a calculated amount of 0.78 g.

**Step 9:** Under a nitrogen atmosphere and at room temperature, to a 100 mL single-neck flask were added 15 mL of DMF, compound **74** (0.78 g, 1.8 mmol), *N-*hydroxysuccinimide (HOSu, 0.32 g, 2.8 mmol), N₃-PEG₃-COOH (0.68 g, 2.8 mmol), and *N,N*'-dicyclohexylcarbodiimide (DCC, 0.76 g, 3.7 mmol), respectively, and the reaction was carried out overnight. TLC (isopropanol:concentrated aqueous ammonia:water = 25:5:3) indicated that the reaction was complete. The mixture was filtered, and the filtrate was concentrated under reduced pressure. The residue was purified by C18 column chromatography (methanol:water = 1:5-1:2) to obtain 0.63 g of compound **75** as a white solid in 52% yield.

**Step 10:** At 0°C, to a solution of compound **75** (130 mg, 0.20 mmol) in water (8 mL) (in a 100 mL three-neck reaction flask) were added potassium phosphate (K₃PO₄, 634 mg, 2.99 mmol) and 2-chloro-1,3-dimethyl-1H-benzimidazol-3-ium chloride (CDMBI, 216 mg, 0.99 mmol), and then the mixture was stirred at 0°C overnight. TLC (isopropanol:concentrated aqueous ammonia:water = 25:5:3) indicated that the reaction was complete. The reaction mixture was filtered, and the filtrate was directly purified by C18 column chromatography (acetonitrile:water = 1:5-1:2, preparative high-performance liquid chromatograph (Wuhan Ruihe Chromatography Technology, LC 2100)) to obtain 82 mg of compound **G24** as a white solid in 65% yield.

ESI-MS calc. for C₂₃H₃₉N₈O₁₃ [M+H]⁺ m/z = 635.2, found m/z =635.2. ¹H NMR (400 MHz, D₂O) *δ* 6.19 (d, *J =* 7.3 Hz, 1H), 4.44 (dd, *J =* 3.1, 1.6 Hz, 1H), 4.37 (d, *J =* 7.8 Hz, 1H), 4.27 (ddd, *J =* 8.6, 3.1, 1.5 Hz, 1H), 4.24 - 4.11 (m, 2H), 3.85 (dd, *J =* 3.4, 0.9 Hz, 1H), 3.81 - 3.74 (m, 3H), 3.72 - 3.63 (m, 13H), 3.58 (dd, *J* = 9.9, 3.4 Hz, 1H), 3.52 - 3.40 (m, 6H), 2.54 (t, *J =* 6.1 Hz, 2H).

### Example 18: Synthesis of compound G25

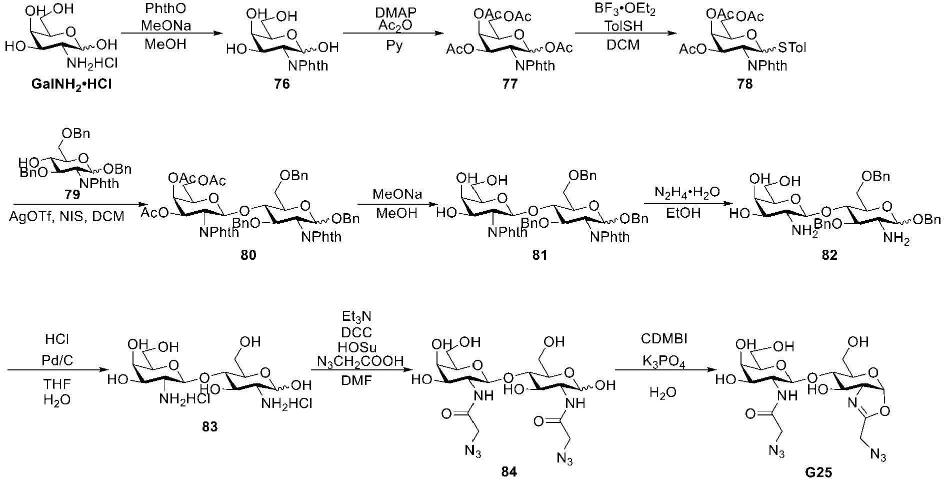

**Step 1:** At room temperature, to a suspension of D-(+)-galactosamine hydrochloride (**GalNH₂.HCl,** 50 g, 0.23 mol) in MeOH (400 mL) (in a 1.0 L single-neck flask) was added MeONa (13.2 g, 0.24 mol), and the mixture was stirred at room temperature for 20 minutes. Then, phthalic anhydride (PhthO, 37.8 g, 0.26 mol) was added, and the mixture was stirred overnight. The reaction mixture was concentrated under reduced pressure to obtain a crude product of compound 76, which was used directly in the next step (calculated based on 71 g for charging).

**Step 2:** To a 2.0 L single-neck reaction flask were added 700 mL of pyridine, compound **76** (71 g, 0.23 mol), and 4-dimethylaminopyridine (DMAP, 1.4 g, 0.01 mol), and the mixture was stirred until dissolved. Then, acetic anhydride (Ac₂O, 195 mL, 2.08 mol) was added to the reaction system, and the mixture was stirred at room temperature overnight. TLC (petroleum ether:ethyl acetate = 1:1) indicated that the reaction was complete. The reaction mixture was concentrated under reduced pressure, and then the residue was purified by silica gel column chromatography (petroleum ether:ethyl acetate = 3:1-2:1) to obtain 59 g of solid compound **77** in 54% yield.

**Step 3:** To a 500 mL single-neck reaction flask were added 180 mL of dichloromethane, compound 77 (59 g, 0.12 mol), 4-toluenethiol (TolSH, 24.5 g, 0.20 mol), and boron trifluoride diethyl etherate (BF₃•Et₂O, 50 mL, 0.40 mol), respectively, and the mixture was stirred at room temperature for 3 hours. TLC (petroleum ether:ethyl acetate = 3:1) indicated that the reaction was complete. The reaction mixture was diluted with dichloromethane (900 mL) and washed with saturated aqueous sodium bicarbonate solution (1200 mL). The organic phase was concentrated under reduced pressure, and then the residue was purified by silica gel column chromatography (petroleum ether:ethyl acetate = 7:1-3:1) to obtain 43 g of compound **78** as a white solid in 64% yield.

**Step 4:** Under a nitrogen atmosphere, to a solution of compound **78** (9.4 g, 17.36 mmol) and compound **79** (10.0 g, 17.25 mmol) in dichloromethane (150 mL) (in a 500 mL three-neck reaction flask) was added dried 4Å molecular sieves (30 g), and the mixture was stirred at room temperature for 2 hours. The temperature was lowered to about -10°C. *N-*Iodosuccinimide (NIS, 7.0 g, 31.11 mmol) and silver trifluoromethanesulfonate (AgOTf, 0.66 g, 2.57 mmol) were added, and then the mixture was warmed to room temperature and stirred overnight. TLC (petroleum ether:ethyl acetate = 3:1) indicated that the reaction was complete. The reaction mixture was filtered, and the filter residue was rinsed with dichloromethane (300 mL). Water (300 mL) was added to the filtrate, and sodium thiosulfate was slowly added with vigorous stirring until the organic phase was decolorized. The organic phase was concentrated under reduced pressure, and the residue was purified by silica gel column chromatography (petroleum ether:ethyl acetate = 3:1-1:1) to obtain 15 g of compound **80** as a colorless gum in 87% yield.

**Step 5:** Under a nitrogen atmosphere and at 0°C, to a solution of compound **80** (15 g, 15.04 mmol) in methanol (300 mL) (in a 1.0 L single-neck reaction flask) was added sodium methoxide (1.5 mL, 5 M in methanol). The reaction was stirred at room temperature for 3 hours. TLC (petroleum ether:ethyl acetate = 1:3) indicated that the reaction was complete. Cation exchange resin (H⁺) was added for neutralization, and the pH was adjusted to 6-7. The mixture was filtered, and the filtrate was concentrated under reduced pressure to obtain 13 g of a crude product of compound **81** as a light yellow solid, which was used directly in the next step without further purification.

**Step 6:** Under a nitrogen atmosphere, hydrazine hydrate (N₂H₄•H₂O, 14.5 mL, 298.91 mmol) was added to a solution of compound **81** (13 g, 14.93 mmol) in ethanol (400 mL) (in a 1.0 L single-neck reaction flask), and the mixture was stirred at 80°C overnight. TLC (dichloromethane:methanol = 10:1) indicated that the reaction was complete. After cooling, the mixture was filtered. The filtrate was concentrated under reduced pressure, and then the residue was purified by C18 column chromatography (methanol:water = 2:3-2:1) to obtain 7.8 g of compound **82** as a white solid in 85% yield.

**Step 7:** To a mixture of compound **82** (2 g, 3.27 mmol) in tetrahydrofuran (240 mL) and water (70 mL) (in a 500 mL single-neck flask) were added Pd/C (2 g, 10 wt%, 1.88 mmol) and 1 M dilute HCl (13 mL). The mixture was charged with H₂ (15 psi) and stirred at room temperature overnight. TLC (isopropanol:concentrated aqueous ammonia:water = 4:2:1) indicated that the reaction was complete. The reaction mixture was filtered, and the filtrate was concentrated under reduced pressure to obtain compound **83** as a white solid, which was used directly in the next step without purification (1.1 g was used in the next step).

**Step 8:** Under a nitrogen atmosphere, to a solution of compound **83** (1.1 g, 2.66 mmol), azidoacetic acid (N₃CH₂COOH, 807 mg, 7.99 mmol), N-hydroxysuccinimide (HOSu, 1.1 g, 9.56 mmol), and triethylamine (3 mL) in DMF (15 mL) (in a 50 mL single-neck flask) was added N,N'-dicyclohexylcarbodiimide (DCC, 2.2 g, 10.66 mmol), and the reaction was carried out at room temperature overnight. TLC (isopropanol:concentrated aqueous ammonia:water = 25:5:3) indicated that the reaction was complete. The mixture was filtered, and the filtrate was concentrated under reduced pressure. The residue was purified by amide column chromatography to obtain 0.8 g of compound **84** as a white solid in 59% yield.

**Step 9:** At 0°C, to a solution of compound **84** (130 mg, 0.26 mmol) in water (10 mL) (in a 100 mL three-neck reaction flask) were added potassium phosphate (K₃PO₄, 817 mg, 3.85 mmol) and 2-chloro-1,3-dimethyl-1H-benzimidazol-3-ium chloride (CDMBI, 278 mg, 1.28 mmol), and then the mixture was stirred at 0°C overnight. TLC (isopropanol:concentrated aqueous ammonia:water = 25:5:3) indicated that the reaction was complete. The precipitate was removed by filtration, and the filtrate was purified by preparative C18 chromatography (acetonitrile:water = 1:20-1:4) to obtain 80 mg of compound **G25** as a white solid in 63% yield.

ESI-MS calc. for C₁₆H₂₅N₈O₁₀ [M+H]⁺ m/z = 489.1, found m/z =489.1. ¹H NMR (400 MHz, D₂O) *δ* 6.18 (d, *J =* 7.3 Hz, 1H), 4.56 (d, *J* = 8.4 Hz, 1H), 4.46 (dd, *J* = 3.1*,* 1.7 Hz, 1H), 4.26 (ddd, *J* = 7.4, 3.2, 1.6 Hz, 1H), 4.17 (dd, *J =* 4.0, 1.8 Hz, 2H), 4.04 (d, *J =* 1.2 Hz, 2H), 3.95 - 3.88 (m, 2H), 3.85 - 3.55 (m, 7H), 3.33 (ddd, *J* = 8.8, 6.4, 2.4 Hz, 1H).

### Example 19: Synthesis of compound G28b

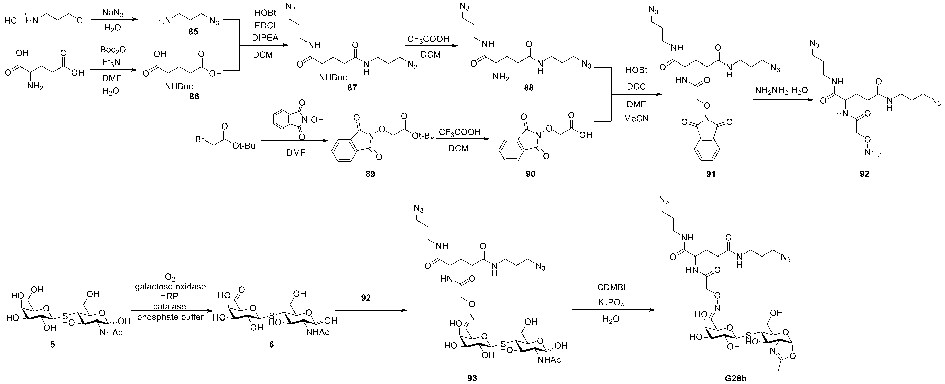

**Step 1:** Under a nitrogen atmosphere, to a solution of 3-chloropropylamine hydrochloride (36 g, 0.28 mol) in water (220 mL) (in a 500 mL reaction flask) was added sodium azide (NaN₃, 54 g, 0.83 mol), and the mixture was stirred at 80°C overnight. Sodium hydroxide (33 g, 0.83 mol) was added, and the mixture was extracted with diethyl ether (200 mL × 5). The combined organic phases were dried over anhydrous sodium sulfate and concentrated under reduced pressure to about 100 mL to obtain compound **85,** which was used directly in the next step (with the next step charged based on 22 g, 79% yield).

**Step 2:** To a mixture of glutamic acid (52 g, 0.19 mol) in DMF (300 mL) and water (200 mL) (in a 1.0 L single-neck reaction flask) was added triethylamine (98 mL), and the reaction was stirred at room temperature for 10 minutes. Then, di-tert-butyl dicarbonate (Boc₂O, 81 g, 0.37 mol) was added, and the reaction was carried out for 2.5 hours. Saturated brine (600 mL) and an aqueous HCl solution (4 M, 160 mL) were added, and the mixture was extracted with ethyl acetate (400 mL × 3). The combined organic phases were dried over anhydrous sodium sulfate and then concentrated under reduced pressure. The residue was slurried with dichloromethane (200 mL) to obtain 85 g of compound **86** as a white powder solid in 97% yield.

**Step 3:** Under a nitrogen atmosphere, to a suspension of compound **85** (10 g, 0.10 mol), compound **86** (14 g, 0.06 mol), and 1-hydroxybenzotriazole (HOBt, 27 g, 0.20 mol) in dichloromethane (200 mL) (in a 500 mL reaction flask) was added EDCI (39 g, 0.20 mol) and N,N-diisopropylethylamine (DIPEA, 84 mL, 0.48 mol), and the mixture was stirred at room temperature overnight. TLC (ethyl acetate:methanol = 1:0) indicated that the reaction was complete. The reaction mixture was concentrated under reduced pressure. To the residue was added saturated sodium bicarbonate (400 mL), and the mixture was extracted with ethyl acetate (200 mL × 3). The combined organic phases were concentrated under reduced pressure, and then the residue was purified by silica gel column chromatography (ethyl acetate:methanol = 1:0-5:1) to obtain 8 g of compound **87** as a light yellow oil in 48% yield.

**Step 4:** To a solution of compound **87** (8 g, 0.02 mol) in dichloromethane (30 mL) (in a 100 mL reaction flask) was added trifluoroacetic acid (14.5 mL), and the reaction was carried out at room temperature for 2 hours. TLC (ethyl acetate:methanol = 10:1) indicated that the reaction was complete. The mixture was concentrated under reduced pressure, and then the residue was purified by C18 column chromatography (methanol:water = 1:10-2:3) to obtain 4.5 g of compound **88** as a colorless oil in 74% yield.

**Step 5:** To a solution of *N*-hydroxyphthalimide (50 g, 0.31 mol) in DMF (30 mL) (in a 2.0 L three-neck reaction flask) were added potassium carbonate (85 g, 0.62 mol) and tert-butyl bromoacetate (47 mL, 0.32 mol), and the mixture was stirred at 60°C for 2 hours. TLC (ethyl acetate:methanol = 10:1) indicated that the reaction was complete. The reaction mixture was cooled to room temperature and then poured into ice water (2 L). The resulting mixture was stirred to precipitate a white solid, which was filtered. The filter cake was washed with water (300 mL) and then slurried with petroleum ether (300 mL) to obtain 60 g of compound **89** as a white solid in 70% yield.

**Step 6:** To a solution of compound **89** (20 g, 0.07 mol) in dichloromethane (80 mL) (in a 500 mL reaction flask) was added trifluoroacetic acid (43 mL), and the mixture was stirred at room temperature for 6 hours. TLC (ethyl acetate:methanol = 10:1) indicated that the reaction was complete. The reaction mixture was concentrated under reduced pressure, and the residue was slurried with ethyl acetate (50 mL) to obtain 12 g of compound **90** as a white solid in 75% yield.

**Step 7:** Under a nitrogen atmosphere, to a mixture of compound **88** (5 g, 16.06 mmol), compound **90** (3.9 g, 17.63 mmol), and 1-hydroxybenzotriazole (HOBt, 2.9 g, 21.46 mmol) in DMF (65 mL) and acetonitrile (65 mL) (in a 500 mL reaction flask) was added *N,N'-*dicyclohexylcarbodiimide (DCC, 4.4 g, 21.33 mmol), and the mixture was stirred at room temperature overnight. TLC (isopropanol:water = 20:1) indicated that the reaction was complete. The white precipitate was removed by filtration, and the filtrate was concentrated under reduced pressure to obtain a crude product of compound **91,** which was used directly in the next step without purification (with the next step charged based on 8.26 g).

**Step 8:** To a solution of compound **91** (8.26 g, 16.05 mmol) in methanol (200 mL) (in a 500 mL reaction flask) was added hydrazine hydrate (5.1 mL, 105.14 mmol), and the reaction was carried out at room temperature for 2 hours. TLC (isopropanol:concentrated aqueous ammonia:water = 20:1:1) indicated that the reaction was complete. The mixture was concentrated under reduced pressure, and the residue was purified by C18 column chromatography (methanol:water = 1:3-1:2) to obtain 6 g of compound **92** as a white solid in 97% yield.

**Step 9:** To a phosphate buffer (5 mL) were sequentially added galactose oxidase (3 mL), horseradish peroxidase (HRP, 50 mg), catalase (1.5 mL), and copper sulfate (CuSO₄, 1 mM, 0.9 mL), and the mixture was activated in a 30°C water bath for 30 minutes. At 30°C, oxygen (O₂) was introduced into a solution of compound **5** (600 mg, 1.50 mmol) in phosphate buffer (25 mL) (in a 100 mL single-neck reaction flask) for 30 minutes, and then the mixed enzyme was added to the reaction system. The reaction was carried out in a 30°C water bath for 6 hours. TLC (isopropanol:concentrated aqueous ammonia:water = 25:5:3) indicated that the reaction was complete, and the introduction of oxygen was stopped. Compound **92** (693 mg, 1.80 mmol) was added, and the reaction was carried out at room temperature overnight. TLC (isopropanol:concentrated aqueous ammonia:water = 25:5:3) indicated that the reaction was complete. Methanol (300 mL) was added. The precipitate was removed by filtration through diatomite, and the precipitate was rinsed with methanol (300 mL). The resulting filtrate was concentrated under reduced pressure, and the residue was purified by C18 column chromatography (methanol:water = 1:10-1:4) to obtain 700 mg of compound **93** as a white powder solid in 61% yield.

**Step 10:** At 0°C, to a solution of compound **93** (170 mg, 0.22 mmol) in water (8 mL) (in a 100 mL three-neck reaction flask) were added potassium phosphate (K₃PO₄, 709 mg, 3.34 mmol) and 2-chloro-1,3-dimethyl-1H-benzimidazol-3-ium chloride (CDMBI, 242 mg, 1.11 mmol), and then the mixture was stirred at 0°C overnight. TLC (isopropanol:concentrated aqueous ammonia:water = 25:5:3) indicated that the reaction was complete. The reaction mixture was filtered, and the filtrate was directly purified by C18 column chromatography (acetonitrile:water = 1:4-7:3) to obtain 112 mg of compound **G28b** as a white solid in 67% yield.

ESI-MS calc. for C₂₇H₄₄N₁₁O₁₂S [M+H]⁺ m/z = 746.2, found m/z =746.2. ¹H NMR (400 MHz, D₂O) *δ* 7.55 (d, *J =* 4.6 Hz, 0.7H), 6.85 (d, *J =* 4.5 Hz, 0.3H), 5.97 (d, *J =* 7.3 Hz, 1H), 4.51 (d, *J* = 5.0 Hz, 2H), 4.44 (d, *J =* 10.1 Hz, 1H), 4.38 (d, *J =* 7.0 Hz, 1H), 4.25 (s, 1H), 4.22 - 4.16 (m, 1H), 4.06 (s, 1H), 4.00 - 3.94 (m, 1H), 3.73 (d, *J* = 11.9 Hz, 1H), 3.61 (d, *J* = 17.7 Hz, 2H), 3.47 (d, *J* = 9.6 Hz, 1H), 3.37 (d, *J* = 7.5 Hz, 1H), 3.24 (d, *J =* 9.5 Hz, 4H), 3.17 (d*, J =* 6.6 Hz, 4H), 2.90 (s, 1H), 2.19 (d, *J* = 6.1 Hz, 2H), 2.04 - 1.96 (m, 1H), 1.92 (s, 3H), 1.84 (dd, *J =* 14.8, 7.6 Hz, 1H), 1.68 - 1.60 (m, 4H).

### Example 20: Synthesis of compound G29

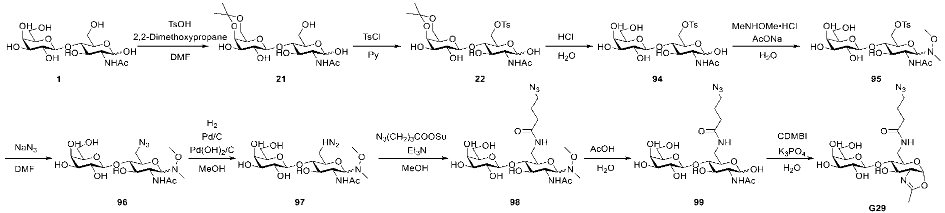

**Step 1:** To a solution of compound **1** (10 g, 26.09 mmol) and TsOH (0.45 g, 2.61 mmol) in DMF (250 mL) (in a 500 mL single-neck reaction flask) was added 2,2-dimethoxypropane (51 mL), and the mixture was stirred at room temperature for 25 minutes. Subsequently, saturated sodium bicarbonate (50 mL) was added to quench the reaction, and the solid was removed by filtration. The filtrate was concentrated under reduced pressure, and the residue was purified by C18 (methanol:water = 1:20) to obtain 4.9 g of compound **21** as a white solid in 44% yield.

**Step 2:** At -20°C, to a solution of compound **21** (4.9 g, 11.57 mmol) in pyridine (200 mL) (in a 500 mL single-neck reaction flask) was added TsCl (4.0 g, 20.98 mmol), and the mixture was stirred overnight. The reaction was quenched by adding 20 mL of methanol, and then concentrated under reduced pressure. The residue was purified by C18 column chromatography (methanol:water = 2:3-1:1) to obtain 2.3 g of compound **22** as a yellow solid in 34% yield.

**Step 3:** Compound **22** (2.3 g, 3.98 mmol) was dissolved in an aqueous solution of HCl (30 mL, 1.5 M) (in a 250 mL single-neck reaction flask), and the mixture was stirred at room temperature for 2 hours. TLC (dichloromethane:methanol = 20:1) indicated that the reaction was complete. The mixture was then concentrated under reduced pressure, and the residue was purified by C18 column chromatography (methanol:water = 1:2-1:1) to obtain 1.8 g of compound **94** as a yellow solid in 86% yield.

**Step 4:** To a solution of compound **94** (2.3 g, 4.28 mmol) in water (15 mL) (in a 100 mL single-neck reaction flask) was added dropwise a solution of *N,O*-dimethylhydroxylamine hydrochloride (MeNHOMe HCl, 1.09 g, 11.17 mmol) and sodium acetate (AcONa, 0.91 g, 11.09 mmol) in water (10 mL), and the reaction was carried out at room temperature overnight. TLC (dichloromethane:methanol = 20:1) indicated that the reaction was complete. The reaction mixture was concentrated under reduced pressure, and the residue was purified by C18 column chromatography (methanol:water = 1:2-1:1) to obtain 1.2 g of compound **95** in 48% yield.

**Step 5:** Under a nitrogen atmosphere, to a solution of compound **95** (1.2 g, 2.07 mmol) in DMF (30 mL) (in a 100 mL three-neck reaction flask) was added NaN₃ (1.1 g, 16.9 mmol), and then the temperature was raised to 60°C and the reaction was carried out overnight. TLC (isopropanol:water:concentrated aqueous ammonia=25:5:3) indicated that the reaction was complete. The reaction mixture was filtered, and the filtrate was concentrated under reduced pressure. The residue was purified by C18 column chromatography (methanol:water = 1:20-1:4) to obtain 0.9 g of compound **96** in 96% yield.

**Step 6:** To a solution of compound **96** (0.9 g, 1.99 mmol) in methanol (20 mL) (in a 100 mL single-neck reaction flask) were added Pd/C (1 g, 5 wt%, 0.47 mmol) and Pd(OH)₂/C (1 g, 20 wt%, 1.42 mmol). The reaction mixture was charged with hydrogen (15 psi) and stirred at room temperature overnight. The reaction mixture was filtered, and the filtrate was concentrated under reduced pressure. The residue was used directly in the next step (calculated based on 0.8 g, 94%).

**Step 7:** To a solution of compound **97** (0.8 g, 1.88 mmol) in methanol (20 mL) (in a 100 mL single-neck reaction flask) were added N₃(CH₂)₃COOSu (1.28 g, 5.66 mmol) and Et₃N (0.4 mL), and the mixture was stirred at room temperature overnight. The reaction mixture was concentrated under reduced pressure, and the residue was purified by C18 column chromatography (methanol:water = 1:20-1:4) to obtain 0.41 g of compound **98** in 40% yield.

**Step 8:** Compound **98** (0.41 g, 0.76 mmol) was dissolved in acetic acid (60 mL) and water (15 mL), and the reaction was carried out at 60°C for 4 hours. TLC (*i*-PrOH:NH₃ H₂O:H₂O = 25:5:3) indicated that the reaction was complete. The reaction mixture was concentrated under reduced pressure, and the residue was purified by C18 column chromatography (methanol:water = 1:4-3:7) to obtain 0.34 g of compound **99** as a white solid in 90% yield.

**Step 9:** At 0°C, to a solution of compound **99** (200 mg, 0.41 mmol) in water (13 mL) (in a 100 mL three-neck reaction flask) were added potassium phosphate (K₃PO₄, 1.29 g, 6.08 mmol) and 2-chloro-1,3-dimethyl-1H-benzimidazol-3-ium chloride (CDMBI, 440 mg, 2.03 mmol), and then the mixture was stirred at 0°C overnight. TLC (isopropanol:concentrated aqueous ammonia:water = 25:5:3) indicated that the reaction was complete. The reaction mixture was filtered, and the filtrate was directly subjected to preparative C18 chromatography (acetonitrile:water = 1:5-2:3, preparative high-performance liquid chromatograph (Wuhan Ruihe Chromatography Technology, LC 2100)) to obtain 140 mg of compound **G29** as a white solid in 72% yield.

ESI-MS calc. for C₁₈H₃₀N₅O₁₀ [M+H]⁺ m/z = 476.2, found m/z = 476.2. ¹H NMR (400 MHz, D₂O) *δ* 6.09 (d, *J =* 7.3 Hz, 1H), 4.48 (d, *J =* 7.8 Hz, 1H), 4.43 (dd, *J* = 3.0, 1.5 Hz, 1H), 4.21 (dq, *J =* 6.7, 1.7 Hz, 1H), 3.96 - 3.90 (m, 1H), 3.87 - 3.62 (m, 5H), 3.61 - 3.35 (m, 6H), 2.39 (t, *J* = 7.2 Hz, 2H), 2.07 (d, *J =* 1.8 Hz, 3H), 1.91 (q, *J* = 7.0 Hz, 2H).

### Example 21: Synthesis of compound G32

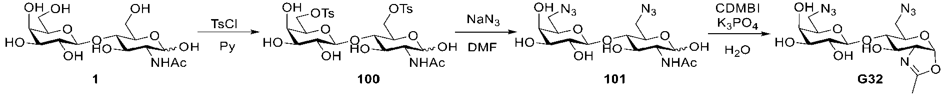

**Step 1:** At 25°C, to a solution of compound 1 (3.0 g, 7.83 mmol) in pyridine (30 mL) (in a 100 mL single-neck reaction flask) was added TsCl (3.7 g, 19.41 mmol), and the mixture was stirred for 3 hours. TLC (acetonitrile:water = 10:1) indicated that the reaction was complete. The mixture was concentrated under reduced pressure, and the residue was purified by silica gel column chromatography (dichloromethane:methanol = 50:1-10:1) to obtain 2.2 g of compound **100** as a yellow solid in 40% yield.

**Step 2:** Under a nitrogen atmosphere, to a solution of compound **100** (2.2 g, 3.18 mmol) in DMF (15 mL) (in a 100 mL three-neck reaction flask) was added NaN₃ (1.03 g, 15.8 mmol), then the temperature was raised to 80°C, and the reaction was stirred for 12 hours. The reaction mixture was cooled to room temperature, filtered, and the filtrate was concentrated under reduced pressure. The residue was subjected to preparative C18 chromatography (acetonitrile:water = 1:10-1:2, preparative high-performance liquid chromatograph (Wuhan Ruihe Chromatography Technology, LC 2100)) to obtain 160 mg of compound **101** as a white solid in 11% yield.

**Step 3:** At 0°C, to a solution of compound **101** (160 mg, 0.37 mmol) in water (12 mL) (in a 100 mL three-neck reaction flask) were added potassium phosphate (K₃PO₄, 1.18 g, 5.56 mmol) and 2-chloro-1,3-dimethyl-1H-benzimidazol-3-ium chloride (CDMBI, 400 mg, 1.84 mmol), and then the mixture was stirred at 0°C overnight. TLC (isopropanol:concentrated aqueous ammonia:water = 25:5:3) indicated that the reaction was complete. Impurities were extracted with toluene (20 mL × 4), and the aqueous layer was directly subjected to preparative C18 chromatography (acetonitrile:water = 1:10-1:1, preparative high-performance liquid chromatograph (Wuhan Ruihe Chromatography Technology, LC 2100)) to obtain 120 mg of compound **G32** as a white solid in 78% yield.

ESI-MS calc. for C₁₄H₂₂N₇O₈ [M+H]⁺ m/z = 416.1, found m/z = 416.1. ¹H NMR (400 MHz, D₂O) *δ* 5.96 (d, *J* = 7.3 Hz, 1H), 4.40 - 4.23 (m, 2H), 4.07 (d, *J =* 8.1 Hz, 1H), 3.74 (s, 1H), 3.71 - 3.61 (m, 2H), 3.57 - 3.42 (m, 4H), 3.43 - 3.33 (m, 2H), 3.29 (dd, *J =* 13.2, 3.7 Hz, 1H), 1.93 (s, 3H).

### Example 22: Synthesis of compound G33

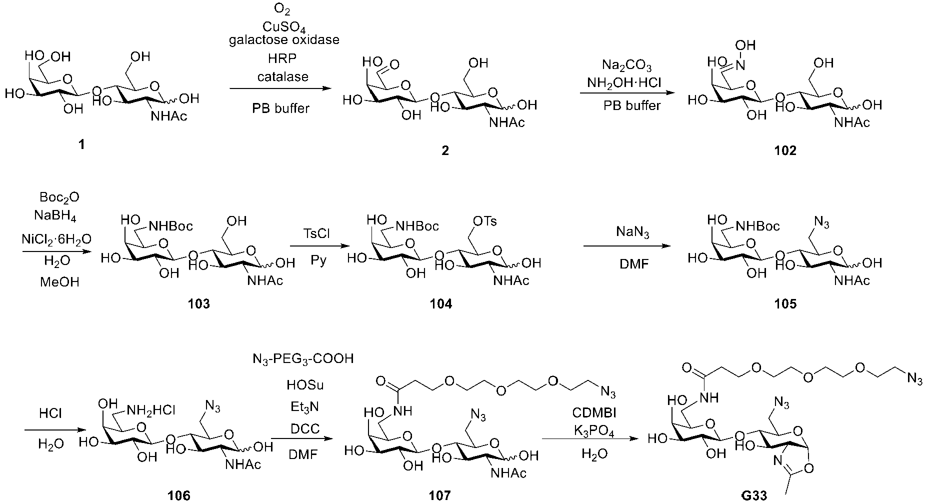

**Steps 1 and 2:** To a phosphate buffer (25 mL) were sequentially added galactose oxidase (12.5 mL), horseradish peroxidase (HRP, 210 mg), catalase (6.25 mL), and copper sulfate (CuSO₄, 1 mM, 4 mL) aqueous solution, and the mixture was activated in a 30°C water bath for 30 minutes. At 30°C, oxygen (O₂) was introduced into a solution of compound 1 (5 g, 13.04 mmol) in phosphate buffer (125 mL) (in a 2.0 L single-neck reaction flask) for 30 minutes, and then the mixed enzyme was added to the reaction system. The reaction was carried out in a 30°C water bath for 6 hours. TLC (isopropanol:concentrated aqueous ammonia:water = 25:5:3) indicated that the reaction was complete, and the introduction of oxygen was stopped. Then, to the reaction mixture were added Na₂CO₃ (691 mg, 6.52 mmol) and NH₂OH HCl (997 mg, 14.35 mmol), and the mixture was stirred at room temperature for 3 hours. TLC (isopropanol:concentrated aqueous ammonia:water = 25:5:3) indicated that the reaction was complete. Methanol (300 mL) was added. The precipitate was removed by filtration through diatomite, and the precipitate was rinsed with methanol (300 mL). The resulting filtrate was concentrated under reduced pressure, and the residue was purified by silica gel column chromatography (dichloromethane:methanol = 6:1-3:1) to obtain 4.0 g of compound **102** in 77% yield.

**Step 3:** At 0°C, to a mixture of compound **102** (5.5 g, 13.88 mmol) and NiCl₂ 6H₂O (8.25 g, 34.71 mmol) in methanol (160 mL) and phosphate buffer (PB, 80 mL) (in a 500 mL single-neck reaction flask) were added NaBH₄ (3.15 g, 83.27 mmol) and Boc₂O (9.09 g, 41.65 mmol), and the mixture was stirred at 0°C overnight. TLC (isopropanol:concentrated aqueous ammonia:water = 25:5:3) indicated that the reaction was complete. The precipitate was removed by filtration. The filtrate was concentrated under reduced pressure, and the residue was purified by C18 column chromatography (methanol:water = 1:5-1:2) to obtain 3.2 g of compound **103** as a white solid in 48% yield.

**Step 4:** At -20°C, to a solution of compound **103** (1.6 g, 3.32 mmol) in pyridine (32 mL) (in a 100 mL three-neck reaction flask) was added TsCl (0.7 g, 3.67 mmol) , and the mixture was stirred overnight. The mixture was concentrated under reduced pressure, and the residue was purified by C18 column chromatography (methanol:water = 1:4-3:1) to obtain 0.6 g of compound **104** in 28% yield.

**Step 5:** Under a nitrogen atmosphere, to a solution of compound **104** (0.6 g, 0.94 mmol) in DMF (20 mL) (in a 100 mL three-neck reaction flask) was added NaN₃ (0.61 g, 9.38 mmol), then the temperature was raised to 60°C, and the reaction was stirred for 24 hours. The reaction mixture was cooled to room temperature and filtered. The filtrate was concentrated under reduced pressure, and the residue was purified by C18 column chromatography (methanol:water = 1:3-1:2) to obtain 0.4 g of compound **105** as a white solid in 84% yield.

**Step 6:** Compound **105** (0.4 g, 0.79 mmol) was dissolved in dilute HCl (5 mL, 1.5 M) (in a 25 mL single-neck reaction flask), and the mixture was stirred at room temperature for 2 hours. The mixture was then concentrated under reduced pressure to obtain a crude product of compound **106,** which was used directly in the next step (calculated based on a yield of 300 mg).

**Step 7:** Under a nitrogen atmosphere and at room temperature, to a solution of compound **106** (300 mg, 0.68 mmol), HOSu (140 mg, 1.22 mmol), N₃-PEG₃-COOH (250 mg, 1.01 mmol), and Et₃N (0.28 mL, 2.01 mmol) in DMF (5 mL) (in a 25 mL single-neck reaction flask) was added DCC (314 mg, 1.52 mmol), and the reaction was carried out overnight. TLC (isopropanol:concentrated aqueous ammonia:water = 25:5:3) indicated that the reaction was complete. The mixture was filtered, and the filtrate was concentrated under reduced pressure. The residue was purified by C18 column chromatography (methanol:water = 1:4-3:7) to obtain 0.23 g of compound **107** as a white solid in 55% yield.

**Step 8:** At 0°C, to a solution of compound **107** (230 mg, 0.36 mmol) in water (10 mL) (in a 50 mL three-neck reaction flask) were added potassium phosphate (K₃PO₄, 1150 mg, 5.42 mmol) and 2-chloro-1,3-dimethyl-1H-benzimidazol-3-ium chloride (CDMBI, 392 mg, 1.81 mmol), and then the mixture was stirred at 0°C overnight. TLC (isopropanol:concentrated aqueous ammonia:water = 25:5:3) indicated that the reaction was complete. The reaction mixture was filtered, and the filtrate was directly purified by C18 column chromatography (acetonitrile:water = 1:4-1:1, preparative high-performance liquid chromatograph (Wuhan Ruihe Chromatography Technology, LC 2100)) to obtain 80 mg of compound G33 as a white solid in 36% yield.

ESI-MS calc. for C₂₃H₃₉N₈O₁₂ [M+H]⁺ m/z = 619.2, found m/z =619.2. ¹H NMR (400 MHz, D₂O) *δ* 6.11 (d, *J =* 7.3 Hz, 1H), 4.43 - 4.36 (m, 2H), 4.22 (dq, *J* = 6.7, 1.6 Hz, 1H), 3.91 - 3.87 (m, 1H), 3.80 (t, *J =* 6.2 Hz, 2H), 3.77 - 3.54 (m, 15H), 3.54 - 3.40 (m, 6H), 2.57 (t, *J* = 6.2 Hz, 2H), 2.07 (d, *J* = 1.8 Hz, 3H).

### Example 23: Synthesis of compound G35

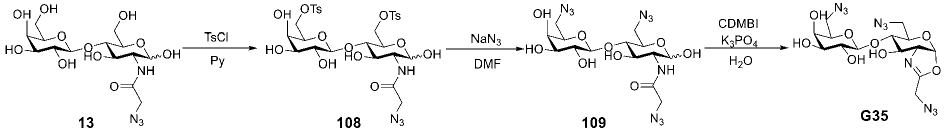

**Step 1:** At -15°C, to a solution of compound **13** (2.0 g, 4.71 mmol) in pyridine (60 mL) (in a 100 mL three-neck reaction flask) was added TsCl (2.7 g, 14.16 mmol), and the mixture was stirred for 13 hours. TLC (acetonitrile:water = 10:1) indicated that the reaction was complete. The mixture was diluted with ethyl acetate (200 mL), followed by the addition of saturated aqueous sodium bicarbonate solution (100 mL). The phases were separated, and the organic phase was washed with saturated brine (150 mL × 3), dried over anhydrous sodium sulfate, then filtered, and concentrated under reduced pressure to obtain 2.8 g of compound **108** as a yellow solid in 81% yield.

**Step 2:** To a solution of compound **108** (2.8 g, 3.82 mmol) in DMF (50 mL) (in a 100 mL three-neck reaction flask) was added sodium azide (3.73 g, 57.38 mmol), and the mixture was stirred at 60°C for 40 hours. TLC (isopropanol:concentrated aqueous ammonia:water = 25:5:3) indicated that the reaction was complete. The mixture was concentrated under reduced pressure, and the residue was purified by C18 column chromatography (acetonitrile:water = 1:10-3:7) to obtain 1.8 g of a crude product of compound **109** as a white solid.

**Step 3:** At 0°C, to a solution of compound **109** (0.35 g, 0.74 mmol) in water (15 mL) (in a 100 mL three-neck reaction flask) were added potassium phosphate (K₃PO₄, 2.35 g, 11.07 mmol) and 2-chloro-1,3-dimethyl-1H-benzimidazol-3-ium chloride (CDMBI, 0.8 g, 3.68 mmol), and then the mixture was stirred at 0°C overnight. TLC (isopropanol:concentrated aqueous ammonia:water = 25:5:3) indicated that the reaction was complete. Impurities were extracted with toluene (20 mL × 4), and the aqueous layer was directly subjected to preparative C18 chromatography (acetonitrile:water = 1:10-1:3, preparative high-performance liquid chromatograph (Wuhan Ruihe Chromatography Technology, LC 2100)) to obtain 166 mg of compound **G35** as a white solid in 49% yield.

ESI-MS calc. for C₁₄H₂₁N₁₀O₈ [M+H]⁺ m/z = 457.1, found m/z = 457.1. ¹H NMR (400 MHz, D₂O) *δ* 6.07 (d, *J =* 7.3 Hz, 1H), 4.40 - 4.25 (m, 2H), 4.20 - 4.11 (m, 1H), 4.10 - 3.92 (m, 1H), 3.72 (d, *J* = 2.8 Hz, 1H), 3.65 (d, *J =* 8.3 Hz, 2H), 3.57 - 3.20 (m, 8H).

### Example 24: Synthesis of compound G36

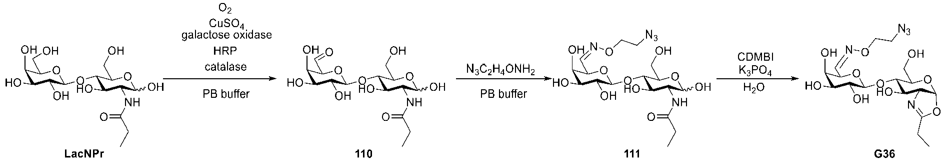

**Steps 1 and 2:** To a phosphate buffer (8 mL) were sequentially added galactose oxidase (4 mL), copper sulfate (CuSO₄, 1 mM, 1.2 mL), horseradish peroxidase (HRP, 66 mg), and catalase (2 mL), and the mixture was activated in a 30°C water bath for 30 minutes. At 30°C, oxygen (O₂) was introduced into a solution of compound **LacNPr** (1.6 g, 4.03 mmol) in phosphate buffer (40 mL) (in a 1.0 L single-neck reaction flask) for 30 minutes, and then the mixed enzyme was added to the reaction system. The reaction was carried out in a 30°C water bath for 6 hours. TLC (isopropanol:concentrated aqueous ammonia:water = 25:5:3) indicated that the reaction was complete. The introduction of oxygen was stopped, and the reaction mixture was used directly in the next step without further treatment. N₃C₂H₄ONH₂ (0.62 g, 6.07 mmol) was added, and the reaction was carried out at room temperature for 14 hours. TLC (isopropanol:concentrated aqueous ammonia:water = 25:5:3) indicated that the reaction was complete. The mixture was concentrated under reduced pressure. The residue was redissolved in 40 mL of methanol, filtered, and the filtrate was concentrated under reduced pressure. The residue was subjected to preparative C18 chromatography (acetonitrile:water = 1:20-1:3, preparative high-performance liquid chromatograph (Wuhan Ruihe Chromatography Technology, LC 2100)) to obtain 0.84 g of compound 111 as a white solid in 43% yield.

**Step 3:** At 0°C, to a solution of compound **111** (840 mg, 1.75 mmol) in water (30 mL) (in a 100 mL three-neck flask) were added potassium phosphate (K₃PO₄, 5.58 g, 26.29 mmol) and 2-chloro-1,3-dimethyl-1H-benzimidazol-3-ium chloride (CDMBI, 1.9 g, 8.75 mmol), and then the mixture was stirred at 0°C overnight. TLC (isopropanol:concentrated aqueous ammonia:water = 25:5:3) indicated that the reaction was complete. The reaction mixture was filtered, and the filtrate was directly subjected to preparative high-performance liquid chromatography (methanol:water = 1:20-1:4, Wuhan Ruihe Chromatography Technology, LC 2100) to obtain 417 mg of compound **G36** as a white solid in 51% yield.

ESI-MS calc. for C₁₇H₂₈N₅O₁₀ [M+H]⁺ m/z = 462.2, found m/z = 462.2. ¹H NMR (400 MHz, D₂O) *δ* 7.51 (d, *J* = 4.8 Hz, 0.7H), 6.85 (d, *J =* 5.9 Hz, 0.3H), 5.95 (d, *J =* 7.3 Hz, 1H), 4.40 - 4.32 (m, 1H), 4.32 - 4.22 (m, 2H), 4.19 - 4.17 (m, 0.3H), 4.17 - 4.11 (m, 2H), 4.04 (d, *J* = 4.1 Hz, 1H), 3.91 (d, *J* = 3.3 Hz, 0.7H), 3.68 (d, *J =* 12.3 Hz, 1H), 3.54 (t, *J* = 7.9 Hz, 3H), 3.43 (dd, *J =* 12.0, 6.3 Hz, 3H), 3.32 (d, *J=* 6.4 Hz, 1H), 2.31 - 2.23 (m, 2H), 1.03 (t, *J =* 8.2 Hz, 3H).

### Example 25: Synthesis of compound G37

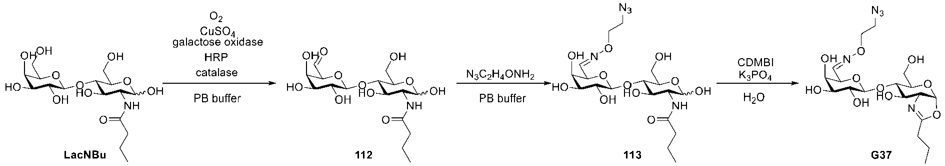

**Steps 1 and 2:** To a phosphate buffer (8 mL) were sequentially added galactose oxidase (4 mL), copper sulfate (CuSO₄, 1 mM, 1.2 mL), horseradish peroxidase (HRP, 66 mg), and catalase (2 mL), and the mixture was activated in a 30°C water bath for 30 minutes. At 30°C, oxygen (O₂) was introduced into a solution of compound **LacNBu** (1.6 g, 3.89 mmol) in phosphate buffer (40 mL) (in a 1.0 L single-neck reaction flask) for 30 minutes, and then the mixed enzyme was added to the reaction system. The reaction was carried out in a 30°C water bath for 5 hours. TLC (isopropanol:concentrated aqueous ammonia:water = 25:5:3) indicated that the reaction was complete, and the introduction of oxygen was stopped. To the reaction mixture was added N₃C₂H₄ONH₂ (0.6 g, 5.88 mmol), and the mixture was stirred at room temperature for 14 hours. TLC (isopropanol:concentrated aqueous ammonia:water = 25:5:3) indicated that the reaction was complete. The mixture was concentrated under reduced pressure, and the residue was redissolved in methanol (40 mL) and filtered. The filtrate was concentrated under reduced pressure, and the residue was subjected to preparative C18 chromatography (acetonitrile:water = 1:20-1:3, preparative high-performance liquid chromatograph (Wuhan Ruihe Chromatography Technology, LC 2100)) to obtain 0.6 g of compound **113** as a white solid in 31% yield.

**Step 3:** At 0°C, to a solution of compound **113** (0.5 g, 1.01 mmol) in water (40 mL) (in a 100 mL three-neck flask) were added potassium phosphate (K₃PO₄, 3.2 g, 15.08 mmol) and 2-chloro-1,3-dimethyl-1H-benzimidazol-3-ium chloride (CDMBI, 1.1 g, 5.07 mmol), and then the mixture was stirred at 0°C overnight. TLC (isopropanol:concentrated aqueous ammonia:water = 25:5:3) indicated that the reaction was complete. The reaction mixture was filtered, and the filtrate was directly purified by C18 column chromatography (acetonitrile:water = 1:10-1:3, preparative high-performance liquid chromatograph (methanol:water = 1:20-1:4, Wuhan Ruihe Chromatography Technology, LC 2100)) to obtain 400 mg of the white solid compound **G37** in 83% yield.

ESI-MS calc. for C₁₈H₃₀N₅O₁₀ [M+H]⁺ m/z = 476.2, found m/z =476.2. ¹H NMR (400 MHz, Deuterium Oxide) *δ* 7.67 (d, *J =* 4.8 Hz, 0.7H), 7.01 (d, *J =* 4.6 Hz, 0.3H), 6.11 (d, *J =* 7.3 Hz, 1H), 4.85 (dd, *J* = 4.6, 1.2 Hz, 0.3H), 4.52 (d, *J =* 7.8 Hz, 0.7H), 4.50 - 4.39 (m, 2H), 4.38 - 4.26 (m, 2.3H), 4.22 (ddd, *J* = 6.6, 3.0, 1.5 Hz, 1H), 4.07 (d, *J =* 3.2 Hz, 0.7H), 3.87 - 3.80 (m, 1H), 3.71 (td, *J =* 10.5, 5.8 Hz, 3H), 3.65 - 3.53 (m, 3H), 3.51 - 3.44 (m, 1H), 2.39 (t, *J =* 7.4 Hz, 2H), 1.68 (h, *J =* 7.5 Hz, 2H), 0.98 (t, *J =* 7.4 Hz, 3H).

### Example 26: Synthesis of compound G38

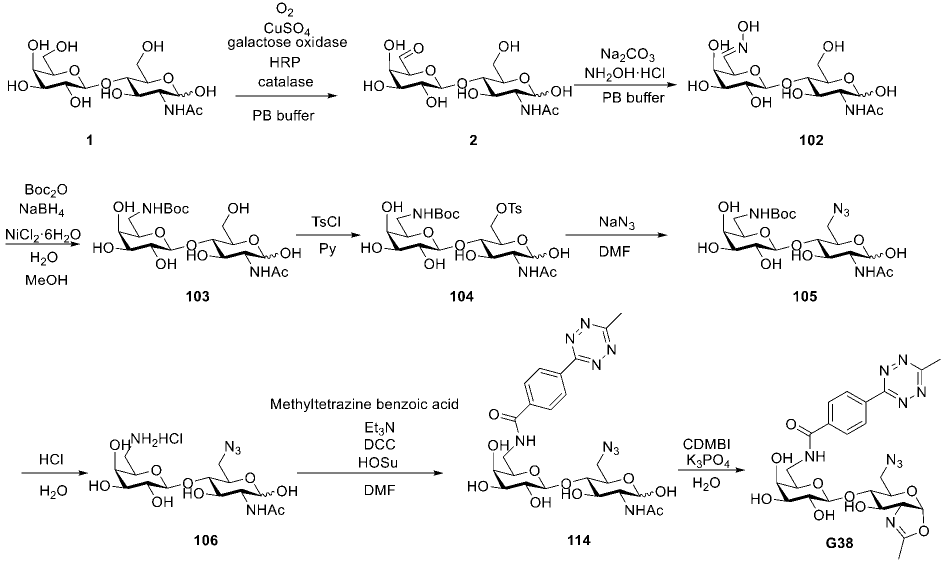

**Steps 1 and 2:** To a phosphate buffer (25 mL) were sequentially added galactose oxidase (12.5 mL), horseradish peroxidase (HRP, 210 mg), catalase (6.25 mL), and copper sulfate (CuSO₄, 1 mM, 4 mL), and the mixture was activated in a 30°C water bath for 30 minutes. At 30°C, oxygen (O₂) was introduced into a solution of compound 1 (5 g, 13.04 mmol) in phosphate buffer (125 mL) (in a 500 mL three-neck reaction flask) for 30 minutes, and then the mixed enzyme was added to the reaction system. The reaction was carried out in a 30°C water bath for 6 hours. TLC (isopropanol:concentrated aqueous ammonia:water = 25:5:3) indicated that the reaction was complete, and the introduction of oxygen was stopped. To the reaction mixture were added Na₂CO₃ (691 mg, 6.52 mmol) and NH₂OH HCl (997 mg, 14.35 mmol), and the reaction was carried out at room temperature for 3 hours. TLC (isopropanol:concentrated aqueous ammonia:water = 25:5:3) indicated that the reaction was complete. Methanol (300 mL) was added. The precipitate was removed by filtration through diatomite, and the precipitate was rinsed with methanol (300 mL). The resulting filtrate was concentrated under reduced pressure, and the residue was purified by silica gel column chromatography (dichloromethane:methanol = 6:1-3:1) to obtain 4.0 g of compound **102** in 77% yield.

**Step 3:** At 0°C, to a mixture of compound **102** (5.5 g, 13.88 mmol) and NiCl₂ 6H₂O (8.25 g, 34.71 mmol) in MeOH (160 mL) and phosphate buffer (PB, 80 mL) (in a 500 mL single-neck reaction flask) were added NaBH₄ (3.15 g, 83.27 mmol) and Boc₂O (9.09 g, 41.65 mmol), and the mixture was stirred at 0°C overnight. TLC (isopropanol:concentrated aqueous ammonia:water = 25:5:3) indicated that the reaction was complete. The precipitate was removed by filtration. The filtrate was concentrated under reduced pressure, and the residue was purified by C18 column chromatography (methanol:water = 1:5-1:2) to obtain 3.2 g of compound **103** as a white solid in 48% yield.

**Step 4:** At -20°C, to a solution of compound **103** (2.5 g, 5.18 mmol) in pyridine (50 mL) (in a 250 mL single-neck reaction flask) was added TsCl (1.83 g, 9.60 mmol), and the mixture was stirred overnight. The mixture was concentrated under reduced pressure, and the residue was purified by C18 column chromatography (methanol:water = 1:4-3:1) to obtain 1.83 g of compound **104** in 55% yield.

**Step 5:** Under a nitrogen atmosphere, to a solution of compound **104** (1.83 g, 2.87 mmol) in DMF (40 mL) (in a 100 mL three-neck reaction flask) was added NaN₃ (1.87 g, 28.76 mmol), then the temperature was raised to 60°C, and the reaction was stirred for 48 hours. The reaction mixture was filtered. The filtrate was concentrated under reduced pressure, and the residue was purified by C18 column chromatography (methanol:water = 1:4-1:2) to obtain 1.35 g of compound **105** as a white solid in 92% yield.

**Step 6:** Compound **105** (0.5 g, 0.99 mmol) was dissolved in dilute HCl (6.6 mL, 1.5 M) (in a 25 mL single-neck reaction flask), and the mixture was stirred at room temperature for 2 hours. The mixture was then concentrated under reduced pressure to obtain a crude product of compound **106,** which was used directly in the next step (calculated based on a product amount of 350 mg and a yield of 80%).

**Step 7:** Under a nitrogen atmosphere and at room temperature, to a solution of compound **106** (350 mg, 0.79 mmol), HOSu (163 mg, 1.42 mmol), 4-(6-methyl-1,2,4,5-tetrazin-3-yl)benzoic acid (239 mg, 1.11 mmol), and Et₃N (0.4 mL, 2.88 mmol) in DMF (10 mL) (in a 25 mL single-neck flask) was added DCC (407 mg, 1.97 mmol), and the mixture was stirred overnight. TLC (isopropanol:concentrated aqueous ammonia:water = 25:5:3) indicated that the reaction was complete. The mixture was filtered, and the filtrate was concentrated under reduced pressure. The residue was purified by C18 column chromatography (methanol:water = 1:4-1:1) to obtain 0.25 g of compound **114** as a red solid in 52% yield.

**Step 8:** At 0°C, to a solution of compound **114** (250 mg, 0.41 mmol) in water (40 mL) and acetonitrile (20 mL) (in a 250 mL three-neck reaction flask) were added potassium phosphate (K₃PO₄, 1310 mg, 6.17 mmol) and 2-chloro-1,3-dimethyl-1H-benzimidazol-3-ium chloride (CDMBI, 448 mg, 2.06 mmol), and then the mixture was stirred at 0°C overnight. TLC (isopropanol:concentrated aqueous ammonia:water = 25:5:3) indicated that the reaction was complete. The reaction mixture was extracted with toluene (30 mL × 4), and the aqueous phase was directly purified by C18 column chromatography (acetonitrile:water = 1:4-1:1, preparative high-performance liquid chromatograph (Wuhan Ruihe Chromatography Technology, LC 2100)) to obtain 80 mg of compound **G38** as a red solid in 33% yield.

ESI-MS calc. for C₂₄H₃₀N₉O₉ [M+H]⁺ m/z = 588.2, found m/z =588.2. ¹H NMR (400 MHz, D₂O) *δ* 8.59 - 8.47 (m, 2H), 8.09 - 7.98 (m, 2H), 6.05 (d, *J =* 7.3 Hz, 1H), 4.44 (d, *J =* 7.8 Hz, 1H), 4.40 (dd, *J =* 3.1, 1.7 Hz, 1H), 4.14 (ddt, *J =* 7.0, 3.3, 1.7 Hz, 1H), 4.00 (d, *J* = 3.5 Hz, 1H), 3.92 (t, *J =* 6.6 Hz, 1H), 3.78 (dt, *J* = 8.7*,* 1.6 Hz, 1H), 3.72 (d, *J =* 6.5 Hz, 2H), 3.68 (dd, *J* = 10.0, 3.4 Hz, 1H), 3.62 (dd, *J =* 13.2, 2.6 Hz, 1H), 3.56 (ddd, *J* = 10.0, 6.8, 4.1 Hz, 2H), 3.46 (dd, *J* = 13.2, 6.1 Hz, 1H), 3.11 (s, 3H), 2.07 (d, *J =* 1.8 Hz, 3H).

### Example 27: Synthesis of compound G39

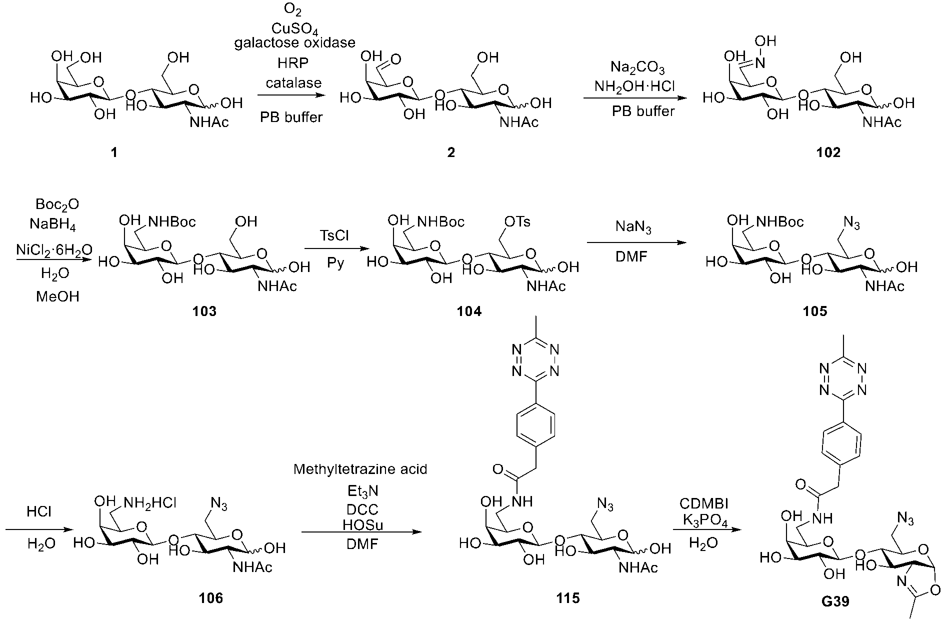

**Steps 1 and 2:** To a phosphate buffer (25 mL) were sequentially added galactose oxidase (12.5 mL), horseradish peroxidase (HRP, 210 mg), catalase (6.25 mL), and copper sulfate (CuSO₄, 1 mM, 4 mL), and the mixture was activated in a 30°C water bath for 30 minutes. At 30°C, oxygen (O₂) was introduced into a solution of compound 1 (5 g, 13.04 mmol) in phosphate buffer (125 mL) (in a 500 mL single-neck reaction flask) for 30 minutes, and then the mixed enzyme was added to the reaction system. The reaction was stirred in a 30°C water bath for 6 hours. TLC (isopropanol:concentrated aqueous ammonia:water = 25:5:3) indicated that the reaction was complete, and the introduction of oxygen was stopped. Na₂CO₃ (691 mg, 6.52 mmol) and NH₂OH·HCl (997 mg, 14.35 mmol) were added, and the reaction was carried out at room temperature for 3 hours. TLC (isopropanol:concentrated aqueous ammonia:water = 25:5:3) indicated that the reaction was complete. Methanol (300 mL) was added. The precipitate was removed by filtration through diatomite, and the precipitate was rinsed with methanol (300 mL). The resulting filtrate was concentrated under reduced pressure, and the residue was purified by silica gel column chromatography (dichloromethane:methanol = 6:1-3:1) to obtain 4.0 g of compound **102** in 77% yield.

**Step 3:** At 0°C, to a mixture of compound **102** (5.5 g, 13.88 mmol) and NiCl₂ 6H₂O (8.25 g, 34.71 mmol) in MeOH (160 mL) and phosphate buffer (PB, 80 mL) (in a 500 mL single-neck reaction flask) were added NaBH₄ (3.15 g, 83.27 mmol) and Boc₂O (9.09 g, 41.65 mmol), and the mixture was stirred at 0°C overnight. TLC (isopropanol:concentrated aqueous ammonia:water = 25:5:3) indicated that the reaction was complete. The precipitate was removed by filtration. The filtrate was concentrated under reduced pressure, and the residue was purified by C18 column chromatography (methanol:water = 1:5-1:2) to obtain 3.2 g of compound **103** as a white solid in 48% yield.

**Step 4:** At -20°C, to a solution of compound **103** (2.5 g, 5.18 mmol) in pyridine (50 mL) (in a 250 mL single-neck reaction flask) was added TsCl (1.83 g, 9.60 mmol), and the mixture was stirred overnight. The mixture was concentrated under reduced pressure, and the residue was purified by C18 column chromatography (methanol:water = 1:4-3:1) to obtain 1.83 g of compound **104** in 55% yield.

**Step 5:** Under a nitrogen atmosphere, to a solution of compound **104** (1.83 g, 2.87 mmol) in DMF (40 mL) (in a 100 mL three-neck reaction flask) was added NaN₃ (1.87 g, 28.76 mmol), then the temperature was raised to 60°C, and the reaction was stirred for 48 hours. The reaction mixture was filtered. The filtrate was concentrated under reduced pressure, and the residue was purified by C18 column chromatography (methanol:water = 1:4-1:2) to obtain 1.35 g of compound **105** as a white solid in 92% yield.

**Step 6:** Compound **105** (0.5 g, 0.99 mmol) was dissolved in dilute HCl (6.6 mL, 1.5 M) (in a 25 mL single-neck reaction flask), and the mixture was stirred at room temperature for 2 hours. The mixture was then concentrated under reduced pressure to obtain a crude product of compound **106,** which was used directly in the next step (calculated based on a product amount of 350 mg and a yield of 80%).

**Step 7:** Under a nitrogen atmosphere and at room temperature, to a solution of compound **106** (500 mg, 1.13 mmol), HOSu (233 mg, 2.02 mmol), 2-(4-(6-methyl-1,2,4,5-tetrazin-3-yl)phenyl)acetic acid (337 mg, 1.46 mmol), and Et₃N (0.5 mL, 3.60 mmol) in DMF (10 mL) (in a 50 mL single-neck flask) was added DCC (581 mg, 2.82 mmol), and the mixture was stirred overnight. TLC (isopropanol:concentrated aqueous ammonia:water = 25:5:3) indicated that the reaction was complete. The mixture was filtered, and the filtrate was concentrated under reduced pressure. The residue was purified by C18 column chromatography (methanol:water = 1:4-1:1) to obtain 0.35 g of compound **115** as a red solid in 50% yield.

**Step 8:** At 0°C, to a solution of compound **115** (150 mg, 0.24 mmol) in water (50 mL) (in a 100 mL three-neck reaction flask) were added potassium phosphate (K₃PO₄, 771 mg, 3.63 mmol) and 2-chloro-1,3-dimethyl-1H-benzimidazol-3-ium chloride (CDMBI, 263 mg, 1.21 mmol), and then the mixture was stirred at 0°C overnight. TLC (isopropanol:concentrated aqueous ammonia:water = 25:5:3) indicated that the reaction was complete. The reaction mixture was extracted with toluene (20 mL × 4), and the aqueous phase was directly purified by C18 column chromatography (acetonitrile:water = 1:4-1:1, preparative high-performance liquid chromatograph (Wuhan Ruihe Chromatography Technology, LC 2100)) to obtain 63 mg of compound **G39** as a red solid in 43% yield.

ESI-MS calc. for C₂₅H₃₂N₉O₉ [M+H]⁺ m/z = 602.2, found m/z = 602.2. ¹H NMR (400 MHz, D₂O) *δ* 8.44 (d, *J =* 8.0 Hz, 2H), 7.62 (d, *J =* 8.0 Hz, 2H), 6.06 (d, *J =* 7.3 Hz, 1H), 4.34 (d, *J =* 7.7 Hz, 1H), 4.27 (t, *J* = 2.7 Hz, 1H), 4.14 (d, *J* = 7.3 Hz, 1H), 3.89 (d, *J =* 3.3 Hz, 1H), 3.80 (s, 2H), 3.74 - 3.68 (m, 1H), 3.63 - 3.40 (m, 8H), 3.09 (d, *J =* 1.4 Hz, 3H), 2.06 (q, *J* = 1.2 Hz, 3H).

### Example 28: Synthesis of compound G40

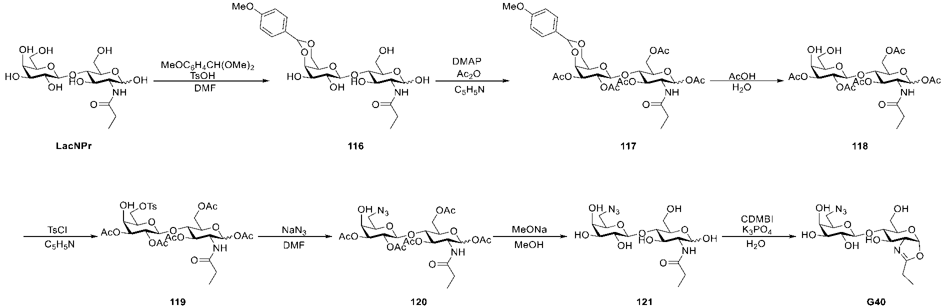

**Step 1:** To a solution of **LacNPr** (10 g, 25.17 mmol) and TsOH (0.56 g, 3.25 mmol) in DMF (220 mL) (in a 500 mL single-neck reaction flask) was added 4-methoxybenzaldehyde dimethyl acetal (11 mL, 64.59 mmol), and the reaction was stirred at room temperature for 12 hours. Then, Et₃N (10 mL) was added to quench the reaction, the mixture was concentrated under reduced pressure, and the residue was purified by silica gel column chromatography (dichloromethane:methanol = 10:1-6:1) to obtain 6.5 g of compound **116** as a white solid in 50% yield.

**Step 2:** To a solution of compound **116** (6.5 g, 12.61 mmol) and DMAP (0.077 g, 0.63 mmol) in pyridine (100 mL) (in a 500 mL single-neck reaction flask) was added Ac₂O (12 mL, 127.77 mmol), and the reaction was stirred at room temperature overnight. TLC (petroleum ether:ethyl acetate = 1:2) indicated that the reaction was complete. The reaction mixture was concentrated under reduced pressure, and the residue was added with ethyl acetate (500 mL), then washed with water (500 mL). The organic phase was concentrated under reduced pressure to obtain 9.1 g of compound **117** as an off-white solid, which was used directly in the next step without further purification in 99% yield.

**Step 3:** A mixture of compound **117** (9.1 g, 12.54 mmol) in acetic acid (80 mL) and water (20 mL) (in a 250 mL single-neck reaction flask) was stirred at room temperature overnight. The reaction mixture was concentrated under reduced pressure, and the residue was purified by silica gel column chromatography (petroleum ether:ethyl acetate = 1:3-0:1) to obtain 2.8 g of solid compound **118** in 36% yield.

**Step 4:** At -20°C, to a solution of compound **118** (2.8 g, 4.64 mmol) in pyridine (100 mL) (in a 250 mL single-neck reaction flask) was added TsCl (1.4 g, 7.34 mmol), and the mixture was stirred overnight. The reaction was quenched by the addition of 20 mL of methanol, and the mixture was subjected to concentration under reduced pressure. The residue was purified by silica gel column chromatography (petroleum ether:ethyl acetate = 1:2-1:3) to obtain 1.9 g of solid compound **119** in 54% yield.

**Step 5:** Under a nitrogen atmosphere, to a solution of compound **119** (1.9 g, 2.99 mmol) in DMF (40 mL) (in a 100 mL three-neck reaction flask) was added NaN₃ (1.46 g, 22.46 mmol), then the temperature was raised to 70°C, and the reaction was stirred for 60 hours. The temperature was cooled to room temperature, the mixture was filtered, and the filtrate was concentrated under reduced pressure. To the residue was added water (100 mL), and the mixture was extracted with ethyl acetate (50 mL × 3). The organic phases were combined and concentrated under reduced pressure to obtain a crude product of compound **120** as a white solid, which was used directly in the next step without further purification (calculated based on a theoretical yield of 1.58 g).

**Step 6:** To a solution of compound **120** (1.58 g, 2.50 mmol) in methanol (30 mL) (in a 100 mL single-neck reaction flask) was added a solution of sodium methoxide (0.5 mL, 5 M) in methanol. The reaction was stirred at room temperature for 3 hours. The pH was adjusted to 6-7 with a cation exchange resin (H⁺), and the mixture was filtered. The filtrate was concentrated under reduced pressure, and the residue was purified by C18 column chromatography (methanol:water = 1:20-1:10) to obtain 380 mg of solid compound **121** in 36% yield.

**Step 7:** At 0°C, to a solution of compound **121** (200 mg, 0.47 mmol) in water (15 mL) (in a 100 mL three-neck reaction flask) were added potassium phosphate (K₃PO₄, 1.51 g, 7.11 mmol) and 2-chloro-1,3-dimethyl-1H-benzimidazol-3-ium chloride (CDMBI, 0.51 g, 2.35 mmol), and then the mixture was stirred at 0°C overnight. TLC (isopropanol:concentrated aqueous ammonia:water = 25:5:3) indicated that the reaction was complete. The reaction mixture was filtered, and the filtrate was directly subjected to preparative C18 chromatography (acetonitrile:water = 1:20-1:4, preparative high-performance liquid chromatograph (Wuhan Ruihe Chromatography Technology, LC 2100)) to obtain 115 mg of compound **G40** as a white solid in 60% yield.

ESI-MS calc. for C₁₅H₂₅N₄O₉ [M+H]⁺ m/z = 405.1, found m/z = 405.1. ¹H NMR (400 MHz, D₂O) *δ* 6.12 (d, *J =* 7.3 Hz, 1H), 4.50 (s, 1H), 4.48 (d, *J =* 1.1 Hz, 1H), 4.22 (ddd, *J =* 6.3, 3.1, 1.6 Hz, 1H), 3.93 - 3.88 (m, 1H), 3.88 - 3.80 (m, 2H), 3.79 - 3.61 (m, 4H), 3.58 - 3.41 (m, 3H), 2.43 (qq, *J* = 7.5, 1.4 Hz, 2H), 1.20 (td, *J* = 7.6*,* 1.2 Hz, 3H).

### Example 29: Synthesis of compound G42

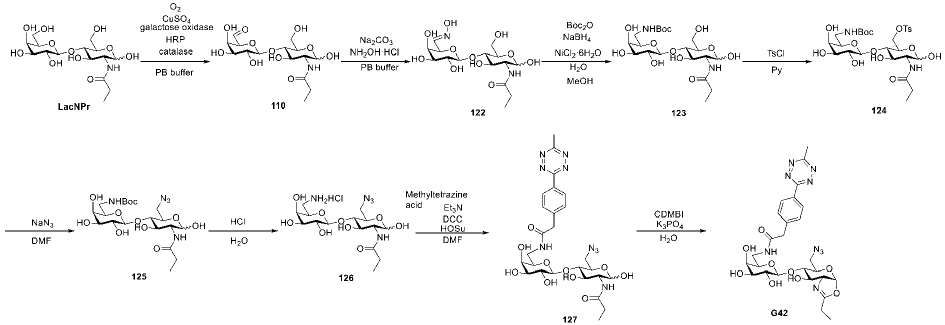

**Steps 1 and 2:** To a phosphate buffer (30 mL) were sequentially added galactose oxidase (11 mL), horseradish peroxidase (HRP, 200 mg), catalase (6 mL), and copper sulfate (CuSO₄, 2 mM, 6 mL) aqueous solution, and the mixture was activated in a 30°C water bath for 30 minutes. At 30°C, oxygen (O₂) was introduced into a solution of compound **LacNPr** (6 g, 15.10 mmol) in phosphate buffer (150 mL) (in a 500 mL single-neck reaction flask) for 30 minutes, and then the mixed enzyme was added to the reaction system. The reaction was carried out in a 30°C water bath for 6 hours. TLC (isopropanol:concentrated aqueous ammonia:water = 4:2:1) indicated that the reaction was complete, and the introduction of oxygen was stopped. To the reaction mixture were added Na₂CO₃ (0.8 g, 7.55 mmol) and NH₂OH HCl (1.15 g, 16.55 mmol), and the mixture was stirred at room temperature for 3 hours. TLC (isopropanol:concentrated aqueous ammonia:water = 4:2:1) indicated that the reaction was complete. Methanol (200 mL) was added. The precipitate was removed by filtration through diatomite, and the filter cake was rinsed with methanol (200 mL). The resulting filtrate was concentrated under reduced pressure, and the residue was purified by C18 column chromatography (methanol:water = 1:20) to obtain 5.0 g of compound **122** in 80% yield.

**Step 3:** At 0°C, to a mixture of compound **122** (5 g, 12.18 mmol) and NiCl₂ 6H₂O (7.24 g, 30.46 mmol) in methanol (400 mL) and phosphate buffer (PB, 200 mL) (in a 500 mL single-neck reaction flask) were added NaBH₄ (2.77 g, 73.22 mmol) and Boc₂O (7.98 g, 36.56 mmol), and the mixture was stirred at 0°C overnight. TLC (isopropanol:concentrated aqueous ammonia:water = 25:5:3) indicated that the reaction was complete. The precipitate was removed by filtration. The filtrate was concentrated under reduced pressure, and the residue was purified by C18 column chromatography (methanol:water = 1:4-1:2) to obtain 5.1 g of compound **123** as a white solid in 84% yield.

**Step 4:** At -20°C, to a solution of compound **123** (5.1 g, 10.27 mmol) in pyridine (100 mL) (in a 250 mL single-neck reaction flask) was added TsCl (3.9 g, 20.46 mmol), and the mixture was stirred overnight. The mixture was concentrated under reduced pressure, and the residue was silica gel column chromatography (dichloromethane:methanol = 20:1-10:1) to obtain 6.6 g of compound **124** in 99% yield.

**Step 5:** Under a nitrogen atmosphere, to a solution of compound **124** (6.6 g, 10.14 mmol) in DMF (120 mL) (in a 500 mL three-neck reaction flask) was added NaN₃ (6.6 g, 101.52 mmol), and then the temperature was raised to 60°C and the reaction was stirred overnight. The reaction mixture was cooled to room temperature and filtered. The filtrate was concentrated under reduced pressure, and the residue was purified by C18 column chromatography (methanol:water = 1:3-2:3) to obtain 2.0 g of compound **125** as a white solid in 38% yield.

**Step 6:** Compound **125** (0.6 g, 1.15 mmol) was dissolved in HCl (7.5 mL, 1.5 M in H₂O) solution (in a 100 mL single-neck reaction flask), and the mixture was stirred at room temperature for 2 hours. The mixture was then concentrated under reduced pressure to obtain a crude product of compound **126,** which was used directly in the next step (calculated based on a product amount of 450 mg and a yield of 85%).

**Step 7:** Under a nitrogen atmosphere and at room temperature, to a solution of compound **126** (450 mg, 0.98 mmol), HOSu (164 mg, 1.42 mmol), 2-(4-(6-methyl-1,2,4,5-tetrazin-3-yl)phenyl)acetic acid (294 mg, 1.28 mmol) and Et₃N (0.5 mL, 3.60 mmol) in DMF (10 mL) (in a 50 mL single-neck flask) was added DCC (324 mg, 1.57 mmol), and the mixture was stirred overnight. TLC (isopropanol:concentrated aqueous ammonia:water = 25:5:3) indicated that the reaction was complete. The mixture was filtered, and the filtrate was concentrated under reduced pressure. The residue was purified by C18 column chromatography (methanol:water = 1:2-1:1) to obtain 0.42 g of compound **127** as a red solid in 67% yield.

**Step 8:** At 0°C, to a solution of compound **127** (200 mg, 0.32 mmol) in water (30 mL) and acetonitrile (10 mL) (in a 50 mL three-neck reaction flask) were added potassium phosphate (K₃PO₄, 1010 mg, 4.76 mmol) and 2-chloro-1,3-dimethyl-1H-benzimidazol-3-ium chloride (CDMBI, 343 mg, 1.49 mmol), and then the mixture was stirred at 0°C overnight. TLC (isopropanol:concentrated aqueous ammonia:water = 25:5:3) indicated that the reaction was complete. The reaction mixture was extracted with toluene (20 mL × 4), and the aqueous phase was directly purified by C18 column chromatography (acetonitrile:water = 1:2-2:1, preparative high-performance liquid chromatograph (Wuhan Ruihe Chromatography Technology, LC 2100)) to obtain 153 mg of compound **G42** as a red solid in 78% yield.

ESI-MS calc. for: C₂₆H₃₄N₉O₉ [M+H]⁺ m/z = 616.2, found m/z = 616.2. ¹H NMR (400 MHz, D₂O) *δ* 8.44 (d, *J =* 7.9 Hz, 2H), 7.62 (d, *J =* 7.9 Hz, 2H), 6.06 (d, *J =* 7.2 Hz, 1H), 4.34 (d, *J* = 7.5 Hz, 1H), 4.29 (d, *J =* 3.3 Hz, 1H), 4.15 (dd, *J =* 7.2, 3.5 Hz, 1H), 3.89 (d, *J =* 3.4 Hz, 1H), 3.83 - 3.66 (m, 3H), 3.63 - 3.40 (m, 8H), 3.09 (d, *J* = 2.3 Hz, 3H), 2.42 - 2.36 (m, 2H), 1.16 (dd, *J* = 8.7, 6.6 Hz, 3H).

### Example 30: Synthesis of compound G44

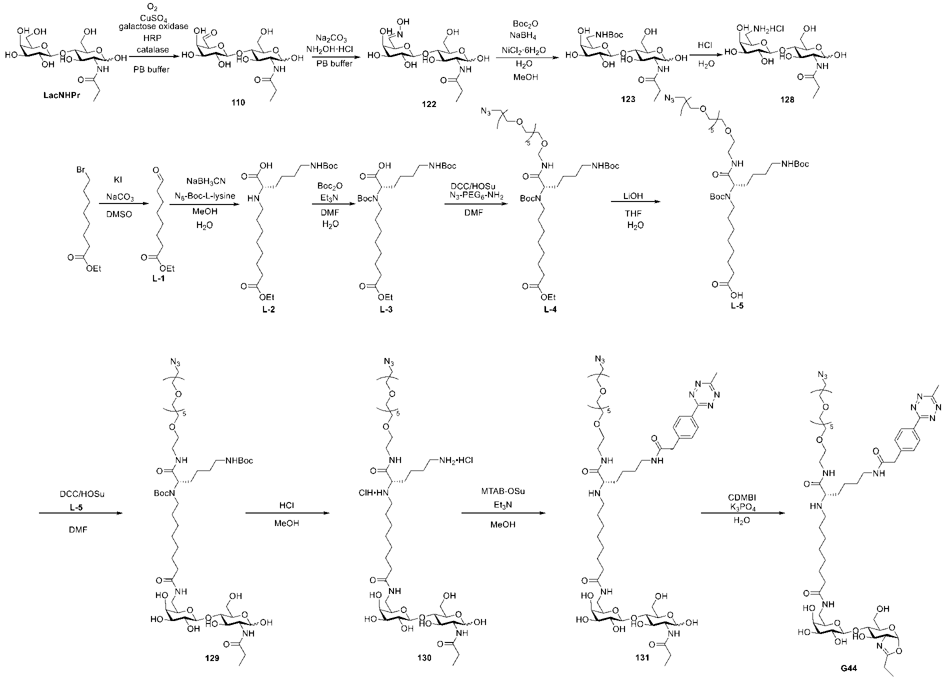

**Steps 1 and 2:** To a phosphate buffer (20 mL) were sequentially added galactose oxidase (9 mL), horseradish peroxidase (HRP, 167 mg), catalase (5 mL), and copper sulfate (CuSO₄, 2 mM, 5 mL) aqueous solution, and the mixture was activated in a 30°C water bath for 30 minutes. At 30°C, oxygen (O₂) was introduced into a solution of compound **LacNPr** (5 g, 12.6 mmol) in phosphate buffer (100 mL) (in a 500 mL single-neck reaction flask) for 30 minutes, and then the mixed enzyme was added to the reaction system. The reaction was stirred in a 30°C water bath for 6 hours. TLC (isopropanol:concentrated aqueous ammonia:water = 4:2:1) indicated that the reaction was complete, and the introduction of oxygen was stopped. To the reaction mixture were added Na₂CO₃ (0.67 g, 6.32 mmol) and NH₂OH HCl (0.96 g, 13.81 mmol), and the reaction was carried out at room temperature for 3 hours. TLC (isopropanol:concentrated aqueous ammonia:water = 4:2:1) indicated that the reaction was complete. Methanol (200 mL) was added. The precipitate was removed by filtration through diatomite, and the filter cake was rinsed with methanol (200 mL). The resulting filtrate was concentrated under reduced pressure, and the residue was purified by C18 column chromatography (methanol:water = 1:20) to obtain 4.0 g of compound **122** in 77% yield.

**Step 3:** At 0°C, to a mixture of compound **122** (4 g, 9.75 mmol) and NiCl₂ 6H₂O (5.79 g, 24.36 mmol) in methanol (400 mL) and phosphate buffer (PB, 200 mL) (in a 1.0 L single-neck reaction flask) were added NaBH₄ (2.21 g, 58.42 mmol) and Boc₂O (6.38 g, 29.23 mmol), and the mixture was stirred at 0°C overnight. TLC (isopropanol:concentrated aqueous ammonia:water = 25:5:3) indicated that the reaction was complete. The precipitate was removed by filtration. The filtrate was concentrated under reduced pressure, and the residue was purified by C18 column chromatography (methanol:water = 1:4-1:2) to obtain 4.5 g of compound 123 as a white solid in 92% yield.

**Step 4:** Compound **123** (1.1 g, 2.22 mmol) was stirred in a dilute hydrochloric acid aqueous solution (13 mL, 1.5 M) at room temperature for 2 hours, and then concentrated under reduced pressure to obtain compound **128** as a white solid, which was used directly in the next step (calculated based on a product amount of 900 mg and a yield of 94%).

**Step 5:** To a solution of ethyl bromooctanoate (27 g, 0.11 mol) in DMSO (500 mL) (in a 1.0 L single-neck reaction flask) were added Na₂CO₃ (11.4 g, 0.11 mol) and KI (17.9 g, 0.11 mol), and the mixture was stirred at 80°C overnight. The reaction mixture was poured into ice water (3 L), and extracted with petroleum ether:ethyl acetate (400 mL:400 mL × 3). The combined organic phases were concentrated under reduced pressure, and the residue was purified by silica gel column chromatography (petroleum ether:ethyl acetate = 20:1-15:1) to obtain 11.0 g of compound **L-1** as a white oil in 55% yield.

**Step 6:** At -10°C, to a mixture of compound **L-1** (3 g, 16.11 mmol) and *N*₆-Boc-*L-*lysine (6 g, 24.36 mmol) in methanol (320 mL) and water (80 mL) (in a 1.0 L single-neck reaction flask) was added NaBH₃CN (2 g, 31.83 mmol), and the mixture was stirred at -10°C overnight. TLC (dichloromethane:methanol = 7:1) indicated that the reaction was complete. The reaction mixture was concentrated under reduced pressure, and the residue was purified by silica gel column chromatography (dichloromethane:methanol = 10:1) to obtain 5.7 g of compound **L-2** as a pale yellow liquid in 85% yield.

**Step 7:** At room temperature, to a mixture of compound **L-2** (3 g, 7.20 mmol) in DMF (200 mL) and H₂O (40 mL) (in a 1.0 L single-neck reaction flask) were added Boc₂O (15.7 g, 71.9 mmol) and Et₃N (2 mL, 14.39 mmol), and the mixture was stirred overnight. TLC (dichloromethane:methanol = 10:1) indicated that the reaction was complete. The reaction mixture was poured into water (2 L) and then extracted with ethyl acetate (400 mL × 3). The organic phases were combined and concentrated under reduced pressure, and the residue was purified by silica gel column chromatography (dichloromethane:methanol = 20:1-15:1) to obtain 1.7 g of compound **L-3** as a pale yellow liquid in 45% yield.

**Step 8:** Under a nitrogen atmosphere, to a solution of compound **L-3** (1.7 g, 3.29 mmol), N₃-PEG₆-NH₂(1.15 g, 3.28 mmol) and HOSu (0.45 g, 3.91 mmol) in DMF (40 mL) (in a 250 mL single-neck reaction flask) was added DCC (1.22 g, 5.91 mmol), and the mixture was stirred at room temperature overnight. TLC (dichloromethane:methanol = 10:1) indicated that the reaction was complete. The reaction mixture was concentrated under reduced pressure, redissolved in DCM (100 mL), and then filtered. The filtrate was concentrated under reduced pressure, and the residue was purified by silica gel column chromatography (dichloromethane:methanol = 100:1-30:1) to obtain 2.3 g of compound **L-4** as a pale yellow liquid in 82% yield.

**Step 9:** At 25°C, to a mixture of compound **L-4** (2.3 g, 2.71 mmol) in THF (20 mL) and H₂O (40 mL) (in a 250 mL single-neck reaction flask) was added LiOH (0.2 g, 8.35 mmol), and the mixture was stirred overnight. TLC (dichloromethane:methanol = 50:1) indicated that the reaction was complete. The reaction mixture was added with water (50 mL), and then extracted with ethyl acetate (150 mL × 3). The organic phases were combined and concentrated under reduced pressure. The residue was purified by C18 column chromatography (methanol:water = 1:1-2:1) to obtain 1.5 g of compound **L-5** as a white solid in 67% yield.

**Step 10:** Under a nitrogen atmosphere and at room temperature, to a solution of compound **128** (0.9 g, 2.08 mmol), compound **L-5** (1.5 g, 1.83 mmol), HOSu (0.32 g, 2.78 mmol), and Et₃N (1 mL, 7.19 mmol) in DMF (40 mL) (in a 250 mL single-neck flask) was added DCC (0.75 g, 3.63 mmol), and the mixture was stirred overnight. TLC (isopropanol:concentrated aqueous ammonia:water = 50:5:3) indicated that the reaction was complete. The mixture was filtered, and the filtrate was concentrated under reduced pressure. The residue was purified by C18 column chromatography (methanol:water = 1:1-7:3) to obtain 0.75 g of compound **129** as a white solid in 34% yield.

**Step 11:** To a solution of compound **129** (250 mg, 0.21 mmol) in methanol (4 mL) (in a 100 mL single-neck flask) was added concentrated hydrochloric acid (HCl, 1 mL, 10 M), and the reaction was carried out at room temperature overnight. TLC (isopropanol:concentrated aqueous ammonia:water = 25:5:3) indicated that the reaction was complete. The mixture was concentrated under reduced pressure to obtain compound **130** as a white solid, which was used directly in the next step (based on a product amount of 200 mg and a yield of 90%).

**Step 12:** Under a nitrogen atmosphere, to a solution of compound **130** (200 mg, 0.19 mol) and Et₃N (0.1 mL, 0.72 mmol) in methanol (5 mL) (in a 100 mL single-neck flask) was added MTAB-OSu (98 mg, 0.30 mmol), and the mixture was stirred at room temperature overnight. TLC (isopropanol:concentrated aqueous ammonia:water = 50:5:3) indicated that the reaction was complete. The mixture was concentrated under reduced pressure, and the residue was purified by C18 column chromatography (methanol:water = 1:1-7:3) to obtain 160 mg of compound **131** as a red solid in 70% yield.

**Step 13:** At 0°C, to a solution of compound **131** (150 mg, 0.12 mmol) in water (60 mL) (in a 100 mL three-neck reaction flask) were added potassium phosphate (K₃PO₄, 394 mg, 1.86 mmol) and 2-chloro-1,3-dimethyl-1H-benzimidazol-3-ium chloride (CDMBI, 134 mg, 0.62 mmol), and then the mixture was stirred at 0°C overnight. TLC (isopropanol:concentrated aqueous ammonia:water = 50:5:3) indicated that the reaction was complete. The reaction mixture was filtered, and the filtrate was purified by C18 column chromatography (acetonitrile:water = 1:2-2:1, preparative high-performance liquid chromatograph (Wuhan Ruihe Chromatography Technology, LC 2100)) to obtain 51 mg of compound **G44** as a red solid in 34% yield.

ESI-MS calc. for: C₅₄H₈₉N₁₂O₁₈ [M+H]⁺ m/z = 1193.6, found m/z = 1193.6. ¹H NMR (400 MHz, D₂O) (5 8.22 (d, *J* = 8.1 Hz, 2H), 7.39 (d, *J* = 8.2 Hz, 2H), 5.89 (d, *J* = 7.3 Hz, 1H), 4.29 - 4.12 (m, 2H), 4.00 (d, *J* = 6.1 Hz, 1H), 3.69 (d, *J* = 3.1 Hz, 1H), 3.61 (d, *J* = 10.4 Hz, 1H), 3.55 - 3.43 (m, 24H), 3.42 - 3.12 (m, 12H), 3.03 (t, *J* = 6.6 Hz, 2H), 2.98 - 2.91 (m, 1H), 2.89 (s, 3H), 2.30 - 2.15 (m, 4H), 2.07 (t, *J* = 7.4 Hz, 2H), 1.52 - 1.30 (m, 6H), 1.28 - 1.13 (m, 4H), 1.14 - 1.02 (m, 8H), 0.98 (t, *J* = 7.6 Hz, 3H).

### Example 31: Synthesis of compound G46

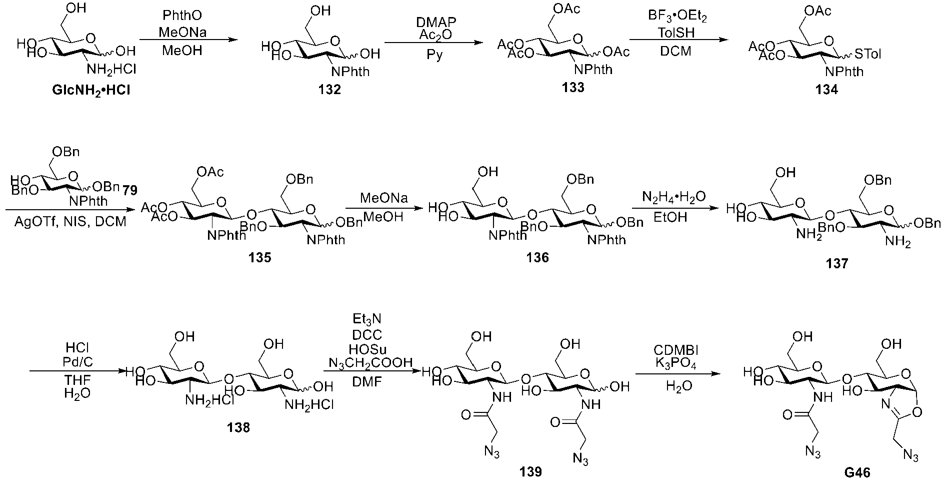

**Step 1:** At room temperature, to a suspension of D-glucosamine hydrochloride **(GlcNH₂ HCl,** 21 g, 0.097 mol) in methanol (200 mL) (in a 500 mL single-neck flask) was added sodium methoxide (5.5 g, 0.102 mol). The mixture was stirred at room temperature for 30 minutes, then phthalic anhydride (PhthO, 15.9 g, 0.107 mol) was added, and the mixture was stirred overnight. The reaction mixture was concentrated under reduced pressure to obtain a crude product of compound **132,** which was used directly in the next step (calculated based on 30 g for charging).

**Step 2:** To a solution of compound **132** (30 g, 0.097 mol) and DMAP (0.6 g, 0.005 mol) in pyridine (500 mL) (in a 2.0 L single-neck reaction flask) was added Ac₂O (100 mL, 1.065 mol), and the reaction was stirred at room temperature overnight. TLC (petroleum ether:ethyl acetate = 1:1) indicated that the reaction was complete. The reaction mixture was concentrated under reduced pressure, and the residue was purified by silica gel column chromatography (petroleum ether:ethyl acetate = 3:1-2:1) to obtain 19.5 g of solid compound **133** in 42% yield.

**Step 3:** To a solution of compound **133** (19.5 g, 0.041 mol) and TolSH (7.6 g, 0.061 mol) in dichloromethane (300 mL) (in a 1.0 L single-neck reaction flask) was added BF₃ Et₂O (23 mL, 0.186 mol). The mixture was stirred at room temperature for 3 hours. TLC (petroleum ether:ethyl acetate = 3:1) indicated that the reaction was complete. The reaction mixture was diluted with dichloromethane (500 mL), washed with saturated sodium bicarbonate (800 mL), and the organic phase was concentrated under reduced pressure. The residue was purified by silica gel column chromatography (petroleum ether:ethyl acetate = 7:1-3:1) to obtain 16 g of compound **134** as a white solid in 72% yield.

**Step 4:** Under a nitrogen atmosphere, to a solution of compound **134** (11.2 g, 0.021 mol) and compound **79** (12.0 g, 0.021 mol) in dichloromethane (400 mL) (in a 1.0 L three-neck reaction flask) was added dried 4Å molecular sieves (30 g), and the mixture was stirred at room temperature for 2 hours. The temperature was lowered to about -10°C, and then NIS (7.0 g, 0.031 mol) and AgOTf (0.53 g, 0.0021 mol) were added. The mixture was then warmed to room temperature and stirred overnight. TLC (petroleum ether:ethyl acetate = 3:1) indicated that the reaction was complete. The reaction mixture was filtered, and the filter cake was rinsed with dichloromethane (400 mL). Water (500 mL) was added to the filtrate, and sodium thiosulfate was slowly added with vigorous stirring until the organic phase was decolorized. The organic phase was concentrated under reduced pressure, and the residue was purified by silica gel column chromatography (petroleum ether:ethyl acetate = 3:1-1:1) to obtain 18.2 g of compound **135** as a colorless gum in 88% yield.

**Step 5:** Under a nitrogen atmosphere and at 0°C, to a solution of compound **135** (18.2 g, 0.018 mol) in methanol (200 mL) (in a 500 mL single-neck reaction flask) was added a solution of sodium methoxide (3.6 mL, 5 M) in methanol. The mixture was stirred at 0°C for 3 hours. TLC (petroleum ether:ethyl acetate = 1:3) indicated that the reaction was complete. Cation exchange resin (H⁺) was added for neutralization, and the pH was adjusted to 6-7. The mixture was filtered, and the filtrate was concentrated under reduced pressure to obtain 15.9 g of a crude product of compound **136** as a light yellow solid, which was used directly in the next step without further purification.

**Step 6:** Under a nitrogen atmosphere, to a solution of compound **136** (13 g, 0.148 mol) in ethanol (700 mL) (in a 2.0 L single-neck reaction flask) was added N₂H₄ H₂O (10.5 mL, 0.216 mol), and the reaction was stirred at 80°C overnight. TLC (dichloromethane:methanol = 10:1) indicated that the reaction was complete. After cooling, the mixture was filtered, and the filtrate was concentrated under reduced pressure to obtain 10.5 g of compound **137** as a white solid in 94% yield. Without further purification, the product was used directly in the next step.

**Step 7:** To a mixture of compound **137** (2 g, 3.17 mmol) in tetrahydrofuran (120 mL) and water (40 mL) (in a 100 mL three-neck flask) were added Pd/C (1.75 g, 10 wt%, 1.64 mmol) and concentrated HCl (1.3 mL, 10 M). The mixture was charged with H₂ (15 psi) and stirred at room temperature overnight. TLC (isopropanol:concentrated aqueous ammonia:water = 4:2:1) indicated that the reaction was complete. The reaction mixture was filtered, and the filtrate was concentrated under reduced pressure to obtain a crude product of compound **138** as a white solid, which was used directly in the next step without purification (1.1 g was used in the next step).

**Step 8:** Under a nitrogen atmosphere, to a solution of compound **138** (1.1 g, 2.66 mmol), N₃CH₂COOH (807 mg, 7.98 mmol), HOSu (1.1 g, 9.56 mmol), and Et₃N (3 mL) in DMF (15 mL) (in a 100 mL single-neck flask) was added DCC (2.2 g, 10.66 mmol). The mixture was stirred at room temperature overnight. TLC (isopropanol:concentrated aqueous ammonia:water = 25:5:3) indicated that the reaction was complete. The mixture was filtered, and the filtrate was concentrated under reduced pressure. The residue was purified by silica gel column chromatography (dichloromethane:methanol = 5:1-4:1) to obtain 0.8 g of a crude product of compound **139** as a white solid, which was further purified by amide column chromatography to obtain 0.26 g of compound **139** as a white solid product in 19% yield.

**Step 9:** At 0°C, to a solution of compound **139** (160 mg, 0.32 mmol) in water (12 mL) (in a 100 mL three-neck reaction flask) were added potassium phosphate (K₃PO₄, 1010 mg, 4.76 mmol) and 2-chloro-1,3-dimethyl-1H-benzimidazol-3-ium chloride (CDMBI, 343 mg, 1.58 mmol), and then the mixture was stirred at 0°C overnight. TLC (isopropanol:concentrated aqueous ammonia:water = 25:5:3) indicated that the reaction was complete. The precipitate was removed by filtration, and the filtrate was purified by preparative C18 chromatography (acetonitrile:water = 1:20-1:4) to obtain 140 mg of compound **G46** as a white solid in 91% yield.

ESI-MS calc. for C₁₆H₂₅N₈O₁₀ [M+H]⁺ m/z = 489.1, found m/z =489.1. ¹H NMR (400 MHz, D₂O) *δ* 6.18 (dd, *J* = 7.3, 1.2 Hz, 1H), 4.65 - 4.59 (m, 1H), 4.46 (dt, *J* = 2.8, 1.3 Hz, 1H), 4.27 (dp, *J* = 7.8, 1.5 Hz, 1H), 4.23 - 4.09 (m, 2H), 4.05 (t, *J* = 1.2 Hz, 2H), 3.95 - 3.86 (m, 1H), 3.78 - 3.54 (m, 6H), 3.50 - 3.39 (m, 2H), 3.36 -3.29 (m, 1H).

### Example 32: Synthesis of compound G47

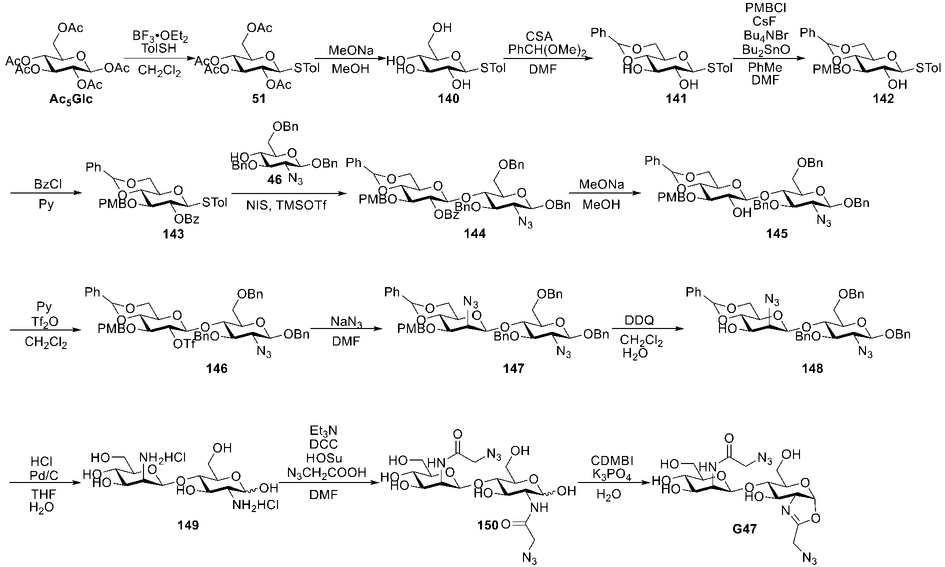

**Step 1:** To a solution of **Ac₅Glc** (45 g, 0.115 mol) and TolSH (21.5 g, 0.173 mol) in DCM (250 mL) (in a 500 mL single-neck reaction flask) was added BF₃ Et₂O (43 mL, 0.348 mol), and the mixture was stirred at room temperature for 3 hours. TLC (petroleum ether:ethyl acetate = 3:1) indicated that the reaction was complete. The reaction mixture was diluted with DCM (600 mL), washed with saturated sodium bicarbonate (1.0 L), and the organic phase was concentrated under reduced pressure. The residue was purified by silica gel column chromatography (petroleum ether:ethyl acetate = 7:1-3:1) to obtain 47 g of compound **51** as a white solid in 90% yield.

**Step 2:** To a solution of compound **51** (47 g, 0.103 mol) in methanol (500 mL) (in a 1.0 L single-neck reaction flask) was added a solution of sodium methoxide (10 mL, 5 M) in methanol, and the mixture was stirred at room temperature for 2 hours. TLC (dichloromethane:methanol = 10:1) indicated that the reaction was complete. Cation exchange resin (H+) was added for neutralization, and the pH was adjusted to 6-7. The mixture was filtered, and the filtrate was concentrated under reduced pressure to obtain 28 g of compound **140** as a white solid in 95% yield, which was used directly in the next step without further purification.

**Step 3:** To a solution of compound **140** (8 g, 0.028 mol) and benzaldehyde dimethyl acetal (8.4 mL) in DMF (100 mL) (in a 250 mL single-neck reaction flask) was added *D-*camphorsulfonic acid (CAS: 3144-16-99, 1.62 g, 0.007 mol), and the mixture was stirred at room temperature overnight. Triethylamine (3 mL) was added to quench the reaction, and the mixture was concentrated under reduced pressure. The residue was washed with dichloromethane (150 mL), saturated sodium bicarbonate (50 mL), and water (50 mL). The organic phase was concentrated under reduced pressure, and the residue was slurried with dichloromethane:*n*-hexane = 1:3 (100 mL) to obtain 9 g of compound **141** as a white solid in 86% yield.

**Step 4:** Under a nitrogen atmosphere, to a solution of compound **141** (9 g, 0.024 mol) in toluene (75 mL) (in a 250 mL single-neck reaction flask) was added Bu₂SnO (6.9 g, 0.028 mol), and the reaction was carried out at 125°C under reflux for 4 hours to remove water. After cooling to room temperature, the mixture was concentrated under reduced pressure. To the residue were added DMF (50 mL), Bu₄NBr (8.5 g, 0.026 mol), CsF (4.0 g, 0.026 mol), and PMBCl (4.1 g, 0.015 mol). The reaction was carried out at room temperature overnight under a nitrogen atmosphere. The reaction mixture was concentrated under reduced pressure. To the residue was added ethyl acetate (200 mL), and the mixture was washed with saturated sodium bicarbonate (100 mL) and water (100 mL). The organic phase was concentrated under reduced pressure, and the residue was purified by silica gel column chromatography (petroleum ether:ethyl acetate:dichloromethane = 2:1:1) to obtain 10 g of compound **142** as a white solid in 84% yield.

**Step 5:** To a solution of compound **142** (10 g, 0.02 mol) and DMAP (0.12 g, 0.001 mol) in pyridine (100 mL) (in a 500 mL single-neck reaction flask) was added benzoyl chloride (BzCl, 3.98 g, 0.028 mol), and the mixture was stirred at room temperature overnight. TLC (petroleum ether:ethyl acetate = 2:1) indicated that the reaction was complete. The reaction mixture was concentrated under reduced pressure, and the residue was purified by silica gel column chromatography (petroleum ether:ethyl acetate = 5:1 to 1:1) to obtain 11.5 g of compound **143** as an off-white solid in 95% yield.

**Step 6:** Under a nitrogen atmosphere, to a solution of compound **143** (11.5 g, 0.019 mol) and compound **46** (7.6 g, 0.016 mol) in DCM (400 mL) (in a 1000 mL three-neck reaction flask) was added dried 4Å molecular sieves (30 g), and the mixture was stirred at room temperature for 2 hours. The temperature was lowered to -10°C, and NIS (5.4 g, 0.024 mol) and TMSOTf (2.1 g, 0.009 mol) were added to the reaction mixture. After the addition was completed, the mixture was warmed to room temperature and stirred at room temperature overnight. TLC (petroleum ether:ethyl acetate = 5:1) indicated that the reaction was complete. The reaction mixture was filtered, and the filter residue was rinsed with DCM (400 mL). Water (400 mL) was added to the filtrate, and sodium thiosulfate was slowly added with vigorous stirring until the organic phase was decolorized. The organic phase was concentrated under reduced pressure, and the residue was purified by silica gel column chromatography (petroleum ether:ethyl acetate = 15:1-7:1) to obtain 12 g of compound **144** as a colorless gum in 79% yield.

**Step 7:** To a solution of compound **144** (12 g, 0.013 mol) in MeOH (200 mL) (in a 500 mL single-neck reaction flask) was added a solution of MeONa (1.26 mL, 5 M) in methanol, and the mixture was stirred at room temperature for 2 hours. TLC (petroleum ether:ethyl acetate = 3:1) indicated that the reaction was complete. Cation exchange resin (H⁺) was added for neutralization, and the pH was adjusted to 6-7. The mixture was filtered, and the filtrate was concentrated under reduced pressure. The residue was purified by silica gel column chromatography (petroleum ether:ethyl acetate = 10:1-3:1) to obtain 10 g of compound **145** as a white solid in 94% yield, which was used directly in the next step without further purification.

**Step 8:** Under a nitrogen atmosphere and at 0°C, to a solution of compound **145** (10 g, 0.012 mol) in dichloromethane (100 mL) and pyridine (3 mL) (in a 250 mL three-neck single-neck reaction flask) was added trifluoromethanesulfonic anhydride (Tf₂O, 3 mL). The reaction was carried out at 0°C with stirring for 2 hours. TLC (petroleum ether:ethyl acetate = 3:1) indicated that the reaction was complete. The mixture was diluted with dichloromethane (100 mL) and then washed with dilute hydrochloric acid (1 M, 100 mL) and saturated sodium bicarbonate (100 mL). The organic phase was concentrated under reduced pressure to obtain 11 g of compound **146** as a light yellow solid in 95% yield, which was used directly in the next step without further purification.

**Step 9:** To a solution of compound **146** (11 g, 11.41 mmol) in DMF (150 mL) (in a 1.0 L single-neck reaction flask) was added NaN₃ (2.9 g, 44.61 mmol). The reaction was stirred at 60°C overnight. TLC (petroleum ether:ethyl acetate = 4:1) indicated that the reaction was complete. The mixture was diluted with water (1000 mL) and extracted with ethyl acetate (250 mL × 2). The organic phases were combined and washed with saturated sodium carbonate (100 mL). The organic phase was concentrated under reduced pressure, and the residue was purified by silica gel column chromatography (petroleum ether:ethyl acetate = 10:1-8:1) to obtain 7.2 g of compound **147** as a white solid in 73% yield.

**Step 10:** At 0°C, to a mixture of compound **147** (3.2 g, 3.73 mmol) in DCM (45 mL) and H₂O (2.5 mL) (in a 250 mL single-neck reaction flask) was added DDQ (1.92 g, 8.46 mmol). The reaction was stirred for 0.5 hours, then warmed to room temperature, and stirred for an additional 1 hour. The reaction mixture was diluted with dichloromethane (200 mL) and washed with saturated sodium bicarbonate (200 mL). The organic phase was concentrated under reduced pressure, and the residue was purified by silica gel column chromatography (petroleum ether:ethyl acetate = 5:1-3:1) to obtain 2.1 g of compound **148** as a light yellow gum in 76% yield.

**Step 11:** To a mixture of compound **148** (2.1 g, 2.85 mmol) in THF (180 mL) and H₂O (60 mL) (in a 500 mL single-neck reaction flask) were added Pd/C (2 g, 10 wt%, 1.88 mmol) and an aqueous solution of HCl (1.12 mL, 10 M). The mixture was charged with H₂ (15 psi) and stirred at room temperature overnight. TLC (isopropanol:concentrated aqueous ammonia:water = 4:2:1) indicated that the reaction was complete. The reaction mixture was filtered, and the filtrate was concentrated under reduced pressure to obtain compound **149** as a light yellow solid, which was used directly in the next step without purification (1.1 g, yield: 95%).

**Step 12:** Under a nitrogen atmosphere, to a solution of compound **149** (1.1 g, 2.66 mmol), N₃CH₂COOH (807 mg, 7.98 mmol), HOSu (1.1 g, 9.56 mmol), and Et₃N (3 mL) in DMF (15 mL) (in a 100 mL single-neck flask) was added DCC (2.2 g, 10.66 mmol). The reaction was carried out at room temperature overnight. TLC (isopropanol:concentrated aqueous ammonia:water = 25:5:3) indicated that the reaction was complete. The reaction mixture was filtered, and the filtrate was concentrated under reduced pressure. The residue was purified by silica gel column chromatography (dichloromethane:methanol = 5:1 to 4:1) to obtain a crude product of compound **150,** which was further purified by amide column chromatography to obtain 0.3 g of compound **150** as a white solid in 22% yield.

**Step 13:** At 0°C, to a solution of compound **150** (150 mg, 0.30 mmol) in water (8 mL) (in a 50 mL three-neck reaction flask) were added potassium phosphate (K₃PO₄, 943 mg, 4.44 mmol) and 2-chloro-1,3-dimethyl-1H-benzimidazol-3-ium chloride (CDMBI, 321 mg, 1.48 mmol), and then the mixture was stirred at 0°C overnight. TLC (isopropanol:concentrated aqueous ammonia:water = 25:5:3) indicated that the reaction was complete. The precipitate was removed by filtration, and the filtrate was purified by preparative C18 chromatography (acetonitrile:water = 1:20-1:5) to obtain 98 mg of compound **G47** as a white solid in 67% yield.

ESI-MS calc. for C₁₆H₂₅N₈O₁₀ [M+H]⁺ m/z = 489.1, found m/z =489.1. ¹H NMR (400 MHz, D₂O) *δ* 6.17 (d, *J* = 7.8 Hz, 1H), 4.85 (s, 1H), 4.47 (d, *J* = 4.3 Hz, 1H), 4.43 -4.38 (m, 1H), 4.25 (d, *J* = 7.4 Hz, 1H), 4.13 (s, 2H), 4.06 (s, 2H), 3.92-3.66 (m, 5H), 3.64-3.55 (m, 1H), 3.53-3.44 (m, 1H), 3.42-3.34 (m, 1H), 3.34-3.29 (m, 1H).

### Example 33: Synthesis of compound G5

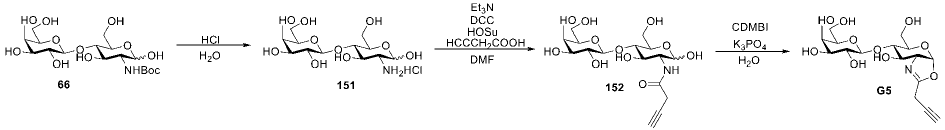

**Step 1:** Compound **66** (0.8 g, 1.81 mmol) was dissolved in an aqueous solution of HCl (10 mL, 1.5 M) (in a 100 mL single-neck reaction flask), and the mixture was stirred at room temperature for 2 hours. TLC (isopropanol:concentrated aqueous ammonia:water = 25:5:3) indicated that the reaction was complete. The reaction mixture was concentrated under reduced pressure to obtain compound **151** as a white solid, which was used directly in the next step without further purification (a product amount of 0.65 g was used in the next step).

**Step 2:** Under a nitrogen atmosphere, to a solution of compound **151** (650 mg, 1.72 mmol), 3-butynoic acid (217 mg, 2.58 mmol), HOSu (356 mg, 3.09 mmol), and Et₃N (0.6 mL) in DMF (10 mL) (in a 100 mL single-neck flask) was added DCC (710 mg, 3.44 mmol), and the reaction was carried out at room temperature overnight. TLC (isopropanol:concentrated aqueous ammonia:water = 25:5:3) indicated that the reaction was complete. The mixture was filtered, and the filtrate was concentrated under reduced pressure. The residue was purified by amide column chromatography to obtain 0.12 g of compound **152** as a white solid in 17% yield.

**Step 3:** At 0°C, to a solution of compound **152** (120 mg, 0.29 mmol) in water (16 mL) (in a 100 mL three-neck reaction flask) were added potassium phosphate (K₃PO₄, 750 mg, 3.53 mmol) and 2-chloro-1,3-dimethyl-1H-benzimidazol-3-ium chloride (CDMBI, 256 mg, 1.18 mmol), and then the mixture was stirred at 0°C overnight. TLC (isopropanol:concentrated aqueous ammonia:water = 25:5:3) indicated that the reaction was complete. The precipitate was removed by filtration, and the filtrate was purified by C18 column chromatography (acetonitrile:water = 1:20-1:5) to obtain 82 mg of compound **G5** as a white solid in 71% yield.

ESI-MS calc. for C₁₆H₂₄NO₁₀ [M+H]⁺ m/z = 390.1, found m/z = 390.1. ¹H NMR (400 MHz, D₂O) *δ* 6.14 (d, *J* = 7.2 Hz, 1H), 5.86 (t, *J* = 6.6 Hz, 1H), 5.37 (dd, *J* = 6.7, 1.8 Hz, 2H), 4.43 (dd, *J* = 3.2, 1.8 Hz, 1H), 4.41 (d, *J* = 7.7 Hz, 1H), 4.28 (dq, *J* = 7.2, 1.6 Hz, 1H), 3.89 (dd, *J* = 3.4, 0.9 Hz, 1H), 3.85 - 3.71 (m, 4H), 3.71 - 3.58 (m, 3H), 3.54 - 3.41 (m, 2H).

### Example 34: Synthesis of compound G45

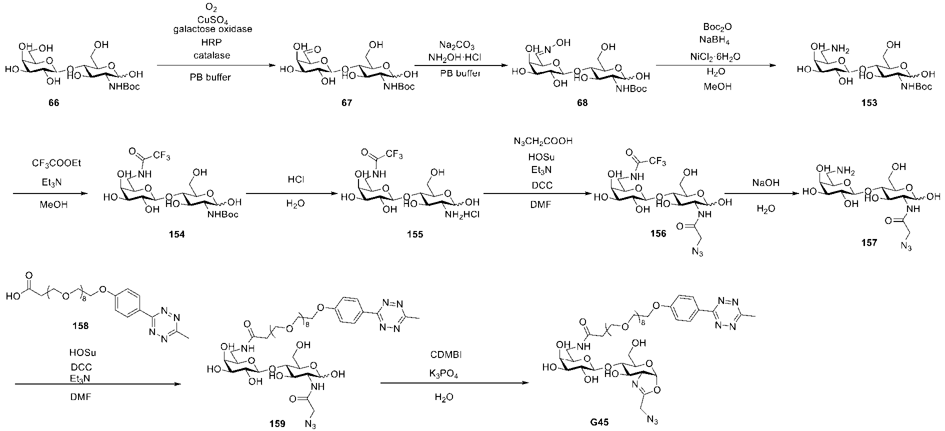

**Step 1:** To a phosphate buffer (30 mL) were sequentially added galactose oxidase (9.5 mL), copper sulfate (CuSO₄, 2 mM, 5 mL), horseradish peroxidase (HRP, 150 mg), and catalase (4.5 mL), and then the mixture was activated in a 30°C water bath for 30 minutes. At 30°C, to a 1.0 L three-neck reaction flask were added phosphate buffer (120 mL) and compound **66** (6 g, 13.59 mmol), respectively. Oxygen (O₂) was introduced for 15-30 minutes, and then the mixed enzyme was added to the reaction system. The reaction was carried out in a 30°C water bath for 6 hours. TLC (isopropanol:concentrated aqueous ammonia:water = 25:5:3) indicated that the reaction was complete. The introduction of oxygen was stopped, and the reaction mixture was used directly in the next step without further treatment.

**Step 2:** To the reaction mixture were added Na₂CO₃ (0.72 g, 6.79 mmol) and hydroxylamine hydrochloride (NH₂OH HCl, 1.04 g, 14.97 mmol), and the reaction was carried out at room temperature for 3 hours. TLC (isopropanol: concentrated aqueous ammonia:water = 25:5:3) indicated that the reaction was complete. Methanol (200 mL) was added, and the precipitate was removed by filtration through diatomite. The precipitate was rinsed with methanol (200 mL), and the obtained filtrate was used directly in the next step (compound 68 therein: 5 g, 81% yield).

**Step 3:** At 0°C, to a mixture of compound **68** (5.0 g, 11.00 mmol) and NiCl₂ 6H₂O (8.25 g, 34.71 mmol) in MeOH (400 mL) and phosphate buffer (PB, 170 mL) (in a 2000 mL single-neck reaction flask) was added NaBH₄ (2.08 g, 55.03 mmol), and the reaction was carried out at 0°C overnight. TLC (isopropanol:concentrated aqueous ammonia:water = 25:5:3) indicated that the reaction was complete. The precipitate was removed by filtration. The filtrate was concentrated under reduced pressure, and the residue was purified by C18 column chromatography (methanol:water = 1:20, hydrochloric acid system) to obtain 2.95 g of compound **153** as a white solid in 56% yield.

**Step 4:** To a solution of compound **153** (1.8 g, 3.77 mmol) and Et₃N (1 mL) in MeOH (40 mL) (in a 100 mL single-neck flask) was added CF₃COOEt (2 mL), and the reaction was carried out overnight. TLC (isopropanol:concentrated aqueous ammonia:water = 25:5:3) indicated that the reaction was complete. The filtrate was concentrated under reduced pressure, and the residue was purified by preparative C18 chromatography (methanol:water = 1:3-1:1) to obtain 1.2 g of compound **154** as a white solid in 54% yield.

**Step 5:** Compound **154** (0.6 g, 1.12 mmol) was dissolved in an aqueous solution of HCl (10 mL, 1.5 M) (in a 100 mL single-neck reaction flask), and the mixture was stirred at room temperature for 2 hours. The mixture was then concentrated under reduced pressure to obtain a crude product of compound **155,** which was used directly in the next step (calculated based on a yield of 500 mg).

**Step 6:** To a solution of compound **155** (500 mg, 1.06 mmol), Et₃N (0.3 mL), N₃CH₂COOH (128 mg, 1.27 mmol), and HOSu (182 mg, 1.58 mmol) in DMF (10 mL) (in a 100 mL single-neck flask) was added DCC (393 mg, 1.90 mmol), and the reaction was carried out at room temperature overnight. TLC (isopropanol:concentrated aqueous ammonia:water = 25:5:3) indicated that the reaction was complete. The mixture was filtered, and the filtrate was concentrated under reduced pressure. The residue was purified by preparative C18 chromatography (methanol:water = 1:20-1:10) to obtain 400 mg of compound **156** as a white solid in 49% yield.

**Step 7:** To a solution of NaOH (50 mg, 1.25 mmol) in H₂O (2.5 mL) (in a 10 mL single-neck reaction flask) was added compound **156** (200 mg, 0.39 mmol), and the mixture was stirred at room temperature for 2 hours. TLC (isopropanol:concentrated aqueous ammonia:water = 25:5:3) indicated that the reaction was complete. The pH was then adjusted to 3-4 with dilute hydrochloric acid, and the mixture was concentrated under reduced pressure to obtain a crude product of compound **157,** which was used directly in the next step (calculated based on a yield of 150 mg).

**Step 8:** To a solution of compound **158** (1-(4-(6-methyl-1,2,4,5-tetrazin-3-yl)phenoxy)-3,6,9,12,15,18,21,24-octaoxaheptacosan-27-oic acid, 325 mg, 0.53 mmol) and HOSu (73 mg, 0.63 mmol) in DMF (5 mL) (in a 25 mL single-neck flask) was added DCC (132 mg, 0.64 mmol), and the reaction was carried out at room temperature overnight. Then, a solution of compound **157** (150 mg, 0.35 mmol) in DMF (5 mL) and Et₃N (0.1 mL) were added, and the reaction was carried out at room temperature for 5 hours. TLC (isopropanol:concentrated aqueous ammonia:water = 25:5:3) indicated that the reaction was complete. The filtrate was concentrated under reduced pressure, and the residue was purified by preparative C18 chromatography (methanol:water = 1:4-1:1) to obtain 150 mg of compound **159** as a red solid in 41% yield.

**Step 9:** At 0°C, to a solution of compound **159** (150 mg, 0.15 mmol) in water (45 mL) (in a 250 mL three-neck reaction flask) were added potassium phosphate (K₃PO₄, 469 mg, 2.21 mmol) and 2-chloro-1,3-dimethyl-1H-benzimidazol-3-ium chloride (CDMBI, 160 mg, 0.74 mmol), and then the mixture was stirred at 0°C overnight. TLC (isopropanol:concentrated aqueous ammonia:water = 25:5:3) indicated that the reaction was complete. The reaction mixture was filtered, and the filtrate was directly purified by C18 column chromatography (acetonitrile:water = 1:10-3:2, preparative high-performance liquid chromatograph (Wuhan Ruihe Chromatography Technology, LC 2100)) to obtain 108 mg of compound **G45** as a red solid in 73% yield.

ESI-MS calc. for C₄₂H₆₆N₉O₁₉ [M+H]⁺ m/z = 1000.4, found m/z =1000.4; C₄₂H₆₅N₉O₁₉K [M+K]⁺ m/z = 1038.3, found m/z =1038.3. ¹H NMR (400 MHz, D₂O) *δ* 8.29 (d, *J* = 8.6 Hz, 2H), 7.15 (d, *J* = 8.6 Hz, 2H), 6.13 (d, *J* = 7.3 Hz, 1H), 4.38 (dd, *J* = 3.1, 1.6 Hz, 1H), 4.31 (d, *J* = 7.7 Hz, 1H), 4.27 (dd, *J* = 5.7, 2.9 Hz, 2H), 4.23 - 4.05 (m, 3H), 3.94 - 3.87 (m, 2H), 3.80 (d, *J* = 3.3 Hz, 1H), 3.77 - 3.49 (m, 35H), 3.48 - 3.29 (m, 4H), 2.97 (s, 3H), 2.48 (t, *J* = 6.1 Hz, 2H).

### Example 35: Synthesis of compound G40-DBCO-PEG₄-VC-PAB-MMAE

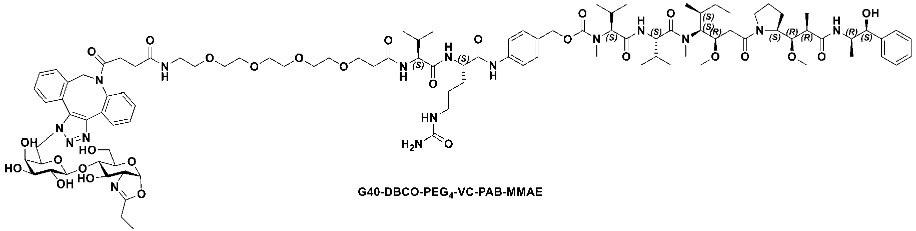

G40 (8.1 mg, 0.02 mmol) and DBCO-VC-PEG₄-PAB-MMAE (16.58 mg, 0.01 mmol) were dissolved in 1 mL of PBS 7.4 and 1 mL of DMSO, respectively. Subsequently, the two solutions were mixed, and the reaction was carried out at 25°C overnight. LC-MS indicated that the reaction was complete. The reaction mixture was subjected to preparative C18 purification (acetonitrile:water = 1:50 to 5:1) to obtain 13 mg of compound **G40-DBCO-PEG₄-VC-PAB-MMAE** as a white solid in 63% yield. The NMR sample was first dissolved in deuterium oxide-deuterated methanol, then lyophilized, and subsequently dissolved in deuterated DMSO for NMR analysis.

ESI-MS calc. for C₁₀₃H₁₅₄N₁₆O₂₈ [M+2H]²⁺ m/z = 1031.2, found m/z =1032.0. 1H NMR (400 MHz, DMSO-d6) *δ* 10.01 (s, 1H), 8.38 - 8.11 (m, 1H), 8.10 - 7.76 (m, 2H), 7.63 (dq, *J* = 28.4, 6.3, 4.0 Hz, 4H), 7.55 - 7.21 (m, 7H), 7.21 - 7.09 (m, 1H), 6.08 - 5.77 (m, 2H), 5.41 (s, 1H), 5.18 - 4.85 (m, 3H), 4.81 - 4.32 (m, 6H), 4.32 - 3.83 (m, 6H), 3.82 - 3.52 (m, 4H), 3.47 (d, *J* = 3.4 Hz, 9H), 3.24 (d, *J* = 6.4 Hz, 3H), 3.19 (d, *J* = 9.9 Hz, 2H), 3.11 (t, 3H), 3.07 = 2.56 (m, 6H), 2.44 - 1.90 (m, 8H), 1.70 (d, *J* = 7.1 Hz, 3H), 1.63 - 1.25 (m, 5H), 1.13 - 0.95 (m, 6H), 0.93 - 0.61 (m, 16H).

### 2. Recombinant expression and purification of enzymes

### Example 36: Recombinant expression and purification of enzymes

The coding genes for the following four enzymes were cloned into the pET22b vector (GenScript). The four enzymes were: Endo Se2 from *Streptococcus equi* subsp. zooepidemicus Sz105, having the amino acid sequence set forth in 37-1011 of SEQ ID NO:1; Endo Si from *Streptococcus iniae,* having the amino acid sequence set forth in 34-928 of SEQ ID NO:2; Endo S2 from *Streptococcus pyogenes* NZ131 (serotype M49), having the amino acid sequence set forth in 38-843 of SEQ ID NO:3; and Endo S from *Streptococcus pyogenes,* having the amino acid sequence set forth in 37-995 of SEQ ID NO:4.

The plasmid containing the target gene was transformed into Escherichia coli BL21 (DE3), spread onto a 2×YT solid plate containing 100 µg/mL ampicillin, followed by overnight culture at 37°C. A single colony was picked and inoculated into 4 mL of 2×YT liquid medium containing 100 µg/mL ampicillin, followed by overnight culture. 4 mL of the bacterial culture was inoculated into 1 L of 2×YT broth medium containing 100 µg/mL ampicillin and cultured at 37°C until OD₆₀₀ reached 0.8-1.0. Then, 0.4 mM of isopropyl β-D-1-thiogalactopyranoside (IPTG) was added to the culture, and the culture was incubated at 20°C to induce overexpression of the protein. After 16 hours, the bacterial cells were harvested by centrifugation. The cell pellet was lysed using B-PER^{™} Bacterial Protein Extraction Reagent (Thermo) according to the manufacturer's instructions. Recombinant proteins of Endo S, Endo S2, Endo Si, and Endo Se2 were purified using a cOmplete^{™} His-Tag Purification Column (Roche) and an SDA030 protein purification system (Sepure). The proteins were concentrated using Amicon centrifugal filters (30 kDa, Millipore), and further purified by size exclusion chromatography using a HiLoad^{™} 26/600 Superdex^{™} 200 prep grade column (Cytiva). The fractions containing the Endo S, Endo S2, Endo Si, and Endo Se2 fusion proteins were respectively concentrated using Amicon centrifugal filters (30 kDa, Millipore) and stored in storage buffer (20 mM PB, pH 7.5). Protein purity was analyzed by sodium dodecyl sulfate-polyacrylamide gel electrophoresis (SDS-PAGE), a gel imaging system (Gel Doc EZ Imager, Bio-RAD), and Image Lab scanning software, and the protein concentration was quantified using a spectrophotometer (Nano-300).

### 3. Study on the transfer activity of disaccharide linkers

### Example 37: Study on the transfer activity of endoglycosidases such as Endo Se2 and Endo Si toward various disaccharide linkers

A wild-type antibody comprising the Fc segment of SEQ ID NO: 5 was dissolved in a buffer containing 20 mM phosphate and 150 mM NaCl at pH 7.4 (10 mg/mL). Wild-type Endo S2 was added to a final concentration of 0.4 mg/mL for hydrolysis, followed by incubation overnight at 37°C. A deglycosylated antibody containing one *N-*acetylglucosamine or core-fucosylated N-acetylglucosamine was obtained through purification using Protein A magnetic beads.

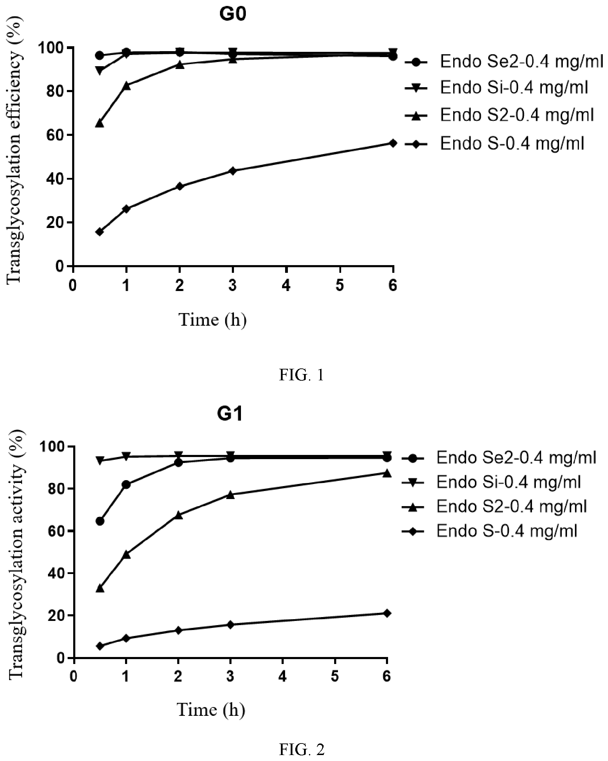

The prepared deglycosylated antibody, disaccharide linker (*i.e.,* compounds G0-G2), and wild-type endoglycosidase (*i.e.,* Endo S, Endo S2, Endo Si, or Endo Se2) were used such that the concentrations of the respective components were sequentially 10 mg/mL, 1.67 mM (25 equivalents relative to the antibody concentration), and 0.4 mg/mL. The pH of the reaction system was adjusted to 7.4, and the reaction temperature was 25°C. At 0.5, 1, 2, 3, and 6 hours, 4 µL of sample was taken out, and 36 µL of PBS solution containing 0.1% formic acid was added to terminate the reaction, followed by analysis by reduced LC-MS. The results are shown in FIGS. 1-3 and Table 1.

For compound G0, at 1 hour, the transglycosylation efficiency of Endo Se2 was comparable to that of Endo Si and significantly superior to those of Endo S2 and Endo S; at 6 hours, the transglycosylation efficiencies of Endo Se2, Endo Si, and Endo S2 were comparable and significantly superior to that of Endo S (FIG. 1, Table 1).

For compound G1, at 1 hour, the transglycosylation efficiency of Endo Si was superior to that of Endo Se2 and significantly superior to that of Endo S2. Endo S showed weak transglycosylation efficiency toward compound G1; at 6 hours, the transglycosylation efficiency of Endo Se2 was comparable to that of Endo Si and superior to Endo S2 (FIG. 2, Table 1).

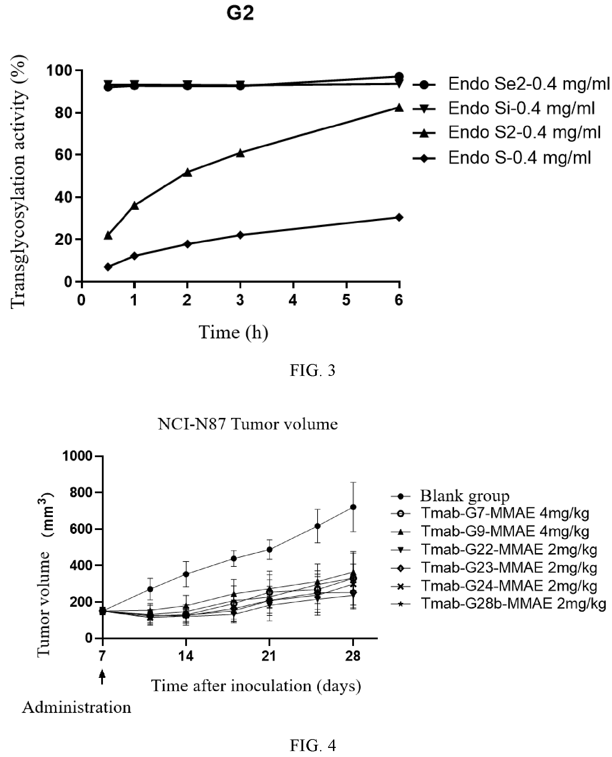

For compound G2, at 1 hour, the transglycosylation efficiency of Endo Si was comparable to that of Endo Se2 and significantly superior to Endo S2. Endo S showed weak transglycosylation efficiency toward compound G2; at 6 hours, the transglycosylation efficiency of Endo Se2 was comparable to that of Endo Si and superior to Endo S2 (FIG. 3, Table 1).

**Table 1 Transglycosylation activities of different endoglycosidases**

| **Disaccharide linker** | **Endoglycosidase** | **Transglycosylation efficiency (%)** | | | | |
|---|---|---|---|---|---|---|
| | | 0.5 hours | 1 hour | 2 hours | 3 hours | 6 hours |
| G0 | Endo Se2 | 96.6 | 97.9 | 98.0 | 97.2 | 96.2 |
| | Endo Si | 89.3 | 97.1 | 97.8 | 97.8 | 97.6 |
| | Endo S2 | 65.7 | 82.7 | 92.4 | 94.8 | 97.3 |
| | Endo S | 15.7 | 26.2 | 36.6 | 43.7 | 56.4 |
| G1 | Endo Se2 | 64.8 | 82.1 | 92.4 | 94.5 | 94.7 |
| | Endo Si | 93.2 | 95.2 | 95.5 | 95.5 | 95.5 |
| | Endo S2 | 33.1 | 49.2 | 67.7 | 77.3 | 87.5 |
| | Endo S | 5.6 | 9.3 | 13.0 | 15.7 | 21.1 |
| G2 | Endo Se2 | 92.2 | 92.9 | 92.8 | 92.7 | 97.2 |
| | Endo Si | 93.4 | 93.4 | 93.3 | 93.1 | 93.8 |
| | Endo S2 | 22.2 | 36.2 | 51.9 | 61.0 | 82.7 |
| | Endo S | 7.0 | 12.3 | 17.9 | 22.1 | 30.5 |

### Example 38: Study on the substrate specificity of endoglycosidases such as Endo Se2 and Endo Si toward various disaccharide linkers

Wild-type pertuzumab, disaccharide linker, and wild-type endoglycosidase (*i*.*e*., Endo S, Endo S2, Endo Si, or Endo Se2) were used such that the concentrations of the respective components were sequentially 10 mg/mL, 1.67 mM (25 equivalents relative to the antibody concentration), and 0.4 mg/mL. The pH of the reaction system was adjusted to 7.4, and the reaction temperature was 25°C. After 6 hours, 4 µL of the sample was taken out, and 36 µL of PBS solution containing 0.1% formic acid was added to terminate the reaction, followed by analysis by LC-MS.

For the disaccharide linker compounds, Endo Se2, Endo Si, Endo S2, and Endo S exhibited differential substrate specificity and transglycosylation efficiency. Endo Se2 and Endo Si demonstrated broader substrate specificity compared to Endo S2 and Endo S, and the optimal endoglycosidase for each substrate was typically Endo Se2 or Endo Si (Table 2).

**Table 2 Transglycosylation activities of different endoglycosidases**

| **Transglycosylation efficiency (%)** | **Enzyme** | | | |
|---|---|---|---|---|
| | **Endo Se2** | **Endo Si** | **Endo S2** | **Endo S** |
| **G0** | >95 | >95 | >95 | 56.4 |
| **G1** | 94.7 | >95 | 87.5 | 21.1 |
| **G2** | >95 | 93.8 | 82.7 | 30.5 |
| **G4** | 94.8 | >95 | 30 | 24 |
| **G4b** | 16.1 | 38.4 | 13.9 | 17.8 |
| **G5** | 10 | 34 | 0 | 0 |
| **G7** | 76.3 | 90.6 | 36.3 | 13 |
| **G9** | 36.3 | >95 | 4.7 | 7.6 |
| **G10** | 13.1 | 10.1 | 8.1 | 17.4 |
| **G13** | 75.3 | 51.8 | 6.9 | 0 |
| **G20** | 89 | 77.7 | 89.8 | 56.7 |
| **G21** | >95 | >95 | 72.6 | 29.4 |
| **G22** | >95 | 68.2 | 7.7 | 0 |
| **G23** | 56.7 | 93.7 | 6.0 | 2.6 |
| **G24** | 88.4 | 60.8 | 29.9 | 11.7 |
| **G25** | 22.5 | 21.2 | 23.6 | 15.9 |
| **G27** | 34.4 | >95 | 10.2 | 5.8 |
| **G28b** | 94.9 | >95 | 55 | 13.6 |
| **G32** | >95 | >95 | 25 | 10.8 |
| **G33** | >95 | >95 | 35.3 | 24.1 |
| **G35** | 80.1 | >95 | 7.8 | 6.6 |
| **G36** | 80.1 | >95 | >95 | 81.4 |
| **G37** | 94.2 | 51.3 | 11.7 | 11.0 |
| **G38** | 17.7 | 60.7 | 6.7 | 5.1 |
| **G39** | 34.1 | >95 | 23.5 | 20.9 |
| **G40** | >95 | >95 | 79.9 | 33.2 |
| **G42** | 59.4 | >95 | 13.1 | 9.7 |
| **G44** | >95 | >95 | 52.9 | 11.7 |
| **G45** | 53.1 | 15.1 | 6.9 | 0 |
| **G46** | 14.9 | 21.1 | 14.0 | 14.8 |

### 4. Glycoengineered antibodies

### Example 39: Preparation of glycoengineered antibody Ab-G1

The non-natural glycoengineered antibody Ab-G1 was obtained from compound G1 and the wild-type antibody Pertuzumab via general procedure I. The observed deconvoluted HRMS value of the heavy chain of the glycoengineered antibody Ab-G1 was 49893.48 Da.

### Example 40: Preparation of glycoengineered antibody Ab-G2

The non-natural glycoengineered antibody Ab-G2 was obtained from compound G2 and the wild-type antibody Pertuzumab via general procedure I. The observed deconvoluted HRMS value of the heavy chain of the glycoengineered antibody Ab-G2 was 49902.46 Da.

### Example 41: Preparation of glycoengineered antibody Ab-G7

The non-natural glycoengineered antibody Ab-G7 was obtained from compound G7 and the wild-type antibody Pertuzumab via general procedure I. The observed deconvoluted HRMS value of the heavy chain of the glycoengineered antibody Ab-G7 was 49810.12 Da.

### Example 42: Preparation of glycoengineered antibody Ab-G9

The non-natural glycoengineered antibody Ab-G9 was obtained from compound G9 and the wild-type antibody Pertuzumab via general procedure I. The observed deconvoluted HRMS value of the heavy chain of the glycoengineered antibody Ab-G9 was 50154.80 Da.

### Example 43: Preparation of glycoengineered antibody Ab-G10

The non-natural glycoengineered antibody Ab-G10 was obtained from compound G10 and the wild-type antibody Pertuzumab via general procedure I. The observed deconvoluted HRMS value of the heavy chain of the glycoengineered antibody Ab-G10 was 50081.15 Da.

### Example 44: Preparation of glycoengineered antibody Ab-G13

The non-natural glycoengineered antibody Ab-G13 was obtained from compound G13 and the wild-type antibody Pertuzumab via general procedure I. The observed deconvoluted HRMS value of the heavy chain of the glycoengineered antibody Ab-G13 was 49807.70 Da.

### Example 45: Preparation of glycoengineered antibody Ab-G27

The non-natural glycoengineered antibody Ab-G27 was obtained from compound G27 and the wild-type antibody Pertuzumab via general procedure I. The observed deconvoluted HRMS value of the heavy chain of the glycoengineered antibody Ab-G27 was 50024.69 Da.

### Example 46: Preparation of glycoengineered antibody Ab-G4

The non-natural glycoengineered antibody Ab-G4 was obtained from compound G4 and the wild-type antibody Pertuzumab via general procedure I. The observed deconvoluted HRMS value of the heavy chain of the glycoengineered antibody Ab-G4 was 49802.29 Da.

### Example 47: Preparation of glycoengineered antibody Ab-G4b

The non-natural glycoengineered antibody Ab-G4b was obtained from compound G4b and the wild-type antibody Pertuzumab via general procedure I. The observed deconvoluted HRMS value of the heavy chain of the glycoengineered antibody Ab-G4b was 49789.06 Da.

### Example 48: Preparation of glycoengineered antibody Ab-G5

The non-natural glycoengineered antibody Ab-G5 was obtained from compound G5 and the wild-type antibody Pertuzumab via general procedure I. The observed deconvoluted HRMS value of the heavy chain of the glycoengineered antibody Ab-G5 was 49818.48 Da.

### Example 49: Preparation of glycoengineered antibody Ab-G20

The non-natural glycoengineered antibody Ab-G20 was obtained from compound G20 and the wild-type antibody Pertuzumab via general procedure I. The observed deconvoluted HRMS value of the heavy chain of the glycoengineered antibody Ab-G20 was 49836.03 Da.

### Example 50: Preparation of glycoengineered antibody Ab-G21

The non-natural glycoengineered antibody Ab-G21 was obtained from compound G21 and the wild-type antibody Pertuzumab via general procedure I. The observed deconvoluted HRMS value of the heavy chain of the glycoengineered antibody Ab-G21 was 49835.47 Da.

### Example 51: Preparation of glycoengineered antibody Ab-G22

The non-natural glycoengineered antibody Ab-G22 was obtained from compound G22 and the wild-type antibody Pertuzumab via general procedure I. The observed deconvoluted HRMS value of the heavy chain of the glycoengineered antibody Ab-G22 was 49835.38 Da.

### Example 52: Preparation of glycoengineered antibody Ab-G23

The non-natural glycoengineered antibody Ab-G23 was obtained from compound G23 and the wild-type antibody Pertuzumab via general procedure I. The observed deconvoluted HRMS value of the heavy chain of the glycoengineered antibody Ab-G23 was 49835.18 Da.

### Example 53: Preparation of glycoengineered antibody Ab-G24

The non-natural glycoengineered antibody Ab-G24 was obtained from compound G24 and the wild-type antibody Pertuzumab via general procedure I. The observed deconvoluted HRMS value of the heavy chain of the glycoengineered antibody Ab-G24 was 50064.00 Da.

### Example 54: Preparation of glycoengineered antibody Ab-G25

The non-natural glycoengineered antibody Ab-G25 was obtained from compound G25 and the wild-type antibody Pertuzumab via general procedure I. The observed deconvoluted HRMS value of the heavy chain of the glycoengineered antibody Ab-G25 was 49893.33 Da.

### Example 55: Preparation of glycoengineered antibody Ab-G28b

The non-natural glycoengineered antibody Ab-G28b was obtained from compound G28b and the wild-type antibody Pertuzumab via general procedure I. The observed deconvoluted HRMS value of the heavy chain of the glycoengineered antibody Ab-G28b was 50175.80 Da.

### Example 56: Preparation of glycoengineered antibody Ab-G32

The non-natural glycoengineered antibody Ab-G32 was obtained from compound G32 and the wild-type antibody Pertuzumab via general procedure I. The observed deconvoluted HRMS value of the heavy chain of the glycoengineered antibody Ab-G32 was 49844.75 Da.

### Example 57: Preparation of glycoengineered antibody Ab-G33

The non-natural glycoengineered antibody Ab-G33 was obtained from compound G33 and the wild-type antibody Pertuzumab via general procedure I. The observed deconvoluted HRMS value of the heavy chain of the glycoengineered antibody Ab-G33 was 50047.05 Da.

### Example 58: Preparation of glycoengineered antibody Ab-G35

The non-natural glycoengineered antibody Ab-G35 was obtained from compound G35 and the wild-type antibody Pertuzumab via general procedure I. The observed deconvoluted HRMS value of the heavy chain of the glycoengineered antibody Ab-G35 was 49886.09 Da.

### Example 59: Preparation of glycoengineered antibody Ab-G36

The non-natural glycoengineered antibody Ab-G36 was obtained from compound G36 and the wild-type antibody Pertuzumab via general procedure I. The observed deconvoluted HRMS value of the heavy chain of the glycoengineered antibody Ab-G36 was 49891.14 Da.

### Example 60: Preparation of glycoengineered antibody Ab-G37

The non-natural glycoengineered antibody Ab-G37 was obtained from compound G37 and the wild-type antibody Pertuzumab via general procedure I. The observed deconvoluted HRMS value of the heavy chain of the glycoengineered antibody Ab-G37 was 49904.60 Da.

### Example 61: Preparation of glycoengineered antibody Ab-G38

The non-natural glycoengineered antibody Ab-G38 was obtained from compound G38 and the wild-type antibody Pertuzumab via general procedure I. The observed deconvoluted HRMS value of the heavy chain of the glycoengineered antibody Ab-G38 was 50018.20 Da.

### Example 62: Preparation of glycoengineered antibody Ab-G39

The non-natural glycoengineered antibody Ab-G39 was obtained from compound G39 and the wild-type antibody Pertuzumab via general procedure I. The observed deconvoluted HRMS value of the heavy chain of the glycoengineered antibody Ab-G39 was 50032.24 Da.

### Example 63: Preparation of glycoengineered antibody Ab-G40

The non-natural glycoengineered antibody Ab-G40 was obtained from compound G40 and the wild-type antibody Pertuzumab via general procedure I. The observed deconvoluted HRMS value of the heavy chain of the glycoengineered antibody Ab-G40 was 49833.19 Da.

### Example 64: Preparation of glycoengineered antibody Ab-G42

The non-natural glycoengineered antibody Ab-G42 was obtained from compound G42 and the wild-type antibody Pertuzumab via general procedure I. The observed deconvoluted HRMS value of the heavy chain of the glycoengineered antibody Ab-G42 was 50046.26 Da.

### Example 65: Preparation of glycoengineered antibody Ab-G44

The non-natural glycoengineered antibody Ab-G44 was obtained from compound G44 and the wild-type antibody Pertuzumab via general procedure I. The observed deconvoluted HRMS value of the heavy chain of the glycoengineered antibody Ab-G44 was 50624.49 Da.

### Example 66: Preparation of glycoengineered antibody Ab-G45

The non-natural glycoengineered antibody Ab-G45 was obtained from compound G45 and the wild-type antibody Pertuzumab via general procedure I. The observed deconvoluted HRMS value of the heavy chain of the glycoengineered antibody Ab-G45 was 50405.15 Da.

### Example 67: Preparation of glycoengineered antibody Ab-G46

The non-natural glycoengineered antibody Ab-G46 was obtained from compound G46 and the wild-type antibody Pertuzumab via general procedure I. The observed deconvoluted HRMS value of the heavy chain of the glycoengineered antibody Ab-G46 was 49865.77 Da.

### Example 68: Preparation of glycoengineered antibody Ab-G47

The non-natural glycoengineered antibody Ab-G47 was obtained from compound G47 and the wild-type antibody Pertuzumab via general procedure I. The observed deconvoluted HRMS value of the heavy chain of the glycoengineered antibody Ab-G47 was 49866.18 Da.

### 5. Preparation of glycosite-specific antibody-drug conjugate

### Example 69: Preparation of glycosite-specific ADC Ab-G1-DXd

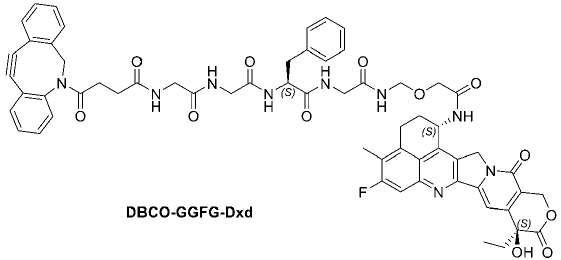

Ab-G1-DXd was obtained from compound DBCO-GGFG-Dxd and the non-natural glycoengineered antibody Ab-G1 via general procedure II. The observed deconvoluted HRMS value of the heavy chain was 51021.77 Da.

### Example 70: Preparation of glycosite-specific ADC Ab-G2-DXd

Ab-G2-DXd was obtained from compound DBCO-GGFG-Dxd and the non-natural glycoengineered antibody Ab-G2 via general procedure II. The observed deconvoluted HRMS value of the heavy chain was 52158.93 Da.

### Example 71: Preparation of glycosite-specific ADC Ab-G7-DXd

Ab-G7-DXd was obtained from compound DBCO-GGFG-Dxd and the non-natural glycoengineered antibody Ab-G7 via general procedure II. The observed deconvoluted HRMS value of the heavy chain was 50964.74 Da.

### Example 72: Preparation of glycosite-specific ADC Ab-G9-MMAE

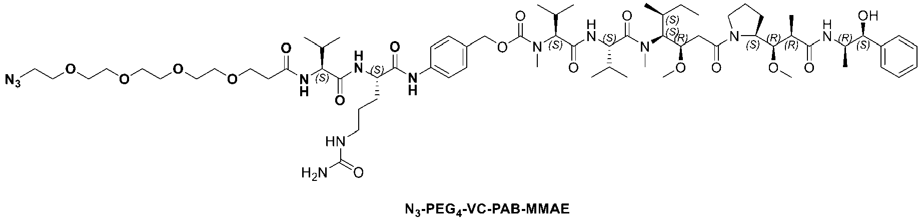

Ab-G9-MMAE was obtained from compound N₃-PEG₄-VC-PAB-MMAE and the non-natural glycoengineered antibody Ab-G9 via general procedure III. The observed deconvoluted HRMS value of the heavy chain was 51551.58 Da.

### Example 73: Preparation of glycosite-specific ADC Ab-G10-MMAE

Ab-G10-MMAE was obtained from compound N₃-PEG₄-VC-PAB-MMAE and the non-natural glycoengineered antibody Ab-G10 via general procedure III. The observed deconvoluted HRMS value of the heavy chain was 51478.22 Da.

### Example 74: Preparation of glycosite-specific ADC Ab-G13-DXd

Ab-G13-DXd was obtained from compound DBCO-GGFG-Dxd and the non-natural glycoengineered antibody Ab-G13 via general procedure II. The observed deconvoluted HRMS value of the heavy chain was 50978.76 Da.

### Example 75: Preparation of glycosite-specific ADC Ab-G27-DXd

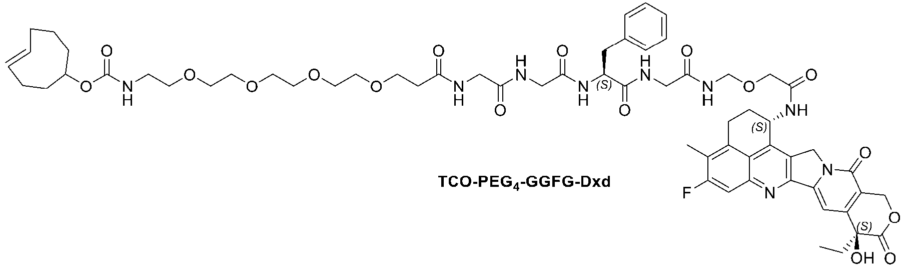

Ab-G27-DXd was obtained from compound TCO-PEG₄-GGFG-Dxd and the non-natural glycoengineered antibody Ab-G27 via general procedure IV. The observed deconvoluted HRMS value of the heavy chain was 51234.41 Da.

### Example 76: Preparation of glycosite-specific ADC Ab-G4b-MMAE

Ab-G4b-MMAE was obtained from compound N₃-PEG₄-VC-PAB-MMAE and the non-natural glycoengineered antibody Ab-G4b via general procedure VII. The observed deconvoluted HRMS value of the heavy chain was 51185.38 Da.

### Example 77: Preparation of glycosite-specific ADC Ab-G20-DXd

Ab-G20-DXd was obtained from compound DBCO-GGFG-Dxd and the non-natural glycoengineered antibody Ab-G20 via general procedure II. The observed deconvoluted HRMS value of the heavy chain was 50963.63 Da.

### Example 78: Preparation of glycosite-specific ADC Ab-G21-DXd

Ab-G21-DXd was obtained from compound DBCO-GGFG-Dxd and the non-natural glycoengineered antibody Ab-G21 via general procedure II. The observed deconvoluted HRMS value of the heavy chain was 50963.46 Da.

### Example 79: Preparation of glycosite-specific ADC Ab-G22-DXd

Ab-G22-DXd was obtained from compound DBCO-GGFG-Dxd and the non-natural glycoengineered antibody Ab-G22 via general procedure II. The observed deconvoluted HRMS value of the heavy chain was 52117.98 Da.

### Example 80: Preparation of glycosite-specific ADC Ab-G23-DXd

Ab-G23-DXd was obtained from compound DBCO-GGFG-Dxd and the non-natural glycoengineered antibody Ab-G23 via general procedure II. The observed deconvoluted HRMS value of the heavy chain was 52116.81 Da.

### Example 81: Preparation of glycosite-specific ADC Ab-G24-DXd

Ab-G24-DXd was obtained from compound DBCO-GGFG-Dxd and the non-natural glycoengineered antibody Ab-G24 via general procedure II. The observed deconvoluted HRMS value of the heavy chain was 52320.37 Da.

### Example 82: Preparation of glycosite-specific ADC Ab-G25-DXd

Ab-G25-DXd was obtained from compound DBCO-GGFG-Dxd and the non-natural glycoengineered antibody Ab-G25 via general procedure II. The observed deconvoluted HRMS value of the heavy chain was 52174.43 Da.

### Example 83: Preparation of glycosite-specific ADC Ab-G28b-DXd

Ab-G28b-DXd was obtained from compound DBCO-GGFG-Dxd and the non-natural glycoengineered antibody Ab-G28b via general procedure II. The observed deconvoluted HRMS value of the heavy chain was 52432.16 Da.

### Example 84: Preparation of glycosite-specific ADC Ab-G32-DXd

Ab-G32-DXd was obtained from compound DBCO-GGFG-Dxd and the non-natural glycoengineered antibody Ab-G32 via general procedure II. The observed deconvoluted HRMS value of the heavy chain was 52101.64 Da.

### Example 85: Preparation of glycosite-specific ADC Ab-G33-DXd

Ab-G33-DXd was obtained from compound DBCO-GGFG-Dxd and the non-natural glycoengineered antibody Ab-G33 via general procedure II. The observed deconvoluted HRMS value of the heavy chain was 52304.05 Da.

### Example 86: Preparation of glycosite-specific ADC Ab-G35-DXd

Ab-G35-DXd was obtained from compound DBCO-GGFG-Dxd and the non-natural glycoengineered antibody Ab-G35 via general procedure II. The observed deconvoluted HRMS value of the heavy chain was 53271.19 Da.

### Example 87: Preparation of glycosite-specific ADC Ab-G36-DXd

Ab-G36-DXd was obtained from compound DBCO-GGFG-Dxd and the non-natural glycoengineered antibody Ab-G36 via general procedure II. The observed deconvoluted HRMS value of the heavy chain was 51018.64 Da.

### Example 88: Preparation of glycosite-specific ADC Ab-G37-DXd

Ab-G37-DXd was obtained from compound DBCO-GGFG-Dxd and the non-natural glycoengineered antibody Ab-G37 via general procedure II. The observed deconvoluted HRMS value of the heavy chain was 51032.43 Da.

### Example 89: Preparation of glycosite-specific ADC Ab-G38-DXd-MMAE

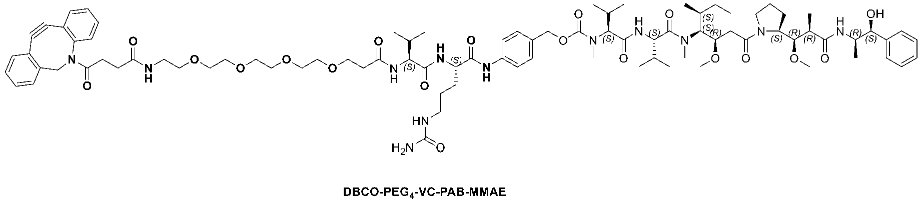

Ab-G38-DXd-MMAE was obtained from compounds TCO-PEG₄-GGFG-Dxd, DBCO-PEG₄-VC-PAB-MMAE, and the non-natural glycoengineered antibody Ab-G38 via general procedure VI. The observed deconvoluted HRMS value of the heavy chain was 52887.29 Da.

### Example 90: Preparation of glycosite-specific ADC Ab-G39-DXd-MMAE

Ab-G39-DXd-MMAE was obtained from compounds TCO-PEG₄-GGFG-Dxd, DBCO-PEG₄-VC-PAB-MMAE, and the non-natural glycoengineered antibody Ab-G39 via general procedure VI. The observed deconvoluted HRMS value of the heavy chain was 52900.70 Da.

### Example 91: Preparation of glycosite-specific ADC Ab-G40-DXd

Ab-G40-DXd was obtained from compound DBCO-GGFG-Dxd and the non-natural glycoengineered antibody Ab-G40 via general procedure II. The observed deconvoluted HRMS value of the heavy chain was 50961.38 Da.

### Example 92: Preparation of glycosite-specific ADC Ab-G42-DXd-MMAE

Ab-G42-DXd-MMAE was obtained from compounds TCO-PEG₄-GGFG-Dxd, DBCO-PEG₄-VC-PAB-MMAE, and the non-natural glycoengineered antibody Ab-G42 via general procedure VI. The observed deconvoluted HRMS value of the heavy chain was 52914.89 Da.

### Example 93: Preparation of glycosite-specific ADC Ab-G44-DXd-MMAE

Ab-G44-DXd-MMAE was obtained from compounds TCO-PEG₄-GGFG-Dxd, DBCO-PEG₄-VC-PAB-MMAE, and the non-natural glycoengineered antibody Ab-G44 via general procedure VI. The observed deconvoluted HRMS value of the heavy chain was 53492.60 Da.

### Example 94: Preparation of glycosite-specific ADC Ab-G45-DXd-MMAE

Ab-G45-DXd-MMAE was obtained from compounds TCO-PEG₄-GGFG-Dxd, DBCO-PEG₄-VC-PAB-MMAE, and the non-natural glycoengineered antibody Ab-G45 via general procedure VI. The observed deconvoluted HRMS value of the heavy chain was 53300.04 Da.

### Example 95: Preparation of glycosite-specific ADC Ab-G46-DXd

Ab-G46-DXd was obtained from compound DBCO-GGFG-Dxd and the non-natural glycoengineered antibody Ab-G46 via general procedure II. The observed deconvoluted HRMS value of the heavy chain was 52174.17 Da.

### Example 96: Preparation of glycosite-specific ADC Ab-G47-DXd

Ab-G47-DXd was obtained from compound DBCO-GGFG-Dxd and the non-natural glycoengineered antibody Ab-G47 via general procedure II. The observed deconvoluted HRMS value of the heavy chain was 52173.90 Da.

### Example 97: Preparation of glycosite-specific ADC Ab-G40-DBCO-PEG₄-VC-PAB-MMAE

Ab-G40-DBCO-PEG₄-VC-PAB-MMAE was obtained from the wild-type antibody Pertuzumab and the payload-disaccharide conjugate G40-DBCO-PEG₄-VC-PAB-MMAE via general procedure V. The observed deconvoluted HRMS value of the heavy chain was 51491.63 Da.

### 6. Preparation of glycosite-specific ADC based on antibody lacking core fucosylation

### Example 98: Preparation of glycosite-specific ADC based on antibody lacking core fucosylation

Wild-type pertuzumab was dissolved in a buffer containing 20 mM phosphate and 150 mM NaCl at pH 7.4 to a concentration of 10 mg/mL. Wild-type Alfc (derived from *Lacticaseibacillus paracasei,* GENBANK accession number WP_012492118.1) was added to a final concentration of 0.5 mg/mL for hydrolysis, followed by overnight incubation at 37°C. A defucosylated antibody (Ab-defuc) containing one N-acetylglucosamine was obtained through purification using Protein A magnetic beads.

The prepared defucosylated antibody, disaccharide linker, and wild-type endoglycosidase (Endo Se2 or Endo Si) were used such that the concentrations of the respective components were sequentially 10 mg/mL, 1.67 mM (25 equivalents relative to the antibody concentration), and 0.4 mg/mL. The pH of the reaction system was adjusted to 7.4, and the reaction temperature was 25°C. After 6 hours, 4 µL of the sample was taken out, and 36 µL of PBS solution containing 0.1% formic acid was added to terminate the reaction, followed by analysis by LC-MS. The observed deconvoluted HRMS values of the heavy chains are shown in Table 3.

The defucosylated non-natural glycoengineered antibodies Ab-defuc-G7, G20, G21, G23, G33, G35, and G40 were reacted with compound DBCO-GGFG-Dxd according to General Procedure II to obtain the corresponding ADCs, respectively. The observed deconvoluted HRMS values of the heavy chains are shown in Table 3.

The defucosylated non-natural glycoengineered antibodies Ab-defuc-G38, G39, G42, G44, and G45 were reacted with compounds TCO-PEG₄-GGFG-Dxd and DBCO-PEG₄-VC-PAB-MMAE according to General Procedure VI to obtain the corresponding ADCs, respectively. The observed deconvoluted HRMS values of the heavy chains are shown in Table 3.

**Table 3 Preparation of glycosite-specific ADC based on antibody lacking core fucosylation**

| Antibody | Disaccharide linker | Molecular weight of transglycosylated antibody (Da) | Linker-payload | Molecular weight of glycosite-specific ADC (Da) |
|---|---|---|---|---|
| Ab-defuc | - | 49283.84 | - | - |
| Ab-defuc | G7 | 49664.23 | DBCO-GGFG-Dxd | 50818.78 |
| Ab-defuc | G20 | 49690.03 | DBCO-GGFG-Dxd | 50817.53 |
| Ab-defuc | G21 | 49689.14 | DBCO-GGFG-Dxd | 50817.49 |
| Ab-defuc | G23 | 49663.11 | DBCO-GGFG-Dxd | 51969.36 |
| Ab-defuc | G33 | 49901.12 | DBCO-GGFG-Dxd | 52157.45 |
| Ab-defuc | G35 | 49646.46 | DBCO-GGFG-Dxd | 53124.32 |
| Ab-defuc | G38 | 49872.52 | TCO-PEG₄-GGFG-Dxd/ DBCO-PEG₄-VC-PAB-MMAE | 52740.79 |
| Ab-defuc | G39 | 49885.94 | TCO-PEG₄-GGFG-Dxd/ DBCO-PEG₄-VC-PAB-MMAE | 52754.62 |
| Ab-defuc | G40 | 49687.05 | DBCO-GGFG-Dxd | 50815.49 |
| Ab-defuc | G42 | 49900.29 | TCO-PEG₄-GGFG-Dxd/ DBCO-PEG₄-VC-PAB-MMAE | 52768.25 |
| Ab-defuc | G44 | 50477.69 | TCO-PEG₄-GGFG-Dxd/ DBCO-PEG₄-VC-PAB-MMAE | 53346.16 |
| Ab-defuc | G45 | 50258.23 | TCO-PEG₄-GGFG-Dxd/ DBCO-PEG₄-VC-PAB-MMAE | 53152.98 |

Conclusion: The ADC preparation method of the present disclosure is applicable to the preparation of ADCs lacking core fucosylation.

### 7. Efficacy of glycosite-specific antibody-drug conjugate

### Example 99: Preparation of HER2-targeted glycosite-specific ADCs

### (1) Synthesis of Tmab-G7, G9, G22, G23, G24, and G28b

The wild-type antibody trastuzumab (Tmab), disaccharide oxazolines (*i.e.,* compounds G7, G9, G22, G23, G24, and G28b), wild-type endoglycosidases Endo Si (for compounds G7, G9, G23, and G28b) and Endo Se2 (for compounds G22 and G24) were used such that the concentrations of the respective components were 10 mg/mL, 1.67 mM (25 equivalents relative to the antibody concentration), and 0.6 mg/mL. The pH of the reaction system was adjusted to 7.4, and the reaction temperature was 25°C with stirring at 800 rpm for 3 hours. The corresponding transglycosylated antibodies were obtained by removing small-molecule compounds and glycoside hydrolases through purification with Protein A.

### (2) Preparation of corresponding ADCs

The prepared transglycosylated antibody Tmab-G7 and DBCO-PEG₄-VC-PAB-MMAE were used such that the concentrations thereof were 5 mg/mL and 0.33 mM, respectively. The pH of the reaction system was adjusted to 7.4, followed by overnight incubation at 25°C. The desired glycosite-specific conjugated ADC, Tmab-G7-MMAE, was obtained by ultrafiltration and analyzed by LC-MS, HIC, and SEC.

The prepared transglycosylated antibody Tmab-G9 and N₃-PEG₄-VC-PAB-MMAE were used such that the concentrations thereof were 5 mg/mL and 0.33 mM, respectively. The pH of the reaction system was adjusted to 7.4, followed by overnight incubation at 25°C. The desired glycosite-specific conjugated ADC, Tmab-G9-MMAE, was obtained by ultrafiltration and analyzed by LC-MS, HIC, and SEC. The prepared transglycosylated antibodies (Tmab-G22, G23, G24, and G28b) and DBCO-PEG₄-VC-PAB-MMAE were used such that the concentrations thereof were 5 mg/mL and 0.33 mM, respectively. The pH of the reaction system was adjusted to 7.4, followed by overnight incubation at 25°C, after which an additional 8 equivalents of DBCO-PEG₄-VC-PAB-MMAE were supplemented. The total reaction time was 24 h. The desired glycosite-specific conjugated ADCs Tmab-G22/G23/G24/G28b-MMAE were obtained by ultrafiltration and analyzed by LC-MS, hydrophobic interaction chromatography (HIC), and size exclusion chromatography (SEC).

### (3) Detection methods

SEC: Chromatographic column: TSKgel G3000SWXL (7.8 mm × 30 cm, 5 µm); Mobile phase: 0.2 M phosphate buffer, 5-15% isopropanol, pH 7.0; Flow rate: 0.5-1 mL/min; Detection wavelength: 280 nm & 248 nm & 360 nm; Column temperature: room temperature; Injection amount: 30 µg; Elution method: isocratic elution.

HIC: Chromatographic column: TSKgel Butyl-NPR (4.6 mm × 10 cm, 2.5 µm); Mobile phase A: 0.05 M phosphate buffer, 1.2 M ammonium sulfate, pH 7.0; Mobile phase B: 0.05 M phosphate buffer, 20% isopropanol, pH 7.0; Flow rate: 0.5 mL/min; Detection wavelength: 280 nm & 248 nm & 360 nm; Column temperature: room temperature; Injection amount: 30 µg; Elution method: mobile phase B was increased from 0% to 100% over 20 minutes.

The quality control data of the prepared HER2-targeted glycosite-specific ADC are shown in Table 4.

**Table 4 Quality control data of the HER2-targeted glycosite-specific ADCs**

| **ADC Name** | **Target** | **Payload** | **Molecular weight (Da)** | **Drug-to-antibody ratio (HIC)** | **SEC (%)** |
|---|---|---|---|---|---|
| Tmab-G7-MMAE | HER2 | MMAE | 149926.45 | 1.95 | 99.29 |
| Tmab-G9-MMAE | HER2 | MMAE | 150041.06 | 2 | 98.21 |
| Tmab-G22-MMAE | HER2 | MMAE | 153293.84 | 3.64 | 99.61 |
| Tmab-G23-MMAE | HER2 | MMAE | 153293.86 | 3.84 | 99.60 |
| Tmab-G24-MMAE | HER2 | MMAE | 153699.92 | 3.71 | 99.86 |
| Tmab-G28b-MMAE | HER2 | MMAE | 153922.45 | 3.7 | 99.77 |

### Example 100: In vitro efficacy of HER2-targeted glycosite-specific ADC

The purpose of this experiment was to evaluate the *in vitro* inhibitory activities of the ADC compounds of the present disclosure against SK-BR-3 (human breast adenocarcinoma cells), NCI-N87 (human gastric cancer cells), and MDA-MB-468 (human breast cancer cells).

Tumor cells SK-BR-3 (source: Cell Resource Center, Shanghai Institutes for Biological Sciences, Chinese Academy of Sciences), NCI-N87 (source: Cell Resource Center, Shanghai Institutes for Biological Sciences, Chinese Academy of Sciences), or MDA-MB-468 (source: Cell Bank of Committee on Type Culture Collection of Chinese Academy of Sciences) in the logarithmic growth phase were taken and added to a cell plate at a density of 2000 cells per well. The cell plate was placed in a 37°C, 5% CO₂ cell incubator and incubated for 12-16 hours. 100 µL of sample was added to each well (starting at 50 µg/mL, with 5-fold serial dilution, 9 concentrations), gently mixed, and then placed in the incubator for incubation. After 144 hours of incubation, 70 µL of CellTiter-Glo^{™} (Promega, Catalog No.: G7572) working solution was added, and the plate was gently shaken to lyse the cells, followed by reading on a microplate reader. The cell proliferation inhibition rate was calculated using the formula: cell proliferation inhibition rate = (1-sample well/control well) × 100%. Using GraphPad Prism 8.0 software, the Log value of the sample concentration was plotted on the x-axis and Cytotoxicity% on the y-axis, and the data were subjected to a Nonlinear regression (curve fit) analysis to obtain the IC₅₀ values of the test samples. The specific results are shown in Table 5.

**Table 5 In vitro anti-tumor activity assay of the ADC compounds of the present disclosure**

| **ADC compound** | **SK-BR-3 (HER2+++)** | | **NCI-N87 (HER2+++)** | | **MDA-MB-468 (HER2-)** | |
|---|---|---|---|---|---|---|
| | **IC₅₀ (µg/mL)** | **Max inhibition(%)** | **IC₅₀ (µg/mL)** | **Max inhibition(%)** | **IC₅₀ (µg/mL)** | **Max inhibition(%)** |
| **Tmab-G7-MMAE** | 0.004 | 92 | 0.005 | 81 | 44 | 60 |
| **Tmab-G9-MMAE** | 0.003 | 92 | 0.003 | 80 | 34 | 76 |
| **Tmab-G22-MMAE** | 0.002 | 92 | 0.003 | 82 | 35 | 77 |
| **Tmab-G23-MMAE** | 0.001 | 92 | 0.002 | 84 | 31 | 81 |
| **Tmab-G24-MMAE** | 0.001 | 92 | 0.002 | 82 | 19 | 84 |
| **Tmab-G28b-MMAE** | 0.002 | 92 | 0.003 | 81 | 15 | 86 |

Conclusion: The ADC drugs of the present disclosure have significant proliferation inhibitory activity against HER2-positive cells SK-BR-3 and NCI-N87; meanwhile, they have weak proliferation inhibitory activity against HER2-negative cells MDA-MB-468, demonstrating good selectivity.

### Example 101: In vivo efficacy of HER2-targeted glycosite-specific ADC

BALB/c Nude mice (purchased from Beijing Vital River) were used as test animals to evaluate the efficacy of anti-HER2 ADC administered via tail vein injection in nude mice bearing human gastric cancer NCI-N87 xenografts.

NCI-N87 cells (source: ATCC) (5 × 10⁶/mouse, containing 50% Matrigel) were subcutaneously inoculated into the right axilla of mice. After tumor growth for 7 days, when the average tumor volume reached about 150 mm³, the animals were randomly grouped based on tumor volume (D7), with 7 mice per group.

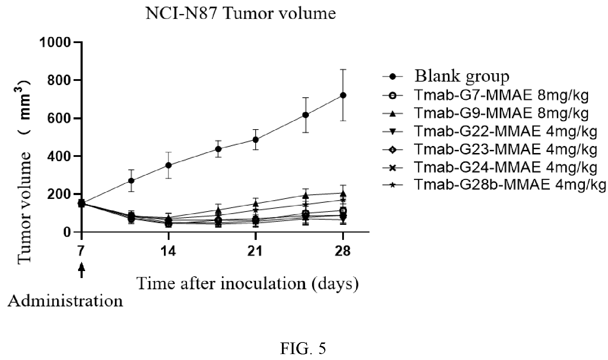

A single dose was administered via tail vein injection. Tumor volume and body weight were measured twice per week, and the data were recorded. The tumor growth inhibition rate TGI (%) was calculated as [1-(T₂₈-T₇) / (V₂₈-V₇)] × 100, where T₂₈ and T₇ represent the tumor volumes of the experimental group on Day 28 and Day 7 post-inoculation, respectively, and V₂₈ and V₇ represent the tumor volumes of the blank control group (Vehicle, PBS) on Day 28 and Day 7 post-inoculation, respectively. The specific experimental results at the end of the experiment on Day 28 post-inoculation are shown in Table 6 and FIGS. 4-5.

**Table 6 In vivo efficacy evaluation of the ADC compounds of the present disclosure**

| **ADC compound** | **Administration dose** | **Tumor growth inhibition rate** |
|---|---|---|
| **Tmab-G7-MMAE** | 4 mpk | 68.6% |
| **Tmab-G9-MMAE** | 4 mpk | 62.4% |
| **Tmab-G22-MMAE** | 2 mpk | 85.3% |
| **Tmab-G23-MMAE** | 2 mpk | 82.2% |
| **Tmab-G24-MMAE** | 2 mpk | 74.1% |
| **Tmab-G28b-MMAE** | 2 mpk | 68.0% |
| **Tmab-G7-MMAE** | 8 mpk | 106.5% |
| **Tmab-G9-MMAE** | 8 mpk | 90.6% |
| **Tmab-G22-MMAE** | 4 mpk | 115.6% |
| **Tmab-G23-MMAE** | 4 mpk | 111.5% |
| **Tmab-G24-MMAE** | 4 mpk | 110.8% |
| **Tmab-G28b-MMAE** | 4 mpk | 96.4% |

Conclusion: The ADC drugs of the present disclosure exhibit excellent anti-tumor activity against NCI-N87 xenograft tumors with high HER2 expression.

## Claims

1. A disaccharide linker represented by formula (I): 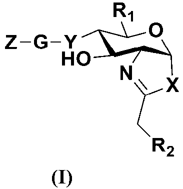 wherein
X is an oxygen atom or a sulfur atom;
Y is an oxygen atom or a sulfur atom;
R₁ is selected from the group consisting of: 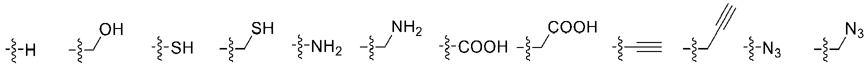 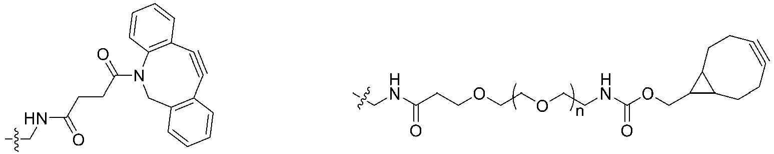 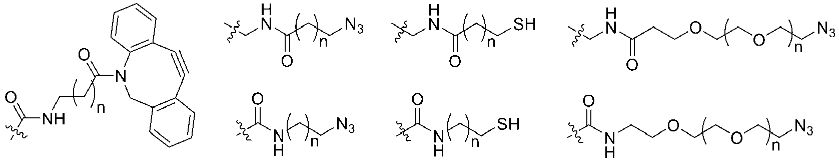 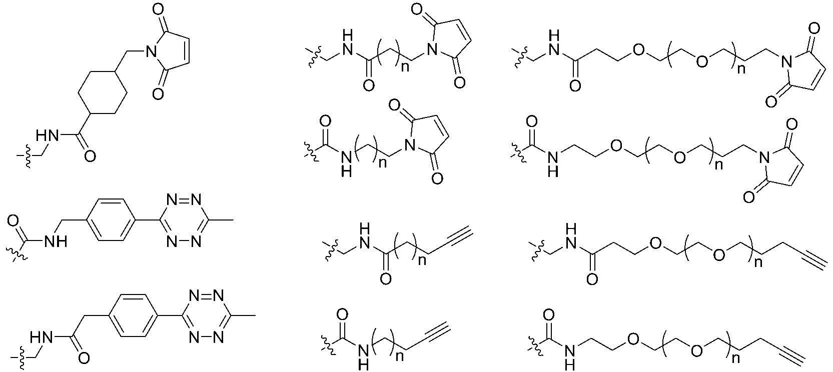
wherein n is an integer selected from the group consisting of 0-30, or an integer from 0-20, or an integer from 0-10;
R₂ is selected from the group consisting of: hydrogen, -N₃, C₁-C₆ alkyl-N₃, -C≡CH, C₁-C₆ alkyl-C≡CH, -SH, C₁-C₆ alkyl-SH, and C₁-C₆ alkyl;
ring G represents a structure derived from a monosaccharide molecule, which may be linked to another monosaccharide via a thioether bond or a glycosidic bond, wherein the monosaccharide molecule is selected from the group consisting of galactose, *N-*acetylgalactosamine, glucose, *N*-acetylglucosamine, *N*-acetylmannosamine, mannose, fucose, and sialic acid; when Y is a sulfur atom, ring G is linked to 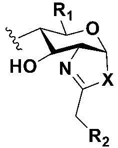 via a thioether bond, wherein the thioether bond is a 1,4-thioether bond, a 2,4-thioether bond, or a 3,4-thioether bond; when Y is an oxygen atom, ring G is linked to 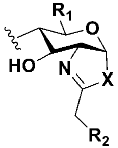 via a glycosidic bond, wherein the glycosidic bond is a 1,4-glycosidic bond, a 2,4-glycosidic bond, or a 3,4-glycosidic bond;
Z is hydrogen, a reactive group containing an azide residue, a reactive group containing a cycloalkyne residue, a reactive group containing a tetrazine residue, a reactive group containing an alkyne residue, a reactive group containing a cycloalkene residue, or a reactive group containing a maleimide residue.

2. The disaccharide linker according to claim 1, wherein the glycosidic bond is a 1,4-glycosidic bond, or the thioether bond is a 1,4-thioether bond.

3. The disaccharide linker according to any one of claims 1-2, wherein the monosaccharide molecule is selected from the group consisting of galactose, glucose, mannose, *N*-acetylglucosamine, *N*-acetylgalactosamine, and *N*-acetylmannosamine.

4. The disaccharide linker according to any one of claims 1-3, wherein the R₁ is selected from the group consisting of: 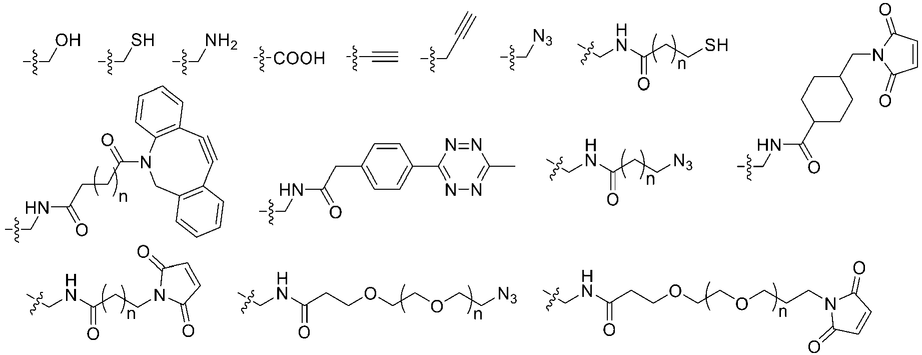 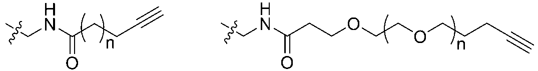 wherein n is an integer selected from the group consisting of 0-30, or an integer from 0-20, or an integer from 0-10.

5. The disaccharide linker according to any one of claims 1-4, wherein R₂ is selected from the group consisting of: hydrogen, -N₃, -CH₂-N₃, -CH₂-CH₂-N₃, -C=CH, -CH₂-C≡CH, -CH₂-CH₂-C≡CH, -SH, -CH₂-SH, -CH₂-CH₂-SH, and C₁-C₆ alkyl; or, R₂ is selected from the group consisting of hydrogen, -N₃, -CH₂-N₃, -CH₂-CH₂-N₃, -C≡CH, -SH, -CH₃, -CH₂-CH₃, and - CH₂-CH₂-CH₃.

6. The disaccharide linker according to any one of claims 1-5, wherein Z is a reactive group containing an azide residue, which is selected from the group consisting of: 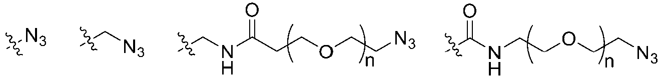 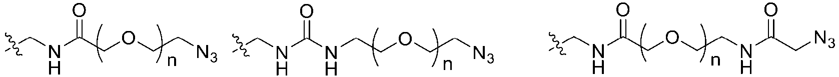 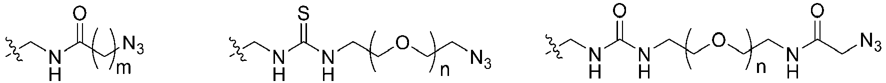 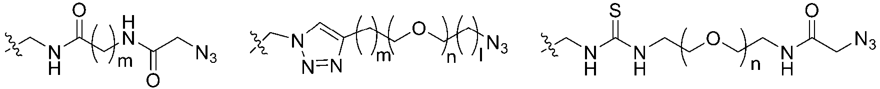 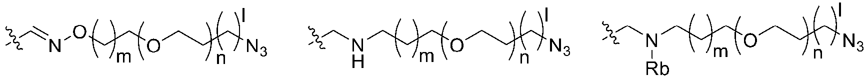 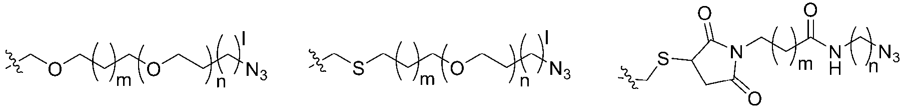 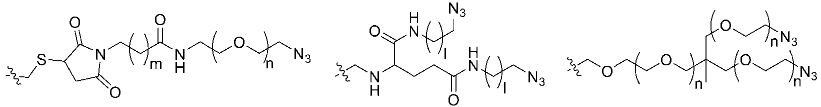 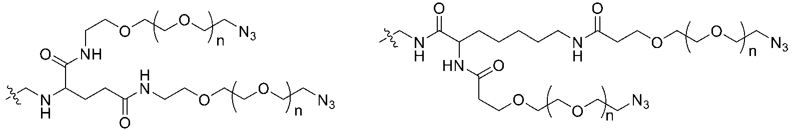 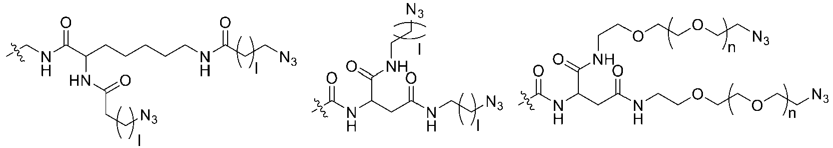 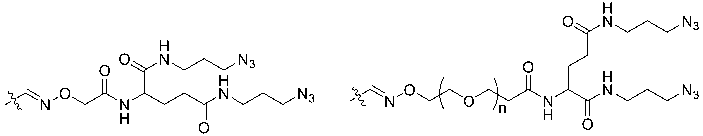 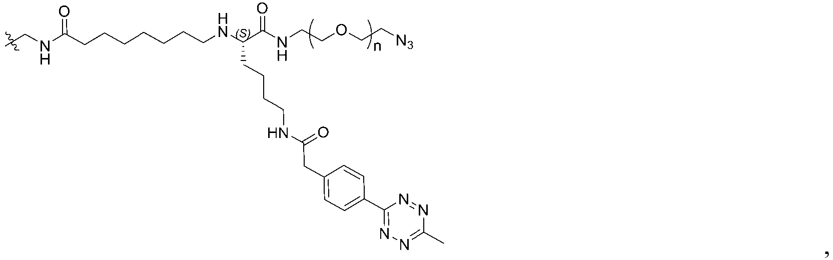 or
the reactive group containing an azide residue is selected from the group consisting of: 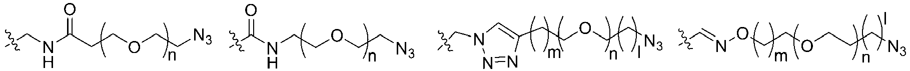 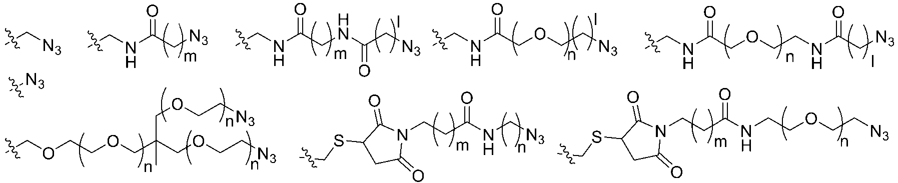 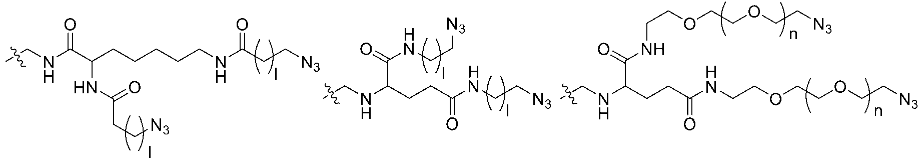 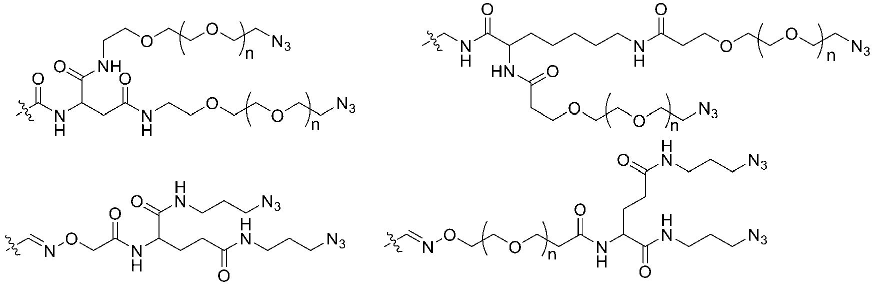 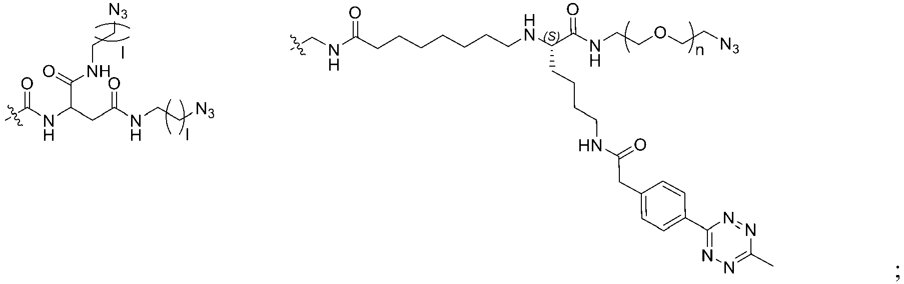 or
Z is a reactive group containing a cycloalkyne residue, which is selected from the group consisting of: 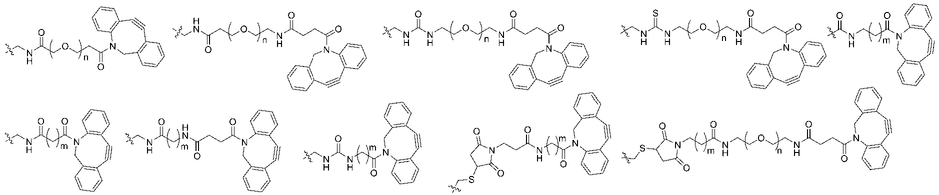 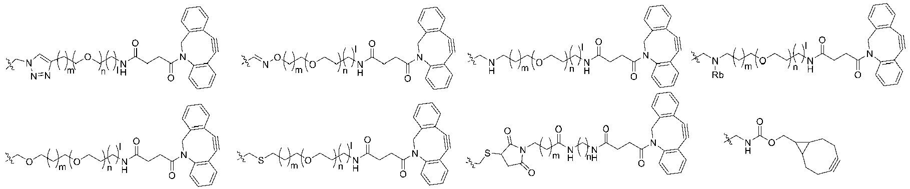 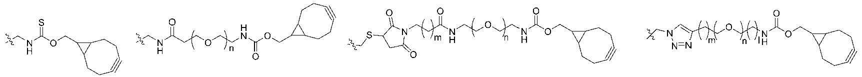 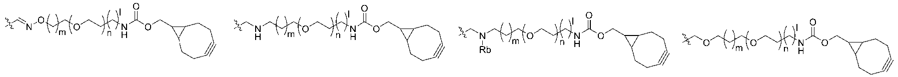 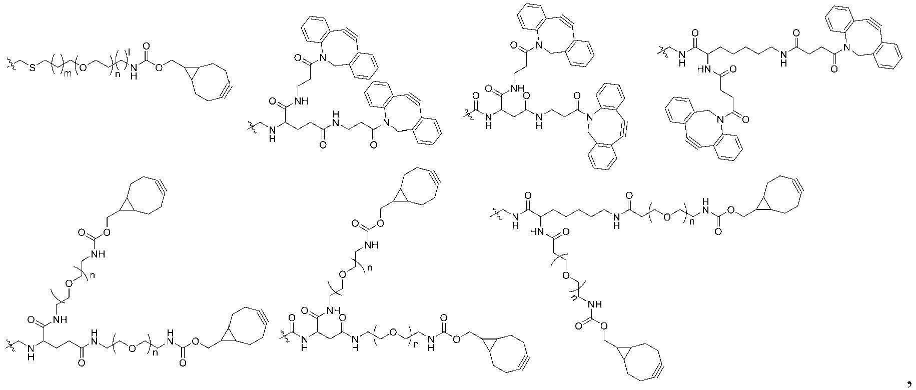 or
the reactive group containing a cycloalkyne residue is selected from the group consisting of: 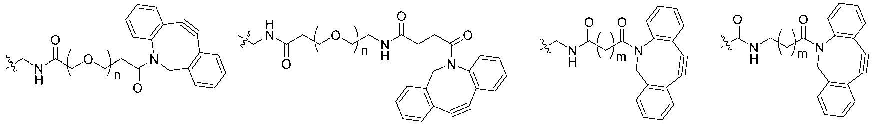 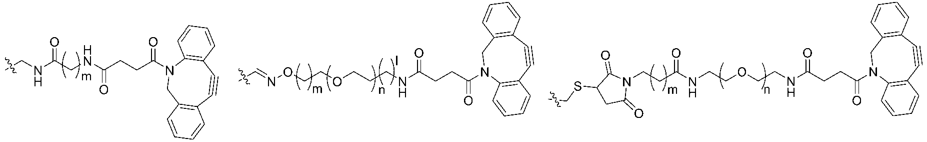 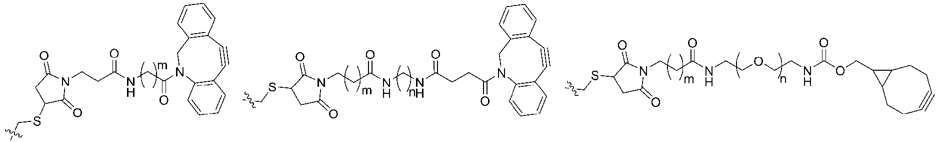 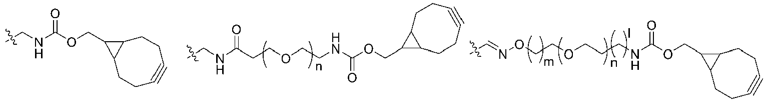 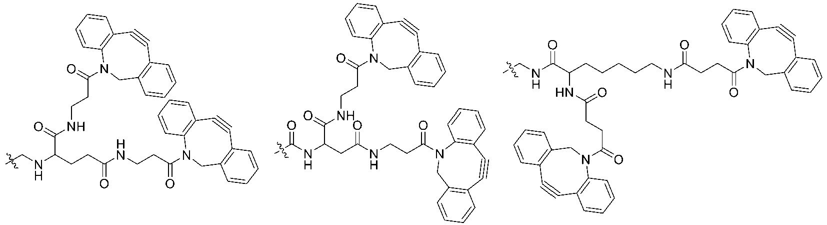 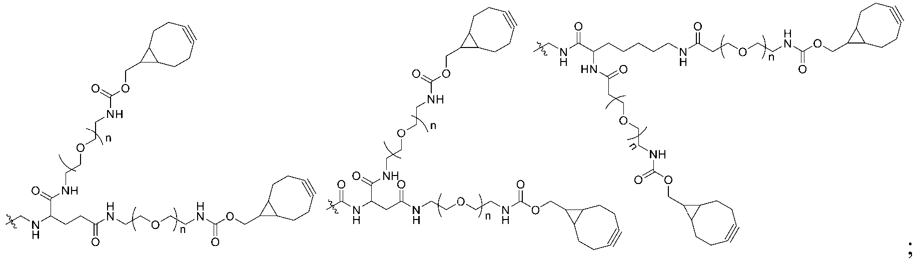 or
Z is a reactive group containing an alkyne residue, which is selected from the group consisting of: 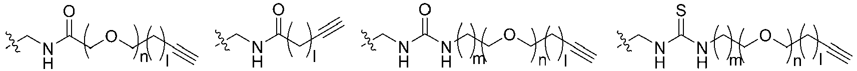 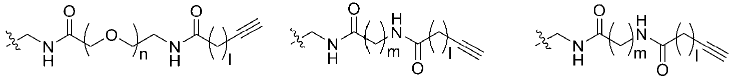 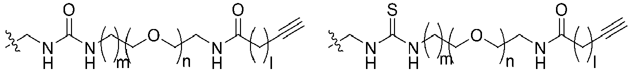 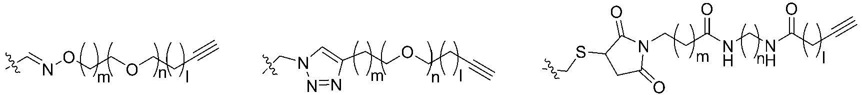 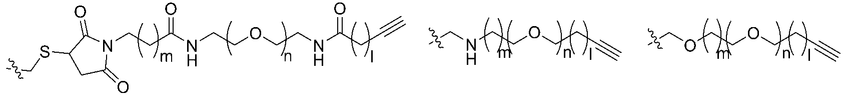 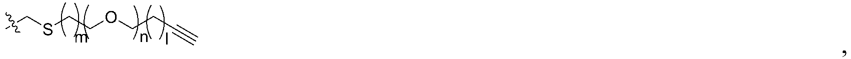 or
the reactive group containing an alkyne residue is selected from the group consisting of: 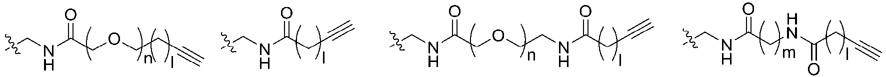 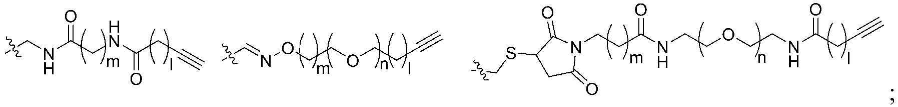 or
Z is a reactive group containing a tetrazine residue, which is selected from the group consisting of: 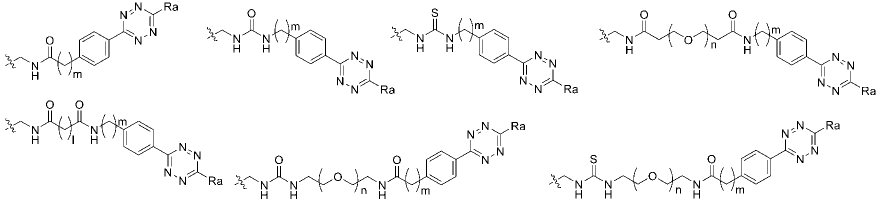 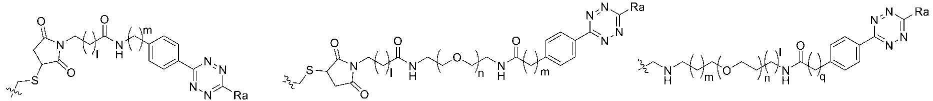 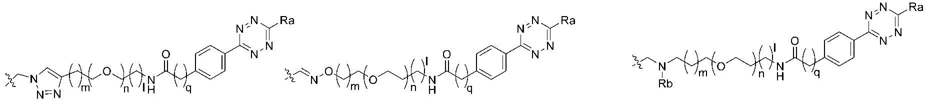 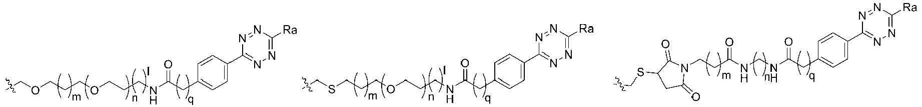 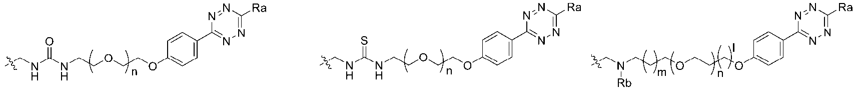 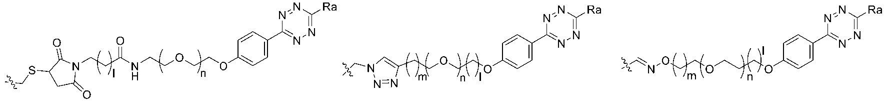 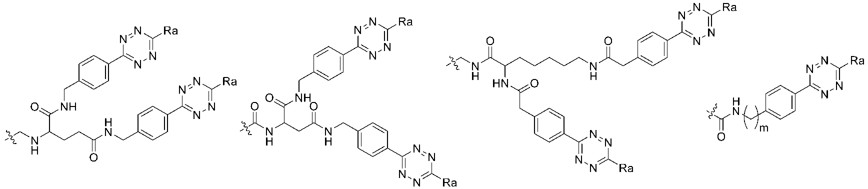 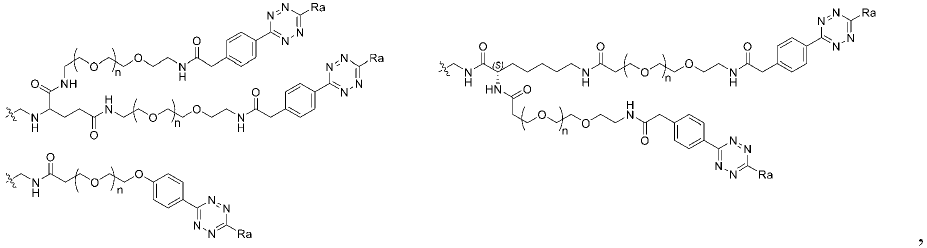 or
the reactive group containing a tetrazine residue is selected from the group consisting of: 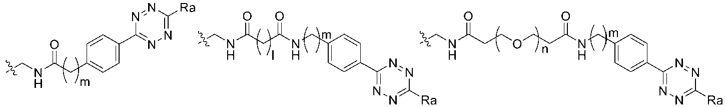 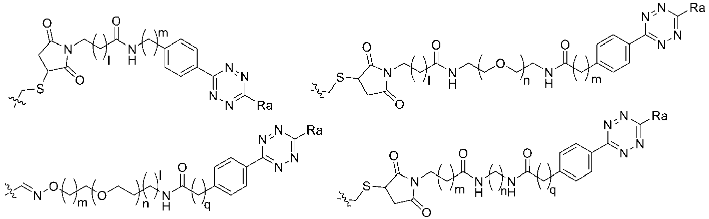 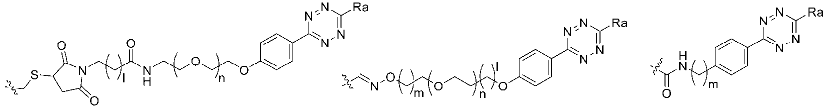 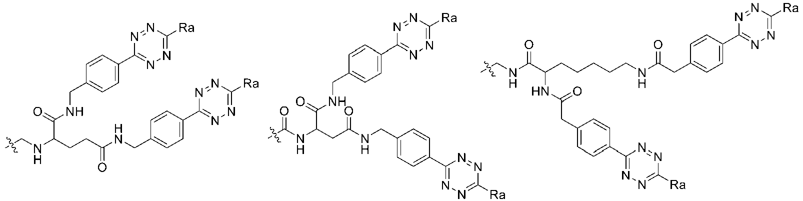 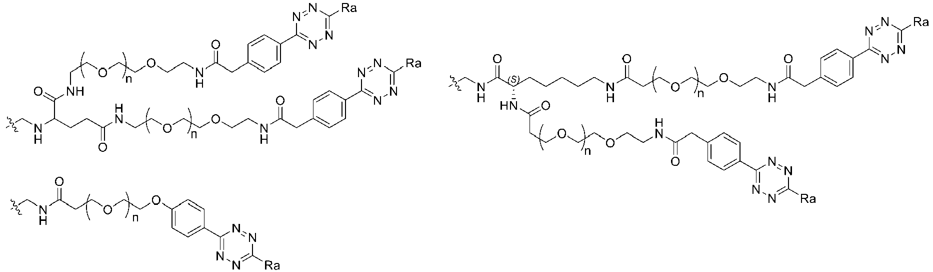 ; or
Z is a reactive group containing a cycloalkene residue, which is selected from the group consisting of: 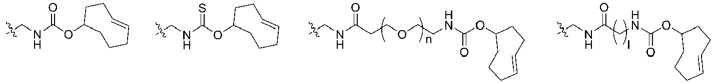 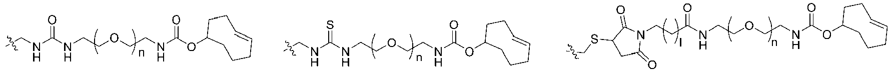 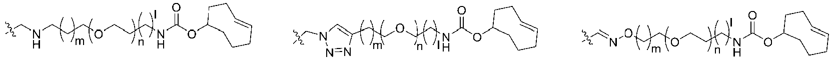 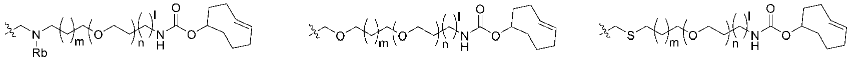 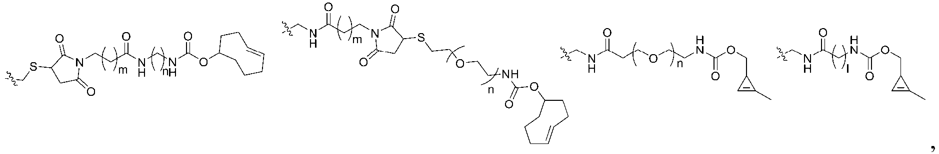 or
the reactive group containing a cycloalkene residue is selected from the group consisting of: 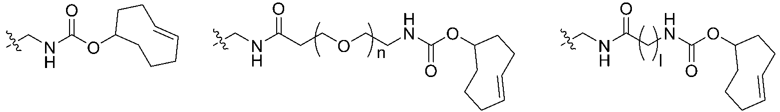 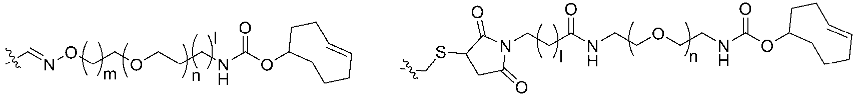 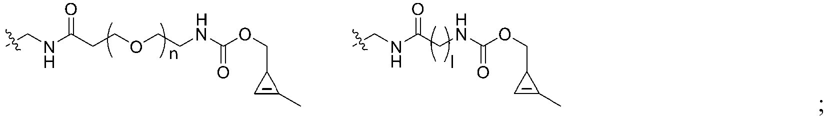 or
Z is a reactive group containing a maleimide residue, which is selected from the group consisting of: 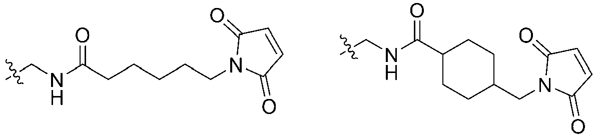 wherein each n, each m, each I, and each q is independently an integer selected from the group consisting of 0-30, or an integer from 0-20, or an integer from 0-10; each Ra is hydrogen or C₁-C₆ alkyl; Rb is hydrogen, C₁-C₆ alkyl, C₆-C₁₀ aryl, or C₅-C₁₀ heteroaryl.

7. The disaccharide linker according to claim 1, selected from the group consisting of the following specific compounds: 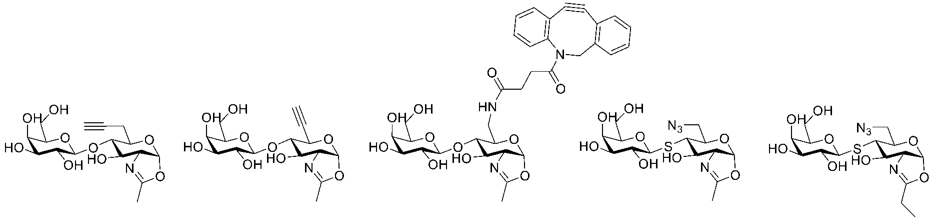 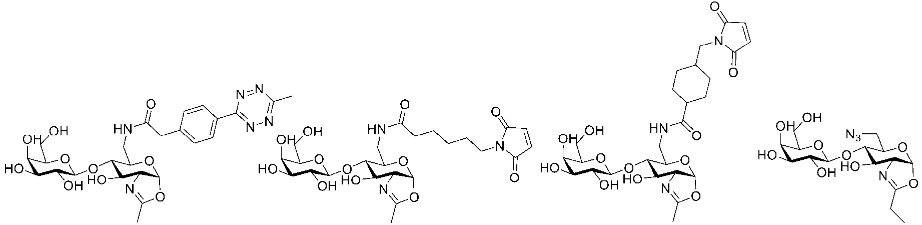 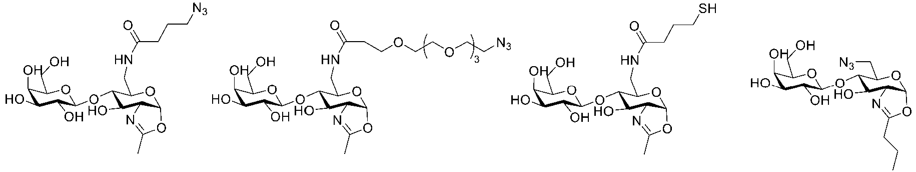 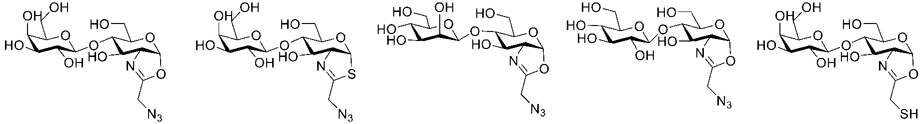 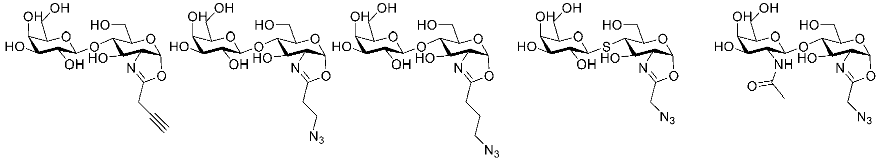 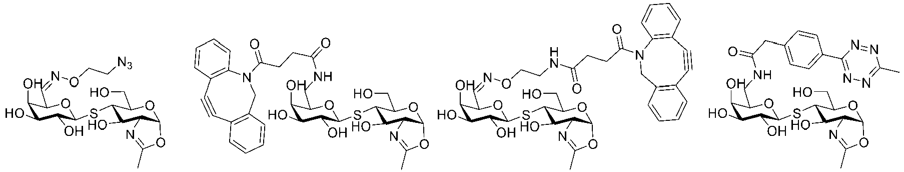 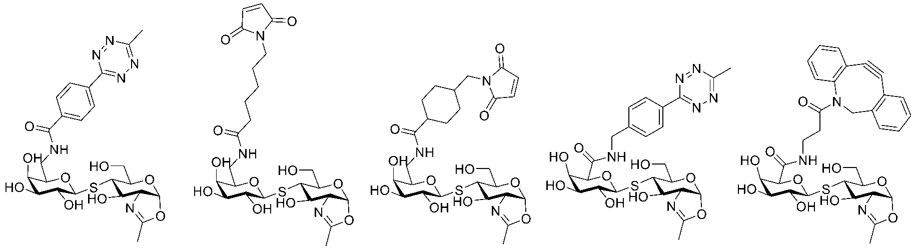 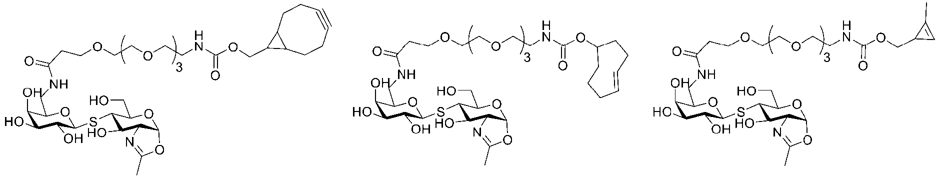 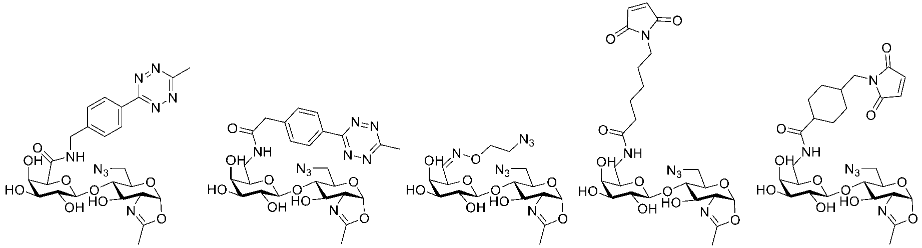 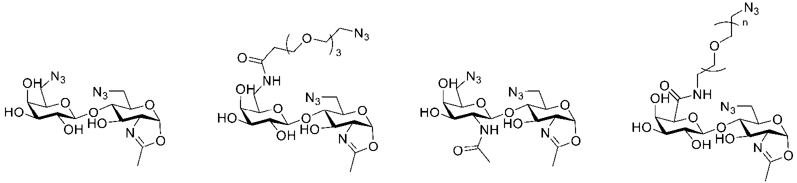 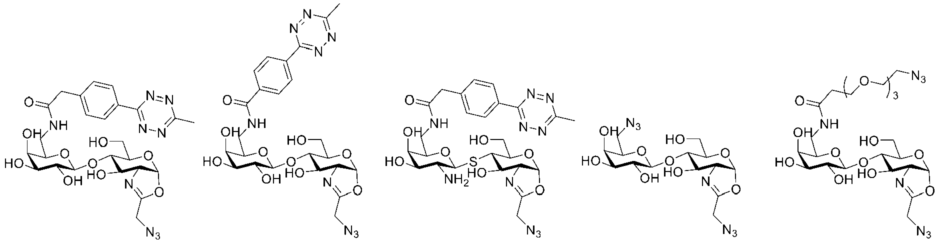 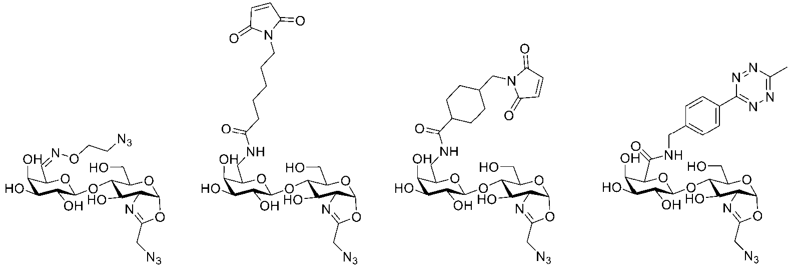 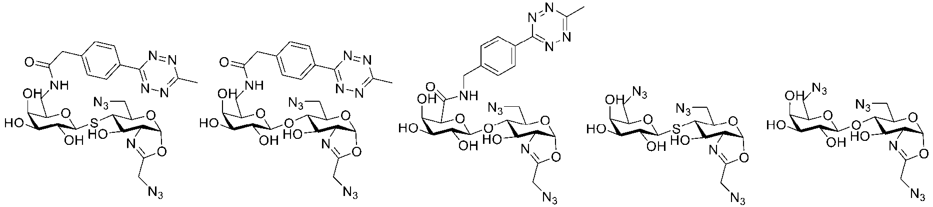 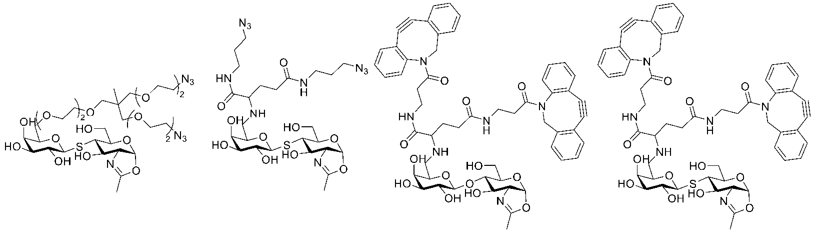 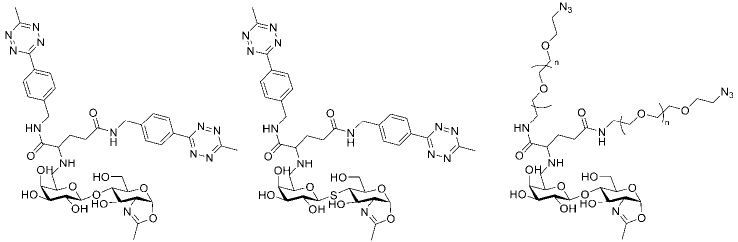 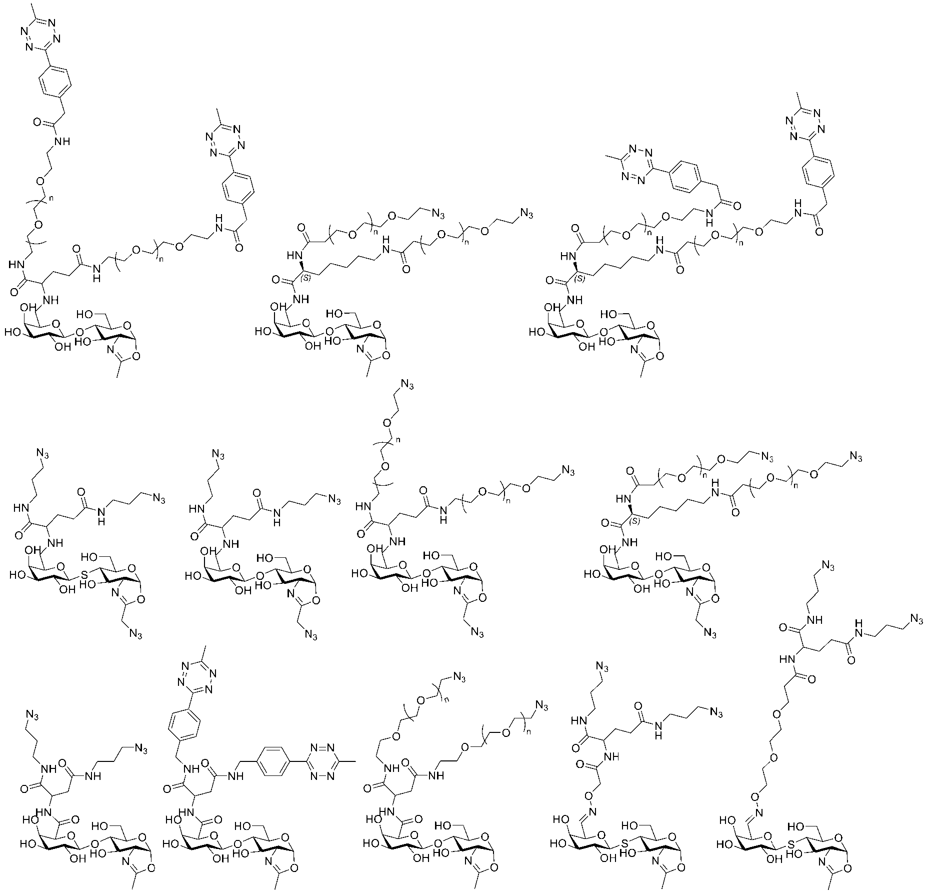 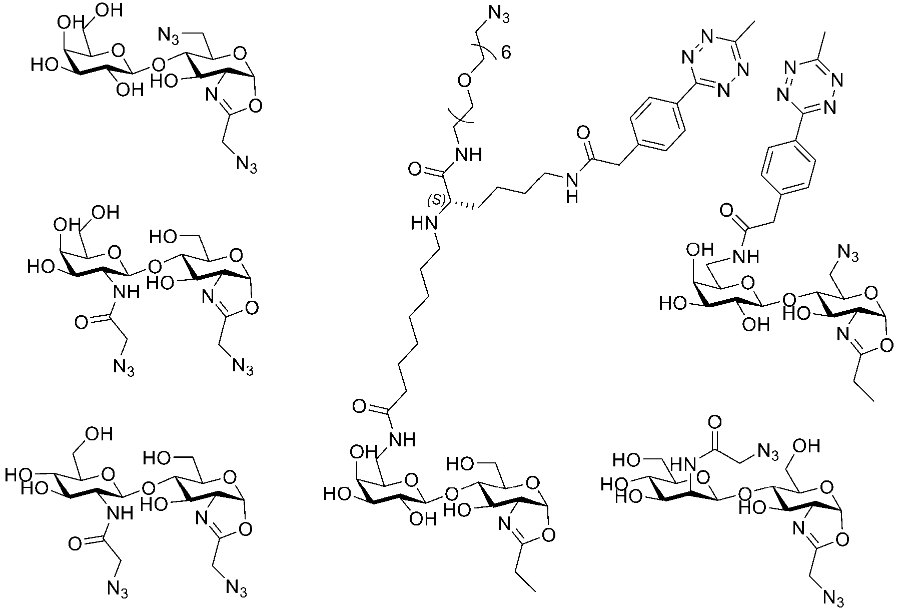 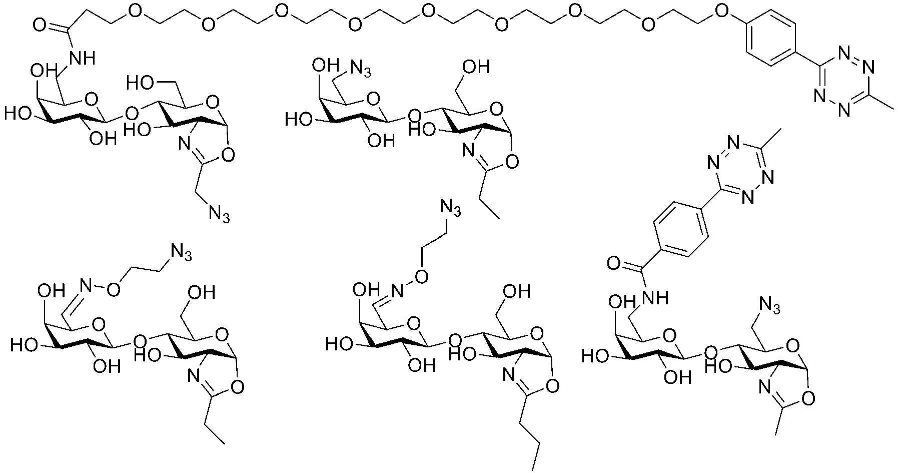 wherein n is an integer selected from the group consisting of 0-30, or an integer from 0-20, or an integer from 0-10.

8. A disaccharide conjugate represented by formula (II): 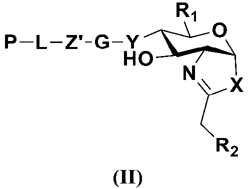 wherein
X, Y, R₁, R₂, and ring G are as defined in any one of claims 1-6;
P-L-Z'- represents a substituent on the ring G, and the substitution position of P-L-Z'- is any position other than position 1 of the ring G derived from the monosaccharide molecule;
L is a divalent or multivalent linker connecting Z' and P, selected from the group consisting of a cleavable linker, a non-cleavable linker, and a combination thereof;
P is selected from the group consisting of a cytotoxin, a small-molecule drug, a near-infrared or fluorescent probe, a peptide, RNA and related drugs, a radioisotope label, a contrast agent, and a magnetic resonance imaging agent; the small-molecule drug is, for example, maytansine, DM-1, DM-4, MMAE, MMAF, Auristatin 0101, SN-38, Dxd, exatecan, duocarmycin, amanitin, PBDs, VP-16, camptothecin, paclitaxel, docetaxel, anthracyclines, or a derivative thereof, or the small-molecule drug is, for example, a radiotherapeutic agent;
Z' is a linking moiety connecting L and ring G, which is independently absent or one or any combination of two or more selected from the group consisting of -(CH₂)ₚ-, -(CH₂-CH₂-O)ₚ-, -(phenyl)ₚ-, and the following groups: 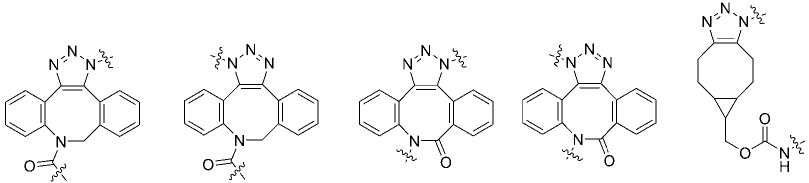 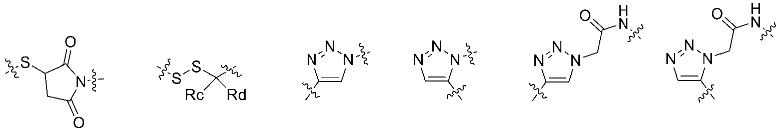 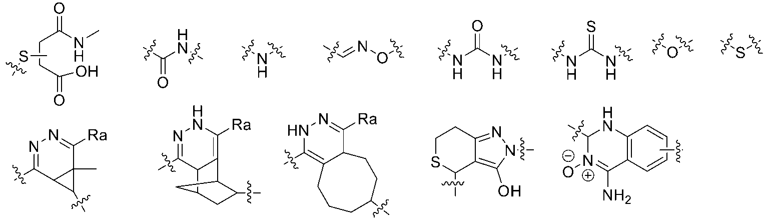
wherein Ra, Rc, and Rd are each independently selected from the group consisting of hydrogen, C₁-C₆ alkyl, C₆-C₁₀ aryl, and C₅-C₁₀ heteroaryl; and
each p is an integer from 1 to 5.

9. The disaccharide conjugate according to claim 8, wherein L is selected from the group consisting of or comprises one or a combination of more than one of the following: Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Gly-Gly-Gly, Gly-Gly-Phe-Gly, and Gly-Gly-Gly-Gly-Gly; or,
L is selected from the group consisting of or comprises one or a combination of more than one of the following: Val-Cit, Val-Ala, Ala-Ala, and Gly-Gly-Phe-Gly; or
L is selected from the group consisting of or comprises one or a combination of more than one of the following cleavable linkers: 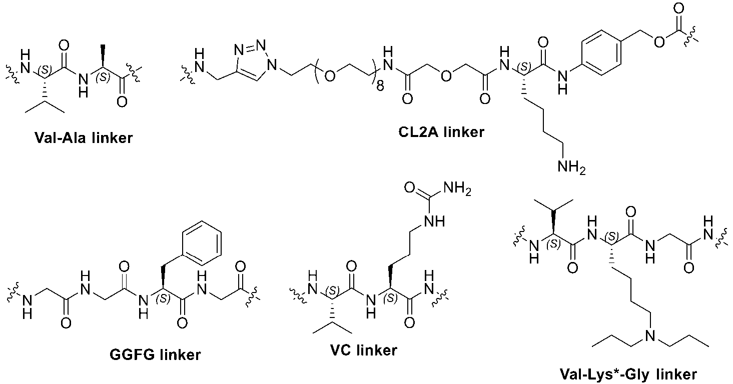 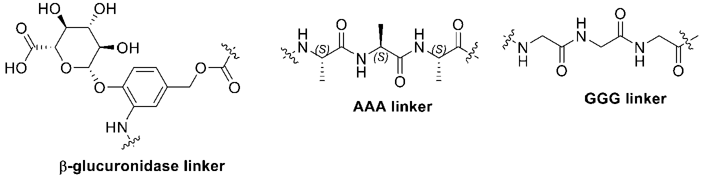 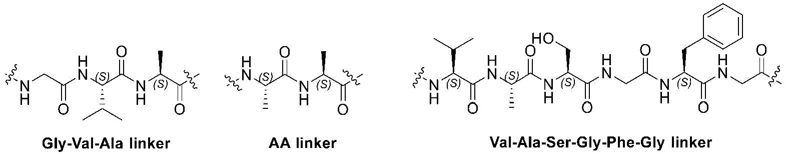 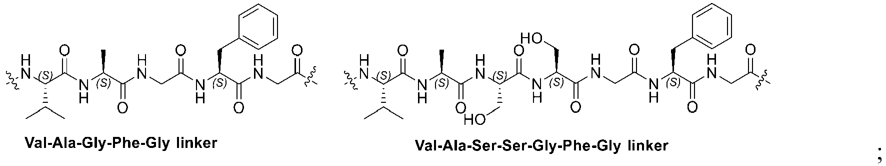 or
L is selected from the group consisting of or comprises one or a combination of more than one of the following non-cleavable linkers: -(CH₂)ₘ-, -(CH₂-CH₂-O)ₘ-, and -(PO₄)ₙ-, wherein each m and each n is independently an integer selected from the group consisting of 0-30, or an integer from 0-20, or an integer from 0-10.

10. The disaccharide conjugate according to any one of claims 8-9, wherein P is a cytotoxin selected from the group consisting of:

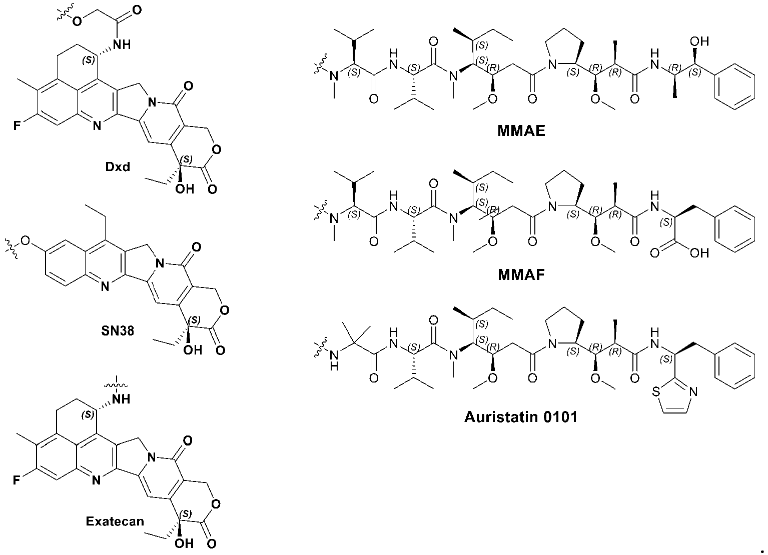

11. The disaccharide conjugate according to claim 8, selected from the group consisting of the following specific compounds: 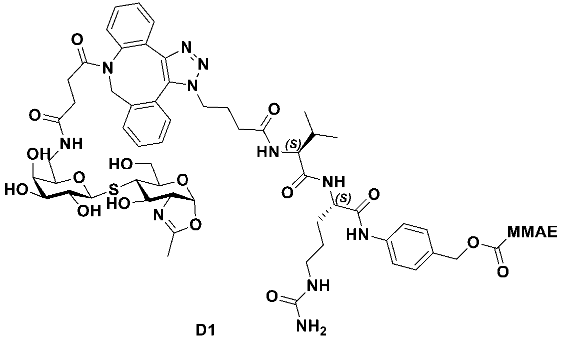 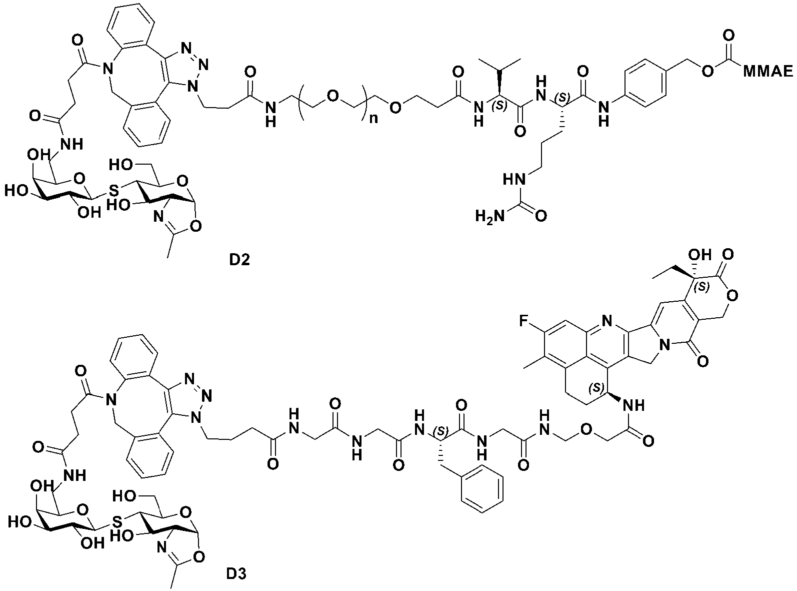 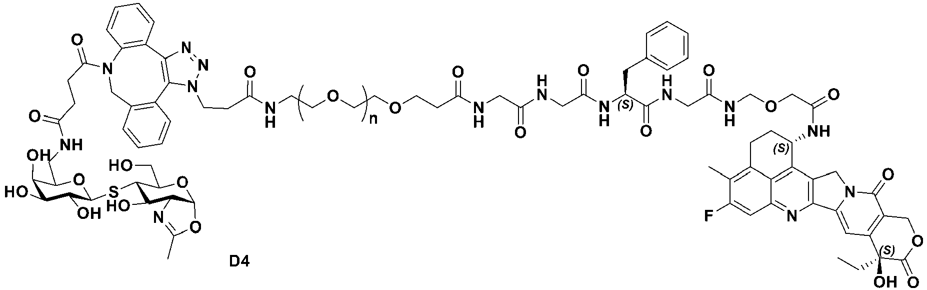 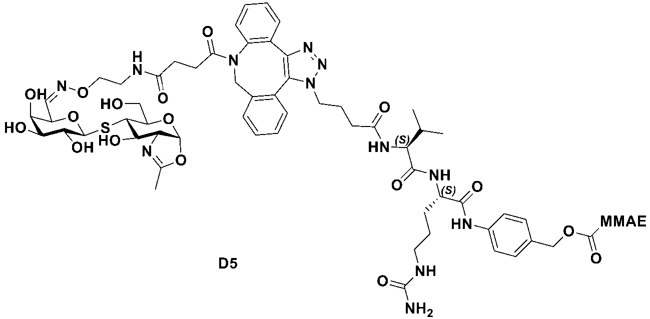 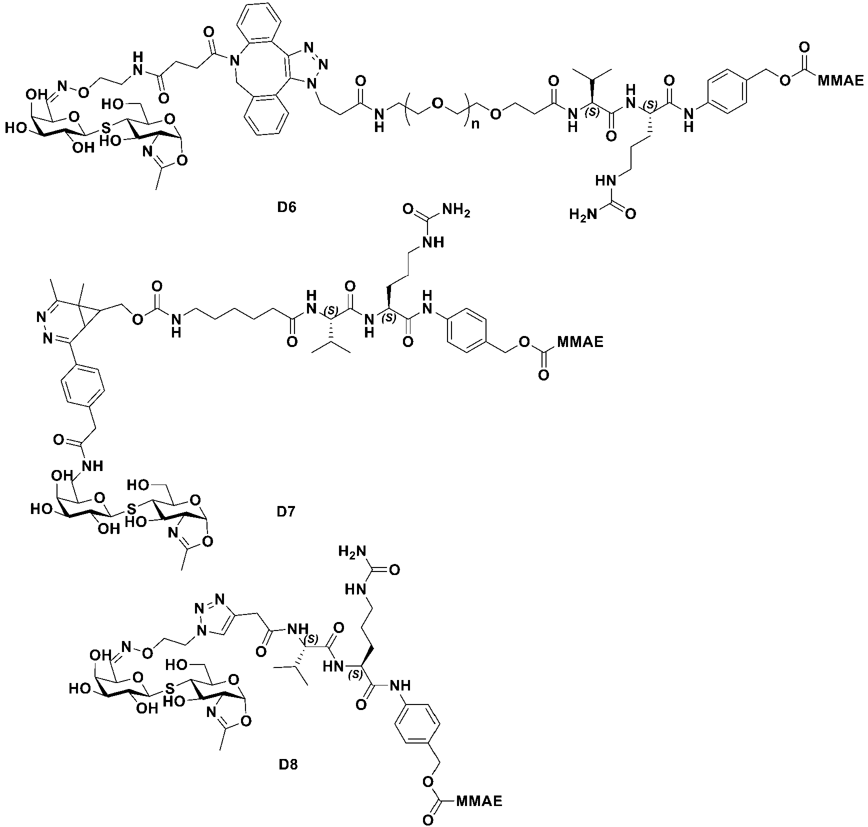 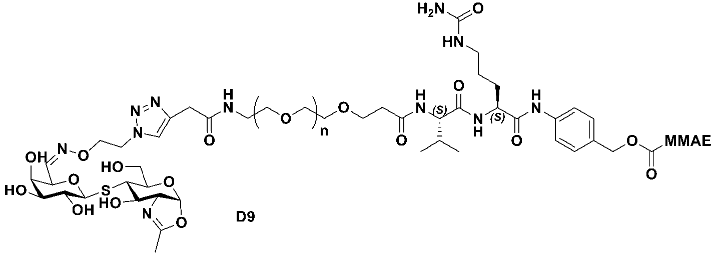 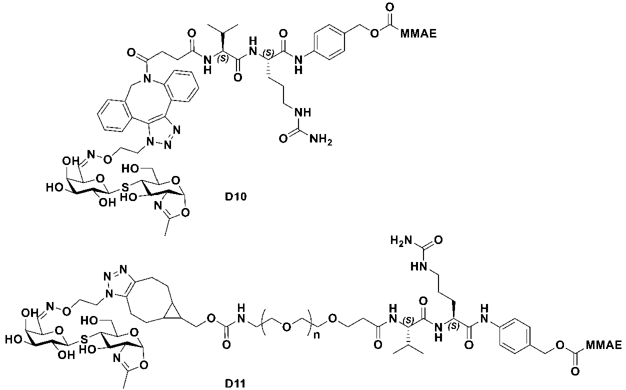 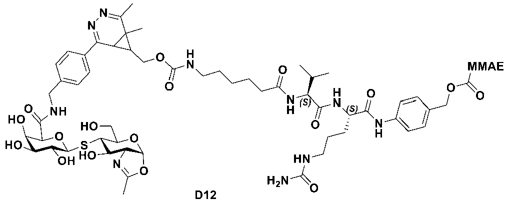 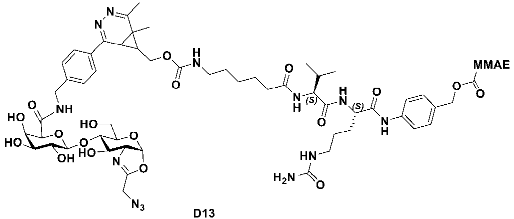 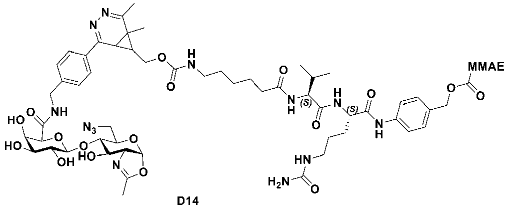 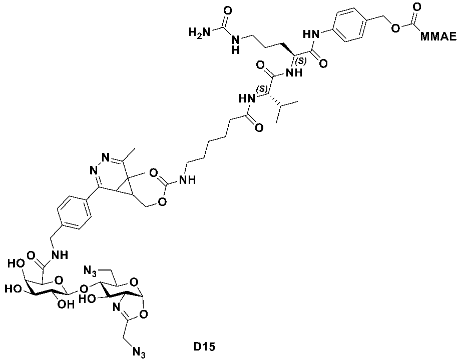 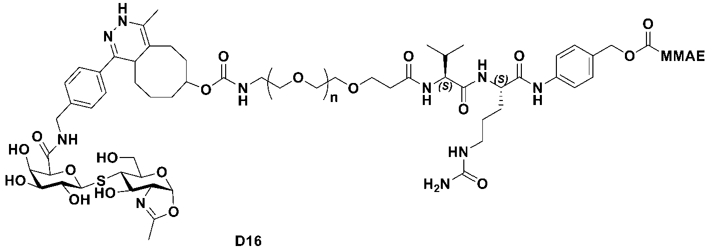 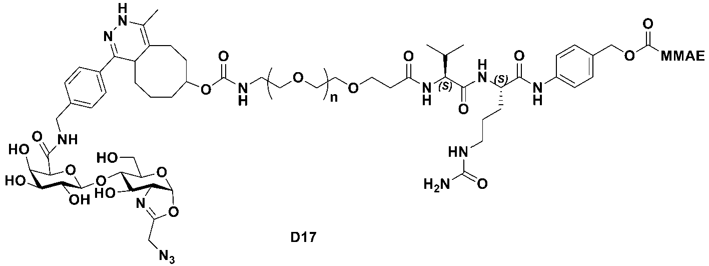 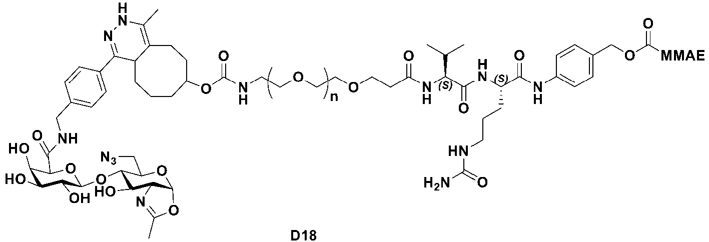 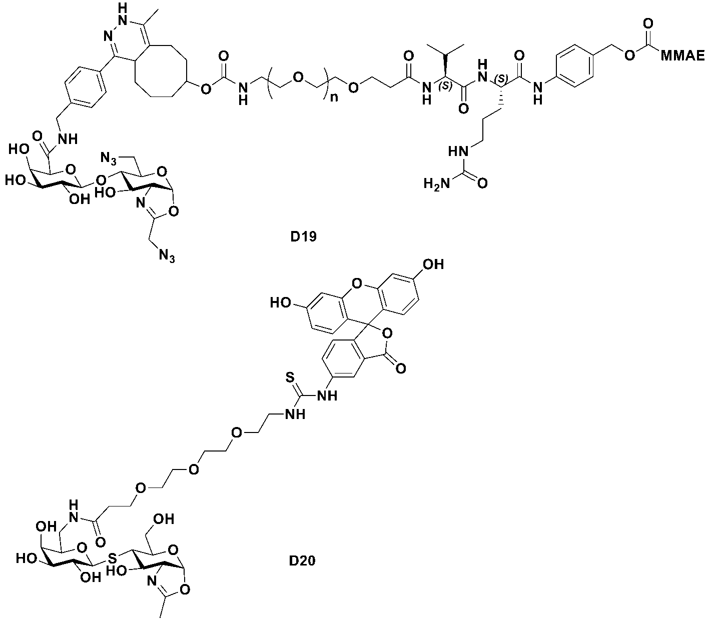 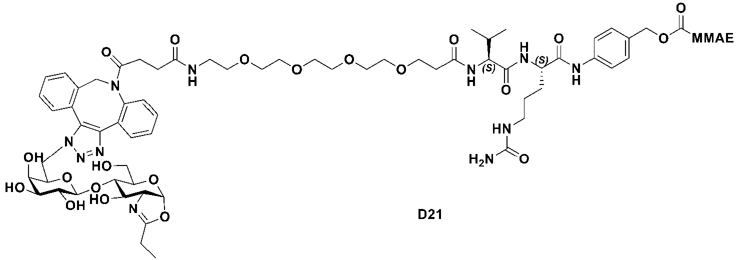 wherein n is an integer selected from the group consisting of 0-30, or an integer from 0-20, or an integer from 0-10.

12. An antibody-based conjugate drug represented by formula (III): 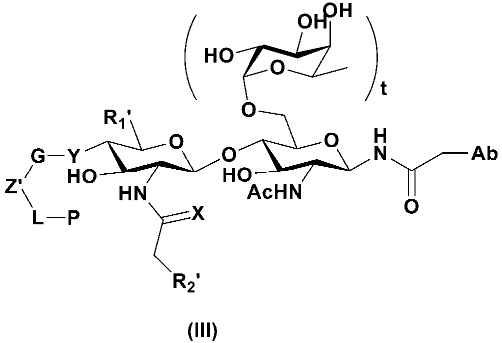
wherein X, Y, and ring G are as defined in any one of claims 1-6;
Z', L, and P are as defined in any one of claims 8-10; or P-L-Z'- is hydrogen;
t is 0 or 1;
R₁' is R₁ or a group formed after R₁ reacts with a drug linker E-L-P, and R₂' is R₂ or a group formed after R₂ reacts with a drug linker E-L-P; wherein R₁ and R₂ are as defined in any one of claims 1 and 4-5; L and P in the drug linker E-L-P are as defined in any one of claims 8-10, and E comprises the following groups: 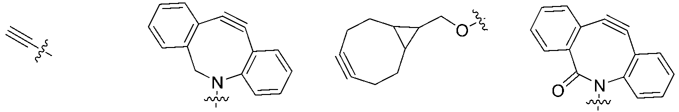 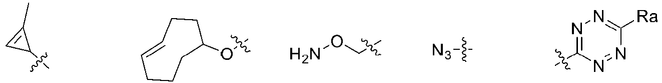 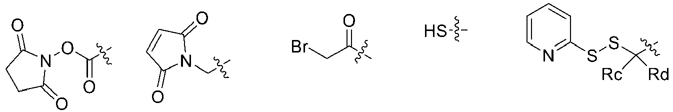 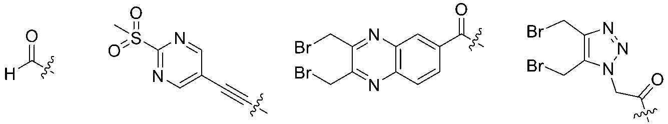
wherein Ra, Rc, and Rd are each hydrogen, C₁-C₆ alkyl, C₆-C₁₀ aryl, or C₅-C₁₀ heteroaryl;
Ab is an antibody or antigen-binding fragment, selected from the group consisting of a monoclonal antibody, a bispecific antibody, and a multispecific antibody.

13. The antibody-based conjugate drug according to claim 12, wherein the drug linker EL-P is selected from the group consisting of the following specific compounds: 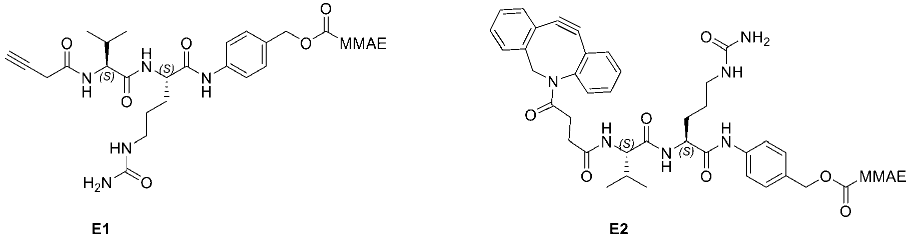 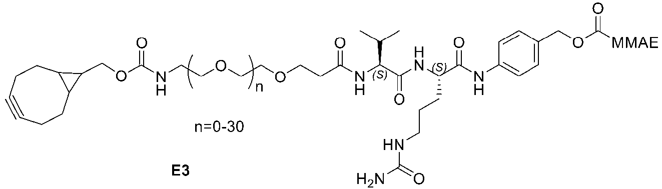 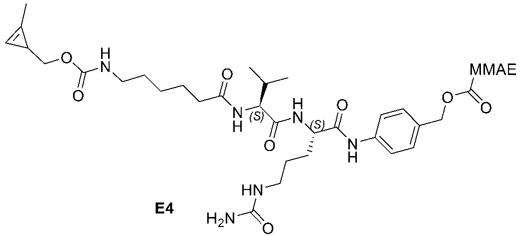 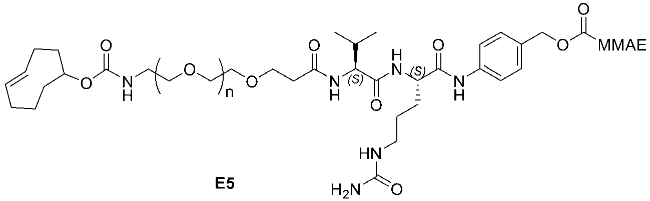 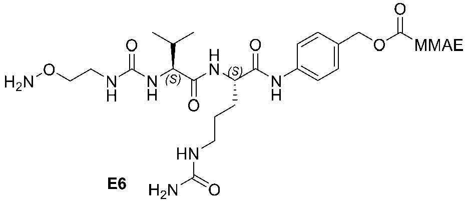 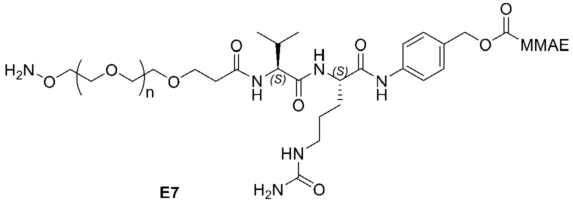 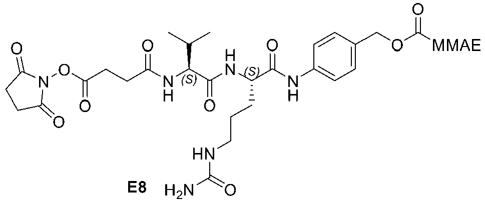 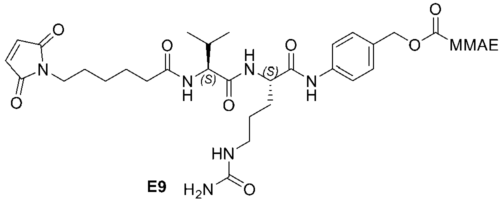 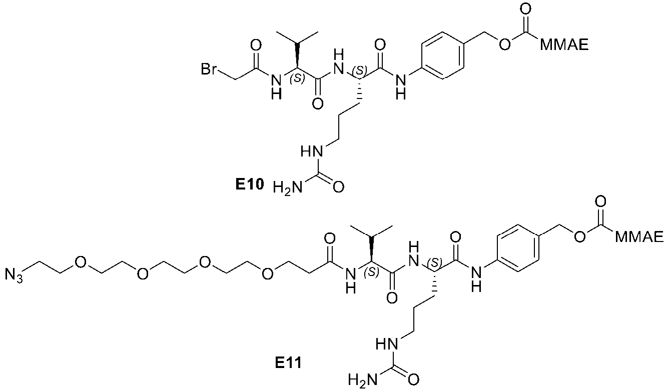 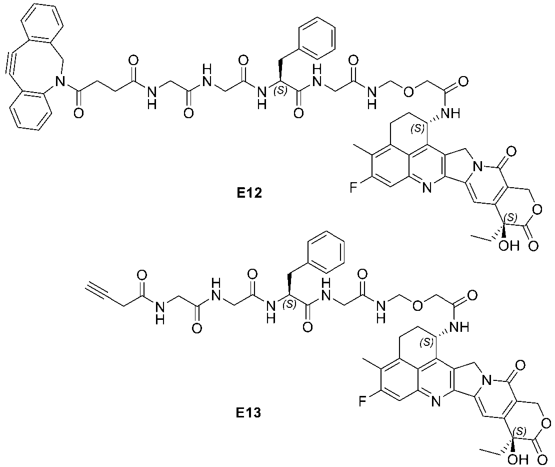 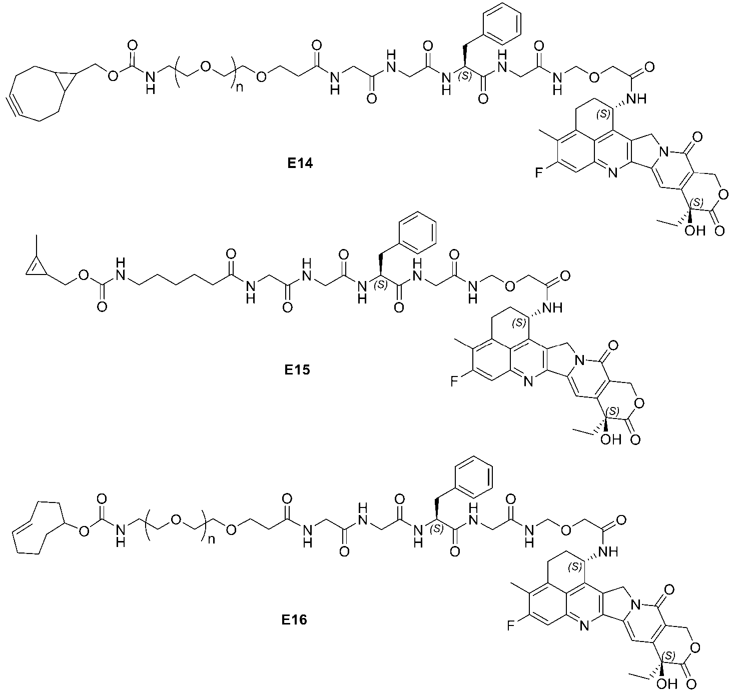 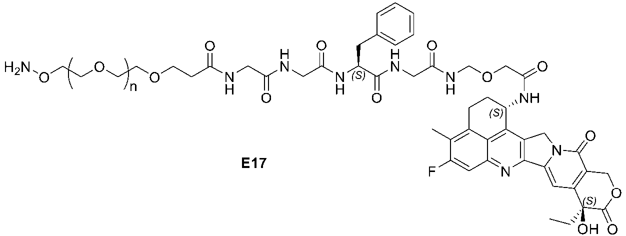 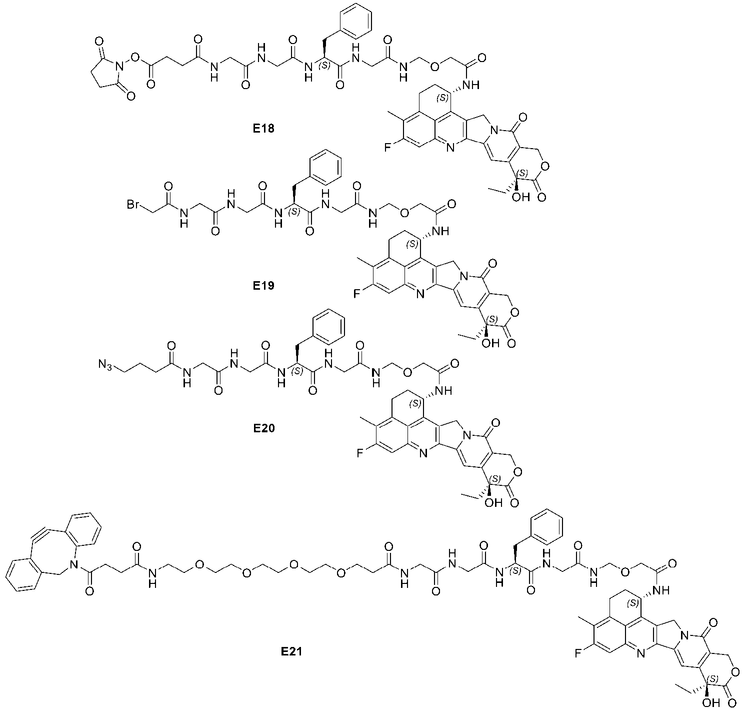 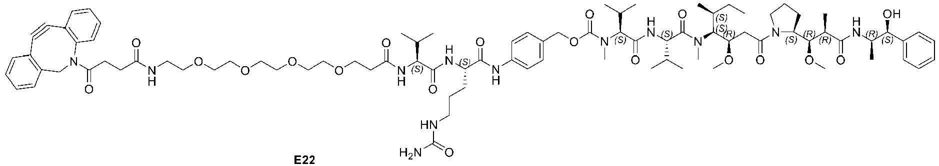 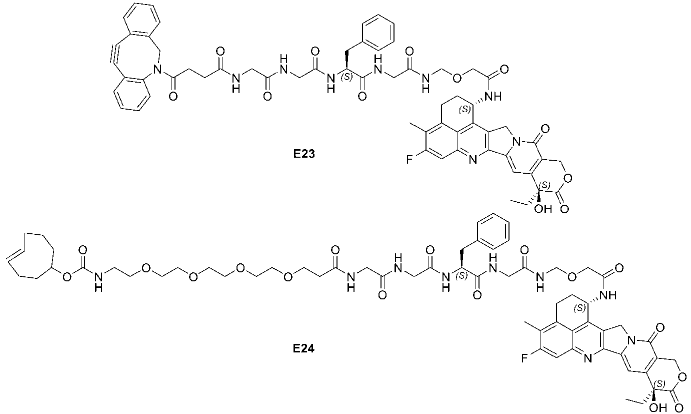 wherein n is an integer selected from the group consisting of 0-30, or an integer from 0-20, or an integer from 0-10.

14. The antibody-based conjugate drug according to claim 12 or 13, wherein the antibody is selected from the group consisting of: Pertuzumab, Trastuzumab, Rituximab, Cetuximab, Margetuximab, Abciximab, Daclizumab, Adalimumab, Palivizumab, Basiliximab, Bevacizumab, Panitumumab, Nimotuzumab, Denosumab, Zolbetuximab, Ramucirumab, Necitumumab, Ipilimumab, Daratumumab, Alemtuzumab, Elotuzumab, Blinatumomab, Nivolumab, Pembrolizumab, Atezolizumab, Avelumab, Durvalumab, Toripalimab, Catumaxomab, Emicizumab, and Amivantamab.

15. The antibody-based conjugate drug according to claim 12 or 14, having a single payload and selected from the group consisting of the following specific structures: 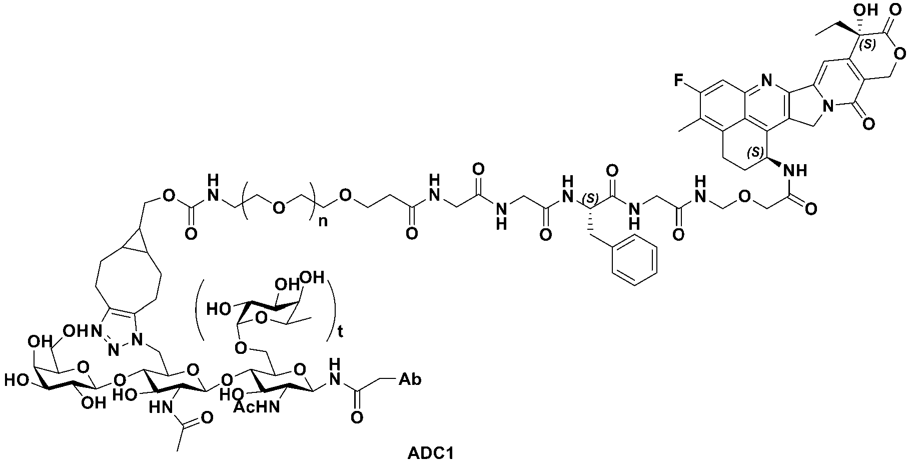 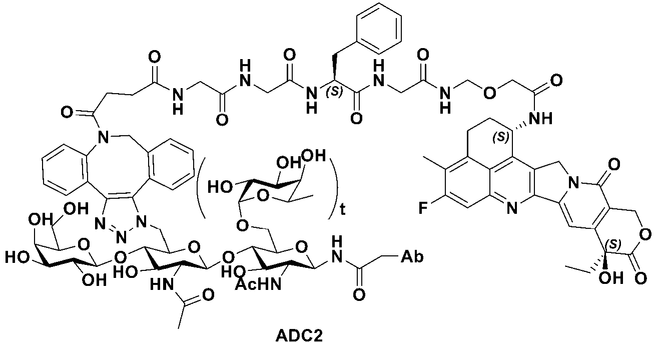 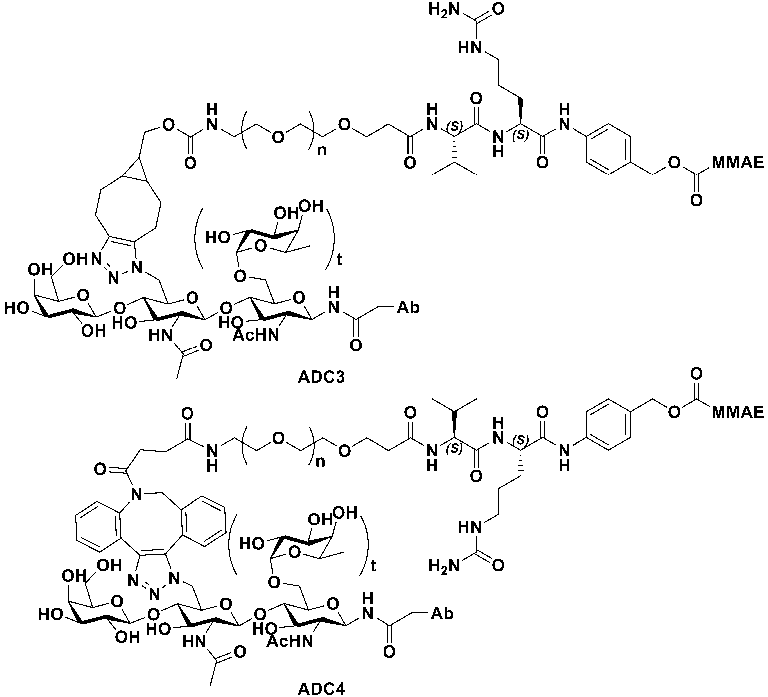 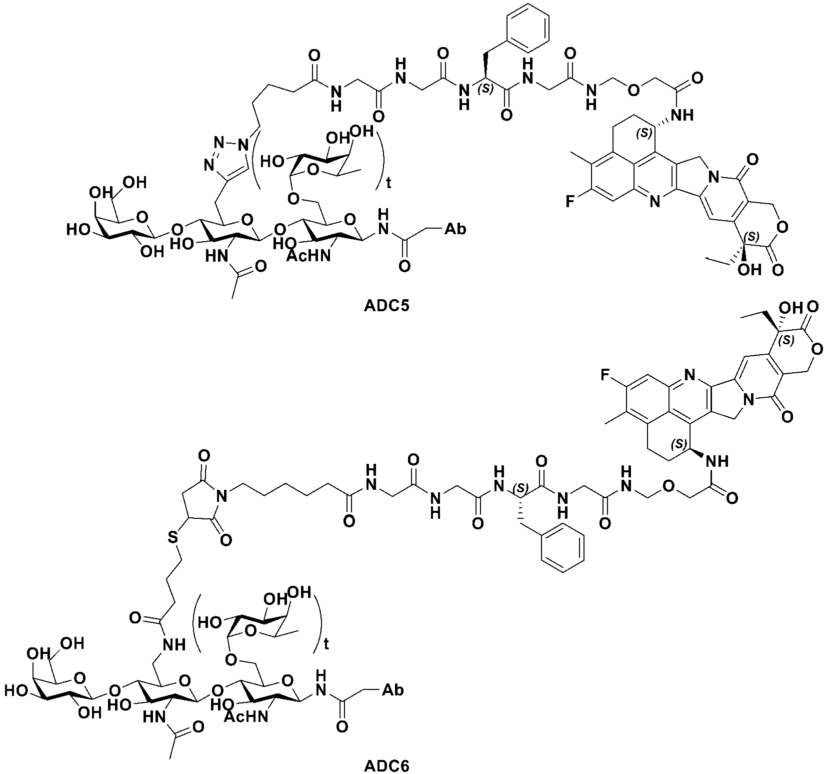 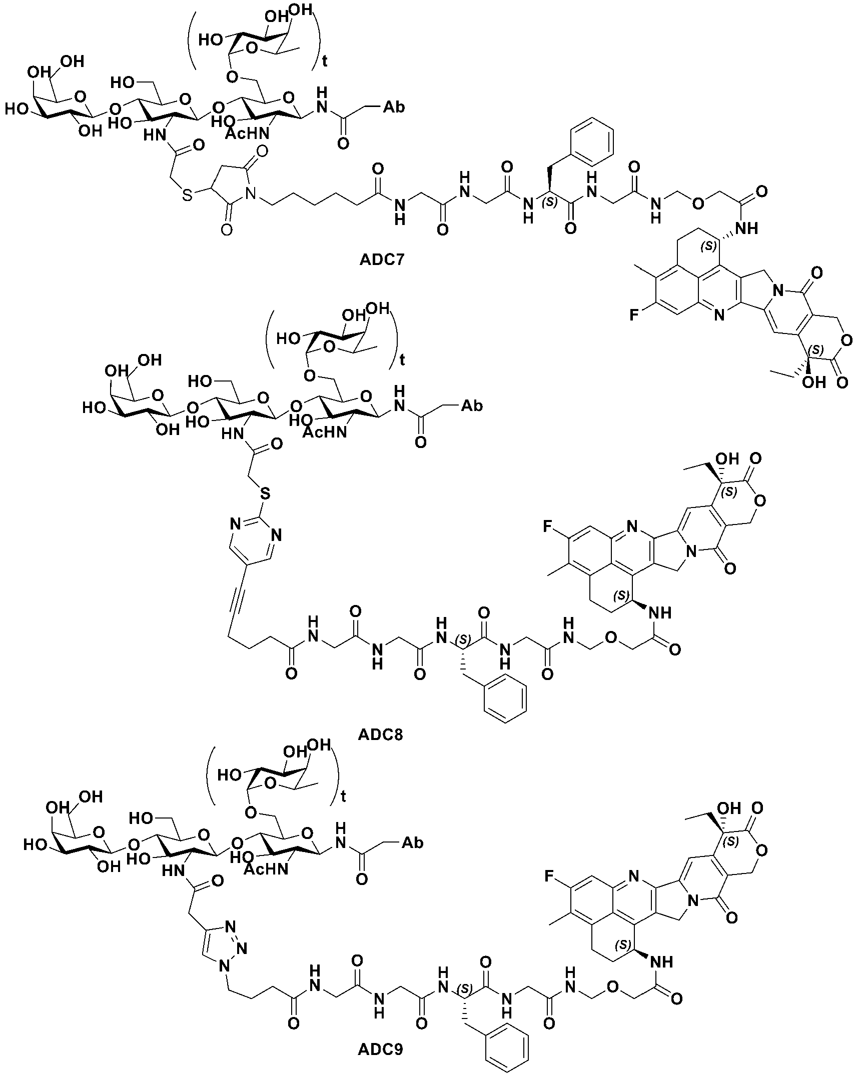 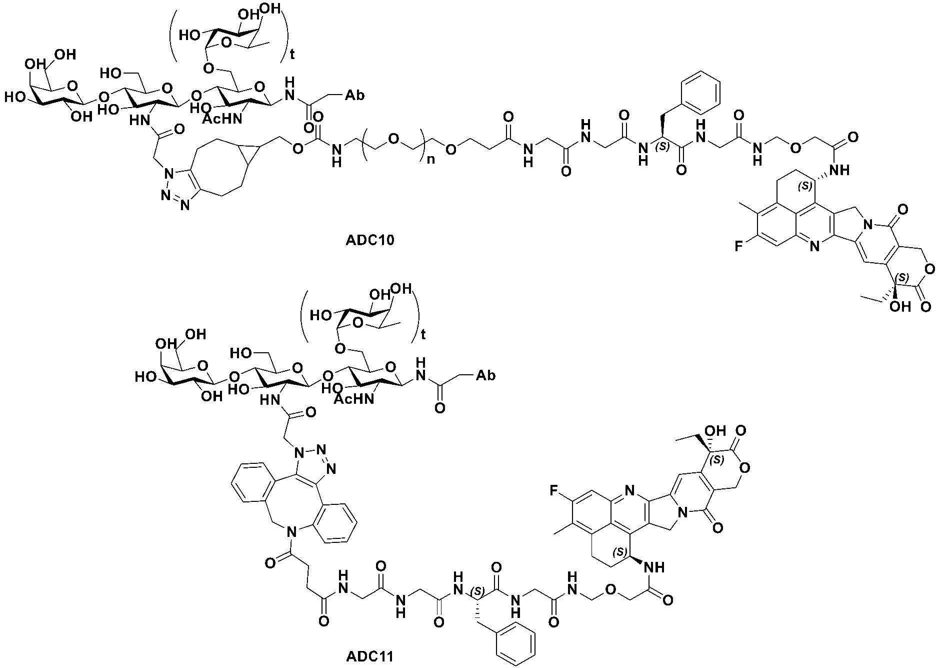 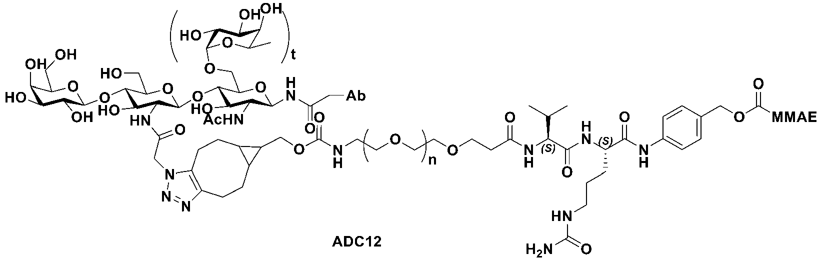 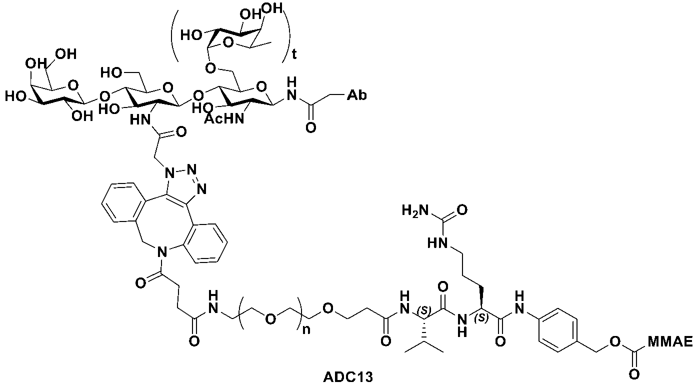 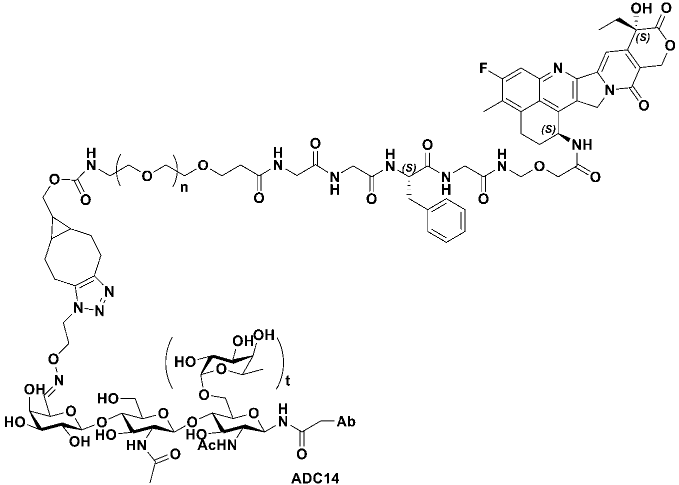 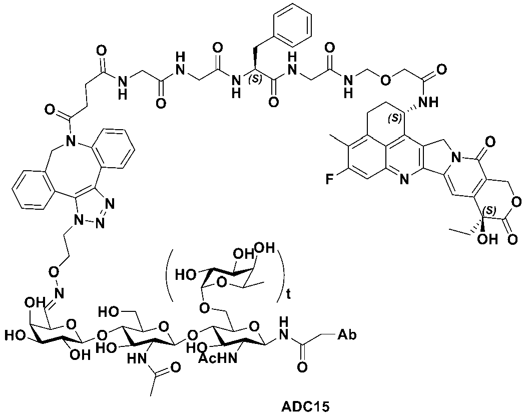 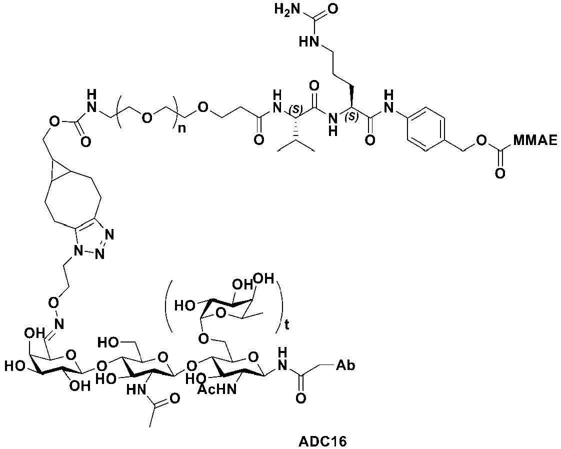 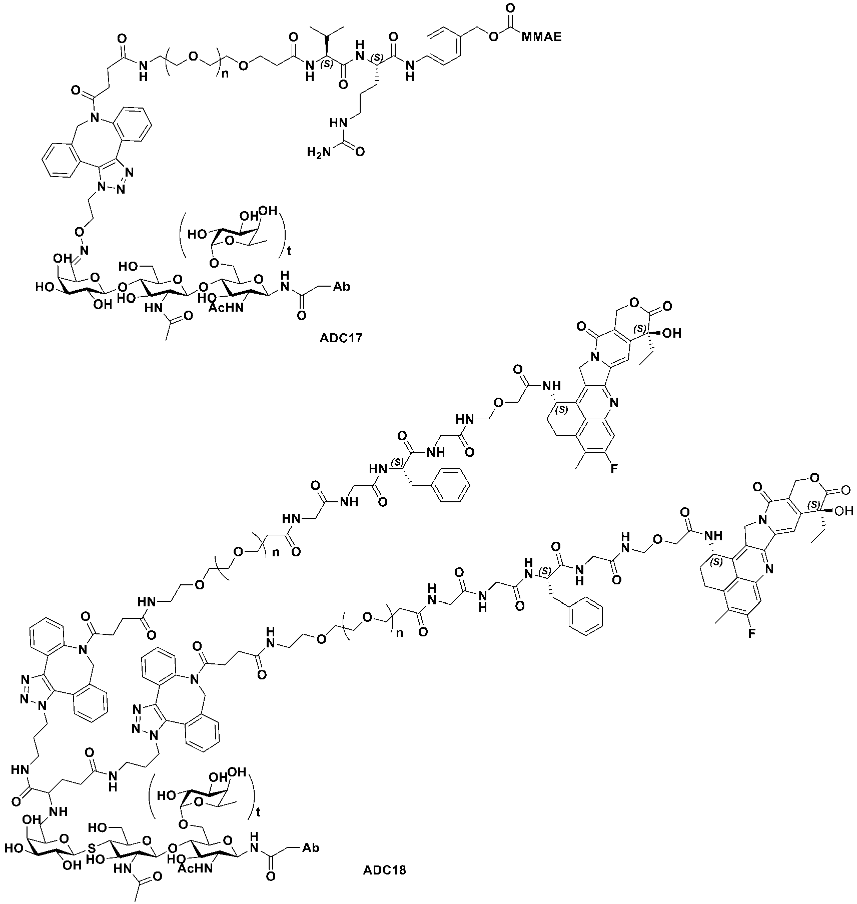 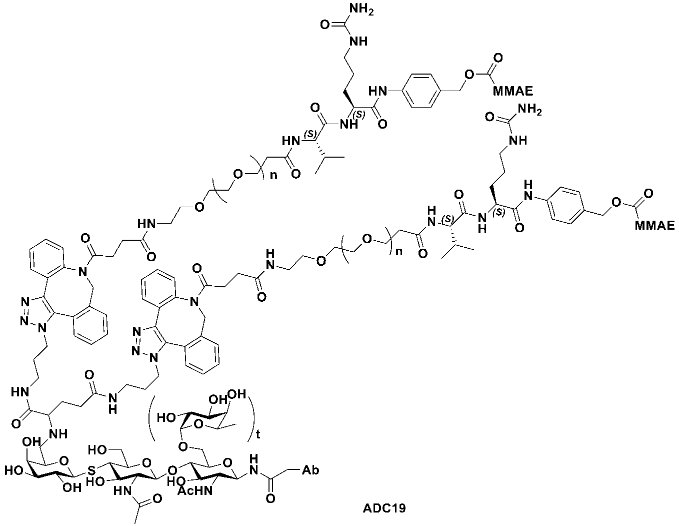 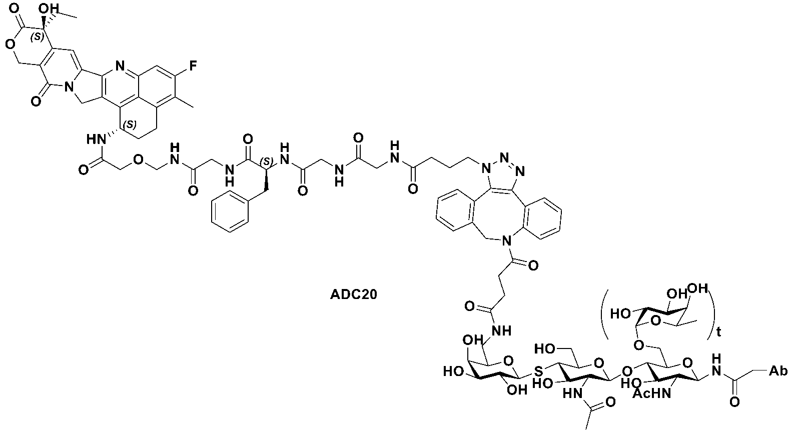 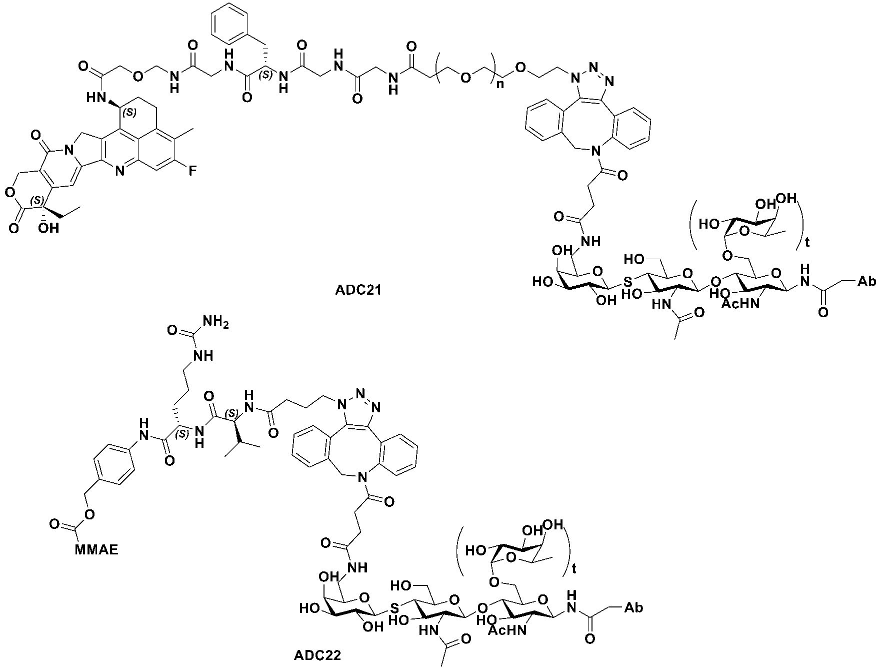 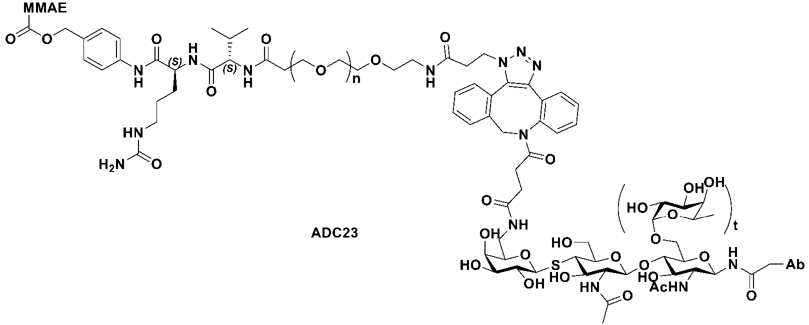 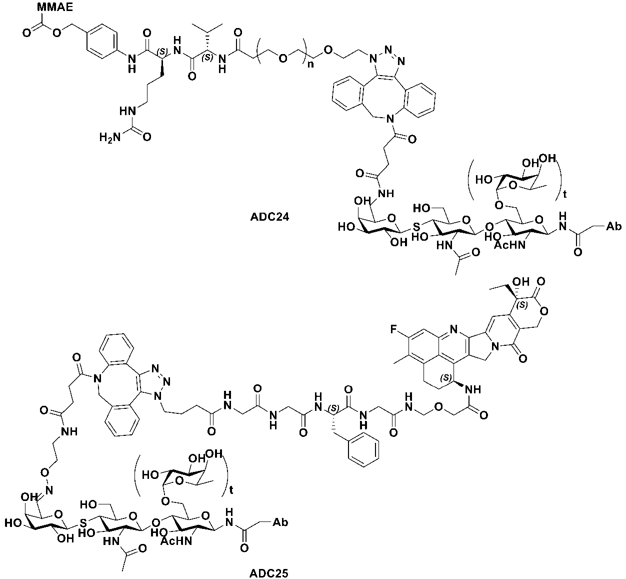 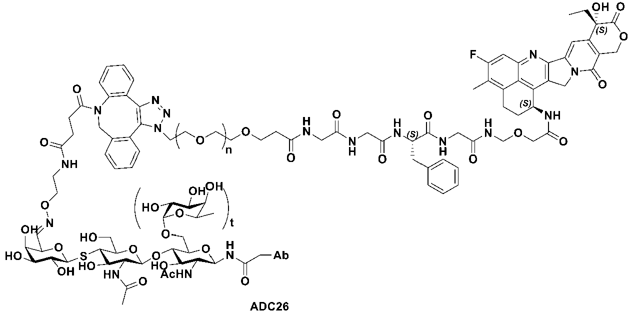 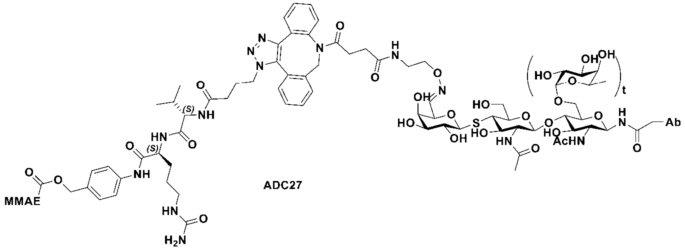 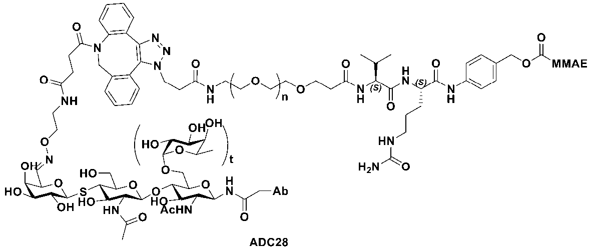 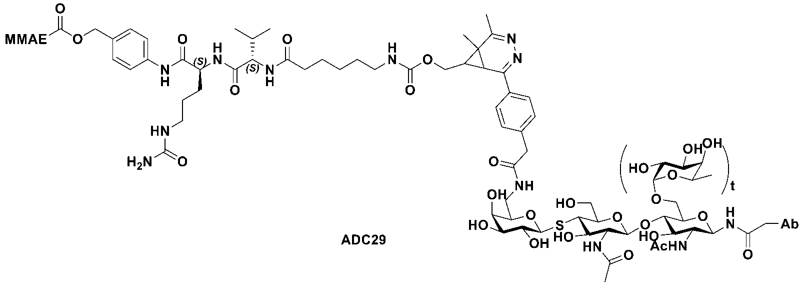 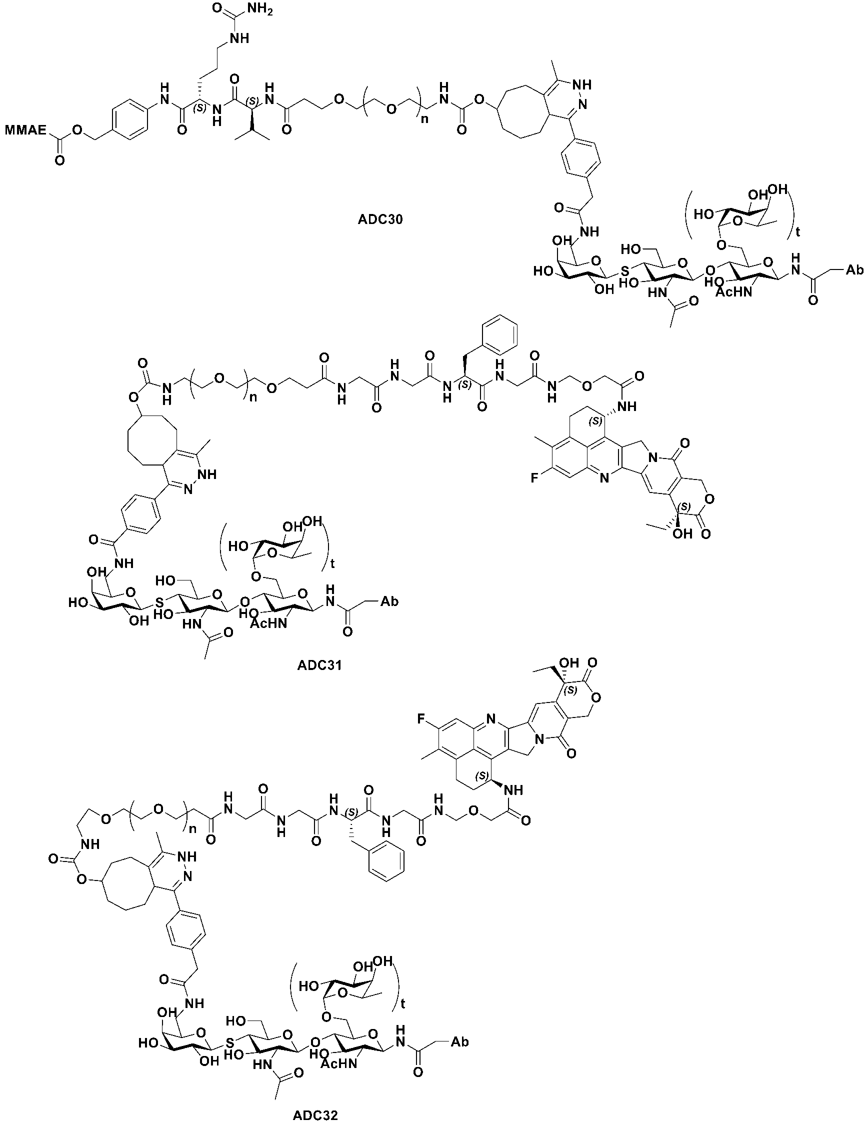 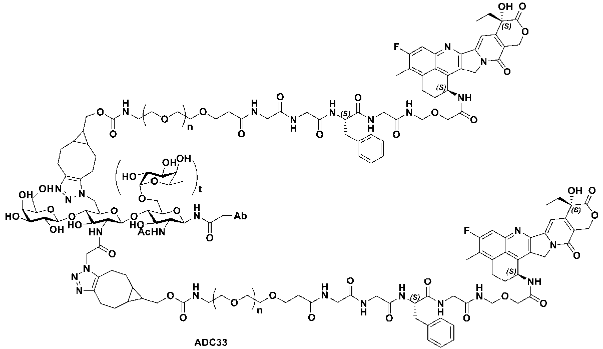 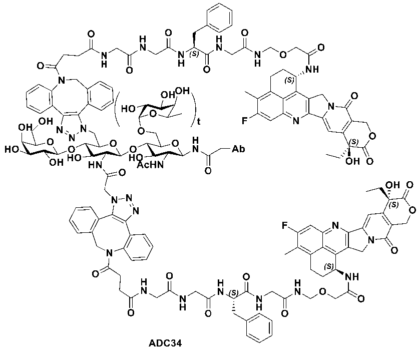 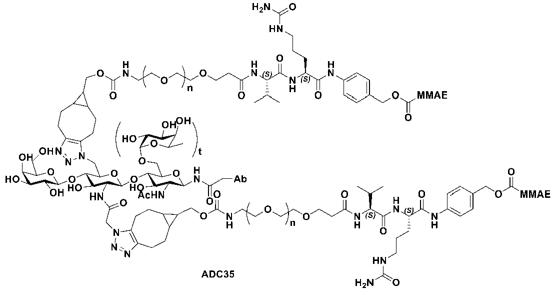 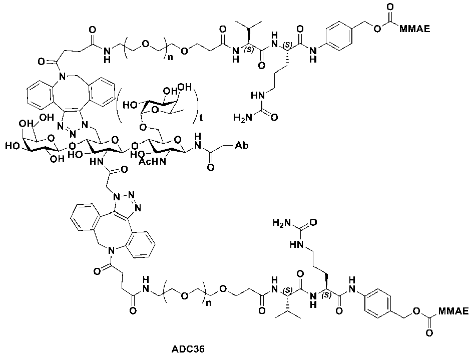 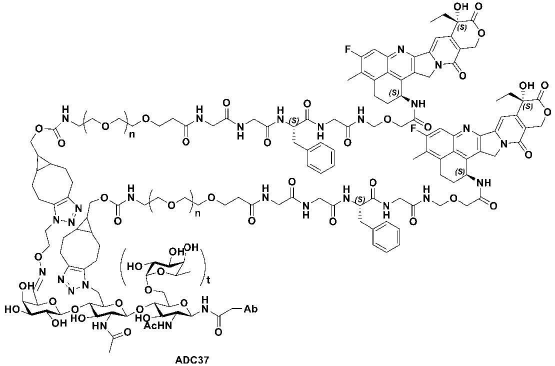 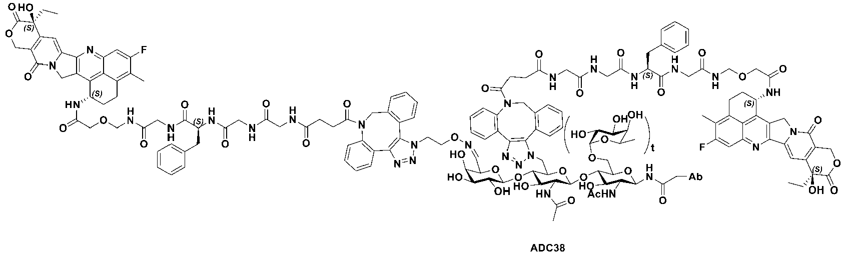 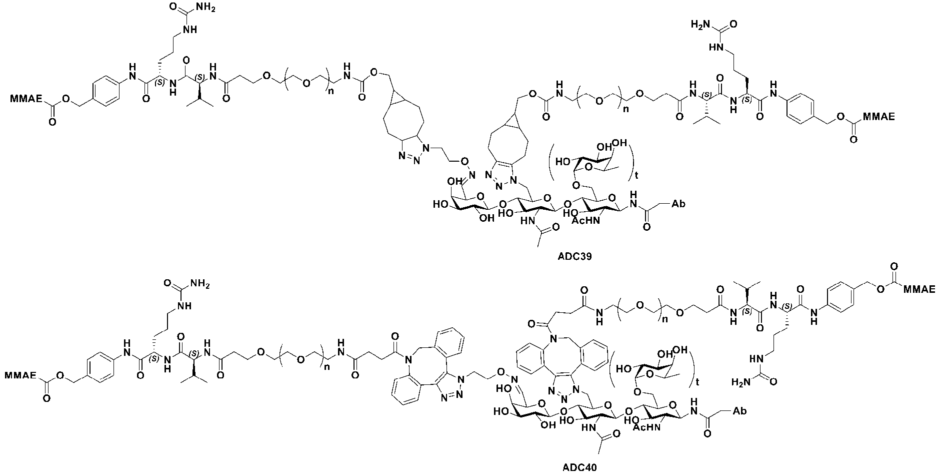 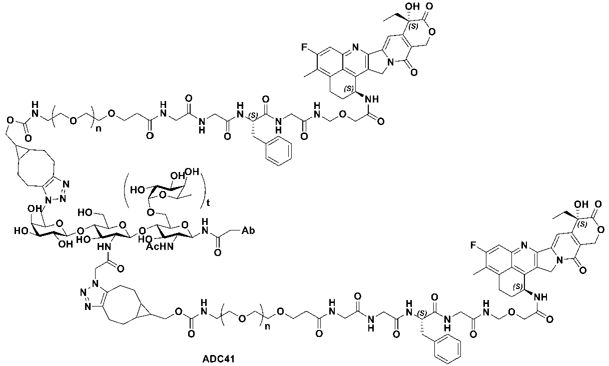 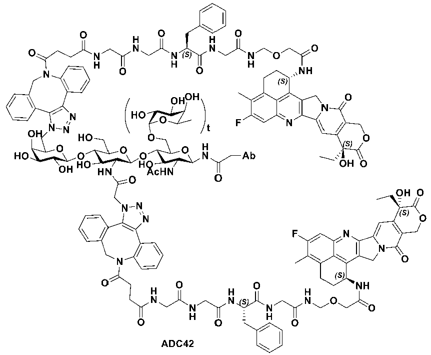 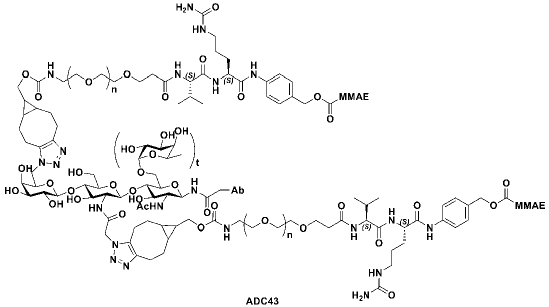 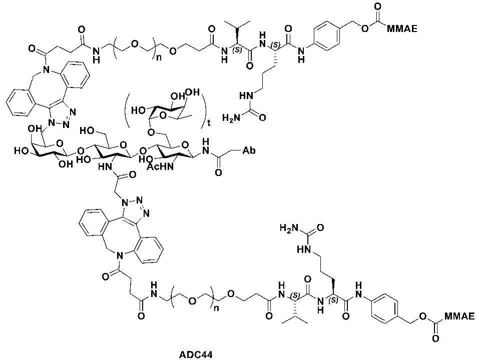 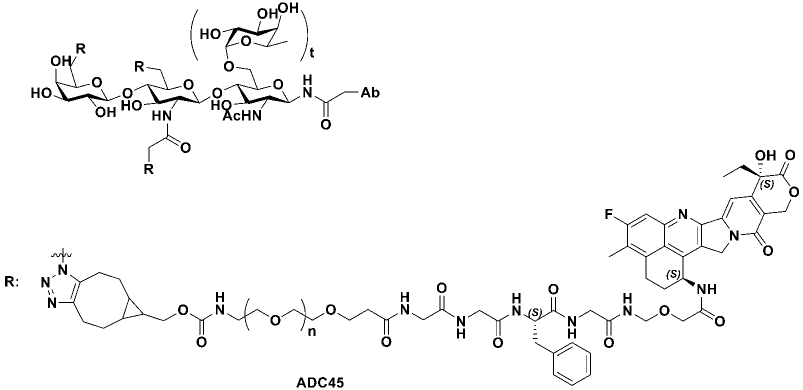 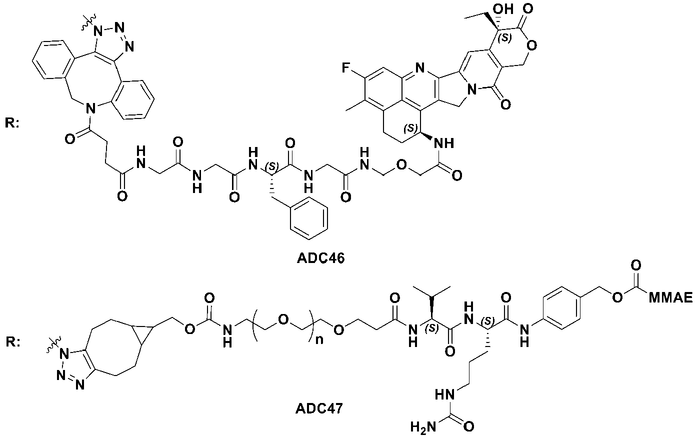 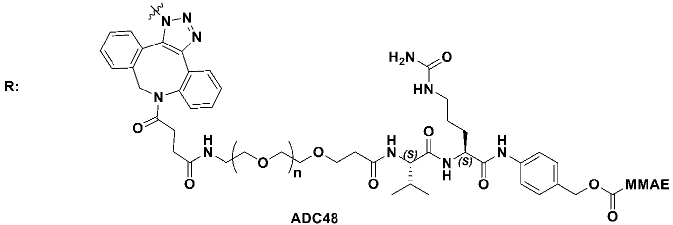 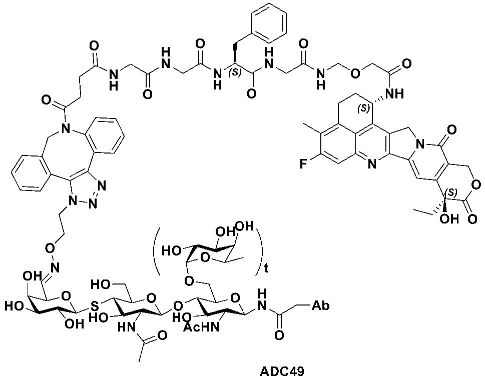 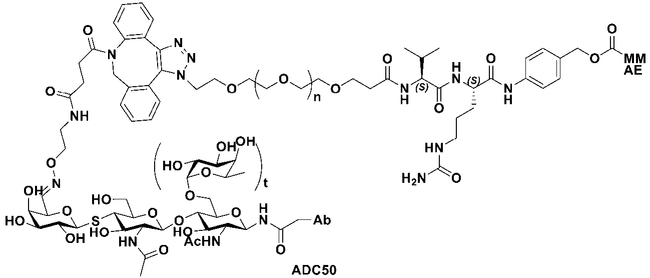 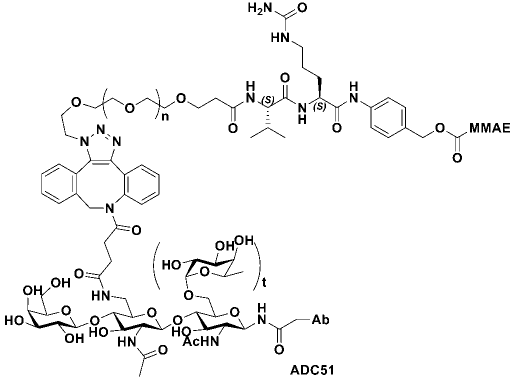 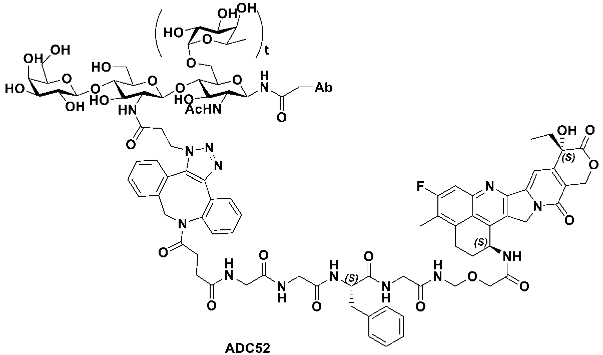 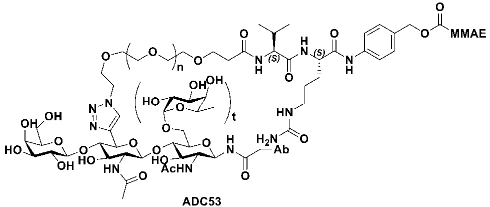 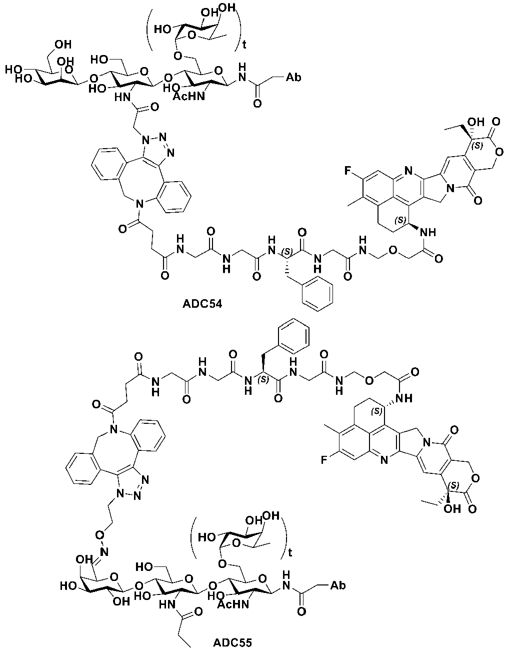 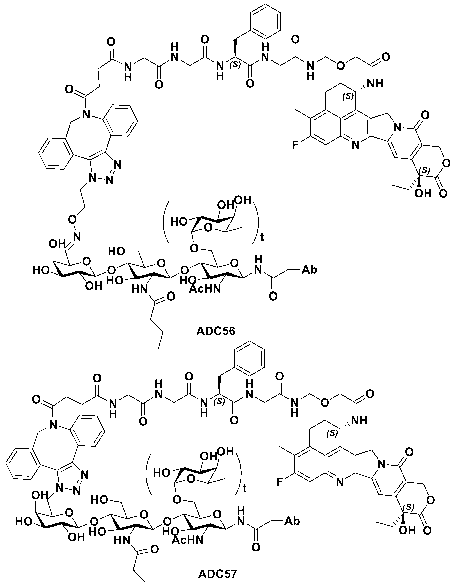 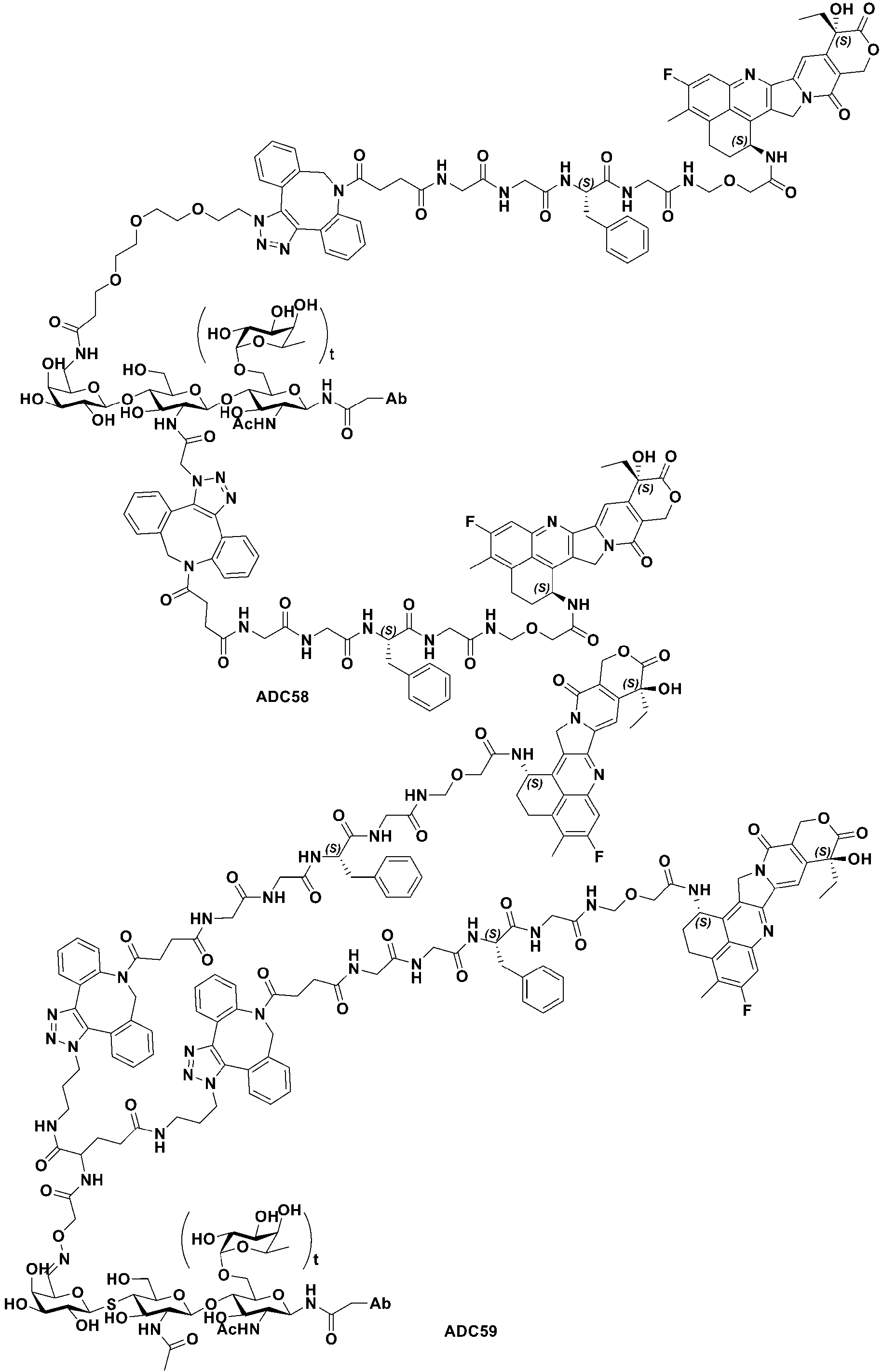 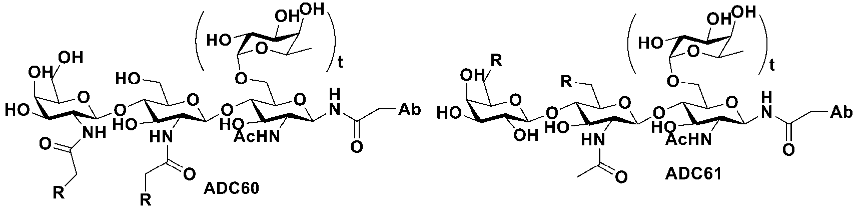 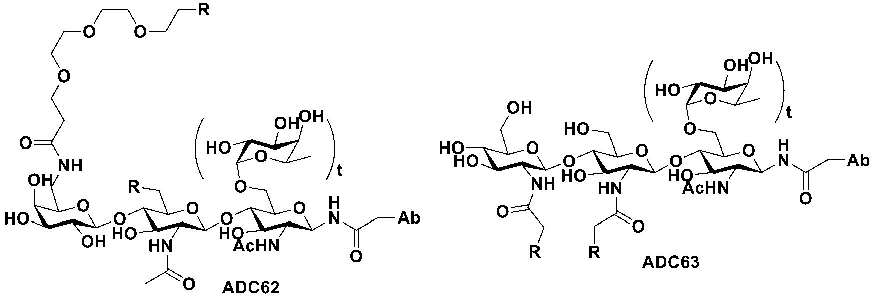 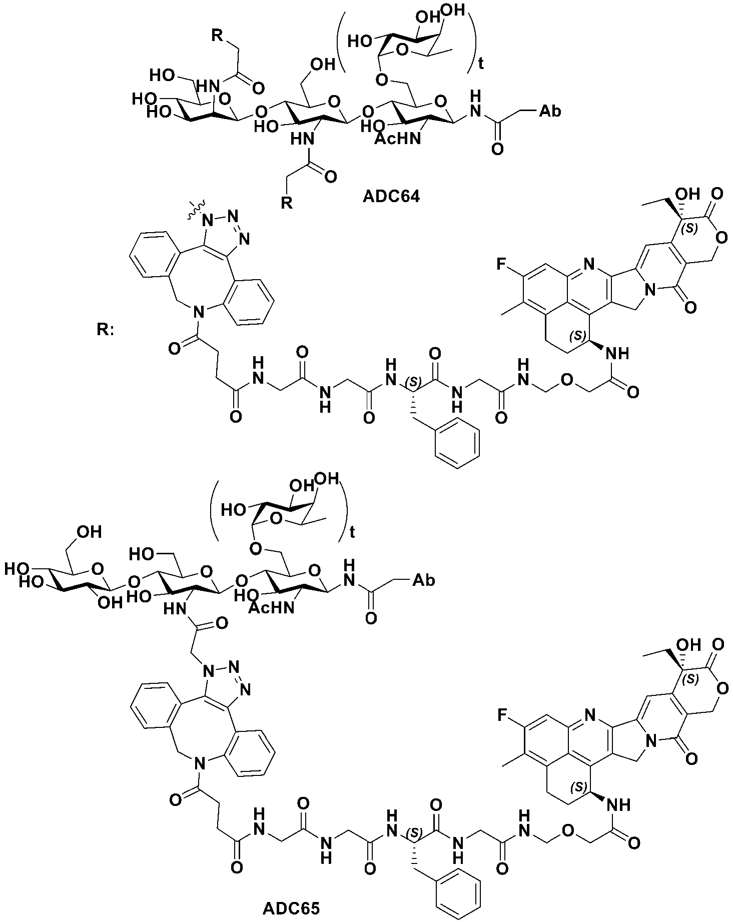 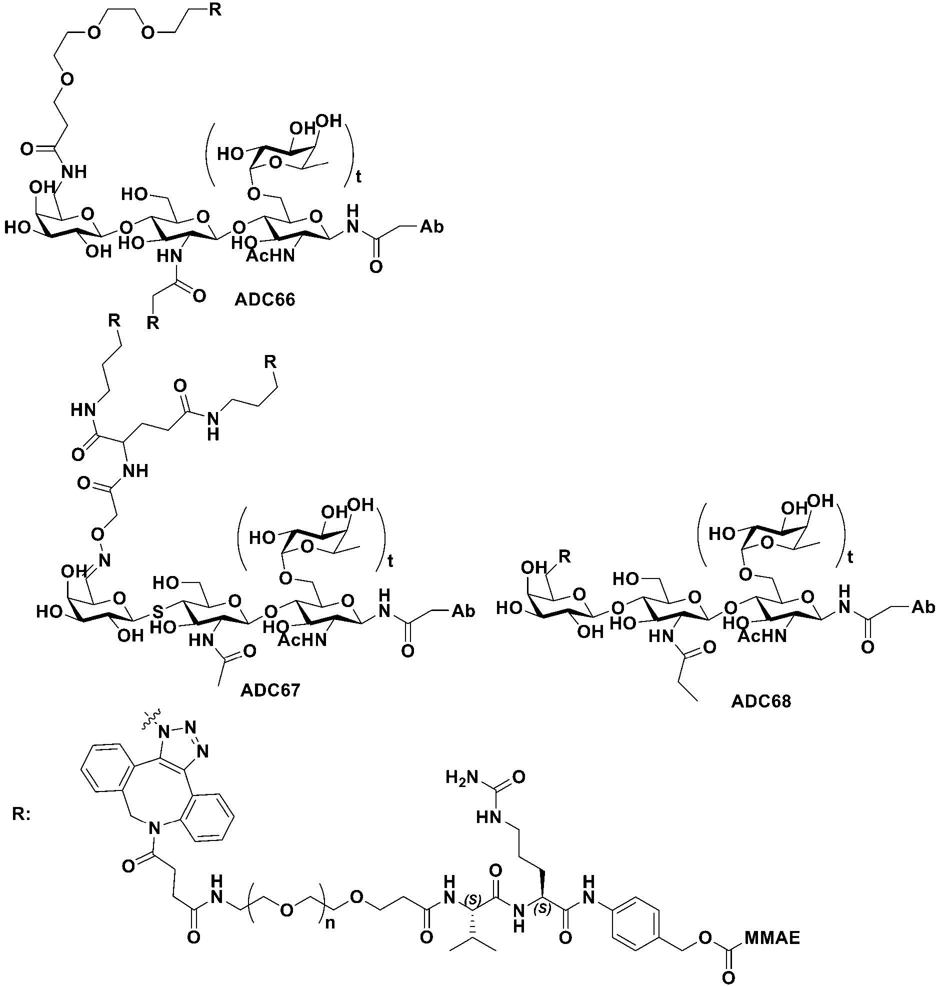
wherein n is an integer selected from the group consisting of 0-30, or an integer from 0-20, or an integer from 0-10;
t is 0 or 1.

16. The antibody-based conjugate drug according to claim 12 or 14, having a dual payload and selected from the group consisting of the following specific structures: 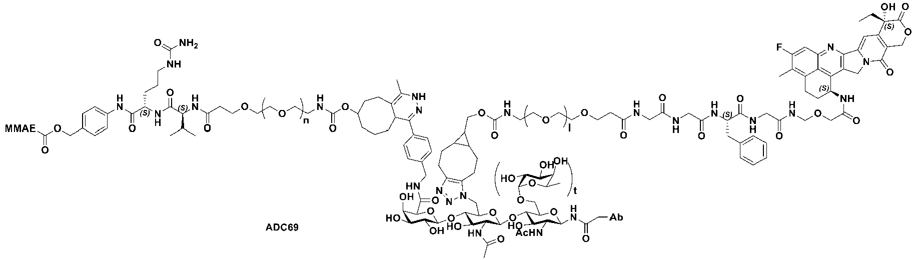 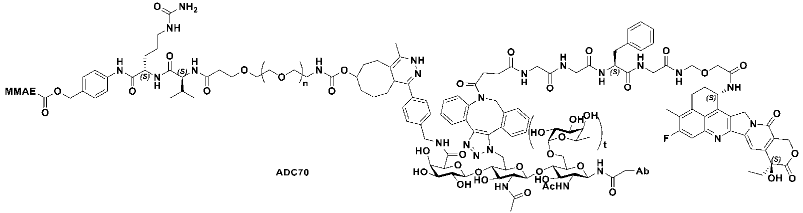 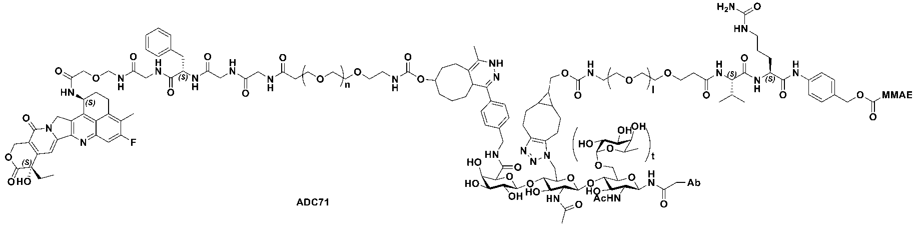 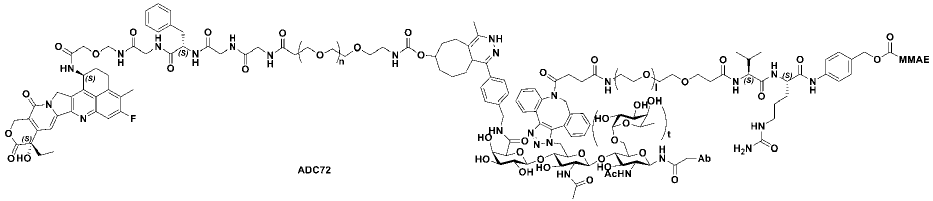 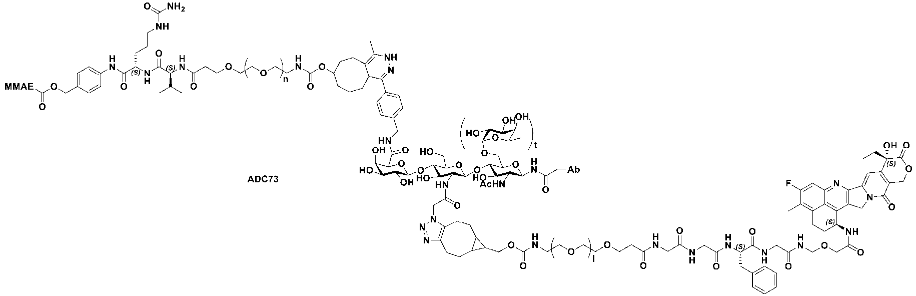 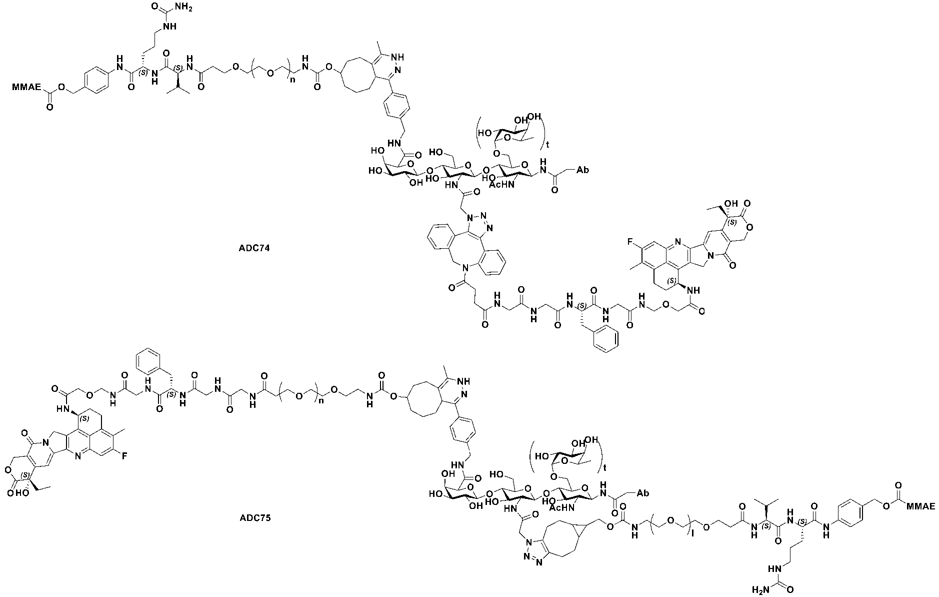 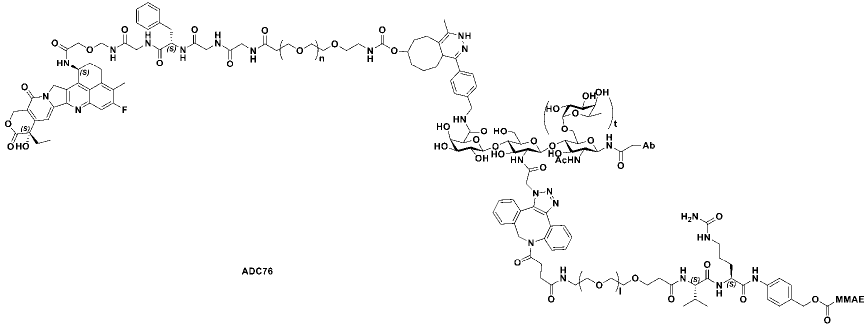 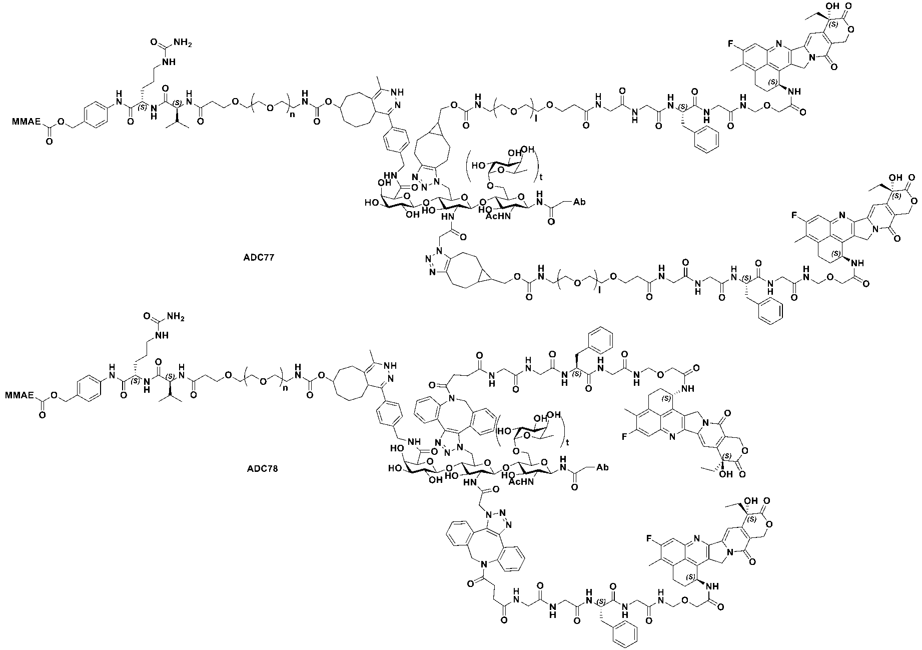 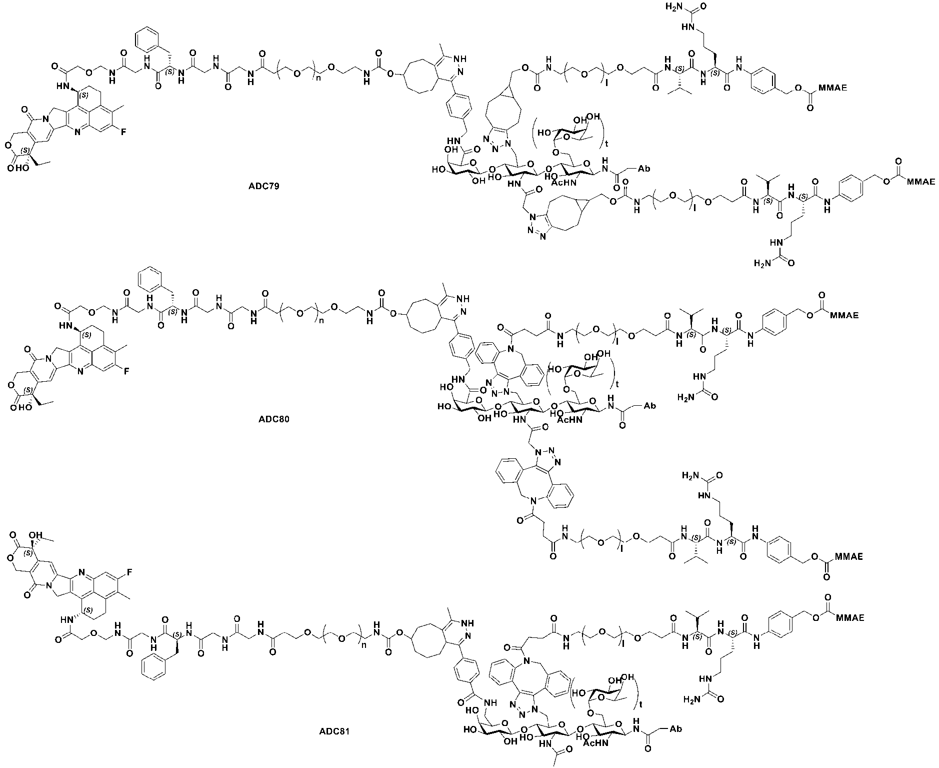 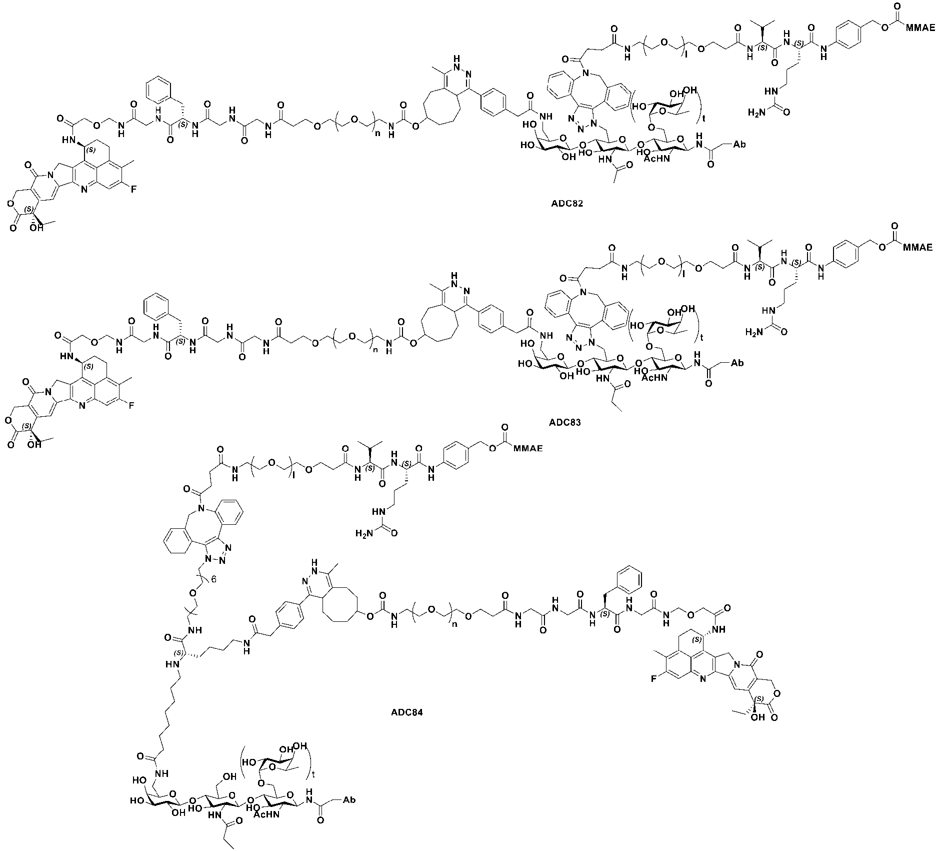 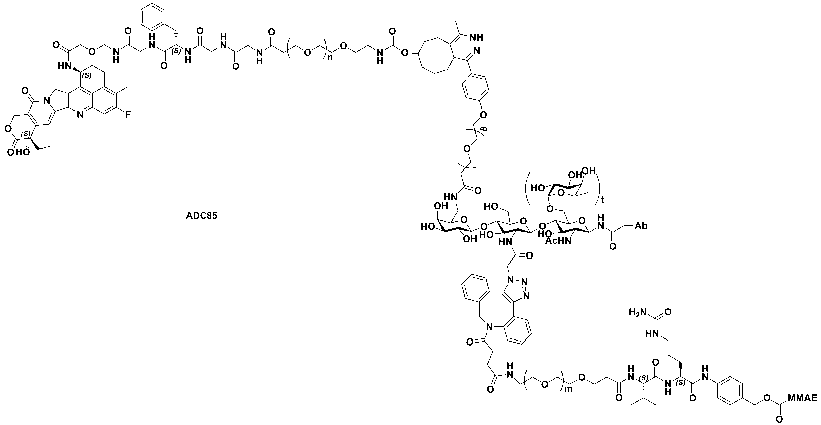
wherein n and I are each independently an integer selected from the group consisting of 0-30, or an integer from 0-20, or an integer from 0-10;
t is 0 or 1.

17. A method for preparing an antibody-based conjugate drug, comprising the following three methods:
**Method 1:**
a) under the action of an endoglycosidase having beta-N-acetylglucosaminidase hydrolytic activity, or an endoglycosidase and a fucosidase, hydrolyzing a wild-type antibody to form a deglycosylated antibody containing one N-acetylglucosamine or core-fucosylated *N-*acetylglucosamine;
b) conjugating the deglycosylated antibody obtained in step a) with the disaccharide linker as defined in any one of claims 1-7 under the catalysis of an endoglycosidase having transglycosylation activity or a mutant thereof, to obtain a glycoengineered antibody;
c) conjugating the glycoengineered antibody containing an orthogonally reactive group obtained in step b) with the drug linker E-L-P as defined in claim 12 or 13, which is modified with a corresponding group capable of undergoing a specific conjugation reaction with the orthogonally reactive group, to prepare the antibody-based conjugate drug;
**Method 2:**
a) under the catalysis of an endoglycosidase having transglycosylation activity or a mutant thereof, conjugating a wild-type antibody with the disaccharide linker as defined in any one of claims 1-7 to obtain a glycoengineered antibody;
b) conjugating the glycoengineered antibody containing an orthogonally reactive group obtained in step a) with the drug linker E-L-P as defined in claim 12 or 13, which is modified with a corresponding group capable of undergoing a specific conjugation reaction with the orthogonally reactive group, to prepare the antibody-based conjugate drug;
**Method 3:**
under the catalysis of an endoglycosidase having transglycosylation activity or a mutant thereof, co-incubating a wild-type antibody and the disaccharide-small-molecule drug conjugate as defined in any one of claims 8-11, or co-incubating a deglycosylated antibody and the disaccharide-small-molecule drug conjugate, to prepare the antibody-based conjugate drug.

18. The method according to claim 17, wherein in the Method 1 and Method 2, the orthogonally reactive group and the corresponding group capable of undergoing a specific conjugation reaction with the orthogonally reactive group are selected from the group consisting of any one of the following combinations: azido and alkynyl, tetrazinyl and trans-cyclooctene (TCO), thiol and maleimide, thiol and thiol or an activated form of thiol, aldehyde and amino, and aldehyde and aminooxy or hydrazino.

19. The method according to claim 17 or 18, wherein the endoglycosidase having beta-N-acetylglucosaminidase hydrolytic activity is selected from the group consisting of Endo Se2, Endo Si, Endo S, Endo S2, Endo F3, Endo A, Endo D, Endo CC, and mutants thereof; and/or the endoglycosidase having transglycosylation activity is selected from the group consisting of Endo Se2, Endo Si, Endo S, Endo S2, Endo F3, and mutants thereof.

20. The method according to claim 17 or 18, wherein when preparing a deglycosylated antibody lacking core fucosylation, a glycoengineered antibody, or an antibody-based conjugate drug, the method comprises using an endoglycosidase having beta-N-acetylglucosaminidase hydrolytic activity in combination with a fucosidase.

21. Use of the disaccharide linker as defined in any one of claims 1-7 or the disaccharide conjugate as defined in any one of claims 8-11 in the manufacture of an antibody-based conjugate drug.

22. Use of the antibody-based conjugate drug as defined in any one of claims 12-16 in the manufacture of a medicament for treating and/or preventing tumors, inflammation, viruses, infectious diseases, or other immune diseases.
